# EUROPEAN PATENT APPLICATION

(11) **EP 4 796 548 A1**
(43) Date of publication of application: **26.08.2026**
(21) Application number: 24879104.8
(22) Date of filing: 18.10.2024
(51) Int. Cl.: C07D 519/00, A61P 35/00

(54) **PROTEOLYSIS-TARGETING CHIMERA COMPOUND FOR DEGRADING IRAK4 AND USE THEREOF**

(30) Priority: 20.10.2023 CN 202311375410; 19.01.2024 CN 202410084340
(71) Applicant: Dovetree Medicines Unus, Inc., Prides Crossing, MA 01965 (US)
(72) Inventor: DU, Xinming, Shenzhen, Guangdong 518017 (CN); MA, Junjun, Shenzhen, Guangdong 518017 (CN); DU, Lifei, Shenzhen, Guangdong 518017 (CN); CHEN, Zhaoqiang, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Latscha Schöllhorn Partner AG
(86) International application number: PCT/CN2024/125690
(87) International publication number: WO 2025/082464

(57) **Abstract**

The present application relates to a proteolysis-targeting chimera compound of formula (A) for degrading IRAK4 and a preparation method therefor, a pharmaceutical composition comprising the compound, and the use of the pharmaceutical composition in treating a disease, disorder or condition related to an IRAK4 protein kinase.

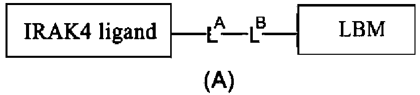

## Description

### Reference to Related Applications

This application claims priority to Chinese patent application CN202311375410.X filed on October 20, 2023 and Chinese patent application CN202410084340.0 filed on January 19, 2024, the contents of which are incorporated herein by reference in their entirety and for all purposes.

### Field of the Invention

The present application relates to a proteolysis-targeting chimera (PROTAC) compound for degrading IRAK4 and a preparation method therefor, a pharmaceutical composition comprising the compound, and the use of the pharmaceutical composition in treating a disease, disorder, or condition related to an IRAK4 protein kinase.

### Background

IRAK4 is a serine/threonine protein kinase belonging to the Interleukin-1 receptor-associated kinase family, the family includes four subtypes IRAK1, IRAK2, IRAK3 (or IRAKM), and IRAK4. Among them, IRAK1, IRAK2, and IRAK4 promote the release of inflammatory factors, whereas IRAK3 participates in anti-inflammatory process. Among the four subtypes, the biological functions of IRAK4 have been clearly elucidated. Upon sensing the stimuli of external signals by TLR or IL-1R, the Myddosome complex formed via IRAK4 activates the MAPK and NF-κB pathways, thereby releasing various inflammatory factors.

Studies have confirmed that IRAK4 is highly expressed in various tumor cells and inflammatory models, and the development of inhibitors targeting IRAK4 is increasingly becoming an important direction for the treatment of autoimmune diseases and tumors. IRAK4 possesses two functions of kinase activity and scaffold activity, and both functions play important roles in the regulation of downstream signaling.

In addition, proteolysis targeting chimera (PROTAC) technology is a new technology that has emerged in recent years. Since its introduction in 2001, this technology has attracted much attention, and currently, several drugs developed based on this technology have entered the clinical research stage, with some entering Phase II clinical trials. PROTAC, as a heterogeneous bifunctional molecule, consists of three parts: a small-molecule inhibitor that can recognize the target protein at one end, a linker strand, and a ligand that can recognize E3 ubiquitin ligase at the other end. This bifunctional molecule recognizes and brings the target protein and E3 ubiquitin ligase into proximity in vivo to form a ternary complex, then ubiquitinates the target protein, thereby initiating a ubiquitin-proteasome-dependent degradation pathway. Compared to conventional small-molecule inhibitors, PROTAC technology simultaneously inhibits both functions of IRAK4 by degrading the IRAK4 protein, can effectively address the issues of insufficient activity or mutations associated with small molecules.

Therefore, developing a new PROTAC molecule that target IRAK4 is of great significance.

### Summary of the Invention

The present invention provides PROTAC compounds targeting IRAK4, which are used to recruit the IRAK4 kinase to an E3 ubiquitin ligase for degradation. In other words, the bifunctional PROTAC compounds of the present invention function as a targeted ubiquitination regulator of the IRAK4 kinase, the IRAK4 kinase is degraded and/or otherwise inhibited by the bifunctional compounds as described herein. The inventors of the present application have demonstrated that the compounds of the present invention can effectively degrade IRAK4 kinase. The compounds of the present invention can be used for treating a disease, disorder, or condition related to an IRAK4 protein kinase. Furthermore, the compounds of the present invention possess superior properties such as enhanced physicochemical properties (e.g., solubility, physical and/or chemical stability), improved pharmacokinetic properties (e.g., improved bioavailability, improved metabolic stability, suitable half-life and duration of action), improved safety profiles (lower toxicity (e.g., reduced cardiotoxicity) and/or fewer side effects), lower tendency to induce drug resistance, and the like.

In one aspect, the present invention provides the compound of formula (A) as defined below: 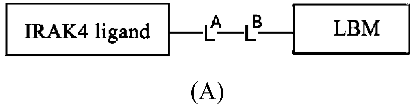 or a stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof.

In another aspect, the present invention provides a method for targeted degradation of IRAK4 protein kinase, comprising contacting the IRAK4 protein kinase with the compound of formula (A) of the present invention in the presence of an E3 ubiquitin ligase.

In another aspect, the present invention provides a pharmaceutical composition, comprising the compound of formula (A) of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate, or pharmaceutically acceptable salt thereof , and a pharmaceutically acceptable excipient, carrier, or diluent. The pharmaceutical composition is preferably a solid formulation, a liquid formulation, or a transdermal formulation.

In another aspect, the present invention provides use of the compound of formula (A) of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate, or pharmaceutically acceptable salt thereof , or the pharmaceutical composition of the present invention in the preparation of a medicament for treating a disease, disorder, or condition related to an IRAK4 protein kinase.

In another aspect, the present invention provides a method for treating a disease, disorder, or condition related to an IRAK4 protein kinase, comprising administering to an individual in need thereof a therapeutically effective amount of the compound of formula (A) of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate or pharmaceutically acceptable salt thereof, or the pharmaceutical composition of the present invention.

In another aspect, the present invention provides a method for preparing the compound of formula (A) of the present invention.

### DETAILED DESCRIPTION

### Definitions

Unless otherwise defined below, all technical and scientific terms used herein are intended to have the same meaning as commonly understood by those skilled in the art. Reference to a technique used herein is intended to refer to a technique as commonly understood in the art, including those technological changes or equivalent replacements that are obvious to those skilled in the art. While it is believed that the following terms will be well understood by those skilled in the art, the following definitions are set forth in order to better explain the present invention.

The terms "including", "comprising", "having", "containing", or "involving" as used herein and their other variant forms, are inclusive or open-ended and do not exclude other unlisted elements or method steps (i.e., these terms also encompass the terms "consisting essentially of" and "consisting of").

As used herein, the term "hydrocarbyl" means straight or branched chain saturated or unsaturated aliphatic hydrocarbon group. Hydrocarbyl includes alkyl, alkenyl, and alkynyl. In some embodiments, hydrocarbyl has 1 to 12, for example 1 to 6 (for example 1, 2, 3, 4, 5, or 6) carbon atoms. For example, as used herein, the term "C₁₋₆ hydrocarbyl" refers to a straight or branched chain group having 1 to 6 carbon atoms, including "C₂₋₆ hydrocarbyl", "C₂₋₅ hydrocarbyl", and "C₁₋₄ hydrocarbyl". As used herein, the term "hydrocarbylene" refers to a group derived from the "hydrocarbyl" as defined above by further loss of one hydrogen atom. As used herein, the term "alkane" means a straight or branched chain saturated aliphatic hydrocarbon.

As used herein, the term "alkyl" means a straight or branched chain monovalent saturated aliphatic hydrocarbon, which can be viewed as a group derived from an alkane by the loss of one hydrogen atom. In some embodiments, alkyl has 1 to 12, for example 1 to 6 (for example 1, 2, 3, 4, 5, or 6) carbon atoms. For example, as used herein, the term "C₁₋₆ alkyl" refers to a straight or branched chain group having 1 to 6 carbon atoms, including "C₂₋₆ alkyl", "C₂₋₅ alkyl", and "C₁₋₄ alkyl". Examples of "C₁₋₆ alkyl" include methyl, ethyl, n-propyl, isopropyl, n-butyl, isobutyl, sec-butyl, tert-butyl, n-pentyl, and n-hexy. The term "C₁₋₄ alkyl" refers to an alkyl having 1 to 4 carbon atoms (namely, methyl, ethyl, n-propyl, isopropyl, n-butyl, isobutyl, sec-butyl, or tert-butyl).

As used herein, the term "alkylene" refers to a group derived from the "alkyl" as defined above by further loss of one hydrogen atom. In some embodiments, alkylene has 1 to 12 carbon atoms, preferably has 1, 2, 3, 4, 5, or 6 carbon atoms. For example, "C₁₋₆ alkylene", "C₂₋₆ alkylene", "C₂₋₅ alkylene", and "C₁₋₄ alkylene". Examples of "C₁₋₆ alkylene" includemethylene, ethylene, n-propylene, isopropylene, n-butylene, isobutylene, sec-butylene, tert-butylene, n-pentylene, and n-hexylene. The term "C₁₋₄ alkylene" refers to an alkylene having 1 to 4 carbon atoms.

As used herein, the term "alkoxy" refers to -O- alkyl, wherein the alkyl is as defined above.

As used herein, the term "heteroalkyl" refers to an alkyl as defined above, wherein one or more, but not all, of the C atoms in the alkyl chain are replaced by a heteroatom or heteroatomic group selected from NR', O, C(O), S, S(O), and S(O)2, wherein R' is a suitable substituent, for example H, alkyl, etc. Heteroalkyl can be attached to the rest of the molecule via C atom or the heteroatom or heteroatomic group. Preferably, heteroalkyl is attached to the rest of the molecule via C atom.

As used herein, the term "alkenyl" means a straight or branched chain monovalent aliphatic hydrocarbon group, containing one or more double bonds. In some embodiments, alkenyl has 2, 3, 4, 5, or 6 carbon atoms ("C₂₋₆ alkenyl", for example "C₂₋₄ alkenyl"). The alkenyl is for example -CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl, and 4-methyl-3-pentenyl. When the compound of the present invention contains an alkenyl, the compound may exist in pure E (entgegen) form, pure Z (zusammen) form, or a form of any mixture thereof. The term "alkenylene" is a corresponding divalent group, including, for example "C₂₋₆ alkenylene", "C₂₋₄ alkenylene", and the like, and specific examples thereof include, but are not limited to: -CH=CH-, -CH₂CH=CH-, -C(CH₃)=CH-, butenylene, pentenylene, hexenylene, cyclopentenylene, cyclohexenylene, and the like.

As used herein, the term "alkynyl" means a straight or branched chain monovalent aliphatic hydrocarbon group, containing one or more triple bonds. In some embodiments, alkynyl has 2, 3, 4, 5, or 6 carbon atoms ("C₂₋₆ alkynyl", for example "C₂₋₄ alkynyl"). The alkynyl is for example -C≡CH, -CH₂C≡CH, -C≡C-CH₃, -CH₂-C≡C-CH₃, 2-pentynyl, 3-pentynyl, 4-pentynyl, 2-hexynyl, 3-hexynyl, 4-hexynyl, 5-hexynyl, 3-methyl-2-butynyl, and 2-methyl-3-pentynyl. The term "alkynylene" is a corresponding divalent group, including, for example "C₂₋₆ alkynylene", "C₂₋₄ alkynylene", and the like, and specific examples thereof include, but are not limited to: -C≡C-, -CH₂C≡C-, -C≡C-CH₂-, -CH₂-C≡C-CH₂-, pentynylene, hexynylene, and the like.

As used herein, the term "fused" means that two or more ring structures share two adjacent atoms with each other.

As used herein, the term "bridged" or "bridging" means that two or more ring structures share two non-adjacent atoms with each other.

As used herein, the term "spiro" or "spiro-linked" means that two or more ring structures share one atom with each other.

As used herein, the terms " cyclohydrocarbyl", "hydrocarbon ring" and "cyclohydrocarbylene" refer to saturated (i.e., " cycloalkyl" and " cycloalkylene") or partially unsaturated (i.e., having one or more double bonds (i.e., "cycloalkenyl" and "cycloalkenylene") and/or triple bonds within the ring) monocyclic or polycyclic hydrocarbon rings having for example 3-12 (suitably having 3-10, 3-8, 3-7, 3-6, 4-6, or 5-6) ring carbon atoms, including but not limited to cyclopropyl(ene)(ring), cyclobutyl(ene)(ring), cyclopentyl(ene)(ring), cyclohexyl(ene)(ring), cycloheptyl(ene)(ring), cyclooctyl(ene)(ring), cyclononyl(ene)(ring), cyclobutenyl(ene)(ring), cyclopentenyl(ene)(ring), cyclohexenyl(ene)(ring), cycloheptenyl(ene)(ring), cyclooctenyl(ene)(ring), cyclononenyl(ene)(ring), and the like. In some embodiments, cyclohydrocarbyl includes an aryl-fused cyclohydrocarbyl, provided that the entire ring system is non-aromatic.

As used herein, the terms "cycloalkyl" and "cycloalkylene" refer to saturated monocyclic or polycyclic (such as bicyclic) hydrocarbon rings (for example, a monocyclic ring, such as cyclopropyl, cyclobutyl, cyclopentyl, cyclohexyl, cycloheptyl, cyclooctyl, cyclononyl, or a bicyclic ring, including a spiro, fused, or bridged system (such as bicyclo[1.1.1]pentyl, bicyclo[2.2.1]heptyl, bicyclo[3.2.1]octyl, or bicyclo[5.2.0]nonyl, decalinyl, etc.) The cycloalkyl has 3-15 carbon atoms, suitably has 3-12, 3-10, 3-8, 3-7, 3-6, 4-6, or 5-6 carbon atoms. For example, the terms "C₃₋₆ cycloalkyl" and "C₃₋₆ cycloalkylene" refer to saturated monocyclic or polycyclic (such as bicyclic) hydrocarbon ring having 3 to 6 ring-forming carbon atoms (for example cyclopropyl, cyclobutyl, cyclopentyl, or cyclohexy).

The terms "spiro cycloalkyl" and "spiro cycloalkylene" refer to polycyclic (such as bicyclic) "cycloalkyl" and "cycloalkylene" defined above, wherein any two connected rings share one carbon atom. For example, "C₇₋₁₂spiro cycloalkyl" and "C₇₋₁₂spiro cycloalkylene" refer to a ring structure containing 7 to 12 (for example 5-12 or 7-11) carbon atoms and formed by at least two rings that share one atom.

The terms "fused cycloalkyl" and "fused cycloalkylene" refer to polycyclic (such as bicyclic) "cycloalkyl" and "cycloalkylene" defined above, wherein any two connected rings share two adjacent carbon atoms. For example, "C₄₋₁₀ fused cycloalkyl" and "C₄₋₁₀ fused cycloalkylene" refer to a fused ring that contains 4 to 10 (for example 6-10 or 8-10) ring carbon atoms and is formed by two or more rings sharing two adjacent carbon atoms.

The terms "bridged cycloalkyl" and "bridged cycloalkylene" refer to polycyclic (such as bicyclic) "cycloalkyl" and "cycloalkylene" defined above, wherein any two connected rings share two carbon atoms that are not adjacent to each other. For example, "C₇₋₁₀ bridged cycloalkyl" and "C₇₋₁₀ bridged cycloalkylene" refer to a ring structure containing 7 to 12 (for example 6-10, 6-9, or 6-8) carbon atoms and formed by two rings sharing two atoms that are not adjacent to each other.

As used herein, the terms "cycloalkenyl" and "cycloalkenylene" refer to a monocyclic or polycyclic (such as bicyclic) fused hydrocarbon ring having one or more double bonds within the ring (for example monocyclic, such as cyclopropenyl, cyclobutenyl, cyclopentenyl, cyclopentadiene, cyclohexenyl, cyclohexadienyl, cycloheptenyl, cyclooctenyl, cyclononenyl, or bicyclic). The cycloalkenyl and "cycloalkenylene" have 3 to 10 carbon atoms, and suitably have 3-8, for example 3-7, 3-6, 4-6, or 5-6.

As used herein, the terms "heterocyclyl", "heterocycle", and "heterocyclylene" refer to a saturated (namely, "heterocycloalkyl" and "heterocycloalkylene") or partially unsaturated (for example, having one or more double bonds within the ring (namely, "heterocycloalkenyl" and "heterocycloalkenylene")) monocyclic or bicyclic ring structure, having 2, 3, 4, 5, 6, 7, 8, or 9 carbon atoms and 1 or more (for example 1, 2, 3, or 4) heteroatom-containing groups selected from O, S, S(=O), S(=O)₂, and NR' in the ring, wherein R^{'} is as defined above. The heterocycle may be attached to the rest of the molecule via any of the carbon atoms or, if present, a nitrogen atom. In particular, a 3-12-membered heterocycle is a group having 3-12 (for example 3-10, 3-8, 3-7, 3-6, 4-11, 4-9, 4-7, 4-6, 5-12, 5-6, 6-10, 6-9, 6-8, 7-11, or 8-12) carbon atoms and heteroatom(s) in the ring. Examples that may be cited include, but are not limited to, oxiranyl, aziridinyl, azetidinyl, oxetanyl, tetrahydrofuranyl, tetrahydrothiophenyl, dioxolinyl, pyrrolidinyl, pyrrolidinonyl, oxazolidine, thiazolidinyl, pyrazolidinyl, imidazolidinyl, pyrazolidinyl, pyrrolinyl, tetrahydropyranyl, piperidinyl, hexahydropyrimidinyl, triazinanyl, morpholinyl, dithianyl, thiomorpholinyl, piperazinyl, trithianyl, azacyclooctyl, dihydropyrrolyl, dihydroimidazolyl, azacyclooctenyl.

As used herein, the heterocycles described above include nitrogen-containing heterocycles, oxygen-containing heterocycles, and sulfur-containing heterocycles. For example, "nitrogen-containing heterocycle" has at least one nitrogen atom and may optionally further have one or more (for example one, two, three, or four) ring members selected from N, O, C=O, S, S=O, and S(=O)₂. Nitrogen-containing heterocycle may be attached to the rest of the molecule via a nitrogen atom. The nitrogen-containing heterocycle is preferably a saturated nitrogen-containing monocyclic ring. In particular, a 3 to 12-membered nitrogen-containing heterocycle is a group having 3-12 carbon atoms and heteroatom(s) (wherein at least one is a nitrogen atom) in the ring, including but not limited to three-membered nitrogen-containing heterocycle (such asaziridinyl), four-membered nitrogen-containing heterocycle (such as azetidinyl), five-membered nitrogen-containing heterocycle (such as pyrrolyl, pyrrolidinyl (pyrrolidine ring), pyrrolinyl, pyrrolidinonyl, imidazolyl, imidazolidinyl, imidazolinyl, pyrazolyl, pyrazolinyl), six-membered nitrogen-containing heterocycle (such as piperidinyl (piperidine ring), morpholinyl, thiomorpholinyl, piperazinyl), seven-membered nitrogen-containing heterocycle, and the like.

As used herein, the heterocycles described above include monocyclic rings, fused rings, bridged rings, and spiro rings, namely, monocyclic heterocycles, bridged heterocycles, spiro heterocycles, and fused heterocycles. The point of attachment of bridged heterocycles, spiro heterocycles, and fused heterocycles to other groups can be on any ring in the structure.

As used herein, a fused heterocycle refers to a polycyclic (such as bicyclic) heterocycle defined above, wherein any two connected rings share two adjacent atoms. The fused heterocycles include, but are not limited to, heterocyclyl-fused-heterocyclyl, heterocyclyl-fused-cycloalkyl, monoheterocyclyl-fused-monoheterocyclyl, monoheterocyclyl-fused-monocycloalkyl, for example 3-7-membered (mono)heterocyclyl-fused-3-7-membered (mono)heterocyclyl, 3-7-membered (mono)heterocyclyl-fused-(mono)cycloalkyl, 3-7-membered (mono)heterocyclyl-fused-C₄₋₆ (mono)cycloalkyl. Preferably, the fused heterocycles are 6 to 10-membered, and more preferably 8-10-membered. Examples of fused heterocycles include, but are not limited to, pyrrolidinyl-fused-cyclopropyl, cyclopentyl-fused-aziridinyl, pyrrolidinyl-fused-cyclobutyl, pyrrolidinyl-fused-pyrrolidinyl, pyrrolidinyl-fused-piperidinyl, pyrrolidinyl-fused-piperazinyl, piperidinyl-fused-morpholinyl, 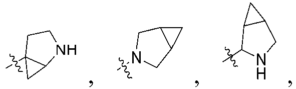 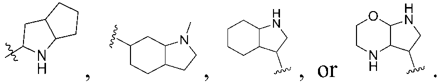 In some embodiments, fused heterocyclyl also includes a heteroaryl-fused-heterocyclyl or -cyclohydrocarbyl, and an aryl-fused-heterocyclyl, provided that the entire ring system is non-aromatic. In some embodiments, fused heterocyclyl includes 5-6-membered monocyclic heteroaryl-fused-C₅₋₆ monocyclic cyclohydrocarbyl, 5-6-membered monocyclic heteroaryl-fused-5-6-membered monocyclic heterocyclyl, and phenyl-fused-5-6-membered monocyclic heterocyclyl, for example pyrrolotetrahydropyridinyl, pyrazolotetrahydropyridinyl, and imidazotetrahydropyridinyl.

As used herein, a spiro heterocycle refers to a polycyclic (such as bicyclic) heterocycle defined above, wherein any two connected rings share one carbon atom. Preferably, the spiro heterocycle is 5-12-membered, and more preferably 7-11-membered. Depending on the number of shared spiro atoms, spiro heterocycles are classified as mono-spiro heterocycles, di-spiro heterocycles, or poly-spiro heterocycles, and preferably refer to mono-spiro heterocycles or di-spiro heterocycles, and more preferably 4-membered/4-membered, 3-membered/5-membered, 4-membered/5-membered, 4-membered/6-membered, 5-membered/5-membered, or 5-membered/6-membered mono-spiro heterocycles.

As used herein, a bridging heterocycle or bridged heterocycle refers to a polycyclic (such as bicyclic) heterocycle defined above, wherein any two connected rings share two non-adjacent atoms. One or more rings of the bridged heterocycle may contain one or more double bonds, but no ring has a fully conjugated π electron system. Preferably, the bridged heterocycle is 6-9-membered, and more preferably 6-8-membered. Depending on the number of member rings, bridged heterocycles are classified as bicyclic , tricyclic, tetracyclic, or polycyclic bridged heterocycles, and preferably refer to bicyclic, tricyclic, or tetracyclic bridged heterocycles, and more preferably bicyclic or tricyclic bridged heterocycles.

As used herein, the term "aryl" refers to an all-carbon monocyclic or fused-ring polycyclic aromatic group having a conjugated π-electron system. For example, as used herein, the term "C₆₋₁₀ aryl" means an aromatic group containing 6 to 10 carbon atoms, such as phenyl or naphthalenyl.

As used herein, the term "heteroaryl" refers to a monocyclic or polycyclic (for example bicyclic or tricyclic) aromatic ring system having 5 to 14 ring atoms, for example 5, 6, 7, 8, 9, 10, 11, 12, 13, or 14 ring atoms, in particular having 1, 2, 3, 4, 5, 6, 7, 8, 9, 10, 11, 12, or 13 carbon atoms and 1, 2, 3, 4, or 5 identical or different heteroatoms independently selected from N, O, S, and S(O)₂. 1 or more ring carbon atoms in the heteroaryl may be replaced by C(O). Heteroaryl can be benzofused. Examples of heteroaryl include, but are not limited to: pyridinyl, pyridonyl, pyrimidinyl, pyrimidinonyl, pyrazinyl, pyridazinyl, thiazolyl, thienyl, oxazolyl, furanyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazopyridinyl, quinolinyl, indolyl, pyrrolopyridazinyl, benzofuranyl, benzothienyl, indazolyl, benzoxazolyl, benzoisoxazolyl, quinazolinyl, pyrrolopyridinyl, pyrazolopyrimidinyl, imidazopyridazinyl, pyrazolopyridinyl, triazolopyridinyl, isoquinolinyl, tetrahydroisoquinolinyl, benzoimidazolyl, cinnolinyl, indolizinyl, phthalazinyl, isoindolyl, pteridinyl, purinyl, furazanyl, benzofurazanyl, quinoxalinyl, naphthyridinyl, or furopyridinyl.

As used herein, the term "halo" or "halogen" group is defined as including F, Cl, Br, or I.

As used herein, the term "haloalkyl" refers to an alkyl substituted by one or more (such as 1 to 3) identical or different halogen atoms, the alkyl is as defined herein. The terms "C₁₋₈ haloalkyl", "C₁₋₆ haloalkyl", and "C₁₋₄ haloalkyl" refer to haloalkyl having 1 to 8 carbon atoms, 1 to 6 carbon atoms, and 1-4 carbon atoms, respectively, for example -CF₃, -C₂F₅, -CHF₂, - CH₂F, -CH₂CF₃, -CH₂Cl, or -CH₂CH₂CF₃, etc.

As used herein, the term "haloalkenyl" refers to an alkenyl substituted by one or more (such as 1 to 3) identical or different halogen atoms, the alkenyl is as defined herein. The terms "C₂₋₈ haloalkenyl", "C₂₋₆ haloalkenyl", and "C₂₋₄ haloalkenyl" refer to haloalkenyl having 2 to 8 carbon atoms, 2 to 6 carbon atoms, and 2-4 carbon atoms, respectively.

The term "substitution" refers to a selective replacement of one or more (e.g., one, two, three, or four) hydrogens on a specified atom by a designated group, with the proviso that the normal valence of the specified atom in the present case is not exceeded and the substitution forms a stable compound. A combination of substituents and/or variables is permitted only when this combination forms a stable compound.

If a group is described as "optionally substituted by ......" or "optionally substituted", the group may be: 1) unsubstituted or (2) substituted. If the carbon of the group is described as being optionally substituted by one or more of the groups in the list, one or more hydrogens on the carbon (to the extent that any hydrogens are present) may be substituted individually and/or together by an independently selected optional substituent. If the nitrogen of the group is described as being optionally substituted by one or more of the groups in the list, each of one or more hydrogens on the nitrogen (to the extent that any hydrogens are present) may be substituted by an independently selected optional substituent.

If a substituent is described as being "independently selected from" a group, each substituent is selected independently of the other. Therefore, each substituent may be the same as or different from another (other) substituent.

As used herein, the term "one or more" means 1 or more than 1 under a reasonable condition, for example, 2, 3, 4, 5, or 10.

Unless otherwise specified, as used herein, the point of attachment of a substituent may be from any suitable position of the substituent.

Unless otherwise stated, when the bond of a substituent is shown as passing through a bond connecting two atoms in a ring (a "floating bond"), such a substituent may be bonded to any ring-forming atom in the substitutable ring. Where an available ring member is shown as carrying a substitutable hydrogen atom, when the floating bond is bonded to the available ring member, the substitutable hydrogen atom is substantially substituted (i.e., absent).

The present invention also includes all pharmaceutically acceptable isotope labeled compounds that are identical to the compounds of the present invention, except that one or more atoms are replaced by atoms having the same atomic number but with an atomic mass or mass number different from which predominates in nature. Examples of an isotope suitable for inclusion in the compound of the present invention include (but are not limited to), a hydrogen isotope (e.g., deuterium (D, ²H), tritium (T, ³H)); a carbon isotope (e.g., ¹¹C, ¹³C, and ¹⁴C); a chlorine isotope (e.g., ³⁶Cl); a fluorine isotope (e.g., ¹⁸F); an iodine isotope (e.g., ¹²³I and ¹²⁵I); a nitrogen isotope (e.g., ¹³N and ¹⁵N); an oxygen isotope (e.g., ¹⁵O, ¹⁷O and ¹⁸O); a phosphorus isotope (e.g., ³²P); and a sulfur isotope (e.g., ³⁵S). Certain isotope labeled compounds of the present invention (e.g., those incorporating a radioisotope) can be used in a pharmaceutical and/or substrate tissue distribution study (e.g., analysis). Radioisotope tritium (i.e., ³H) and carbon-14 (i.e., ¹⁴C) are particularly useful for this purpose due to their ease of incorporation and detection. Substitution with a positron emission isotope (e.g., ¹¹C, ¹⁸F, ¹⁵O, and ¹³N) can be used to examine substrate receptor occupancy in a positron emission tomography (PET) study. An isotope labeled compound of the present invention can be prepared by a method similar to those described in the accompanying routes and/or an example and preparation, by using an appropriate isotope labeled reagent instead of a previously used unlabeled reagent. The pharmaceutically acceptable solvate of the present invention includes those in which a crystallization solvent can be substituted by an isotope, for example, D₂O, acetone-*d₆* or DMSO-*d₆*. In some embodiments, the isotope-labeled compound of the present invention is a deuterated compound.

The term "stereoisomer" refers an isomer formed due to at least one asymmetric center, with the same chemical composition but different in the spatial arrangement of atoms or groups. In compounds with one or more (e.g., 1, 2, 3, or 4) asymmetric centers, racemic mixtures, single enantiomers, diastereomer mixtures, and individual diastereomers can be produced. A specific individual molecule can also exist as a geometric isomer (cis/trans). Similarly, the compound of the present invention can exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present application covers all such isomers or mixtures thereof in any proportion (e.g., 60%, 65%, 70%, 75%, 80%, 85%, 90%, 95%, 96%, 97%, 98%, 99%).

"Diastereomer" refers to stereoisomers that have two or more chiral centers and whose molecules are not mirror images of each other. Diastereomers have different physical properties, for example melting point, boiling point, spectral properties, and reactivity. The mixture of diastereomers can be separated by a high-resolution analytical method, for example an electrophoresis method and a chromatography.

"Enantiomer" refers to two stereoisomers of a compound that are non-superimposable mirror images of each other.

The term "chiral" refers to molecules that are not superimposable on their mirror images, while the term "achiral" refers to molecules that are superimposable on their mirror images.

The compounds of the present invention can be prepared in a racemic form, or single enantiomers can be prepared by enantioselective synthesis or by resolution.

As used herein, the term "cis/trans isomer" or "geometric isomer" refers to isomers that arises from the inability of double bonds or single bonds of ring-forming carbon atoms to rotate freely. The compounds provided herein include all cis, trans, syn, anti, entgegen (E), and zusammen (Z) isomers and their corresponding mixtures.

The chemical bonds of the compounds of the present invention may be depicted herein using solid lines ( 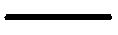 ), dashed bars ( 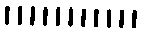 ), solid bars ( 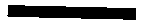 ), solid wedges ( 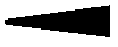 ), or dashed wedges ( 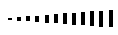 ). The use of a solid line to depict a bond attached to an asymmetric carbon atom is intended to indicate that all possible stereoisomers at that carbon atom (e.g., particular enantiomers, racemic mixtures, etc.) are included. The use of a solid or dashed wedge to depict a bond attached to an asymmetric carbon atom is intended to indicate the presence of the indicated stereoisomer. Isomers arising from the inability of single bonds of ring atoms to rotate freely are depicted with dashed or solid rods to indicate that two substituents on the ring atoms are on the same side or opposite sides, for example, 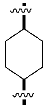 indicates a cis configuration, and 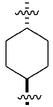 indicates a trans configuration. When present in a racemic mixture, solid and dashed wedges are used to define relative stereochemistry, not absolute stereochemistry. Unless otherwise indicated, the compounds of the present invention are intended to exist in the form of stereoisomers (including cis and trans isomers, optical isomers (e.g., R and S enantiomers), diastereomers, geometric isomers, rotamers, conformational isomers, atropisomers, and mixtures thereof). The compounds of the present invention may exhibit more than one type of isomerism and are composed of mixtures thereof (e.g., racemic mixtures and diastereomeric pairs).

It should also be understood that certain compounds of the present invention may exist in a free form for treatment, or where appropriate, exist in their pharmaceutically acceptable derivative forms. In the present invention, a pharmaceutically acceptable derivative includes, but is not limited to, a pharmaceutically acceptable salt, ester, solvate, metabolite or prodrug that, when administered to a patient in need thereof, can directly or indirectly provide the compound of the present invention or its metabolite or residue. Therefore, when referring to "the compound of the present invention" herein, it is also intended to cover the various derivative forms of the compound described above.

The term "pharmaceutically acceptable" means that the substance or composition must be chemically and/or toxicologically compatible with the other components constituting the formulation and/or the mammal being treated therewith.

The pharmaceutically acceptable salt of the compound of the present invention includes an acid addition salt and a base addition salt thereof.

A suitable acid addition salt is formed from an acid that forms a pharmaceutically acceptable salt. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methyl sulfate, naphthylate, nicotinate, nitrate, orotate, oxalate, palmitate, and other similar salts.

A suitable base addition salt is formed from a base that forms a pharmaceutically acceptable salt. Examples include aluminum salt, arginine salt, choline salt, diethylamine salt, lysine salt, magnesium salt, meglumine salt, potassium salt, and other similar salts.

For a review of a suitable salt, see "Handbook of Pharmaceutical Salts: Properties, Selection, and Use" by Stahl and Wermuth (Wiley-VCH, 2002). The methods for preparing a pharmaceutically acceptable salt of the compound of the present invention are known to those skilled in the art.

As used herein, the term "ester" means an ester derived from the various general formula compounds of the present application, including physiologically hydrolyzable esters (the compounds of the present invention that can be hydrolyzed under physiological conditions to release a free acid or alcohol). The compound of the present invention itself may also be an ester.

The present invention covers all possible crystalline forms or polymorphs of the compound of the present invention, which may be a single polymorph or a mixture of more than one polymorphs in any proportion.

The compound of the present invention may exist in the form of a solvate (preferably a hydrate), wherein the compound of the present invention contains a polar solvent, particularly, for example, water, methanol, or ethanol, as a structural element of the lattice of the compound. The amount of the polar solvent, particularly water, can exist in a stoichiometric or non-stoichiometric ratio.

Those skilled in the art will understand that not all nitrogen-containing heterocycles are capable of forming *N*-oxides because nitrogen requires available lone pairs of electrons to be oxidized to an oxide; those skilled in the art will identify nitrogen-containing heterocycles that are capable of forming *N*-oxides. Those skilled in the art will also recognize that tertiary amines can form *N*-oxides*.* The synthetic methods for preparing *N-*oxides of heterocycles and tertiary amines are well known to those skilled in the art, including the oxidation of heterocycles and tertiary amines with peroxy acids such as peracetic acid and m-chloroperoxybenzoic acid (MCPBA), hydrogen peroxide, alkyl hydrogen peroxide such as tert-butyl hydrogen peroxide, sodium perborate, and dioxirane such as dimethyldioxirane. These methods for preparing N-oxides have been extensively described and reviewed in literatures, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The scope of the present invention also includes a metabolite of the compound of the present invention, i.e., a substance formed in the body upon administration of the compound of the present invention. Such products can be generated, for example, by oxidation, reduction, hydrolysis, amidation, deamidation, esterification, deesterification, enzymatic hydrolysis, etc., of the administered compound. Therefore, the present invention includes a metabolite of the compound of the present invention, including a compound prepared by a method of contacting the compound of the present invention with a mammal for a time sufficient to produce its metabolite.

The present invention further includes, within its scope, prodrugs of the compound of the present invention, which are certain derivatives of the compound of the present invention that may themselves have a little or no pharmacological activity, and which, when administered to or on the body, can be converted, for example, by hydrolytic cleavage into the compound of the present invention having the desired activity. Typically, such a prodrug will be a functional group derivative of the compound, which is readily converted in vivo into a compound with the desired therapeutic activity. Other information regarding the use of prodrugs can be found in "Pro-drugs as Novel Delivery Systems", Vol. 14, ACS Symposium Series (T. Higuchi and V. Stella), and "Bioreversible Carriers in Drug Design," Pergamon Press, 1987 (edited by E. B. Roche, American Pharmaceutical Association). The prodrug of the present invention can be prepared, for example, by replacing an appropriate functional group present in the compound of the present invention with a certain moiety known to those skilled in the art as "pro-moiety (e.g., described in "Design of Prodrugs", H. Bundgaard (Elsevier, 1985))".

The present invention also covers the compound of the present invention containing a protecting group. In any process for preparing the compound of the present invention, it may be necessary and/or desirable to protect sensitive or reactive groups on any relevant molecules, thereby forming a chemically protected form of the compound of the present invention. This can be achieved by a conventional protecting group, for example, those protecting groups described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, these references are incorporated herein by reference. The protecting group can be removed at an appropriate subsequent stage using methods known in the art.

As used herein, the term "about" refers to within the range of ±10%, preferably ±5%, more preferably ±2% of the stated numerical value.

### Compounds

In one aspect, the present invention provides a compound of formula (A): 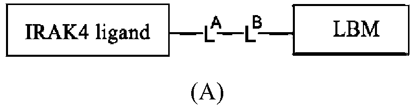 or a stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof, wherein:
L^{A} is selected from a bond and straight or branched chain C₁₋₄ alkylene, the C₁₋₄ alkylene is optionally substituted by one or more substituents independently selected from C₁₋₄ alkyl, C₁₋₄ haloalkyl, halogen, oxo (=O), OH, CN, NH₂, -NH(C₁₋₄ alkyl), and -N(C₁₋₄ alkyl)₂;
L^{B} is selected from groups (1)-(21):

   (1) -CyL1-,

   (2) -CyL1-La-,

   (3) -CyL1-Lb-,

   (4) -CyL1-La-CyL2-La-,

   (5) -CyL1-NR^{L1}-,

   (6) -CyL1-C(O)-,

   (7) -CyL1-C(O)-NR^{L1}-,

   (8) -CyL1-NR^{L1}-C(O)-,

   (9) -CyL1-CyL2-,

   (10) -NR^{L1}-CyL1-La-,

   (11) -NR^{L1}-CyL1-Lb-,

   (12) -NR^{L1}-CyL3-NR^{L2}-

   (13) -NR^{L1}-CyL3-La-NR^{L2}-,

   (14) -NR^{L1}-CyL1-C(O)-,

   (15) -NR^{L1}-La-CyL1-La-,

   (16) -La-CyL1-,

   (17) -O-La-,

   (18) -S-La-,

   (19) -NR^{L1}-La-,

   (20) -CyL1-La-CyL2-,

   and

   (21) -CyL1-Lc-CyL4-,

   wherein:
   In the groups (1)-(21), the bond extending from the leftmost end of each group is attached to L^{A} and the bond extending from the rightmost end is attached to the 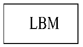 moiety, or the bond extending from the leftmost end of each group is attached to the 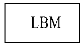 moiety and the bond extending from the rightmost end is attached to L^{A},
   CyL1 and CyL2 at each occurrence are each independently selected from C₃₋₁₂ cycloalkylene or 3-12-membered heterocycloalkylene, wherein the heterocycloalkylene preferably has 1, 2, or more nitrogen heteroatoms and 0, 1, or 2 heteroatoms selected from O and S,
   CyL3 at each occurrence is independently selected from C₃₋₁₂ cycloalkylene,
   CyL4 at each occurrence is independently selected from 5-12-membered heteroarylene,
   La at each occurrence is independently selected from C₁₋₄ hydrocarbylene,
   Lb at each occurrence is independently selected from straight chain C₂₋₄ hydrocarbylene, wherein 1 or 2, but not all, of the CH₂ groups in the straight chain C₂₋₄ hydrocarbylene are replaced by 1 or 2 groups selected from O, S, NR^{L1}, and C(O),
   Lc at each occurrence is independently selected from a bond or C₁₋₄ hydrocarbylene,
   each of CyL1, CyL2, CyL3, CyL4, La, Lb, and Lc is optionally substituted by one or more groups independently selected from the following: C₁₋₄ alkyl, C₁₋₄ haloalkyl, halogen, OH, CN, NH₂, -NH(C₁₋₄ alkyl), and -N(C₁₋₄ alkyl)₂, preferably methyl, ethyl, F, Cl, Br, OH, CN, and NH₂, more preferably methyl, F, Cl, and OH; and
   R^{L1} and R^{L2} at each occurrence are each independently selected from H and C₁₋₄ alkyl;
   In some embodiments,
   CyL1 and CyL2 at each occurrence are each independently selected from 3-12-membered heterocycloalkylene, wherein the heterocycloalkylene preferably has 1, 2, or more nitrogen heteroatoms and 0, 1, or 2 heteroatoms selected from O and S;
   Lb at each occurrence is independently selected from straight chain C₂₋₄ alkylene, wherein 1 or 2, but not all, of the CH₂ groups in the straight chain C₂₋₄ alkylene are replaced by 1 or 2 groups selected from O, S, NR^{L1}, and C(O);
   the 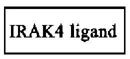 moiety has the structure of formula (1): 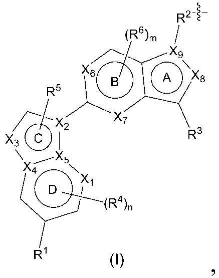 wherein:
   X₁, X₂, X₃, X₄, X₅, X₆, X₇, X₈, and X₉ can each independently be C, CR, or N, with the proviso that the valencies of all atoms are satisfied, and the X₄ and X₅ are not simultaneously N;
   R, R¹, R⁴, and R⁵ are each independently selected from: hydrogen, deuterium, R⁷, halogen, CN, NO₂, C₁₋₆ alkyl, -OR^{1f}, -SR^{1f}, -NR^{1d}R^{1e}, -S(O)2R^{1f}, -S(O)R^{1f}, -S(O)2-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), -CF₃, -CCl(R^{1f})₂, -CCl₂(R^{1f}), - CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1c}, -C(O)R^{1f}, -C(O)OR^{1f}, - C(O)NR^{1d}R^{1e}, -C(O)NR^{1f}-OR^{1f}, -OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, and -NR^{1f}-S(O)₂R^{1f},
   p is 1, 2, or 3;
   n is 0, 1, or 2, wherein when n is 1 or 2, R⁴ is attached to an available ring member of ring D; and
   R⁵ is attached to an available ring member of ring C;
   R² is selected from: saturated or partially unsaturated C₃₋₇ cyclohydrocarbyl; 3-7-membered saturated or partially unsaturated heterocyclyl having 1-3 heteroatoms independently selected from nitrogen, oxygen, and sulfur; C₆₋₁₀ aryl; and 5-10-membered heteroaryl having 1-4 heteroatoms independently selected from nitrogen, oxygen, and sulfur, wherein the cyclohydrocarbyl, heterocyclyl, aryl, and heteroaryl are optionally substituted by 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cyclohydrocarbyl or heterocyclyl, the two substituents optionally together with the ring carbon atoms that they are attached to, form saturated or partially unsaturated optionally substituted C₃₋₇ cyclohydrocarbyl, or 3-7-membered saturated or partially unsaturated optionally substituted heterocyclyl having 1-3 heteroatoms independently selected from nitrogen, oxygen, and sulfur;
   R³ and R⁶ are each independently selected from: H, R⁸, halogen, CN, NO₂, C₁₋₆ alkyl, - OR^{3f}, -SR^{3f}, -NR^{3d}R^{3e}, -S(O)₂R^{3f}, -S(O)R^{3f}, -S(O)₂-NR^{3d}R^{3e}, -S(O)-NR^{3d}R^{3e}, -P(O)(OR^{3f})₂,-P(O)(NR^{3d}R^{3e})₂, -CF(R^{3f})₂, -CF₂(R^{3f}), -CF₃, -CCl(R^{3f})₂, -CCl₂(R^{3f}), -CCl₃, -(CR^{3a}R^{3b})_{q}-OR^{3f},-(CR^{3a}R^{3b})_{q}-C(O)OR^{3f}, -(CR^{3a}R^{3b})_{q}-NR^{3d}R^{3e}, -C(O)R^{3f}, -C(O)OR^{3f}, and -C(O)NR^{3d}R^{3e}
   q is 1, 2, or 3; and
   m is 0, 1, 2, or 3, wherein when m is 1, 2, or 3, R⁶ is attached to an available ring member of ring B;
   R^{1a}, R^{1b}, R^{3a}, and R^{3b} are each independently selected from hydrogen, deuterium, halogen, CN, OH, SH, NO₂, C₁₋₆ alkyl, C₁₋₆ haloalkyl, C₁₋₆ alkoxy, C₁₋₆ alkylthio, and -NR^{7d}R^{7e}; or R^{1a} and R^{1b}, or R^{3a} and R^{3b}, together with the carbon atoms that they are commonly attached to, form R⁹;
   R^{1d}, R^{1e}, R^{2d}, R^{2e}, R^{3d}, R^{3e}, R^{7d}, and R^{7e} are each independently selected from hydrogen, deuterium, R¹¹, C₁₋₆ alkyl, and C₁₋₆ haloalkyl; or, R^{1d} and R^{1e}, or R^{2d} and R^{2e}, or R^{3d} and R^{3e}, or R^{7d} and R^{7e}, together with the nitrogen atoms that they are commonly attached to, form R¹⁰;
   R^{1f}, R^{2f}, and R^{3f} are independently selected from hydrogen, deuterium, C₁₋₆ alkyl, C₁₋₆ haloalkyl, and R¹²;
   R⁷, R⁸, R⁹, R¹¹, and R¹² are each independently selected from: saturated or partially unsaturated C₃₋₇ cyclohydrocarbyl; 3-12-membered saturated or partially unsaturated heterocyclyl having 1-3 heteroatoms independently selected from nitrogen, oxygen, and sulfur; C₆₋₁₀ aryl; and 5-10-membered heteroaryl having 1-4 heteroatoms independently selected from nitrogen, oxygen, and sulfur, wherein the cyclohydrocarbyl, heterocyclyl, aryl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
   R¹⁰ is selected from: 3-7-membered saturated or partially unsaturated heterocyclyl having one nitrogen heteroatom and optionally 1-2 additional heteroatoms independently selected from nitrogen, oxygen, and sulfur; and 5-10-membered heteroaryl having one nitrogen heteroatom and optionally 1-3 additional heteroatoms independently selected from nitrogen, oxygen, and sulfur, wherein the heterocyclyl and heteroaryl are optionally substituted by one or more substituents independently selected from halogen, CN, OH, SH, NO₂, oxo, C₁₋₆ alkyl, C₁₋₆ haloalkyl, C₁₋₆ alkoxy, C₁₋₆ alkylthio, and -NR^{10d}R^{10e}; and
   R^{8d}, R^{8e}, R^{10d}, and R^{10e} are each independently selected from hydrogen, deuterium, C₁₋₆ alkyl, and C₁₋₆ haloalkyl; and
   the LBM moiety is a ligase binding moiety.

In some embodiments,
R⁷, R⁸, R⁹, R¹¹, and R¹² are each independently selected from: saturated or partially unsaturated C₃₋₇ cyclohydrocarbyl;3-10-membered saturated or partially unsaturated heterocyclyl having 1-3 heteroatoms independently selected from nitrogen, oxygen, and sulfur; C₆₋₁₀ aryl; and 5-10-membered heteroaryl having 1-4 heteroatoms independently selected from nitrogen, oxygen, and sulfur, wherein the cyclohydrocarbyl, heterocyclyl, aryl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
R¹⁰ is selected from: 3-7-membered saturated or partially unsaturated heterocyclyl having one nitrogen heteroatom and optionally 1-2 additional heteroatoms independently selected from nitrogen, oxygen, and sulfur; and 5-10-membered heteroaryl having one nitrogen heteroatom and optionally 1-3 additional heteroatoms independently selected from nitrogen, oxygen, and sulfur, wherein the heterocyclyl and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{10d}R^{10e};

### Linking group L^{A}

In some embodiments, the present invention provides the compound of formula (A) according to the present invention, which has the L^{A} group as defined above.

In some embodiments, L^{A} is selected from a bond, C₁₋₂ alkylene, the C₁₋₂ alkylene is optionally substituted by one or more substituents independently selected from C₁₋₂ alkyl, C₁₋₂ haloalkyl, halogen, oxo (=O) , OH, CN, NH₂, -NH(C₁₋₂ alkyl), and -N(C₁₋₂ alkyl)₂.

In some embodiments, L^{A} is selected from a bond, C₁₋₂ alkylene, the C₁₋₂ alkylene is optionally substituted by a substituent selected from C₁₋₂ alkyl, halogen, oxo (=O).

In some embodiments, L^{A} is selected from a bond, -CH₂-, -CH₂-CH₂-, -CH(CH₃)-, and - C(=O)-.

In some embodiments, L^{A} is a bond, -CH₂-, or -CH₂-CH₂-, preferably -CH₂-.

### IRAK4 ligand

In some embodiments, the present invention provides the compound of formula (1) according to the present invention, which has the IRAK ligand as defined above.

In some embodiments, the present invention provides the IRAK ligand as described above, wherein, R¹ and R⁴ are not simultaneously hydrogen or deuterium.

In some embodiments, the present invention provides the IRAK ligand as described above, wherein, R^{1a}, R^{1b}, R^{3a}, and R^{3b} are each independently selected from hydrogen, halogen, CN, OH, SH, NO₂, C₁₋₄ alkyl, C₁₋₄ haloalkyl, C₁₋₄ alkoxy, C₁₋₄ alkylthio, and -NR^{7d}R^{7e}; or R^{1a} and R¹¹, or R^{3a} and R^{3b}, together with the carbon atoms that they are commonly attached to, form R⁹.

In some embodiments, the present invention provides the IRAK ligand as described above, wherein, R^{1d}, R^{1e}, R^{2d}, R^{2e}, R^{3d}, R^{3e}, R^{7d}, and R^{7e} are each independently selected from hydrogen, R¹¹, C₁₋₄ alkyl, and C₁₋₄ haloalkyl; or, R^{1d} and R^{1e}, or R^{2d} and R^{2e}, or R^{3d} and R^{3e}, or R^{7d} and R^{7e}, together with the nitrogen atoms that they are commonly attached to, form R¹⁰.

In some embodiments, the present invention provides the IRAK ligand as described above, wherein, R^{1f}, R^{2f}, and R^{3f} are independently selected from hydrogen, C₁₋₄ alkyl, C₁₋₄ haloalkyl, and R¹².

In some embodiments, the present invention provides the IRAK ligand as described above, wherein, R⁷, R⁸, R⁹, R¹¹, and R¹² are each independently selected from: saturated or partially unsaturated C₃₋₆ cyclohydrocarbyl; 3-10-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cyclohydrocarbyl, heterocyclyl, aryl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}.

In some embodiments, the present invention provides the IRAK ligand as described above, wherein, R¹⁰ is selected from: 3-6-membered saturated or partially unsaturated heterocyclyl having one nitrogen heteroatom and optionally 1-2 additional heteroatoms independently selected from nitrogen, oxygen, and sulfur; and 5-6-membered heteroaryl having one nitrogen heteroatom and optionally 1-2 additional heteroatoms independently selected from nitrogen, oxygen, and sulfur, wherein the heterocyclyl and heteroaryl are optionally substituted by one or more substituents independently selected from halogen, CN, OH, SH, NO₂, oxo, C₁₋₆ alkyl, C₁₋₆ haloalkyl, C₁₋₆ alkoxy, C₁₋₆ alkylthio, and -NR^{10d}R^{10e}.

In some embodiments, the present invention provides the IRAK ligand as described above, wherein, R^{8d}, R^{8e}, R^{10d}, and R^{10e} are each independently selected from hydrogen, C₁₋₆ alkyl, and C₁₋₆ haloalkyl.

In a first sub-aspect, the present invention provides the IRAK ligand as described above, wherein, R¹ is not hydrogen.

In some embodiments, R¹ is selected from: R⁷, halogen, CN, NO₂, C₁₋₄ alkyl, -OR^{1f}, -SR^{1f}, -NR^{1d}R^{1e}, -S(O)₂R^{1f}, -S(O)R^{1f}, -S(O)₂-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), -CF₃, -CCl(R^{lf})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1c}, -C(O)R^{1f}, -C(O)OR^{1f}, -C(O)NR^{1d}R^{1e}, -C(O)NR^{1f}-OR^{1f}, - OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, or -NR^{1f} -S(O)₂R^{1f}.

In some embodiments, R¹ is selected from: R⁷, halogen, CN, NO₂, C₁₋₄ alkyl, -OR^{1f}, -SR^{1f}, -NR^{1d}R^{1e}, -CF(R^{1f})₂, -CF₂(R^{1f}), -CF₃, -CCl(R^{1f})₂, -CCl₂(R^{1f}), -CCl₃, -(CR^{1a}R^{1b})ₚ-OR^{1f}, - (CR^{1a}R^{1b})ₚ-C(O)OR^{1f}, -(CR^{1a}R^{1b})ₚ-NR^{1d}R^{1c}, -C(O)OR^{1f}, or -C(O)NR^{1d}R^{1c}; or
In some embodiments, R¹ is selected from: R⁷, halogen, CN, -OR^{1f}, -NR^{1d}R^{1e}, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e}.

In some embodiments, R⁷ is selected from: C₃₋₆ cycloalkyl; 4-6-membered saturated or partially unsaturated heterocyclyl having 1-3 heteroatoms independently selected from nitrogen, oxygen, and sulfur; phenyl; and 5-6-membered heteroaryl having 1-3 heteroatoms independently selected from nitrogen, oxygen, and sulfur, wherein the cycloalkyl, heterocyclyl, phenyl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}.

In some embodiments, R⁷ is selected from: C₃₋₆ cycloalkyl; 4-6-membered heterocycloalkyl having 1-3 heteroatoms independently selected from nitrogen, oxygen, and sulfur; phenyl; and 5-6-membered heteroaryl having 1-3 heteroatoms independently selected from nitrogen, oxygen, and sulfur, wherein the cycloalkyl, heterocycloalkyl, phenyl, and heteroaryl are optionally substituted by one or more substituents independently selected from halogen, CN, OH, NO₂, C₁₋₄ alkyl, C₁₋₄ haloalkyl, C₁₋₄ alkoxy, and -NR^{8d}R^{8e}.

In some embodiments, R^{8d} and R^{8e} are each independently selected from hydrogen and C₁₋₆ alkyl; or, preferably, R^{8d} and R^{8e} are each independently selected from hydrogen and C₁₋₄ alkyl.

In some embodiments, R⁷ is selected from: 4-6-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R⁷ is selected from: azetidinyl, pyrrolidinyl, and piperidinyl.

In some embodiments, p is 1.

In some embodiments, R^{1a} and R^{1b} are each independently selected from hydrogen and C₁₋₆ alkyl.

In some embodiments, R^{1a} and R^{1b} are each independently selected from hydrogen and C₁₋₄ alkyl.

In some embodiments, R^{1a} and R^{1b} are each independently hydrogen.

In some embodiments, R^{1a} and R^{1b} together with the carbon atoms that they are commonly attached to, form R⁹.

In some embodiments, R⁹ is selected from: C₃₋₆ cycloalkyl; 3-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}.

In some embodiments, R^{8d} and R^{8e} are each independently selected from hydrogen and C₁₋₆ alkyl; or preferably, R^{8d} and R^{8e} are each independently selected from hydrogen and C₁₋₄ alkyl.

In some embodiments, R⁹ is selected from: C₃₋₆ cycloalkyl, wherein the cycloalkyl is optionally substituted by one or more substituents independently selected from F, Cl, Br, CN, OH, NO₂, C₁₋₄ alkyl, C₁₋₄ haloalkyl, C₁₋₄ alkoxy, NH₂, -NH(C₁₋₄ alkyl), and N(C₁₋₄ alkyl)₂.

In some embodiments, R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R⁹ is cyclopropyl.

In some embodiments, R^{1d} and R^{1e} are each independently selected from hydrogen, R¹¹, and C₁₋₆ alkyl.

In some embodiments, R^{1d} and R^{1e} are each independently selected from hydrogen, R¹¹, and C₁₋₄ alkyl.

In some embodiments, R^{1d} and R^{1e} are each independently selected from hydrogen, R¹¹, methyl, ethyl, propyl, isopropyl, n-butyl, isobutyl, sec-butyl, and tert-butyl.

In some embodiments, R^{1d} and R^{1e} together with the nitrogen atoms that they are commonly attached to, form R¹⁰.

In some embodiments, R¹¹ is selected from: C₃₋₆ cycloalkyl; 3-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}.

In some embodiments, R^{8d} and R^{8e} are each independently selected from hydrogen and C₁₋₆ alkyl; preferably, R^{8d} and R^{8e} are each independently selected from hydrogen and C₁₋₄ alkyl.

In some embodiments, R¹¹ is selected from: C₃₋₆ cycloalkyl; and 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹¹ is selected from: cyclopropyl, cyclobutyl, cyclopentyl, cyclohexyl, azetidinyl, pyrrolidinyl, oxazolidine, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R¹¹ is selected from: cyclopropyl, cyclobutyl, cyclopentyl, cyclohexyl, azetidinyl, pyrrolidinyl, and piperidinyl, each of which is optionally substituted by one or more substituents independently selected from F and Cl.

In some embodiments, R¹¹ is selected from cyclopropyl and

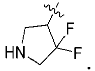

In some embodiments, R¹⁰ is selected from: 3-6-membered heterocycloalkyl having one nitrogen heteroatom and optionally 1-2 additional heteroatoms independently selected from nitrogen, oxygen, and sulfur, and 5-6-membered heteroaryl having one nitrogen heteroatom and optionally 1-2 additional heteroatoms independently selected from nitrogen, oxygen, and sulfur, wherein the heterocycloalkyl and heteroaryl are optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: 3-6-membered heterocycloalkyl having one nitrogen heteroatom and optionally 1-2 additional heteroatoms independently selected from nitrogen, oxygen, and sulfur, and 5-6-membered heteroaryl having one nitrogen heteroatom and optionally 1-2 additional heteroatoms independently selected from nitrogen, oxygen, and sulfur, wherein the heterocycloalkyl and heteroaryl are optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: 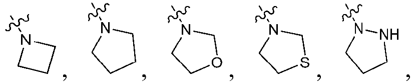 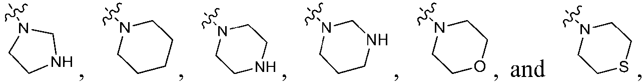 each of which is optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R¹⁰ is selected from: 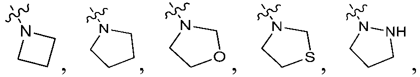 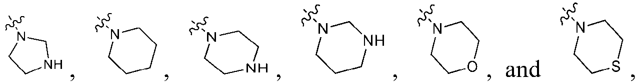 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R¹⁰ is selected from: 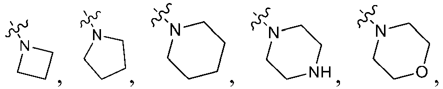 and 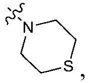 each of which is optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R¹⁰ is selected from: 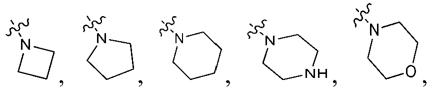 and 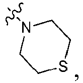 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1f} is selected from hydrogen and C₁₋₆ alkyl.

In some embodiments, R^{1f} is selected from hydrogen and C₁₋₄ alkyl.

In some embodiments, R^{1f} is hydrogen.

In some embodiments, wherein R¹ is selected from: F, Cl, Br, CN, OH, NH₂, -NHCH₃, - C(O)OH, -C(O)NH₂, -CH₂NH₂, -CH₂OH,

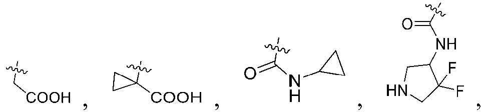

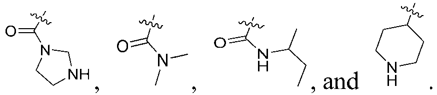

In some embodiments, R¹ is selected from -CN, -C(O)NH₂, F, -NHCH₃, -C(O)OH,

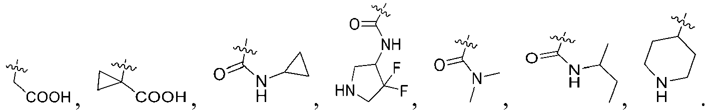

In some embodiments according to the first sub-aspect, the 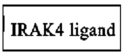 moiety has the structure of formula (I-1): 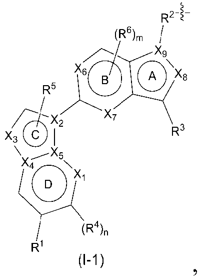 wherein, n is 0 or 1.

In a second sub-aspect, the present invention provides the IRAK ligand as described above, wherein, R¹ is selected from hydrogen and deuterium, and n is 1 or 2.

In some embodiments, R¹ is hydrogen, and n is 1.

In some embodiments according to the second sub-aspect, the 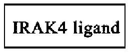 moiety has the structure of formula (I-2): 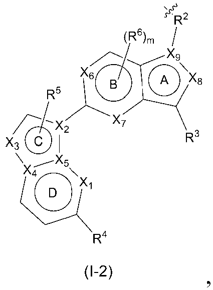 with the proviso that: R⁴ is not hydrogen or deuterium.

In some embodiments, the present invention provides the compound according to any of the above-described embodiments, wherein, R⁴ is selected from: hydrogen, R⁷, halogen, CN, NO₂, -OR^{1f}, -SR^{1f}, and -NR^{1d}R^{1e}; with the proviso that when R¹ is hydrogen and deuterium, R⁴ is not hydrogen.

In some embodiments, R^{1d} and R^{1e} are each independently selected from hydrogen and C₁₋₆ alkyl.

In some embodiments, R^{1d} and R^{1e} are each independently selected from hydrogen and C₁₋₄ alkyl.

In some embodiments, R^{1f} is selected from hydrogen and C₁₋₆ alkyl.

In some embodiments, R^{1f} is selected from hydrogen and C₁₋₄ alkyl.

In some embodiments, R⁴ is selected from: hydrogen, R⁷, F, Cl, Br, CN, NO₂, OH, and NH₂; with the proviso that when R¹ is hydrogen and deuterium, R⁴ is not hydrogen.

In some embodiments, R⁴ is selected from: hydrogen, R⁷, and CN; with the proviso that when R¹ is hydrogen and deuterium, R⁴ is not hydrogen.

In some embodiments, R⁷ is selected from: 4-6-membered saturated monocyclic heterocyclyl, 8-10-membered saturated fused bicyclic heterocyclyl, 6-11-membered saturated spiro heterocyclyl, and 7-10-membered saturated bridged heterocyclyl, each of which has 1-3 heteroatoms independently selected from nitrogen, oxygen, and sulfur, and is optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}.

In some embodiments, R⁷ is selected from: 4-6-membered saturated monocyclic heterocyclyl and 7-10-membered saturated bridged heterocyclyl, the monocyclic heterocyclyl and bridged heterocyclyl have one nitrogen heteroatom and optionally 1-2 additional heteroatoms independently selected from nitrogen, oxygen, and sulfur, and are optionally substituted by one or more substituents independently selected from halogen, CN, OH, NO₂, C₁₋₄ alkyl, C₁₋₄ haloalkyl, C₁₋₄ alkoxy, and -NR^{8d}R^{8e}.

In some embodiments, R^{8d} and R^{8e} are each independently selected from hydrogen and C₁₋₆ alkyl; preferably, R^{8d} and R^{8e} are each independently selected from hydrogen and C₁₋₄ alkyl.

In some embodiments, R⁷ is selected from: 4-6-membered saturated monocyclic heterocyclyl (for example azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl) and 7-10-membered saturated bridged heterocyclyl, the monocyclic heterocyclyl and bridged heterocyclyl have one nitrogen heteroatom and optionally 1-2 additional heteroatoms independently selected from nitrogen, oxygen, and sulfur, and are optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R⁷ is selected from: 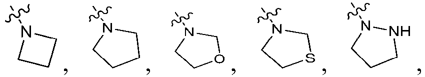 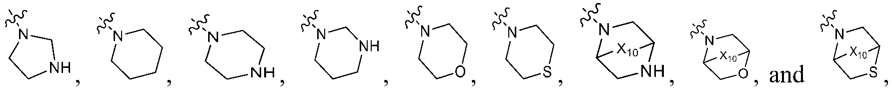 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂, and wherein X₁₀ is CH₂, (CH₂)₂, or (CH₂)₃.

In some embodiments, R⁷ is selected from:

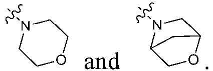

In some embodiments, R⁴ is selected from: hydrogen, CN, 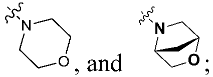 with the proviso that: when R¹ is hydrogen or deuterium, R⁴ is not hydrogen.

In some embodiments, the present invention provides the compound according to any of the above-described embodiments, wherein, R and R⁵ are each independently selected from hydrogen.

In some embodiments, the present invention provides the compound according to any of the above-described embodiments, wherein, R² is selected from: C₃₋₆ cycloalkylene; 4-6-membered saturated or partially unsaturated heterocyclylene having 1-3 heteroatoms independently selected from nitrogen, oxygen, and sulfur; C₆₋₁₀ arylene; and 5-6-membered heteroarylene having 1-3 heteroatoms independently selected from nitrogen, oxygen, and sulfur, wherein the cycloalkylene, heterocyclylene, arylene, and heteroarylene are optionally substituted by 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and - C(O)NR^{2d}R^{2e}, and in the case where two substituents are attached to the same ring carbon atom of the cycloalkylene or heterocyclylene, the two substituents optionally together with the ring carbon atoms that they are attached to, form optionally substituted C₃₋₆ cycloalkyl, or optionally substituted 4-6-membered heterocycloalkyl having 1-3 heteroatoms independently selected from nitrogen, oxygen, and sulfur; wherein preferably, R^{2d} and R^{2e} are each independently selected from hydrogen, C₁₋₆ alkyl, and C₁₋₆ haloalkyl, and/or R^{2f} is selected from hydrogen and C₁₋₆ alkyl.

In some embodiments, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₄ alkyl, -C(O)OH, - C(O)O-C₁₋₄ alkyl, -C(O)NH₂, -C(O)NH(C₁₋₄ alkyl), and -C(O)NH(C₁₋₄ alkyl)₂, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl.

In some embodiments, R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylidene, piperazinylene, hexahydropyrimidinylene, triazinylene, morpholinylene, thiomorpholinylidene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1, 2, or more substituents independently selected from F, Cl, Br, CN, OH, NH₂, NO₂, methyl, ethyl, isopropyl, tert-butyl, C(O)OH, -C(O)OCH₃, -C(O)OCH₂CH₃, - C(O)NH₂, -C(O)NHCH₃, and -C(O)N(CH₃)₂, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylidene, piperazinylene, hexahydropyrimidinylene, morpholinylene, or thiomorpholinylidene, the two substituents optionally together with the ring carbon atoms that they are attached to, form optionally substituted C₃₋₆ cycloalkyl.

In some embodiments, R² is selected from: cyclohexylidene, pyrrolidinylene, piperidinylene, phenylene, and pyridylene, each of which is optionally substituted by 1 or 2 substituents independently selected from F, Cl, Br, OH, NH₂, and methyl, and in the case where two substituents are attached to the same ring carbon atom of the cyclohexylidene, pyrrolidinylene, or piperidinylene, the two substituents optionally together with the ring carbon atoms that they are attached to, form optionally substituted C₃₋₆ cycloalkyl.

In some embodiments, R² is selected from: 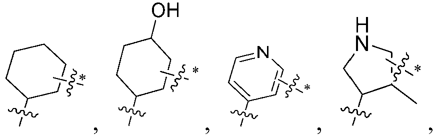 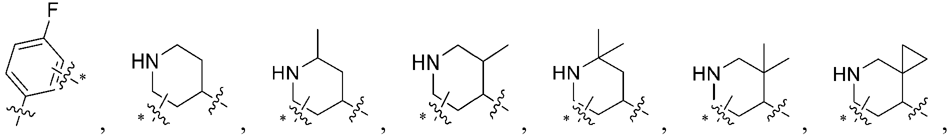 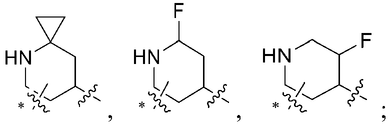 wherein, the wavy line " 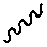 " represents the site of attachment, the bond indicated by * is attached to L^{A}.

In some embodiments, R² is selected from: 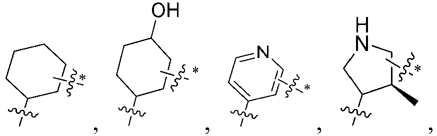 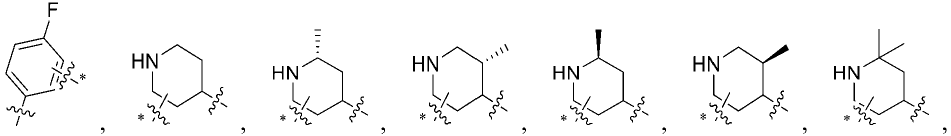 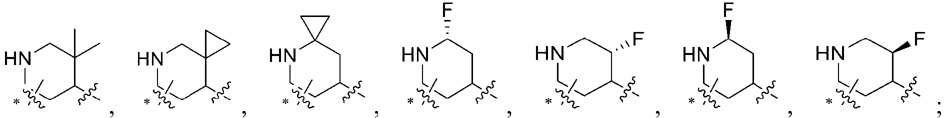 wherein, the wavy line " 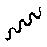 " represents the site of attachment, the bond indicated by * is attached to L^{A}.

In some embodiments, the present invention provides the compound according to any of the above-described embodiments, wherein, R³ is selected from: halogen, CN, NO₂, C₁₋₆ alkyl, -OR^{3f}, -SR^{3f}, -NR^{3d}R^{3e}, -S(O)₂-NR^{3d}R^{3e}, -S(O)-NR^{3d}R^{3e}, -CF(R^{3f})₂, -CF₂(R^{3f}), -CF₃, -CCl(R^{3f})₂, -CCl₂(R^{3f}), -CCl₃, -C(O)OR^{3f}, and -C(O)NR^{3d}R^{3e}.

In some embodiments, R³ is selected from: halogen, CN, C₁₋₄ alkyl, -OR^{3f}, -NR^{3d}R^{3e}, - CF(R^{3f})2, -CF₂(R^{3f}), -CF₃, -CCl(R^{3f})₂, -CCl₂(R^{3f}), -CCl₃, -C(O)OR^{3f}, and -C(O)NR^{3d}R^{3e}.

In some embodiments, R³ is selected from: halogen, CN, C₁₋₄ alkyl, -OR^{3f}, -NR^{3d}R^{3e}, - CF(R^{3f})₂, -CF₂(R^{3f}), -CF₃, -CCl(R^{3f})₂, -CCl₂(R^{3f}), -CCl₃, and -C(O)NR^{3d}R^{3e}.

In some embodiments, R^{3d} and R^{3e} are each independently selected from hydrogen, deuterium, R¹¹, C₁₋₄ alkyl, and C₁₋₄ haloalkyl. In some embodiments, wherein R¹¹ is preferably selected from: C₃₋₆ cycloalkyl; 4-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}, wherein preferably, R^{8d} and R^{8e} are each independently selected from hydrogen, C₁₋₄ alkyl, and C₁₋₄ haloalkyl.

In some embodiments, R^{3d} and R^{3e} are each independently selected from hydrogen, methyl, and ethyl.

In some embodiments, R^{3f} is selected from hydrogen and C₁₋₆ alkyl.

In some embodiments, R^{3f} is selected from hydrogen and C₁₋₄ alkyl.

In some embodiments, R^{3d} and R^{3e} together with the nitrogen atoms that they are commonly attached to, form R¹⁰. In some embodiments, wherein R¹⁰ is preferably selected from: 3-6-membered heterocycloalkyl having one nitrogen heteroatom and optionally 1-2 additional heteroatoms independently selected from nitrogen, oxygen, and sulfur, and 5-6-membered heteroaryl having one nitrogen heteroatom and optionally 1-2 additional heteroatoms independently selected from nitrogen, oxygen, and sulfur, wherein the heterocycloalkyl and heteroaryl are optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, - C(O)NH₂, -C(O)NH(C₁₋₄ alkyl), and -C(O)N(C₁₋₄ alkyl)₂.

In some embodiments, R³ is selected from: F, Cl, Br, OH, NH₂, CN, methyl, ethyl, propyl, isopropyl, n-butyl, isobutyl, sec-butyl, tert-butyl, methoxy, ethoxy, -N(CH₃)₂, -CHF₂, -CHCl₂, and -C(O)NH₂.

In some embodiments, R⁶ is selected from: halogen, CN, NO₂, C₁₋₄ alkyl, -OR^{3f}, -SR^{3f}, - NR^{3d}R^{3e}, -CF(R^{3f})₂, -CF₂(R^{3f}), -CF₃, -CCl(R^{3f})₂, -CCl₂(R^{3f}), and -CCl₃, wherein R^{3f} is preferably selected from hydrogen and C₁₋₆ alkyl, or R^{3f} is preferably selected from hydrogen and C₁₋₄ alkyl.

In some embodiments, R⁶ is selected from: F, Cl, Br, OH, NH₂, CN, NO₂, methyl, ethyl, propyl, isopropyl, n-butyl, isobutyl, sec-butyl, tert-butyl, methoxy, ethoxy, -CH₂F, -CHF₂, -CF₃, -CH₂Cl, -CHCl₂, and -CCl₃.

In some embodiments, the present invention provides the compound according to any of the above-described embodiments, m is 0.

In some embodiments, the present invention provides the compound according to any of the above-described embodiments, at least 2 of X₁, X₂, X₃, X₄, and X₅ are N, with the proviso that X₄ and X₅ are not simultaneously N.

In some embodiments, 3 of X₁, X₂, X₃, X₄, and X₅ are N, with the proviso that X₄ and X₅ are not simultaneously N.

In some embodiments, 4 of X₁, X₂, X₃, X₄, and X₅ are N, with the proviso that X₄ and X₅ are not simultaneously N.

In some embodiments, X₁ is N.

In some embodiments, at least 2 of X₆, X₇, X₈, and X₉ are N.

In some embodiments, at least 3 of X₆, X₇, X₈, and X₉ are N.

In some embodiments, at least 4 of X₆, X₇, X₈, and X₉ are N.

In some embodiments, X₈ and X₉ are each N.

In some embodiments, the present invention provides the compound according to any of the above-described embodiments, 2 or 3 of X₁, X₂, X₃, X₄, and X₅ are N, and X₁ is N, with the proviso that X₄ and X₅ are not simultaneously N; and
3 or 4 of X₆, X₇, X₈, and X₉ are N, and X₈ and X₉ are each N.

In a third sub-aspect, the present invention provides the 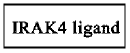 moiety according to formula (I), formula (1-1), and formula (I-2) described above, wherein, X₁ and X₂ are each N; X₃ is CH; X₄ and X₅ are each C; X₆, X₈, and X₉ are each N; and X₇ is CH.

In some embodiments, the 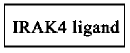 moiety has the structure of formula (I-i) or formula (I-ii): 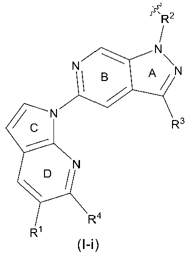 wherein: R¹, R², R³, and R⁴, are each as defined in any of the embodiments described above with respect to the 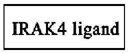 moiety of the formula (I), formula (1-1), and formula (I-2), with the proviso that: R¹ is not hydrogen or deuterium; 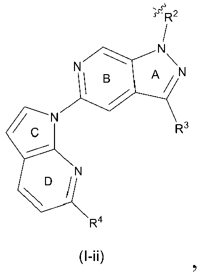 wherein: R², R³, and R⁴, are each as defined in any of the embodiments described above with respect to the 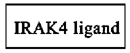 moiety of the formula (I), formula (1-1), and formula (I-2), with the proviso that: R¹ is not hydrogen or deuterium.

In some embodiments, R¹ is selected from: halogen, CN, NO₂, -OR^{1f}, -(CR^{1a}R^{1b})ₚ-OR^{1f}, - (CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}.

In some embodiments, R¹ is selected from: halogen, CN, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e}.

In some embodiments, p is 1.

In some embodiments, R^{1a} and R^{1b} are each independently selected from hydrogen and C₁₋₄ alkyl, preferably each independently are hydrogen; or R^{1a} and R^{1b} together with the carbon atoms that they are commonly attached to, form R⁹.

In some embodiments, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂.

In some embodiments, R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R⁹ is cyclopropyl.

In some embodiments, R^{1d} and R^{1e} are each independently selected from hydrogen and R¹¹; or R^{1d} and R^{1e} together with the nitrogen atoms that they are commonly attached to, form R¹⁰.

In some embodiments, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋
4 alkoxy, NH₂, NH(C₁₋₄ alkyl), and N(C₁₋₄ alkyl)₂.

In some embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂.

In some embodiments, R¹¹ is selected from: cyclopropyl, cyclobutyl, cyclopentyl, cyclohexyl, azetidinyl, pyrrolidinyl, piperidinyl, piperazinyl, and morpholinyl, each of which is optionally substituted by one or more substituents independently selected from F, Cl, Br, CN, OH, NO₂, and NH₂.

In some embodiments, R¹¹ is selected from cyclopropyl and

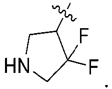

In some embodiments, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R¹⁰ is selected from: 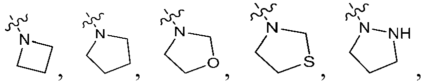 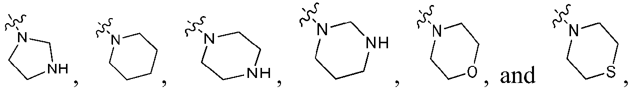 preferably 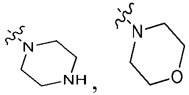 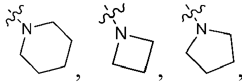 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1f} is selected from hydrogen and C₁₋₄ alkyl, preferably hydrogen.

In some embodiments, R¹ is selected from: F, Cl, Br, CN, OH, NH₂, C(O)OH, -C(O)NH₂,

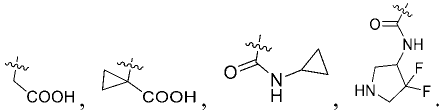

In some embodiments, R¹ is selected from CN and -C(O)NH₂.

In some embodiments, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl.

In some embodiments, R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl.

In some embodiments, R² is selected from cyclopentylidene, cyclohexylidene, pyrrolidinylene, piperidinylene, phenylene, and pyridylene, each of which is optionally substituted by 1 or 2 substituents independently selected from F, Cl, Br, CN, OH, NH₂, NO₂, methyl, ethyl, and isopropyl, and in the case where two substituents are attached to the same ring carbon atom of the cyclopentylidene, cyclohexylidene, pyrrolidinealkylene, or piperidinylene, the two substituents optionally together with the ring carbon atoms that they are attached to, form optionally substituted C₃₋₆ cycloalkyl.

In some embodiments, R² is selected from: 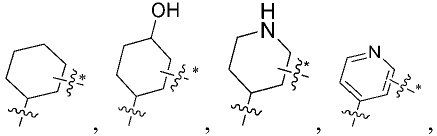 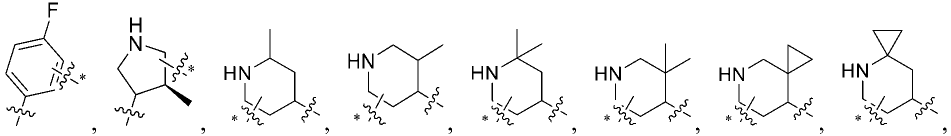 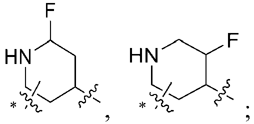 wherein, the wavy line " 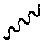 " represents the site of attachment, the bond indicated by * is attached to L^{A}.

In some embodiments, R² is selected from 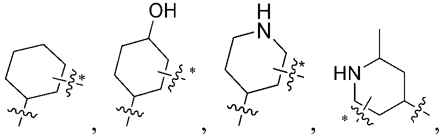 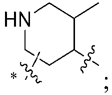 wherein, the wavy line " 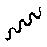 " represents the site of attachment, the bond indicated by * is attached to L^{A}.

In some embodiments, R² is 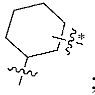 wherein, the wavy line " 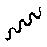 " represents the site of attachment, the bond indicated by * is attached to L^{A}.

In some embodiments, R³ is selected from: halogen, CN, NO₂, C₁₋₆ alkyl, OH, NH₂, -CH₂F, -CHF₂, -CF₃, -CH₂Cl, -CHCl₂, and -CCl₃.

In some embodiments, R³ is -CHF₂.

In some embodiments, R⁴ is selected from: hydrogen, R⁷, halogen, CN, NO₂, OH, and NH₂.

In some embodiments, R⁴ is selected from: hydrogen, R⁷, F, Cl, Br, CN, NO₂, OH, and NH₂.

In some embodiments, R⁷ is selected from: 4-6-membered saturated monocyclic heterocyclyl, the heterocyclyl has one nitrogen heteroatom and optionally 1-2 additional heteroatoms independently selected from nitrogen, oxygen, and sulfur, and is optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, NH₂, NH(C₁₋₄ alkyl), and N(C₁₋₄ alkyl)₂.

In some embodiments, R⁷ is selected from:

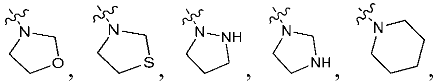

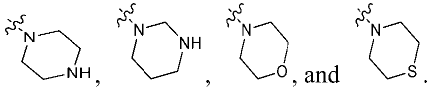

In some embodiments, R⁴ is selected from: hydrogen, CN, and 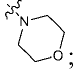 with the proviso that: when R¹ is hydrogen, R⁴ is not hydrogen.

In a fourth sub-aspect, the present invention provides the compound according to formula (I), formula (I-1), and formula (I-2) described above, wherein, X₁ and X₅ are each N; X₂ and X₄ are C; X₃ is CH; X₆, X₈, and X₉ are each N; and X₇ is CH.

In some embodiments, the 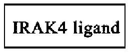 moiety of the present invention has the structure of formula (I-iii): 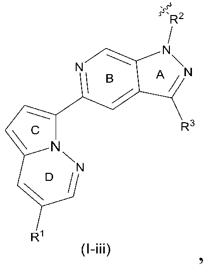 wherein: R¹, R², and R³, are each as defined in any of the embodiments described above with respect to the 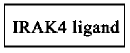 moiety of the formula (I), formula (1-1), and formula (I-2), with the proviso that: R¹ is not hydrogen or deuterium.

In some embodiments, R¹ is selected from: R⁷, halogen, NO₂, -OR^{1f}, -NR^{1d}R^{1e}, - (CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}.

In some embodiments, R¹ is selected from: R⁷, halogen, -NR^{1d}R^{1e}, -(CR^{1a}R^{1b})ₚ-OR^{1f}, - (CR^{1a}R^{1b})ₚ-C(O)OR^{1f}, -C(O)OR^{1f}, or -C(O)NR^{1d}R^{1e}.

In some embodiments, p is 1.

In some embodiments, R⁷ is selected from: 4-6-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R⁷ is selected from: azetidinyl, pyrrolidinyl, oxazolidine, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R⁷ is piperidinyl.

In some embodiments, R^{1a} and R^{1b} are each independently selected from hydrogen and C₁₋₄ alkyl, preferably each independently are hydrogen.

In some embodiments, R^{1a} and R^{1b} together with the carbon atoms that they are commonly attached to, form R⁹.

In some embodiments, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂.

In some embodiments, R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1d} and R^{1e} are each independently selected from hydrogen, C₁₋₄ alkyl, and R¹¹; or R^{1d} and R^{1e} together with the nitrogen atoms that they are commonly attached to, form R¹⁰.

In some embodiments, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂.

In some embodiments, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1f} is selected from hydrogen and C₁₋₄ alkyl, preferably hydrogen;

In some embodiments, R¹ is selected from: F, Cl, Br, OH, NH₂, -NHCH₃, C(O)OH, - C(O)NH₂, -CH₂OH,

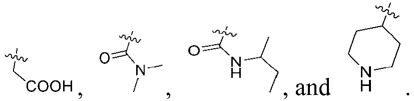

In some embodiments, R¹ is selected from -CN and -C(O)NH₂.

In some embodiments, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl.

In some embodiments, R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl.

In some embodiments, R² is selected from: cyclopentylidene, cyclohexylidene, pyrrolidinylidene, piperidinylidene, phenylene, and pyridylene, each of which is optionally substituted by 1 or 2 substituents independently selected from F, Cl, Br, CN, OH, NH₂, NO₂, methyl, ethyl, and isopropyl, and in the case where two substituents are attached to the same ring carbon atom of the cyclopentylidene, cyclohexylidene, pyrrolidinylidene, or piperidinylidene, the two substituents optionally together with the ring carbon atoms that they are attached to, form optionally substituted C₃₋₆ cycloalkyl.

In some embodiments, R² is selected from: 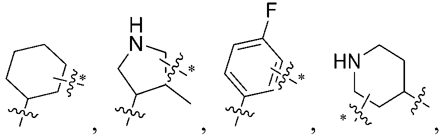 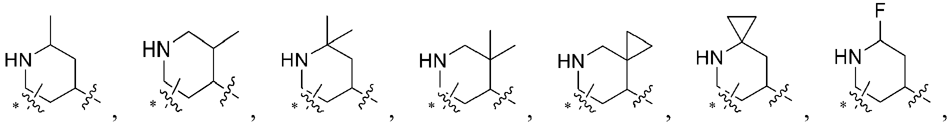 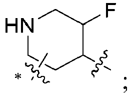 wherein, the wavy line " 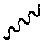 " represents the site of attachment, the bond indicated by * is attached to L^{A}.

In some embodiments, R² is selected from: 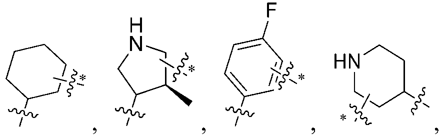 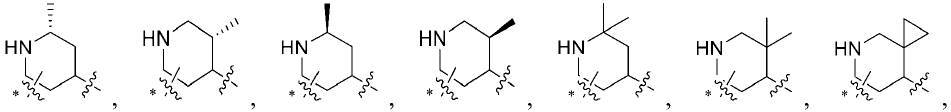 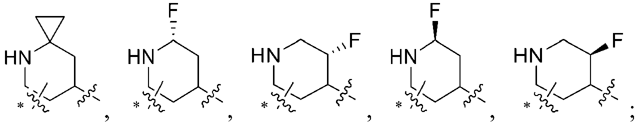 wherein, the wavy line " 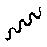 " represents the site of attachment, the bond indicated by * is attached to L^{A}.

In some embodiments, R³ is selected from: halogen, CN, C₁₋₄ alkyl, -OR^{3f}, -SR^{3f}, -NR^{3d}R^{3e}, -CF(R^{3f})₂, -CF₂(R^{3f}), -CF₃, -CCl(R^{3f})₂, -CCl₂(R^{3f}), -CCl₃, and -C(O)NR^{3d}R^{3e}.

In some embodiments, R^{3d} and R^{3e} are each independently selected from hydrogen and C₁₋₄ alkyl, preferably hydrogen and methyl.

In some embodiments, R^{3f} is selected from hydrogen and C₁₋₄ alkyl, preferably hydrogen and methyl.

In some embodiments, R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, - C(O)NH₂, -C(O)NH(C₁₋₄ alkyl), and -C(O)N(C₁₋₄ alkyl)₂.

In some embodiments, R³ is selected from: F, OH, NH₂, CN, methyl, ethyl, isopropyl, tert-butyl, methoxy, -N(CH₃)₂, -CHF₂, and -C(O)NH₂.

In some embodiments, R³ is selected from: F, CN, methyl, isopropyl, methoxy, -N(CH₃)₂, -CHF₂, and -C(O)NH₂.

In a fifth sub-aspect, the present invention provides the compound according to formula (I), formula (I-1), and formula (I-2) described above, wherein, X₁, X₃, and X₄ are each N; X₂ and are C; X₆, X₈, and X₉ are each N; and X₇ is CH.

In some embodiments, the IRAK4 ligand moiety has the structure of formula (I-iv): 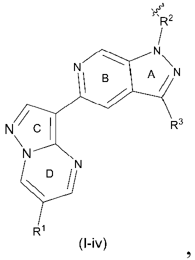 wherein: R¹, R², and R³, are each as defined in any of the embodiments described above with respect to the 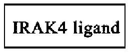 moiety of the formula (I), formula (1-1), and formula (I-2), with the proviso that: R¹ is not hydrogen or deuterium.

In some embodiments, R¹ is selected from: halogen, CN, NO₂, -OR^{1f}, -(CR^{1a}R^{1b})ₚ-OR^{1f}, - (CR^{1a}R^{1b})ₚ-C(O)OR^{1f}, -(CR^{1a}R^{1b})ₚ-NR^{1d}R^{1e}, or -C(O)OR^{1f}.

In some embodiments, R¹ is selected from: halogen, CN, -(CR^{1a}R^{1b})ₚ-OR^{1f}, -(CR^{1a}R^{1b})ₚ-C(O)OR^{1f}, or -C(O)OR^{1f}.

In some embodiments, p is 1.

In some embodiments, R^{1a} and R¹¹ are each independently selected from hydrogen and C₁₋₄ alkyl, preferably each independently are hydrogen; or R^{1a} and R¹¹ together with the carbon atoms that they are commonly attached to, form R⁹.

In some embodiments, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂.

In some embodiments, R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1d} and R^{1e} are each independently selected from hydrogen and R¹¹; or R^{1d} and R^{1e} together with the nitrogen atoms that they are commonly attached to, form R¹⁰.

In some embodiments, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂.

In some embodiments, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some embodiments, R¹⁰ is selected from: azetidinyl, pyrrolidinyl, oxazolidine, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some embodiments, R^{1f} is selected from hydrogen and C₁₋₄ alkyl, preferably hydrogen.

In some embodiments, R¹ is selected from: F, Cl, Br, CN, OH, NH₂, C(O)OH, 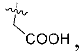 and

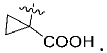

In some embodiments, R¹ is CN.

In some embodiments, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl.

In some embodiments, R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl.

In some embodiments, R² is selected from: 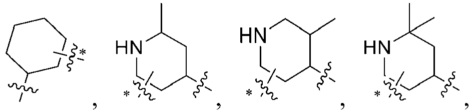 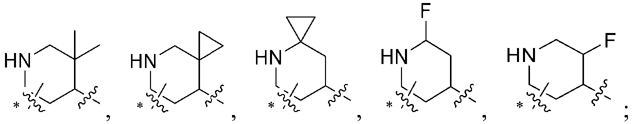 wherein, the wavy line " 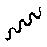 " represents the site of attachment, the bond indicated by * is attached to L^{A}.

In some embodiments, R² is selected from: 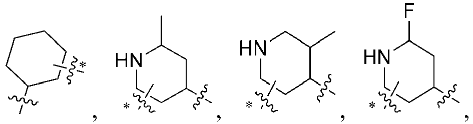 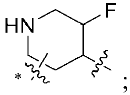 wherein, the wavy line " 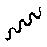 " represents the site of attachment, the bond indicated by * is attached to L^{A}.

In some embodiments, R² is 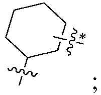 wherein, the wavy line " 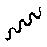 " represents the site of attachment, the bond indicated by * is attached to L^{A}.

In some embodiments, R³ is selected from: halogen, CN, NO₂, C₁₋₆ alkyl, OH, NH₂, -CH₂F, -CHF₂, -CF₃, -CH₂Cl, -CHCl₂, and -CCl₃.

In some embodiments, R³ is -CHF₂.

In a sixth sub-aspect, the present invention provides the compound according to formula (I), formula (I-1), and formula (I-2) described above, wherein, X₁ and are each N; X₂ and X₄ are C; and X₃ is CH; X₆ is CH; and X₇, X₈, and X₉ are each N.

In some embodiments, the IRAK4 ligand moiety has the structure of formula (I-v): 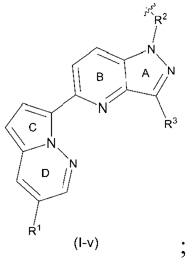 wherein, R¹, R², and R³, are each as defined in any of the embodiments described above with respect to the 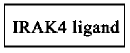 moiety of the formula (I), formula (1-1), and formula (I-2), with the proviso that: R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -C(O)NH₂; and/or R² is 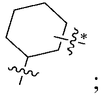 wherein, the wavy line " 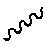 " represents the site of attachment, the bond indicated by * is attached to L^{A}; and/or R³ is - CHF₂.

In a seventh sub-aspect, the present invention is defined according to any of the embodiments described above with respect to the 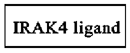 moiety of the formula (I), formula (I-1), and formula (I-2), wherein X₁ and are each N; X₂ and X₄ are C; X₃ is CH; and X₆, X₇, X₈, and X₉ are each N.

In some embodiments, the 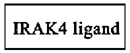 moiety of the present invention has the structure of formula (I-vi): 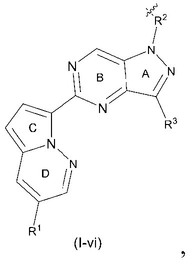 wherein, R¹, R², and R³, are each as defined in any of the embodiments described above with respect to the 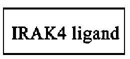 moiety of the formula (I), formula (I-1), and formula (I-2), with the proviso that: R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -C(O)NH₂; and/or R² is 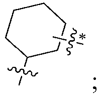 wherein, the wavy line " 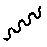 " represents the site of attachment, the bond indicated by * is attached to L^{A}; and/or R³ is - CHF₂;

In an eighth sub-aspect, the present invention is defined according to any of the embodiments described above with respect to the 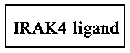 moiety of the formula (I), formula (I-1), and formula (I-2), wherein: X₁ and X₂ are each N; X₃ is CH; X₄ and are each C; and X₆, X₇, X₈, and X₉ are each N.

In some embodiments, the IRAK4 ligand moiety has the structure of formula (I-vii): 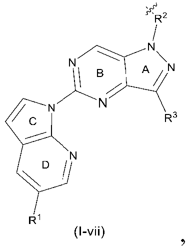 wherein, R¹, R², and R³, are each as defined in any of the embodiments described above with respect to the 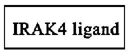 moiety of the formula (I), formula (1-1), and formula (I-2), with the proviso that: R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -CN; and/or R² is 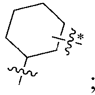 wherein, the wavy line " 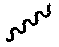 " represents the site of attachment, the bond indicated by * is attached to L^{A}; and/or R³ is -CHF₂.

In a ninth sub-aspect, the present invention is defined according to any of the embodiments described above with respect to the 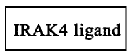 moiety of the formula (I), formula (I-1), and formula (I-2), wherein: X₁, X₃, and X₄ are each N; X₂ and are C; and X₆, X₇, X₈, and X₉ are each N.

In some embodiments, the 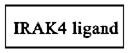 moiety of the present invention has the structure of formula (I-viii): 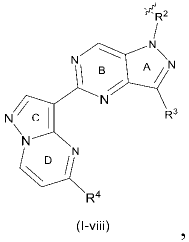 wherein, R², R³, and R⁴, are each as defined in any of the embodiments described above with respect to the 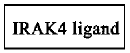 moiety of the formula (I), formula (I-1), and formula (I-2), with the proviso that: R¹ is not hydrogen or deuterium.

In some embodiments, R², R³, and R⁴ are each as defined in any of the embodiments according to the third sub-aspect, the fourth sub-aspect, and/or the fifth sub-aspect.

In some embodiments, R² is 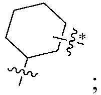 wherein, the wavy line " 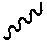 " represents the site of attachment, the bond indicated by * is attached to L^{A}; and/or, in some embodiments, R³ is - CHF₂.

In some embodiments, R⁴ is selected from: R⁷, halogen, CN, NO₂, OH, and NH₂.

In some embodiments, R⁴ is selected from: R⁷, F, Cl, Br, CN, NO₂, OH, and NH₂.

In some embodiments, R⁷ is selected from: 7-10-membered saturated bridged heterocyclyl, the bridged heterocyclyl has one nitrogen heteroatom and optionally 1-2 additional heteroatoms independently selected from nitrogen, oxygen, and sulfur, and is optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some embodiments, R⁷ is selected from: 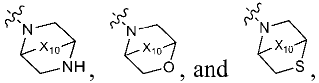 wherein X₁₀ is CH₂, (CH₂)₂, or (CH₂)₃.

In some embodiments, R⁴ is selected from: CN and 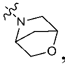 more preferably

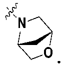

In some embodiments, the present invention provides a compound of formula (A), or a stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof, wherein: the IRAK4 ligand moiety is selected from: 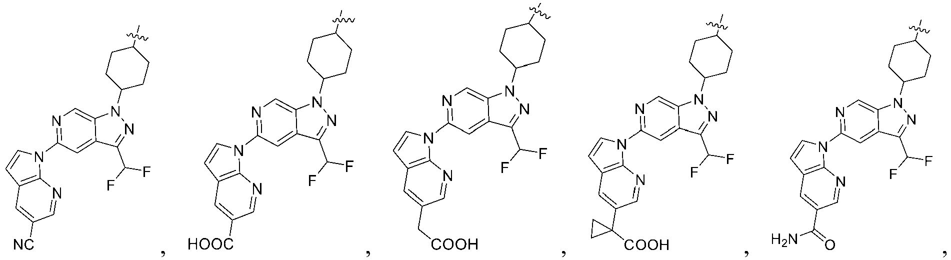 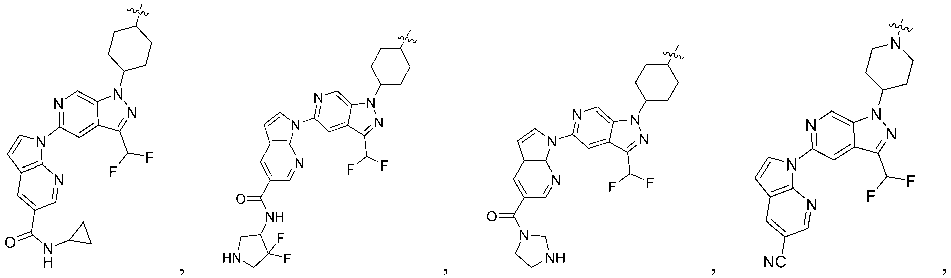 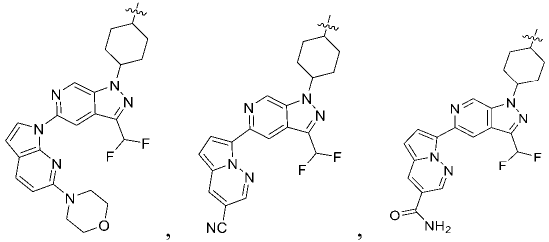 (preferably 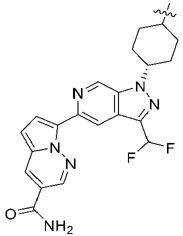 and 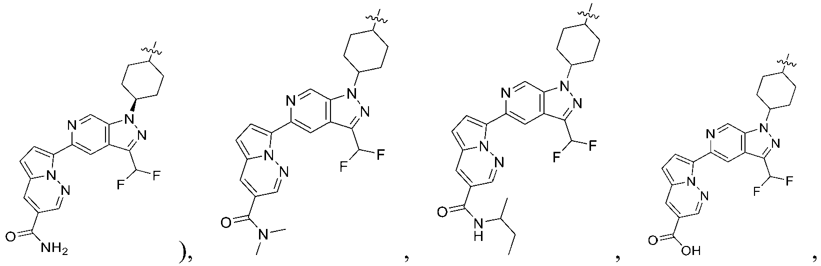 (preferably 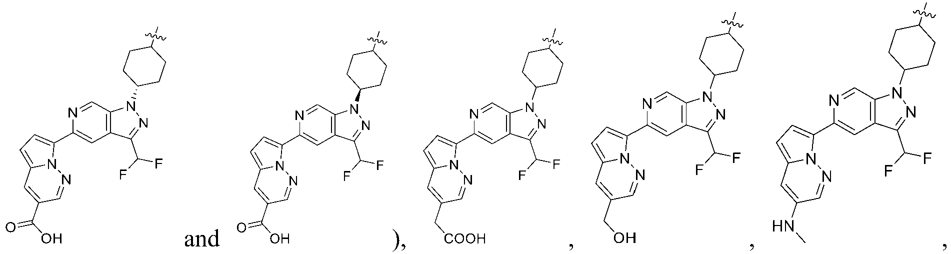 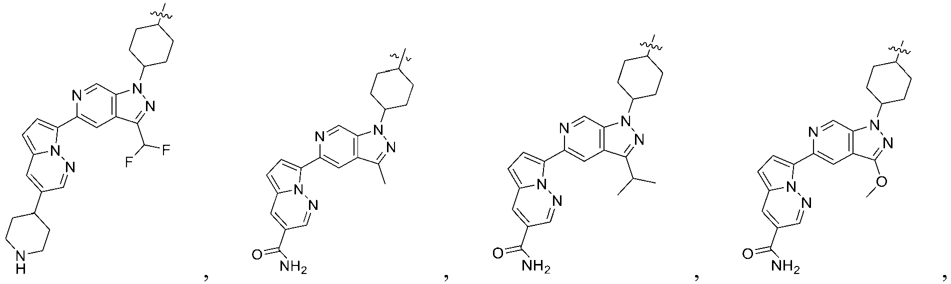 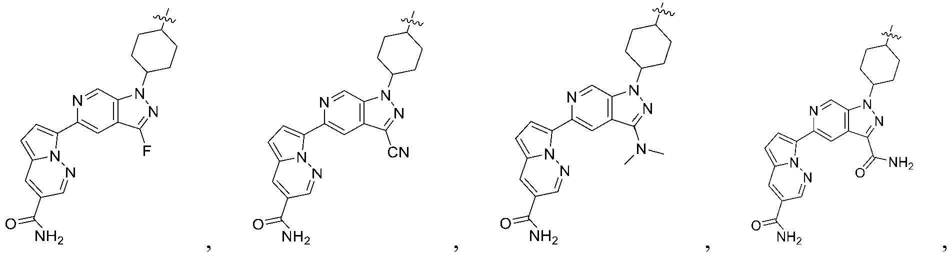 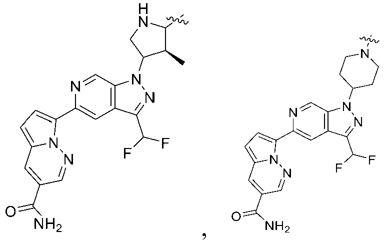 (preferably 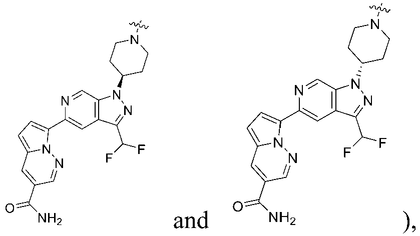 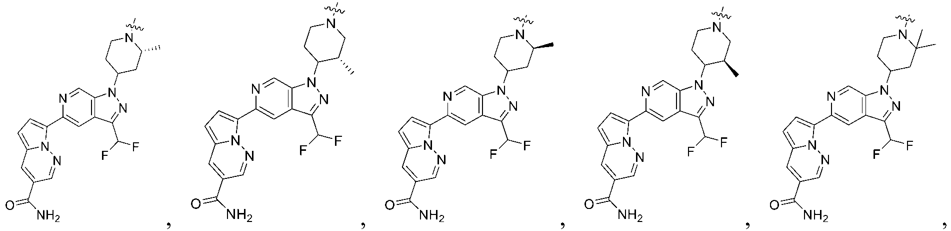 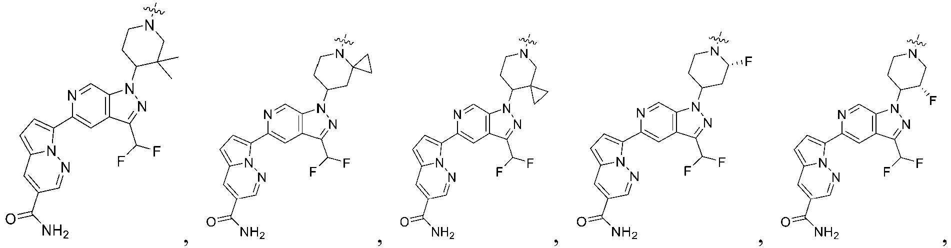 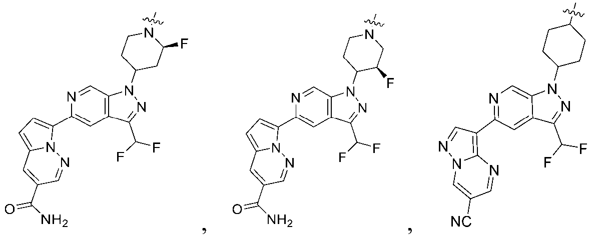 (preferably 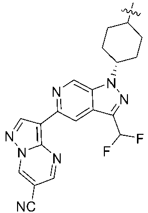 and 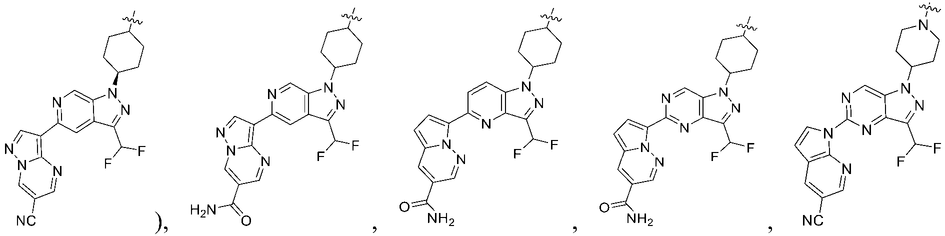 (preferably 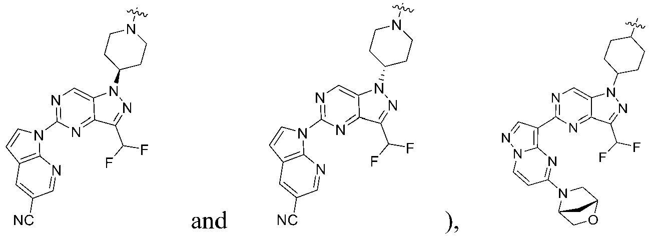 (preferably 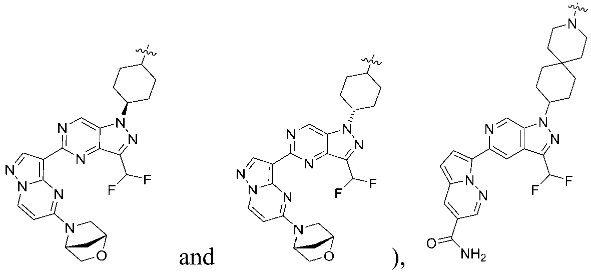 (preferably 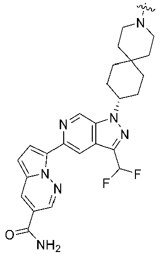 and 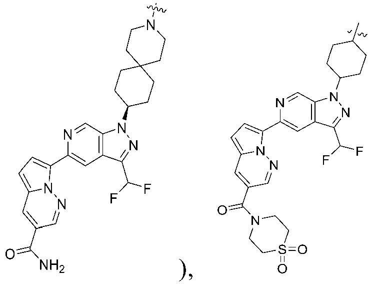 (preferably 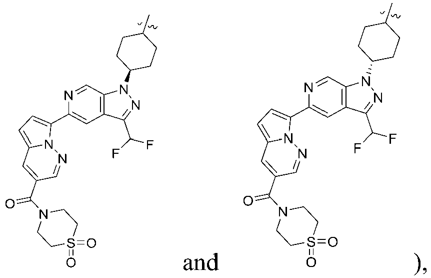 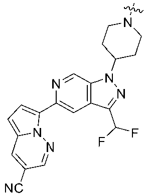 (preferably 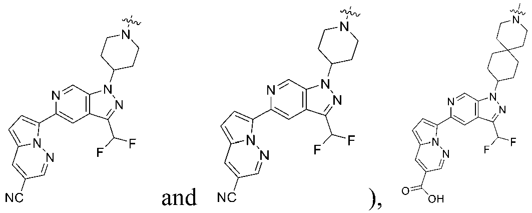 (preferably 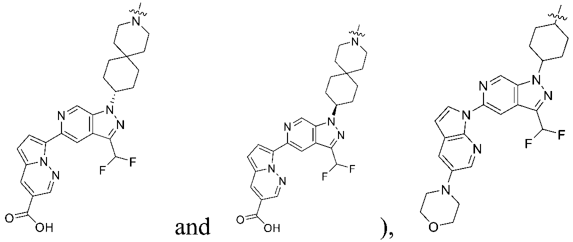 (preferably 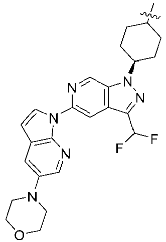 and 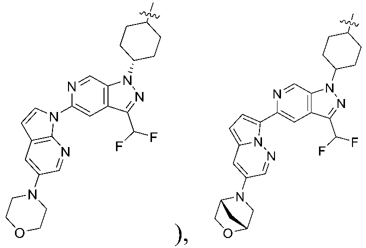 (preferably 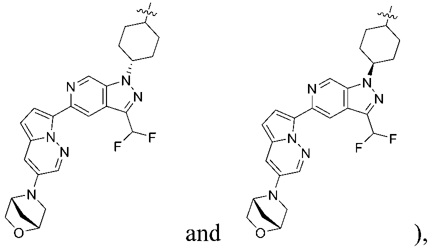 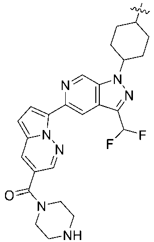 (preferably 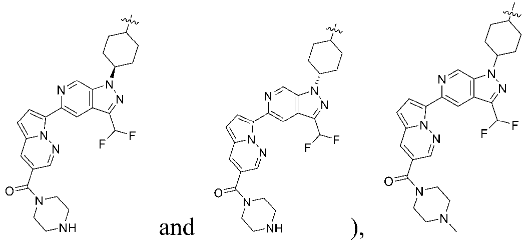 (preferably 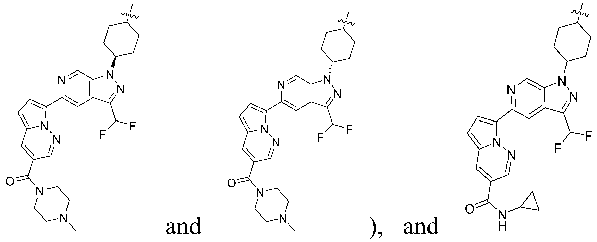 (preferably 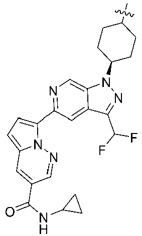 and

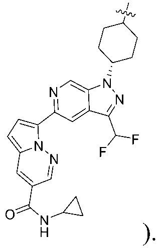

### Linking group L^{B}

In some embodiments, the present invention provides the compound of formula (A) according to the present invention, which has the L^{B} group as defined above.

In some embodiments, the CyL1 and CyL2 groups at each occurrence are each independently selected from C₄₋₁₁ cycloalkylene, 4-11-membered heterocycloalkylene, preferably C₄₋₇ monocyclic cycloalkylene, 4-7-membered monocyclic heterocycloalkylene, 6-10-membered fused bicyclic heterocycloalkylene, 6-9-membered bridged heterocycloalkylene, and 5-12-membered spiro heterocycloalkylene, more preferably C₄₋₆ monocyclic cycloalkylene, 4-6-membered monocyclic heterocycloalkylene, 8-10-membered fused bicyclic heterocycloalkylene, 6-8-membered bridged heterocycloalkylene, and 7-11-membered spiro heterocycloalkylene, further preferably, CyL1 at each occurrence is independently selected from C₅₋₆ monocyclic cycloalkylene, 4-6-membered monocyclic heterocycloalkylene, 8-10-membered fused bicyclic heterocycloalkylene, 7-8-membered bridged bicyclic heterocycloalkylene, 7-11-membered spiro bicyclic heterocycloalkylene, CyL2 at each occurrence is independently selected from 4-6-membered monocyclic heterocycloalkylene, wherein any of the above heterocycloalkylene preferably has 1, 2, or more nitrogen heteroatoms and 0, 1, or 2 heteroatoms selected from O and S.

In some embodiments, the CyL1 and CyL2 groups at each occurrence are each independently selected from 4-11-membered heterocycloalkylene, preferably 4-7-membered monocyclic heterocycloalkylene, 6-10-membered fused bicyclic heterocycloalkylene, 6-9-membered bridged heterocycloalkylene, and 5-12-membered spiro heterocycloalkylene, more preferably 4-6-membered monocyclic heterocycloalkylene, 8-10-membered fused bicyclic heterocycloalkylene, 6-8-membered bridged heterocycloalkylene, and 7-11-membered spiro heterocycloalkylene, wherein any of the above heterocycloalkylene preferably has 1, 2, or more nitrogen heteroatoms and 0, 1, or 2 heteroatoms selected from O and S.

In some embodiments, the CyL3 group at each occurrence is independently selected from C₄₋₁₁ cycloalkylene, preferably C₄₋₆ monocyclic cycloalkylene, C₆₋₁₀ fused bicyclic cycloalkylene, C₆₋₉ bridged cycloalkylene, and C₅₋₁₂ spiro cycloalkylene, more preferably C₅₋₆ monocyclic cycloalkylene, C₈₋₁₀ fused bicyclic cycloalkylene, C₆₋₈ bridged cycloalkylene, and C₇₋₁₁ spiro cycloalkylene.

In some embodiments, the CyL3 group at each occurrence is independently selected from C₅₋₆ monocyclic cycloalkylene, C₇₋₁₁ spiro bicyclic cycloalkylene.

In some embodiments, the CyL4 group at each occurrence is independently selected from 5-10-membered heteroarylene, preferably 5-6-membered heteroarylene, more preferably 5-6-membered nitrogen-containing heteroarylene.

In some embodiments, La at each occurrence is independently selected from C₁₋₄ alkylene, C₂₋₄ alkenylene, and C₂₋₄ alkynylene, preferably -CH₂-, -(CH₂)₂-, -(CH₂)₃-, -(CH₂)₄-, -CH=CH-, -CH₂-CH=CH-, -CH=CH-CH₂-, -C≡C-, -CH₂-C≡C-, -C≡C-CH₂-, -C≡C-CH₂CH₂-, -CH₂CH₂-C≡C-, and -CH₂-C≡C-CH₂-, more preferably -CH₂-, -(CH₂)₂-, -(CH₂)₃-, -C≡C-, -CH₂-C≡C-, - C≡C-CH₂-, -C≡C-CH₂CH₂-, and -CH₂CH₂-C≡C-.

In some embodiments, Lb at each occurrence is independently selected from -O-straight chain C₁₋₃ alkylene-, -straight chain C₁₋₃ alkylene-O-, -O-C₂₋₃ alkenylene-, -C₂₋₃ alkenylene-O-, -O-C₂₋₃ alkynylene-, -C₂₋₃ alkynylene-O-, -NR^{8'}-straight chain C₁₋₃ alkylene-, -straight chain C₁₋₃ alkylene-NR^{8'}-, -straight chain C₁₋₂ alkylene-NR^{8'}-straight chain C₁₋₂ alkylene-, -straight chain C₁₋₂ alkylene-C(O)-NR^{8'}-, -NR^{8'}-C(O)-straight chain C₁₋₂ alkylene-, -straight chain C₁₋₂ alkylene-NR^{8'}-C(O)-, -C(O)-straight chain C₁₋₃ alkylene-, and -straight chain C₁₋₃ alkylene-C(O)-, preferably -O-C₂₋₃ alkynylene-, -NR^{8'}-straight chain C₁₋₃ alkylene-, -straight chain C₁₋₃ alkylene-NR^{8'}-, -straight chain C₁₋₂ alkylene-NR^{8'}-straight chain C₁₋₂ alkylene-, -straight chain C₁₋₂ alkylene -C(O)-NR^{8'}-, -straight chain C₁₋₂ alkylene-NR^{8'}-C(O)-, and -C(O)-straight chain C₁₋₃ alkylene-, wherein R^{8'} at each occurrence is independently selected from H and C₁₋₄ alkyl.

In some embodiments, Lb at each occurrence is independently selected from -O-straight chain C₁₋₃ alkylene, -straight chain C₁₋₃ alkylene-O-, -O-C₂₋₃ alkenylene, -C₂₋₃ alkenylene-O-, - O-C₂₋₃ alkynylene, -C₂₋₃ alkynylene-O-, -NR^{8'}-straight chain C₁₋₃ alkylene-, -straight chain C₁₋₃ alkylene-NR^{8'}-, -straight chain C₁₋₂ alkylene-NR^{8'}-straight chain C₁₋₂ alkylene-, -straight chain C₁₋₂ alkylene-C(O)-NR^{8'}-, -NR^{8'}-C(O)-straight chain C₁₋₂ alkylene-, -C(O)-straight chain C₁₋₃ alkylene-, and -straight chain C₁₋₃ alkylene-C(O)-, wherein R^{8'} at each occurrence is independently selected from H and C₁₋₄ alkyl.

In some embodiments, Lc at each occurrence is independently selected from a bond or straight chain C₁₋₃ alkylene, preferably a bond, methylene, or ethylene, more preferably a bond or methylene.

In some embodiments, R^{L1}, R^{L2}, and R^{8'}at each occurrence are each independently selected from H, methyl, and ethyl, more preferably H and methyl.

In some embodiments, L^{B} is selected from groups (1)-(21):
(1) 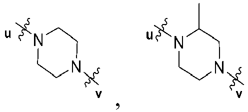 (preferably 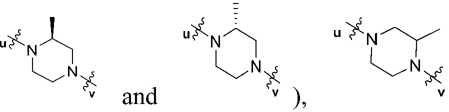 (preferably 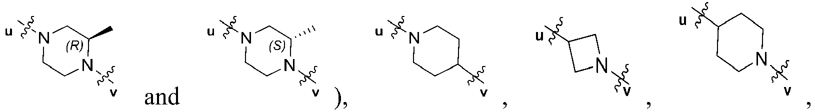 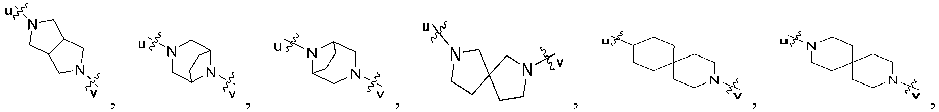 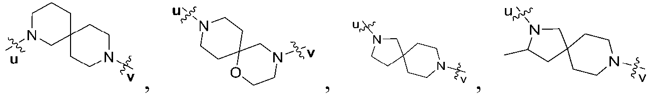 (preferably 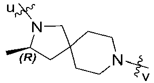 and 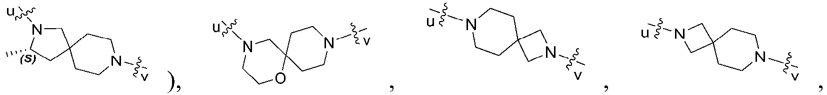 and 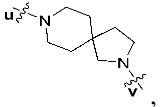
(2) 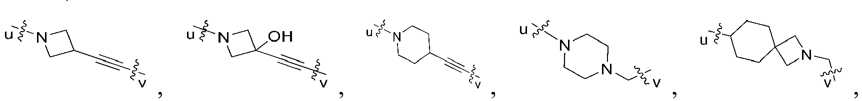 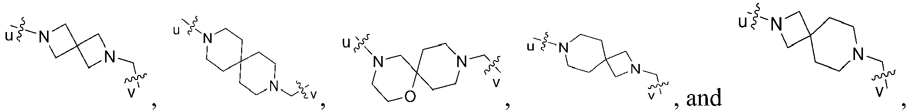
(3) 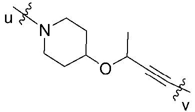 (preferably 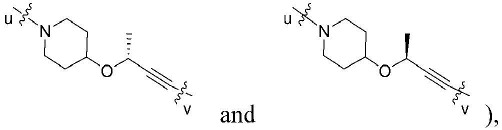 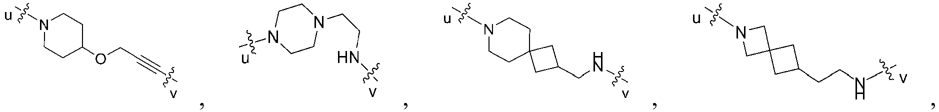 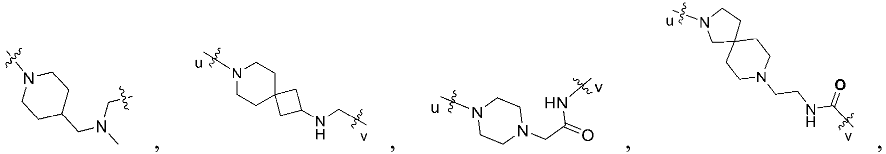 and 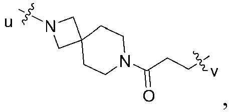
(4) 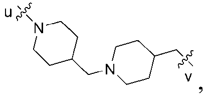
(5) 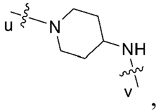
(6) 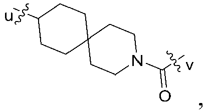
(7) 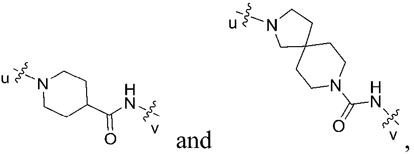
(8) 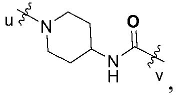
(9) 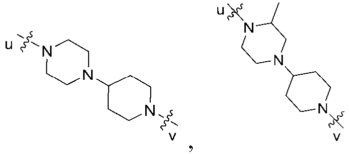 (preferably 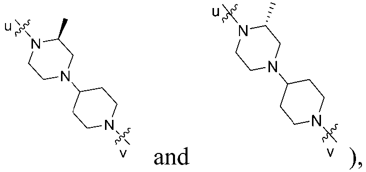 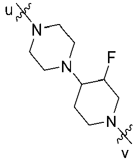 (preferably 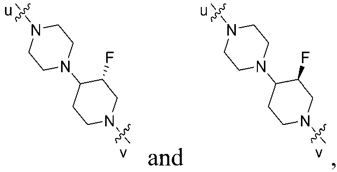 further preferably 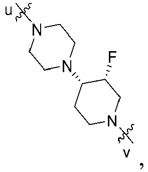 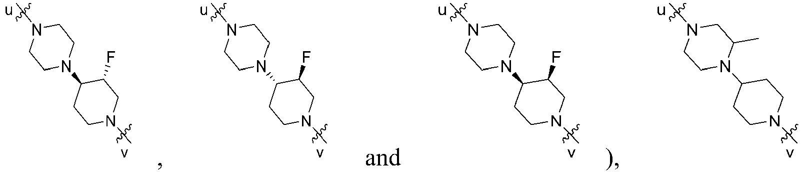 (preferably 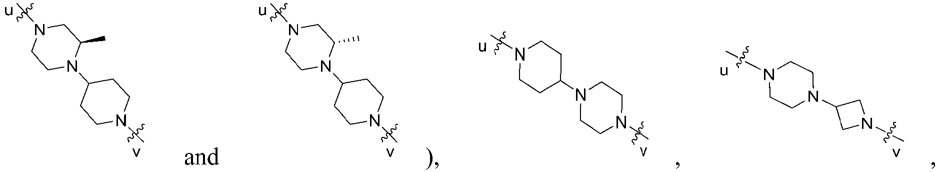 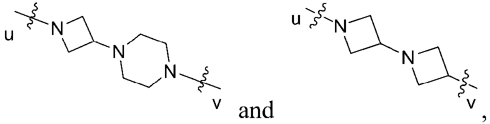
(10) 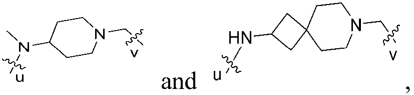
(11) 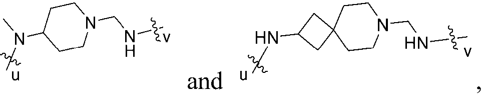
(12) 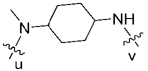 (preferably 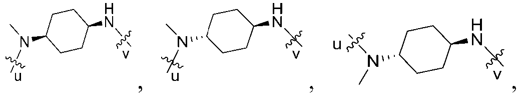 and 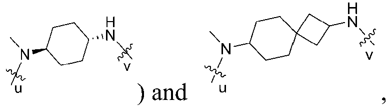
(13) 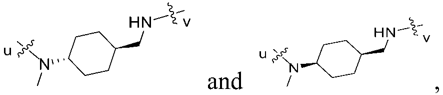
(14) 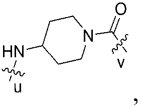
(15) 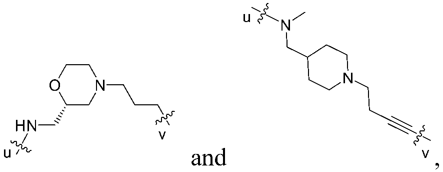
(16) 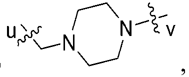
(17) 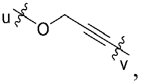
(18) 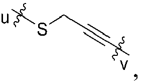
(19) 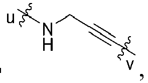
(20) 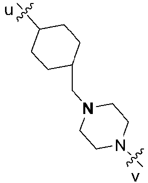 (preferably 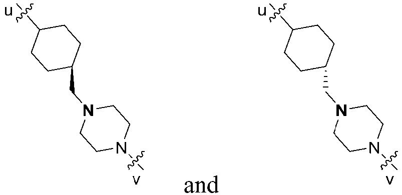 ) and 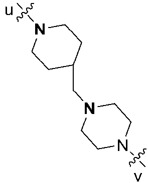 and
(21) 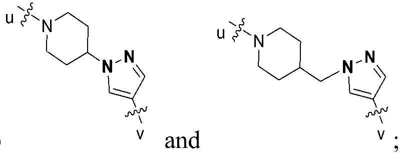 wherein preferably, in any group of the above groups (1)-(21), the bond marked with "u" is attached to the LA, and the bond marked with "v" is attached to the LBM moiety.

### Ligase binding moiety

In some embodiments, the present invention provides the compound of formula (A) according to the present invention, wherein the LBM moiety is an E3 ubiquitin ligase ligand.

In some embodiments, the LBM moiety is selected from: 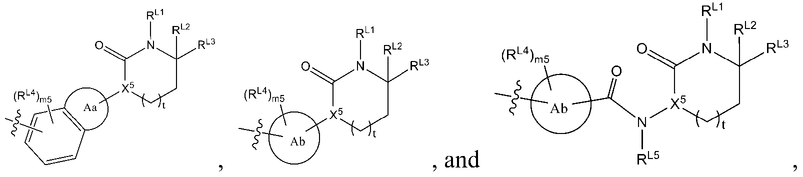 wherein:
ring Aa 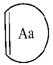 is 5-membered heterocyclyl or 5-membered heteroaryl, preferably 5-membered heterocyclyl or 5-membered heteroaryl having 1, 2, or more N heteroatoms, wherein the 5-membered heterocyclyl and 5-membered heteroaryl are optionally substituted by one or more substituents independently selected from H, halogen, OH, NH₂, CN, oxos and C₁₋₄ alkyl,
preferably, 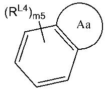 moiety is selected from 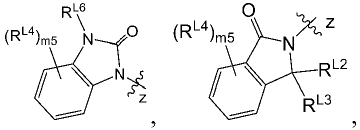 and 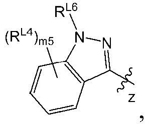 wherein the bond marked with "z" is attached to X⁵;
each ring 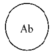 is independently phenyl or 5-6-membered heteroaryl, preferably phenyl;
is CR^{L7} or N;
t is 0 or 1, preferably 1;
R^{L1}, R^{L5}, and R^{L6} at each occurrence are each independently selected from H and C₁₋₄ alkyl, preferably H and methyl;
R^{L2} and R^{L3} at each occurrence are each independently selected from H and C₁₋₄ alkyl, preferably H and methyl; or R^{L2} and R^{L3} together form oxo;
R^{L4} and R^{L7} at each occurrence are each independently selected from H, halogen, OH, NH₂, CN, and C₁₋₄ alkyl, preferably H, F, Cl, Br, and C₁₋₂ alkyl, more preferably H, F, Cl, and methyl;
m5 is 0, 1, 2, 3, or 4, preferably 1 or 2.

In some embodiments, m5 is selected from 0 or 1.

In some embodiments, the LBM moiety is selected from 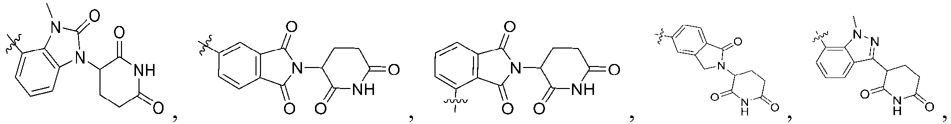 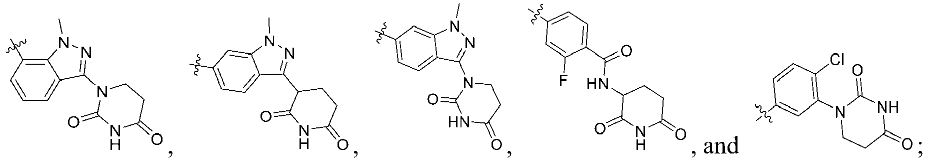 more preferably, the LBM moiety is selected from 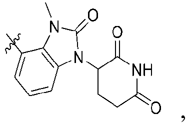 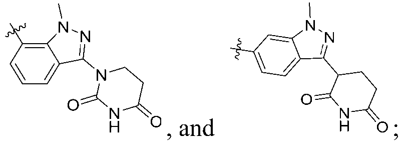 further preferably, the LBM moiety is selected from

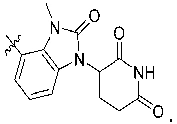

In some embodiments, wherein 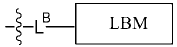 moiety is selected from:

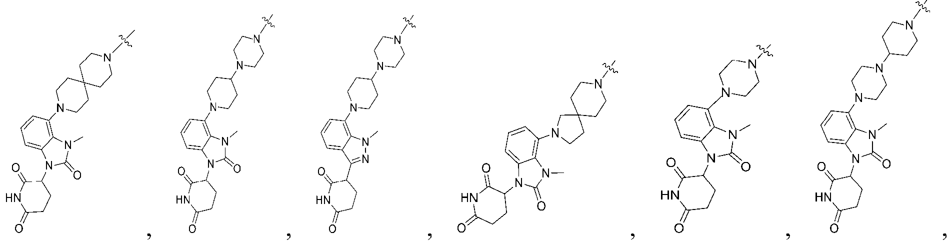

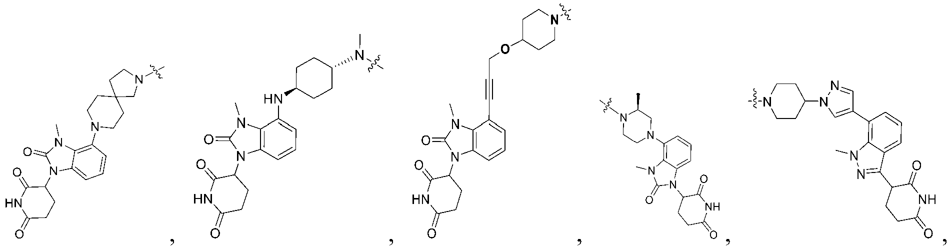

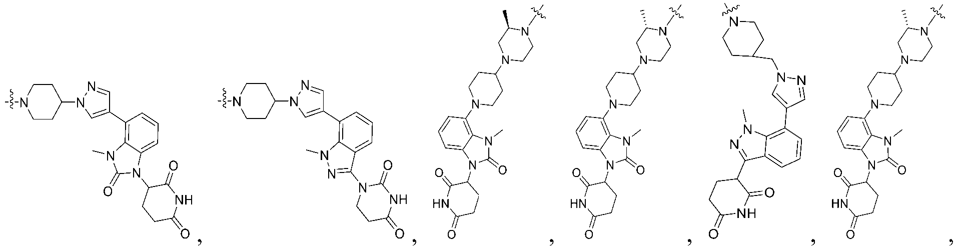

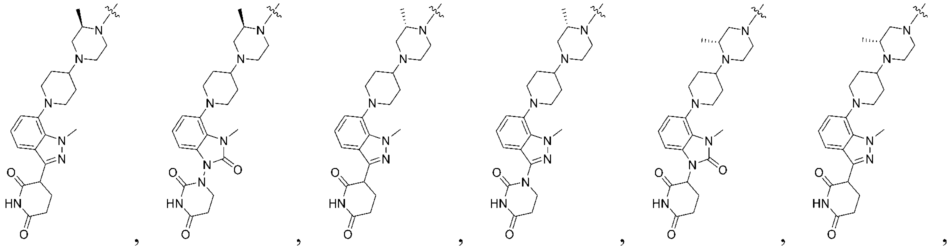

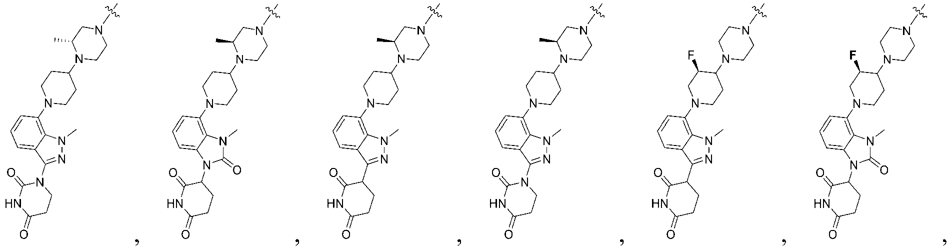

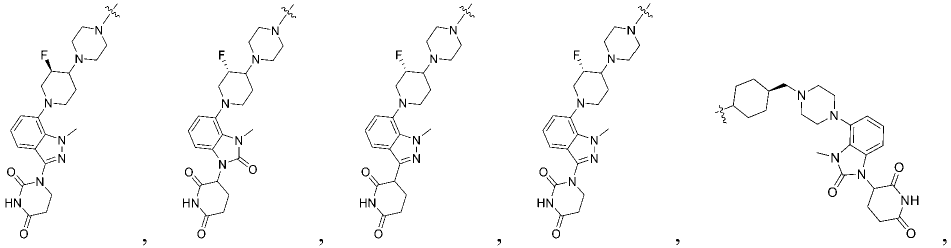

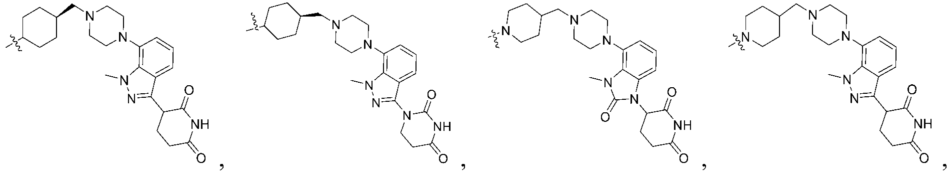

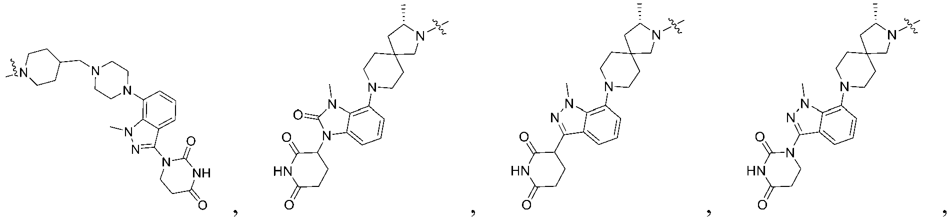

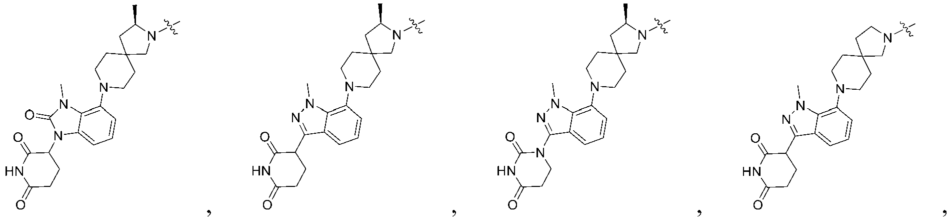

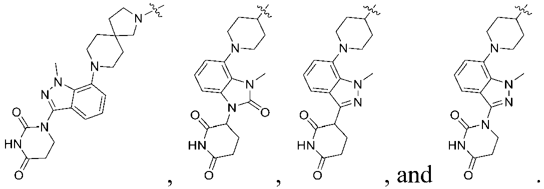

In some embodiments, wherein 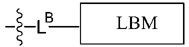 moiety is selected from: 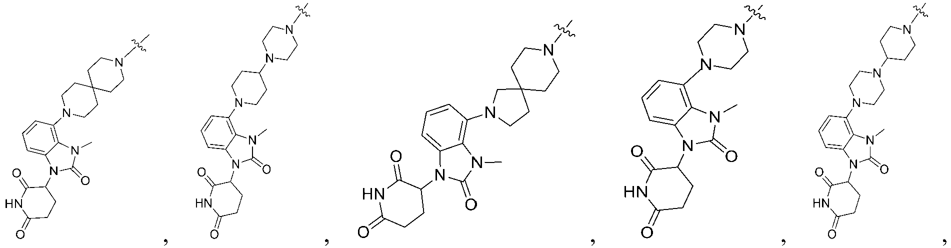 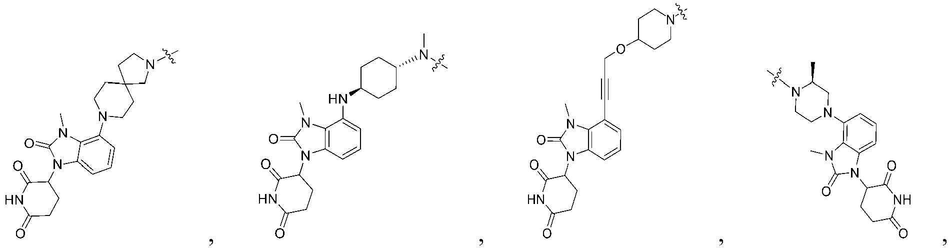 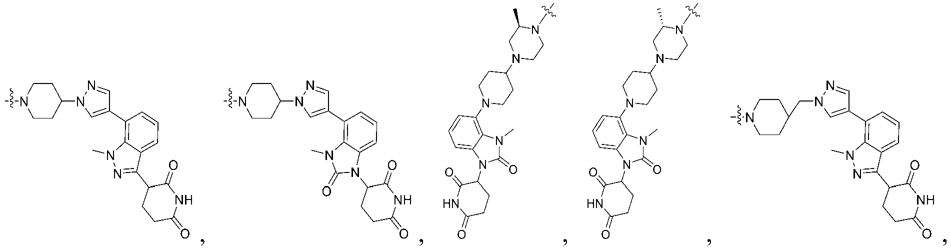 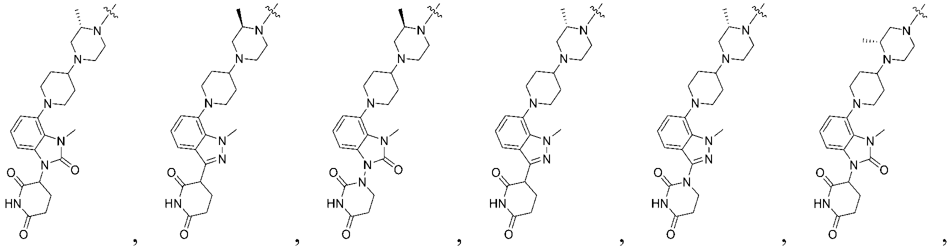 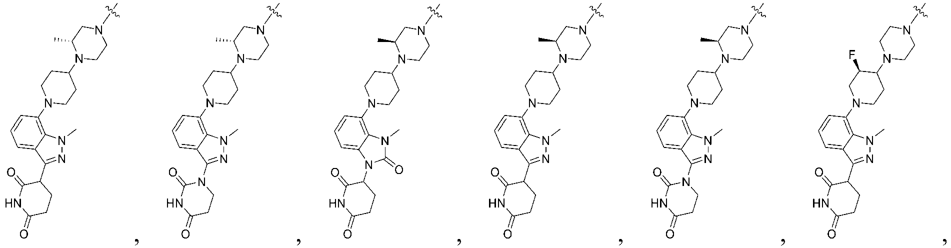 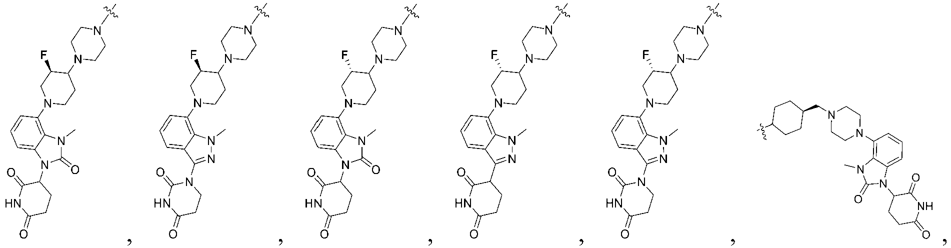 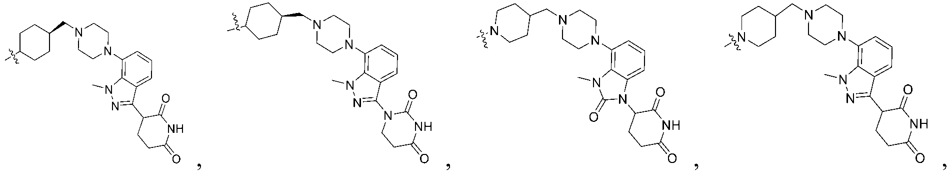 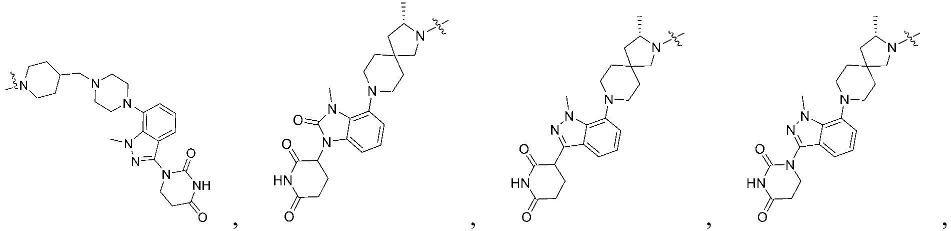 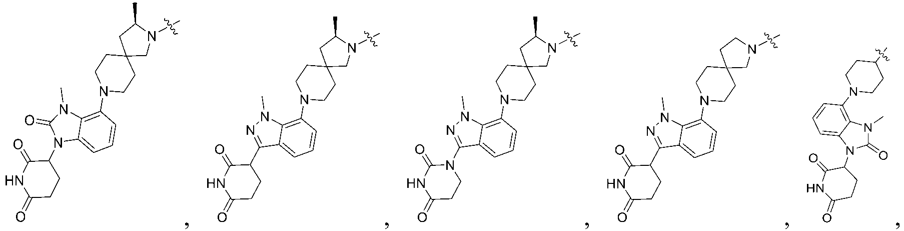 and

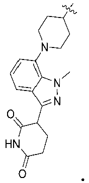

In some preferred embodiments, the compound of the present invention has the structure represented by formula (B0): 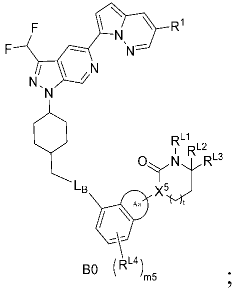
wherein, R¹, L_{B}, Aa, R^{L1} R^{L2}, R^{L3}, R^{L4}, X⁵, t, m5 are as defined in any of the preceding embodiments;
preferably, R¹ is selected from CN, R⁷, -C(O)OR^{1f} or -C(O)NR^{1d}R^{1e}; more preferably, R¹ is selected from -C(O)NR^{1d}R^{1e};
wherein:
   preferably, R⁷ is selected from: 4-7-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
   R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, morpholinyl, thiomorpholinyl, and 2-oxa-5-azabicyclo[2.2.1]heptane, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
   R⁷ is selected from piperidinyl, morpholinyl, and 2-oxa-5-azabicyclo[2.2.1]heptane; and/or
   preferably, R^{1f} is selected from hydrogen and C₁₋₄ alkyl, preferably hydrogen; and/or
   preferably, R^{1d} and R^{1e} are each independently selected from hydrogen, C₁₋₆ alkyl, and R¹¹; or R^{1d} and R^{1e} together with the nitrogen atoms that they are commonly attached to, form R¹⁰;
   wherein:
      preferably, R¹¹ is selected from: C₃₋₆ cycloalkyl; wherein the cycloalkyl is optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
      R¹¹ is selected from: cyclopropyl, cyclobutyl, cyclopentyl, cyclohexyl, each of which is optionally substituted by one or more substituents independently selected from F, Cl, Br, CN, OH, NO₂, and NH₂; and/or
      R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, oxo, C₁₋₄ alkyl, C₁₋₄ haloalkyl, C₁₋₄ alkoxy, - NH₂, -NH(C₁₋₄ alkyl), and -N(C₁₋₄ alkyl)₂; or
      R¹⁰ is selected from: piperidinyl, piperazinyl, morpholinyl, and thiomorpholinyl, each of which is optionally substituted by one or more substituents independently selected from F, Cl, Br, CN, OH, oxo, C₁₋₄ alkyl, C₁₋₄ haloalkyl, C₁₋₄ alkoxy, -NH₂, -NH(C₁₋₄ alkyl), and -N(C₁₋₄ alkyl)₂;
      preferably, R¹ is selected from CN, 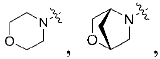 -C(O)OH, -C(O)NH₂, 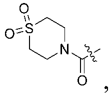 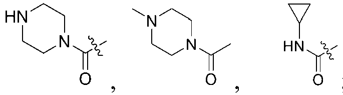 ; more preferably, R¹ is selected from -C(O)NH₂; and/or
      L_{B} is selected from:
         (1) -CyL1-, wherein the CyL1 here is selected from 4-7-membered monocyclic heterocycloalkylene, 7-11-membered spiro bicyclic heterocycloalkylene; preferably 5-6-membered monocyclic heterocycloalkylene, 9-11-membered spiro bicyclic heterocycloalkylene; wherein any of the heterocycloalkylenes each has 1 or 2, preferably 2 nitrogen heteroatoms, and any of the heterocycloalkylenes is optionally substituted by one or more groups independently selected from the following: C₁₋₄ alkyl and halogen, preferably methyl, F, and Cl, more preferably methyl and F;
         (3) -CyL1-Lb-, wherein the CyL1 here is selected from 4-7-membered monocyclic heterocycloalkylene; preferably 5-6-membered monocyclic heterocycloalkylene; Lb group is selected from -O-C₂₋₃ alkynylene;
         (9) -CyL1-CyL2-, wherein the CyL1 and CyL2 groups here are each independently selected from 4-7-membered monocyclic heterocycloalkylene, more preferably 5-6-membered monocyclic heterocycloalkylene, wherein any of the heterocycloalkylenes each has 1 or 2 nitrogen heteroatoms, and is optionally substituted by one or more groups independently selected from the following: C₁₋₄ alkyl and halogen, preferably methyl, F, and Cl, more preferably methyl and F;
         (12) -NR^{L1}-CyL3-NR^{L2}-, wherein the CyL3 group here is selected from C₅₋₆ monocyclic cycloalkylene, R^{L1}, R^{L2} groups are each independently selected from H and C₁₋₄ alkyl, preferably H and methyl;
         (20) -CyL1-La-CyL2-, wherein the CyL1 and CyL2 groups here are each independently selected from 4-7-membered monocyclic heterocycloalkylene or C₄₋₇ monocyclic cycloalkylene; preferably 5-6-membered monocyclic heterocycloalkylene or C₅₋₆ monocyclic cycloalkylene; La is independently selected from C₁₋₄ alkylene, preferably methylene and ethylene;
         (21) -CyL1-Lc-CyL4-, wherein the CyL1 group here is selected from 4-7-membered heterocycloalkylene, preferably 5-6-membered heterocycloalkylene; the CyL4 here is selected from 5-6-membered monocyclic heteroarylene, preferably 5-membered monocyclic nitrogen-containing heteroarylene, the Lc group here is selected from a bond or C₁₋₄ alkylene, preferably a bond, methylene, or ethylene; or
         L_{B} is selected from: 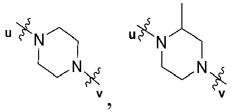 (preferably 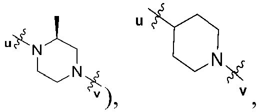 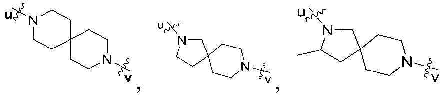 (preferably 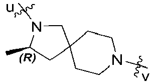 and 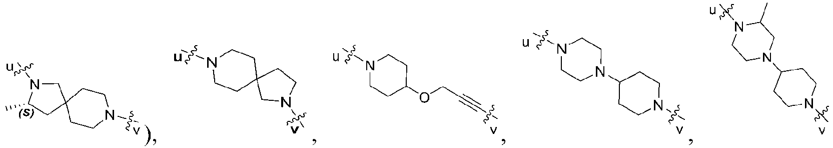 (preferably 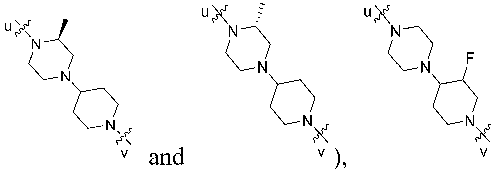 (preferably 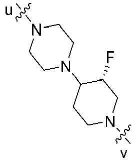 and 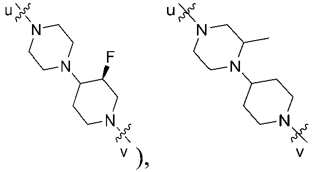 (preferably 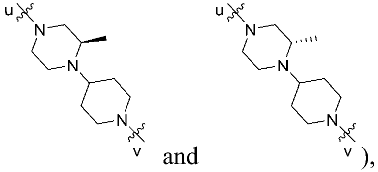 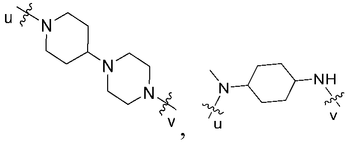 (preferably 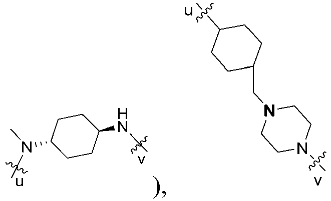 (preferably 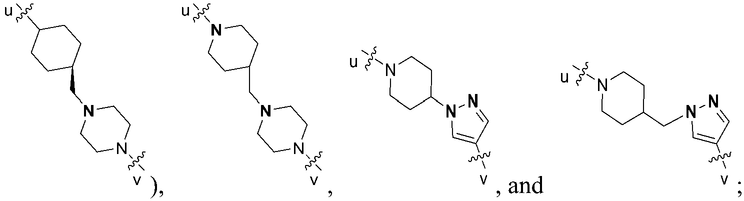 wherein in any of the above groups, the bond marked with "u" is attached to CH₂ moiety, and the bond marked with "v" is attached to the moiety containing phenyl; and/or
         preferably, 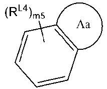 moiety is selected from 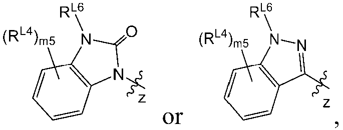 wherein the bond marked with "z" is attached to
         wherein:
            preferably, R^{L4} is selected from H, halogen, and C₁₋₄ alkyl, more preferably H, F, Cl and methyl; and/or
            preferably, R^{L6} is selected from H and C₁₋₄ alkyl, more preferably H and methyl; and/or
            preferably, m5 is selected from 0 or 1, more preferably 0; and/or
            is CR^{L7} or N;
            wherein: R^{L7} is selected from H, halogen, and C₁₋₄ alkyl, more preferably H; and/or
            t is preferably 1; and/or
            preferably, R^{L1} is selected from H and C₁₋₄ alkyl, more preferably H and methyl; and/or
            preferably, R^{L2} and R^{L3} at each occurrence are each independently selected from H and C₁₋₄ alkyl, preferably H and methyl; or R^{L2} and R^{L3} together form oxo; more preferably R^{L2} and R^{L3} together form oxo;
            preferably, the formula (B0) is selected from the structure represented by formula (B0-1), (B0-2), or (B0-3):

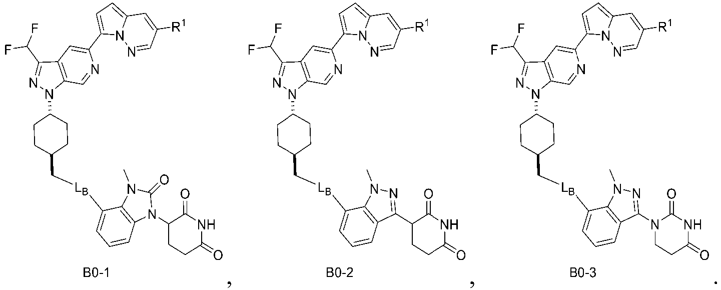

In some preferred embodiments, the present invention provides the compound of formula (A), wherein the compound has the structure represented by formula (B): 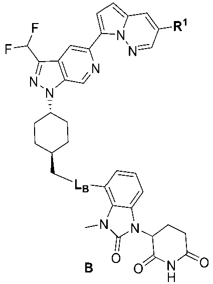 wherein:
L^{B} is selected from:
   (1) -CyL1-, wherein the CyL1 group here is selected from 5-9-membered heterocycloalkylene, 7-11-membered spiro heterocycloalkylene, preferably 5-6-membered heterocycloalkylene, 9-11-membered spiro heterocycloalkylene; more preferably 9-11-membered spiro heterocycloalkylene; wherein the 7-11-membered spiro heterocycloalkylene and 9-11-membered spiro heterocycloalkylene each have 1 or 2, preferably 2 nitrogen heteroatoms, and the 7-11-membered spiro heterocycloalkylene and 9-11-membered spiro heterocycloalkylene are optionally substituted by one or more groups independently selected from the following: C₁₋₄ alkyl and halogen, preferably methyl, F, and Cl, more preferably methyl and F; wherein the 5-9-membered heterocycloalkylene and 5-6-membered heterocycloalkylene each have 1 or 2, preferably 2 nitrogen heteroatoms, and the 5-9-membered heterocycloalkylene and 5-6-membered heterocycloalkylene are optionally substituted by 1 or more groups independently selected from the following: C₁₋₄ alkyl and halogen, preferably methyl, F, and Cl, more preferably methyl and F;
   (2) -CyL1-Lb-, wherein the CyL1 group here is selected from 5-9-membered heterocycloalkylene, more preferably 5-6-membered heterocycloalkylene; Lb group is selected from -O-C₂₋₃ alkynylene;
   (3) -CyL1-CyL2-, wherein the CyL1 and CyL2 groups here are each independently selected from 4-7-membered monocyclic heterocycloalkylene, more preferably 4-6-membered monocyclic heterocycloalkylene, wherein the 4-7-membered monocyclic heterocycloalkylene and 4-6-membered monocyclic heterocycloalkylene each have 1 or 2 nitrogen heteroatoms, and are optionally substituted by one or more groups independently selected from the following: C₁₋₄ alkyl and halogen, preferably methyl, F, and Cl, more preferably methyl and F;
   (4) -NR^{L1}-CyL3-NR^{L2}-, wherein the CyL3 group here is selected from C₄₋₆ monocyclic cycloalkylene, R^{L1}, R^{L2} groups are each independently selected from H, methyl, and ethyl, preferably H and methyl;
   (5) -CyL1-La-CyL2, wherein the CyL1 and CyL2 groups here are each independently selected from 4-7-membered monocyclic heterocycloalkylene or C₄₋₇ monocyclic cycloalkylene; La is independently selected from C₁₋₄ alkylene, preferably methylene and ethylene;
   (6) -CyL1-Lc-CyL4-, wherein the CyL1 group here is selected from C₄₋₆ monocyclic heterocycloalkylene, preferably CyL1 group is selected from 5-6-membered monocyclic heterocycloalkylene; the CyL4 group here is selected from 5-6-membered monocyclic heteroarylene, preferably 5-membered monocyclic nitrogen-containing heteroarylene, the Lc group here is selected from a bond or C₁₋₄ hydrocarbylene, preferably a bond, methylene, or ethylene;
and, R¹ is selected from 3-10-membered saturated or partially unsaturated heterocyclyl having 1-3 heteroatoms independently selected from nitrogen, oxygen, and sulfur, CN, - C(O)R^{1f}, -C(O)OR^{1f}, -C(O)NR^{1d}R^{1e};
R^{1d}, R^{1e} are each independently selected from hydrogen, deuterium, R¹¹, C₁₋₆ alkyl, and C₁₋₆ haloalkyl; preferably hydrogen, R¹¹, C₁₋₃ alkyl, and C₁₋₃ haloalkyl;
R^{1f} is independently selected from hydrogen, deuterium, C₁₋₆ alkyl, C₁₋₆ haloalkyl, and R¹²; preferably hydrogen, C₁₋₃ alkyl, C₁₋₃ haloalkyl, and R¹²;
R¹¹, R¹² are independently selected from: saturated or partially unsaturated C₃₋₇ cyclohydrocarbyl; 3-10-membered saturated or partially unsaturated heterocyclyl having 1-3 heteroatoms independently selected from nitrogen, oxygen, and sulfur; wherein the cyclohydrocarbyl and heterocyclyl are optionally substituted by one or more substituents independently selected from C₁₋₆ alkyl, C₁₋₆ haloalkyl; preferably saturated C₃₋₇ cyclohydrocarbyl; 3-10-membered saturated heterocyclyl having 1-3 heteroatoms independently selected from nitrogen, oxygen, and sulfur; preferably the cyclohydrocarbyl and heterocyclyl are optionally substituted by one or more substituents independently selected from C₁₋₃ alkyl, C₁₋₃ haloalkyl;
R¹ is preferably selected from -CONH₂, -CN, -COOH, 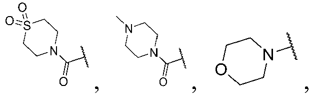 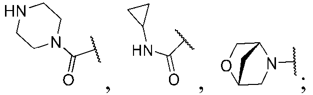 more preferably R¹ is preferably selected from -CONH₂.

In some embodiments, in the structure represented by the formula (B), wherein:
L^{B} is selected from:
(1) -CyL1-, wherein the CyL1 group here is selected from 5-9-membered monocyclic heterocycloalkylene, 7-11-membered spiro heterocycloalkylene; preferably 5-6-membered monocyclic heterocycloalkylene, 9-11-membered spiro heterocycloalkylene; more preferably 9-11-membered spiro heterocycloalkylene; wherein the 7-11-membered spiro heterocycloalkylene and 9-11-membered spiro heterocycloalkylene each have 1 or 2, preferably 2 nitrogen heteroatoms, and the 7-11-membered spiro heterocycloalkylene and 9-11-membered spiro heterocycloalkylene are optionally substituted by one or more groups independently selected from the following: C₁₋₄ alkyl and halogen, preferably methyl, F, and Cl, more preferably methyl and F; wherein the 5-9-membered monocyclic heterocycloalkylene and 5-6-membered monocyclic heterocycloalkylene each have 1 or 2, preferably 2 nitrogen heteroatoms, and the 5-9-membered monocyclic heterocycloalkylene and 5-6-membered monocyclic heterocycloalkylene are optionally substituted by one or more groups independently selected from the following: C₁₋₄ alkyl and halogen, preferably methyl, F, and Cl, more preferably methyl and F;
(2) -CyL1-Lb-, wherein the CyL1 group here is selected from 5-9-membered monocyclic heterocycloalkylene, more preferably 5-6-membered monocyclic heterocycloalkylene; Lb group is selected from -O-C₂₋₃ alkynylene;
(3) -CyL1-CyL2-, wherein the CyL1 and CyL2 groups here are each independently selected from 4-7-membered monocyclic heterocycloalkylene, more preferably 4-6-membered monocyclic heterocycloalkylene, wherein the 4-7-membered monocyclic heterocycloalkylene and 4-6-membered monocyclic heterocycloalkylene each have 1 or 2 nitrogen heteroatoms, and are optionally substituted by one or more groups independently selected from the following: C₁₋₄ alkyl and halogen, preferably methyl, F, and Cl, more preferably methyl and F;
(4) -NR^{L1}-CyL3-NR^{L2}-, wherein the CyL3 group here is selected from C₄₋₆ monocyclic cycloalkylene, R^{L1}, R^{L2} groups are each independently selected from H, methyl, and ethyl, preferably H and methyl;
(5) -CyL1-La-CyL2-, wherein the CyL1 and CyL2 groups here are each independently selected from 4-7-membered monocyclic heterocycloalkylene or C₄₋₇ monocyclic cycloalkylene; La is independently selected from C₁₋₄ alkylene, preferably methylene and ethylene;
(6) -CyL1-Lc-CyL4-, wherein the CyL1 group here is selected from 4-6-membered monocyclic heterocycloalkylene, preferably CyL1 group is selected from 5-6-membered monocyclic heterocycloalkylene; the CyL4 group here is selected from 5-6-membered monocyclic heteroarylene, preferably 5-membered monocyclic nitrogen-containing heteroarylene, the Lc group here is selected from a bond or C₁₋₄ hydrocarbylene, preferably a bond, methylene, or ethylene.

In some embodiments, L^{B} is selected from: 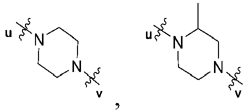 (preferably 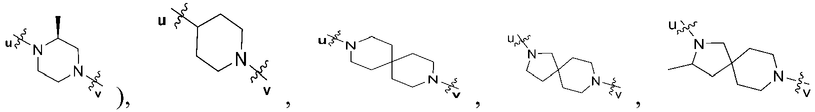 (preferably 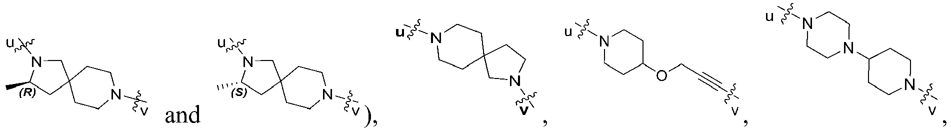 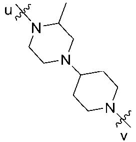 (preferably 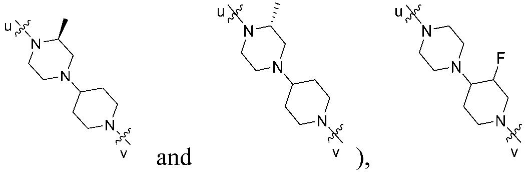 (preferably 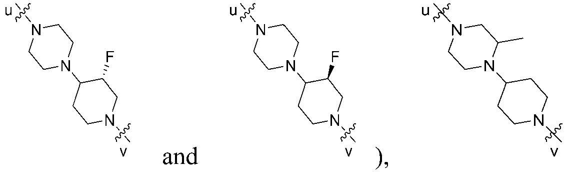 (preferably 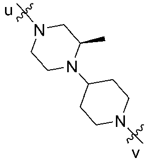 and 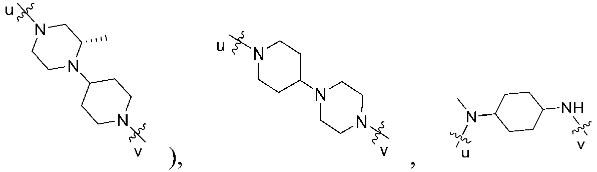 (preferably 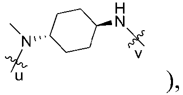 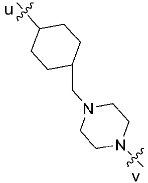 preferably 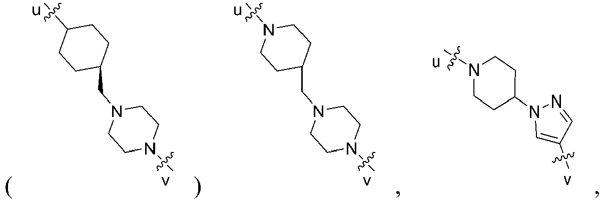 and 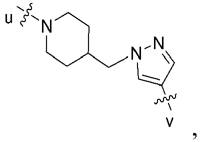 more preferably 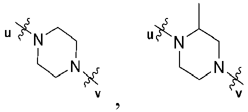 (preferably 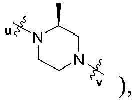 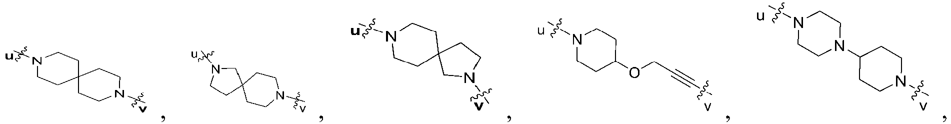 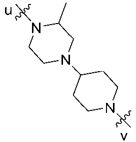 (preferably 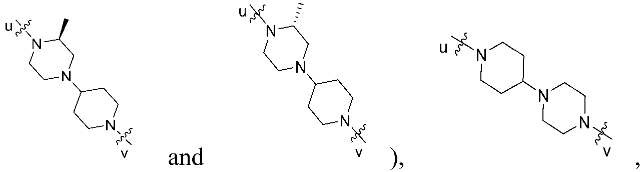 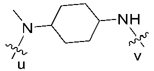 (preferably 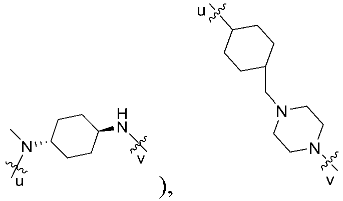 preferably 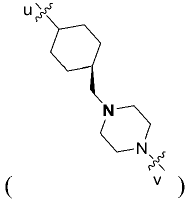 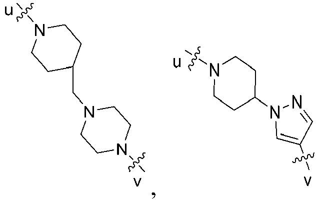 and , wherein in any of the above groups, the bond marked with "u" is attached to CH₂ moiety, and the bond marked with "v" is attached to the moiety containing phenyl.

In some embodiments, R¹ is selected from -CONR_{Na}R_{Nb}, wherein R_{Na}, R_{Nb} are each independently H or C₁₋₄ alkyl. -CONH₂ is preferable.

The present invention covers the compounds obtained by any combination of the various embodiments.

In some embodiments, the present invention provides the compound of formula (A), or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof, wherein the compound is selected from table 1 below:

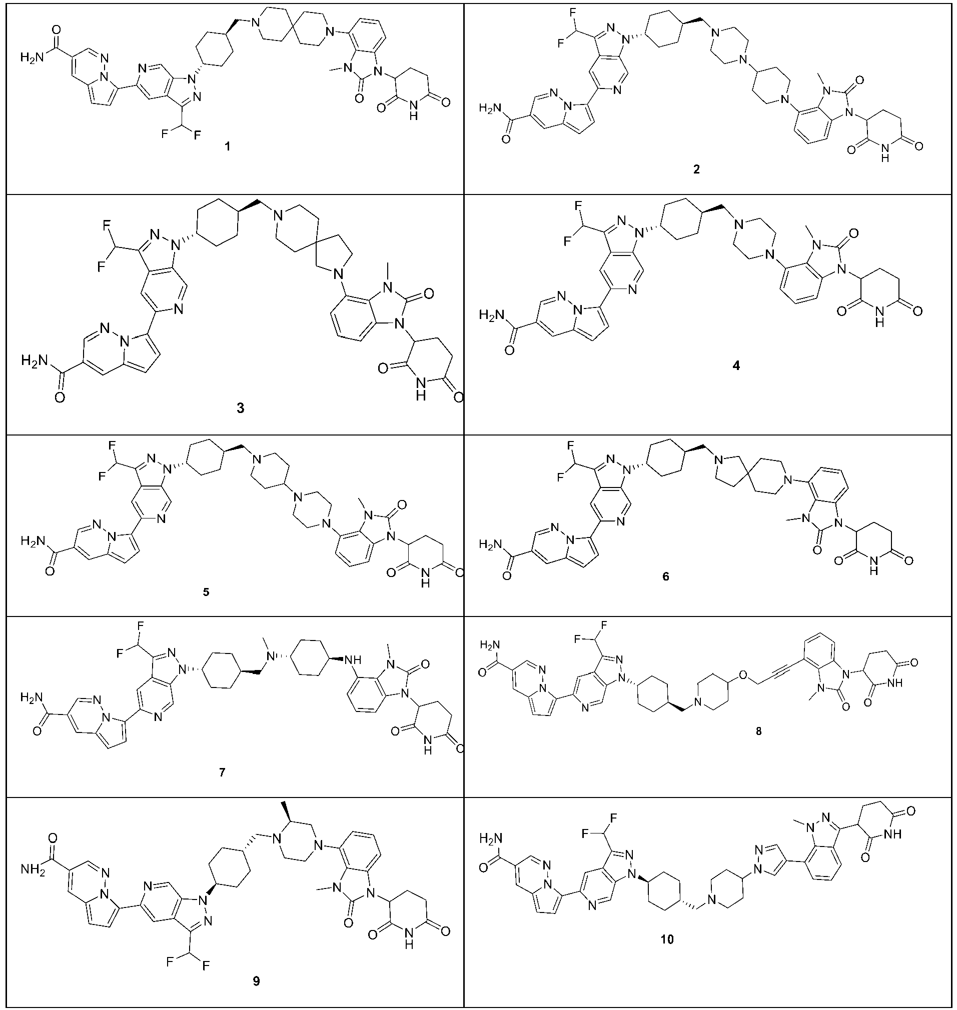

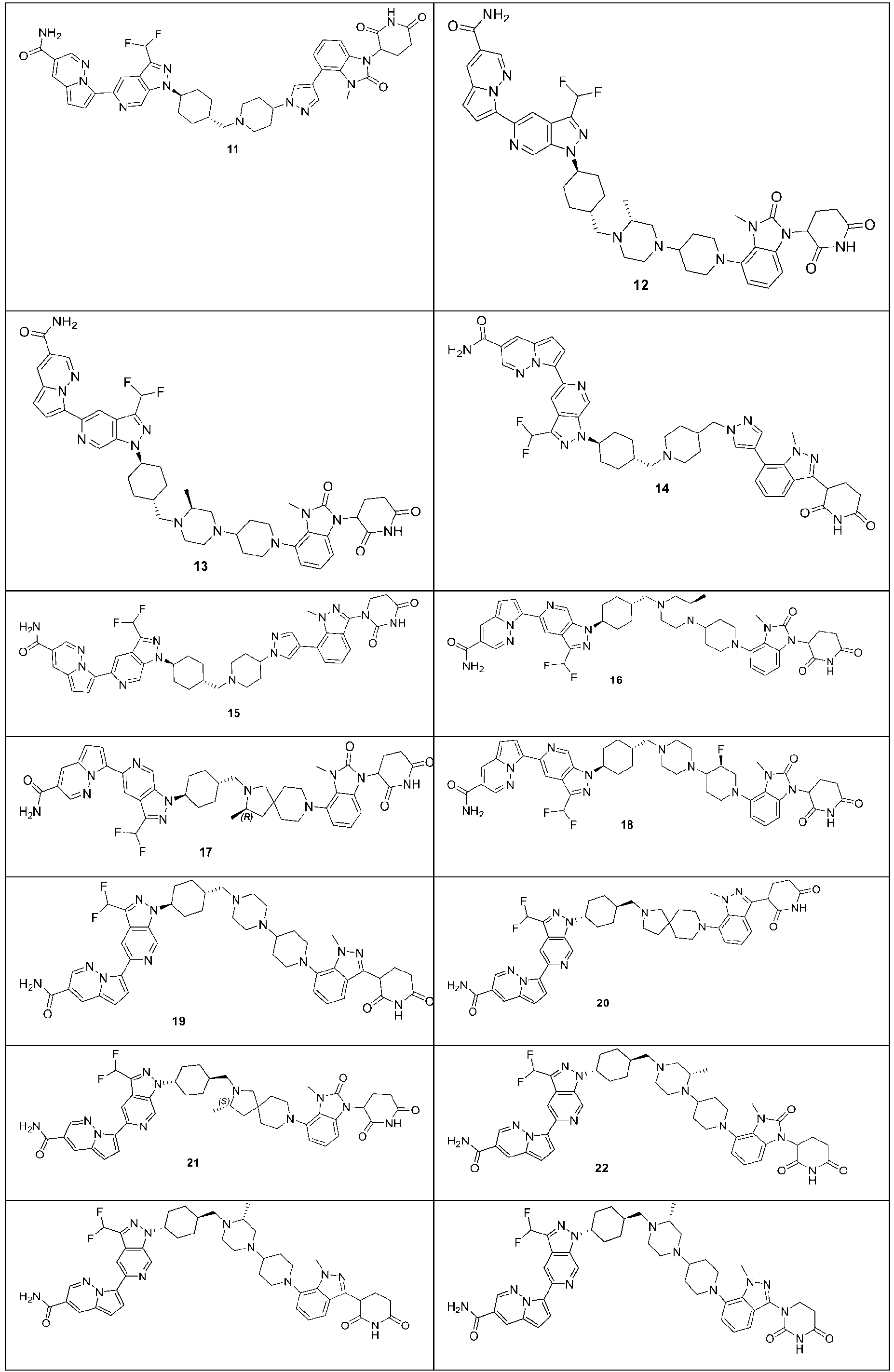

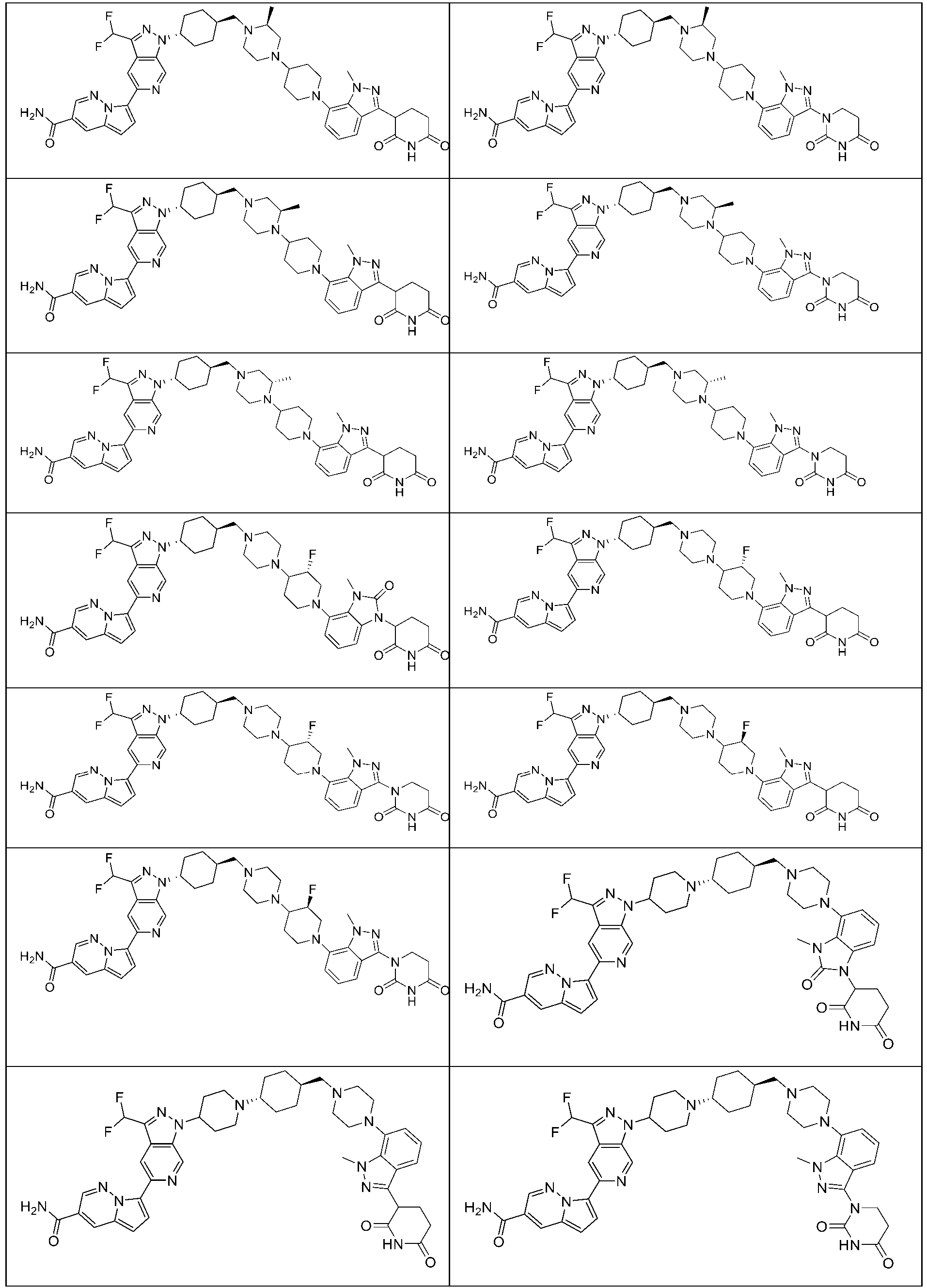

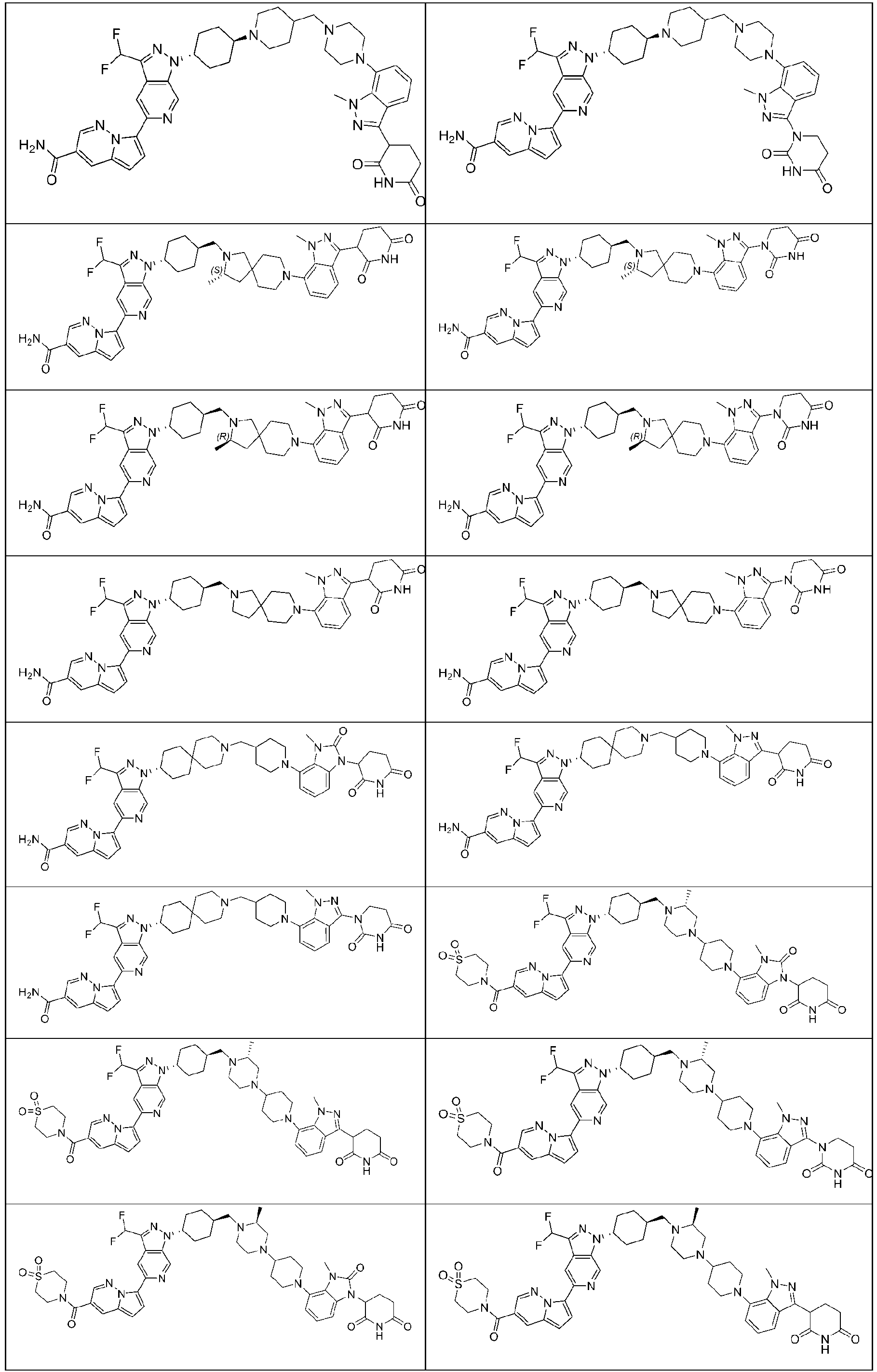

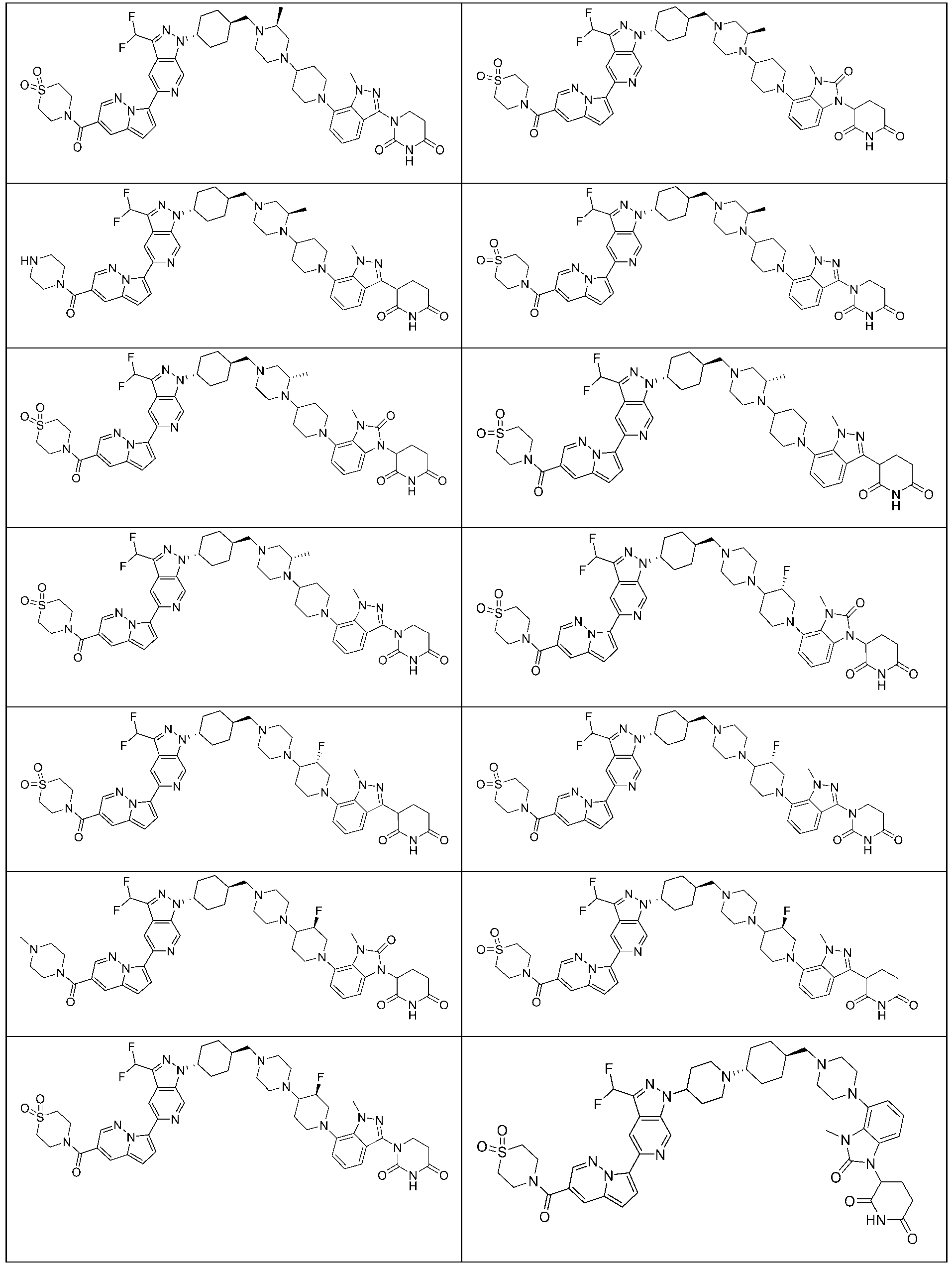

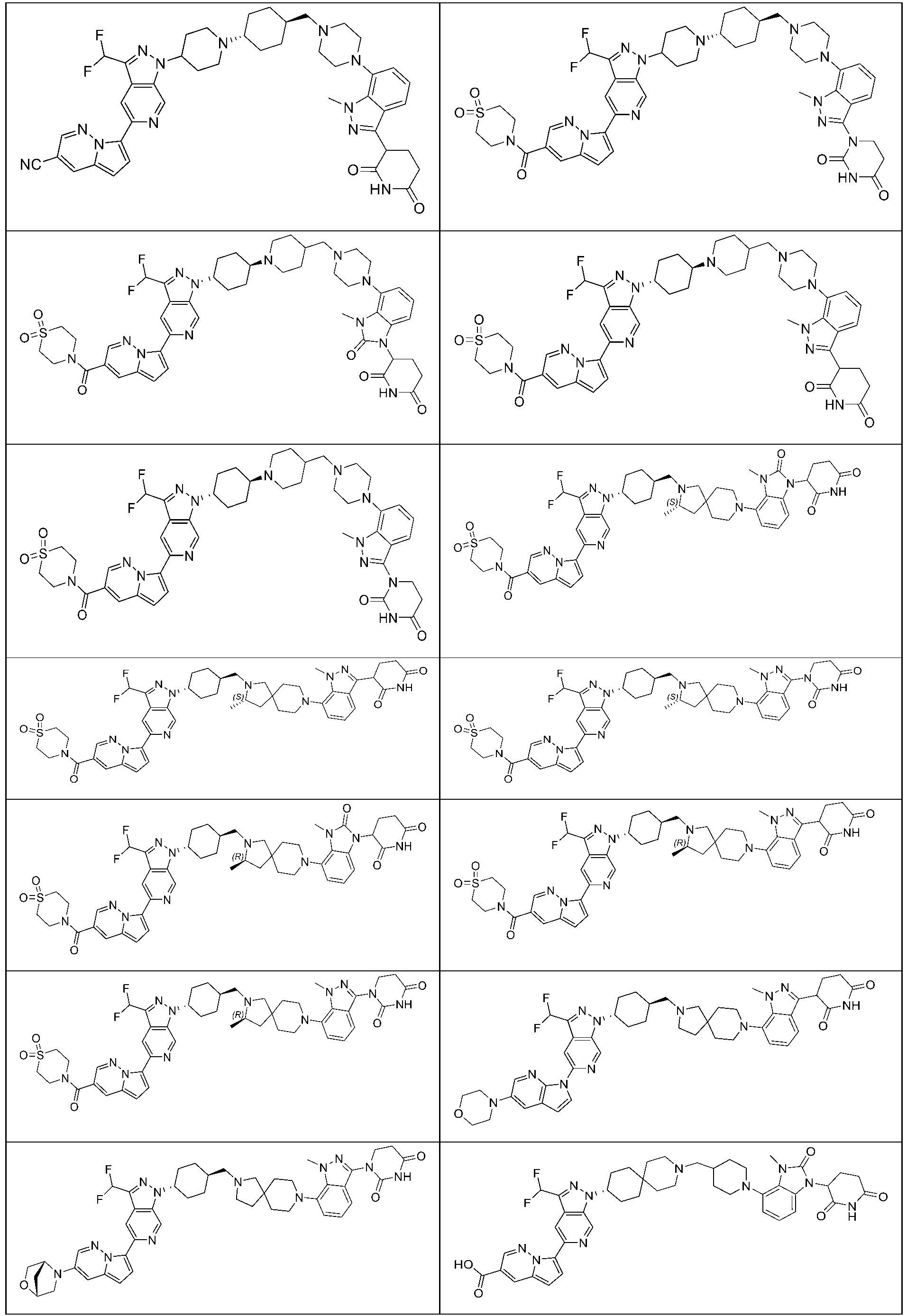

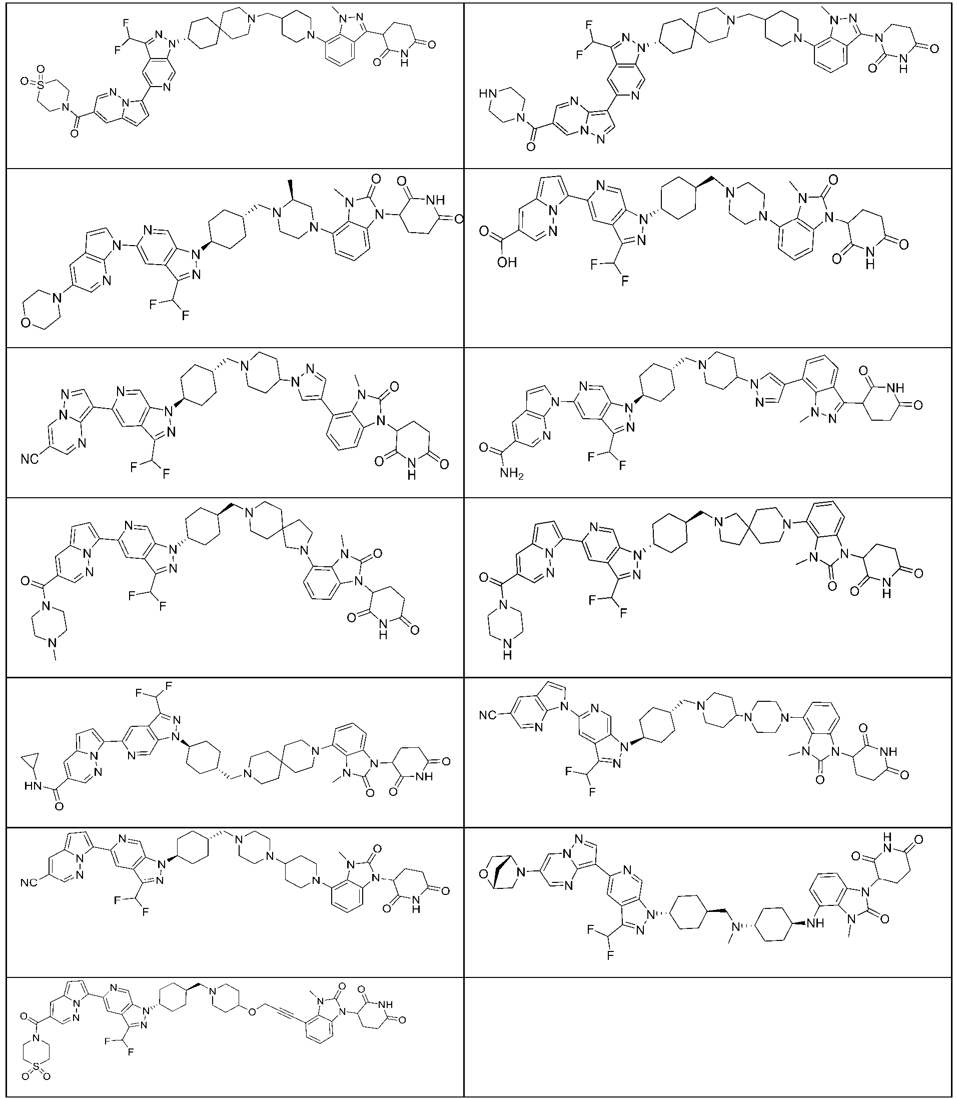

In some preferred embodiments, the compound is selected from compound 1 to compound 22.

### Pharmaceutical compositions and use

In another aspect, the present invention provides a pharmaceutical composition, comprising a therapeutically effective amount of the compound of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate, or pharmaceutically acceptable salt thereof , and one or more pharmaceutically acceptable carriers. The pharmaceutical composition is preferably a solid formulation, a liquid formulation, or a transdermal formulation.

In another aspect, the present invention provides use of the compound of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate, or pharmaceutically acceptable salt thereof, or the pharmaceutical composition of the present invention in the preparation of a medicament.

In some embodiments, the compound of the present invention, the pharmaceutical composition of the present invention, or the medicament is used for treating a disease, disorder, or condition related to an IRAK4 protein kinase.

In another aspect, the present invention also provides a method for treating, alleviating symptoms of, delaying the progression or onset of a disease, disorder, or condition related to an IRAK4 protein kinase, comprising administering to an individual in need thereof an effective amount of the compound of the present invention or stereoisomer, tautomer, diastereomer, racemate, cis/trans isomer, isotope-labeled compound (preferably deuterated compound), N-oxide, metabolite, ester, prodrug, crystal form, hydrate, solvate or pharmaceutically acceptable salt thereof , or the pharmaceutical composition of the present invention.

In yet another aspect, the present invention provides the compound of the present invention or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof, or the pharmaceutical composition of the present invention, for use in treating a disease, disorder, or condition related to an IRAK4 protein kinase.

In another aspect, the present invention also provides use of the compound of the present invention or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof, or the pharmaceutical composition of the present invention in the preparation of a medicament as an IRAK4 inhibitor.

In yet another aspect, the present invention provides a method of inhibiting IRAK4 activity in an individual, comprising administering to an individual in need thereof an effective amount of the compound of the present invention or stereoisomer, tautomer, diastereomer, racemate, cis/trans isomer, isotope-labeled compound (preferably deuterated compound) , N-oxide, metabolite, ester, prodrug, crystal form, hydrate, solvate, or pharmaceutically acceptable salt thereof , or the pharmaceutical composition of the present invention.

In another aspect, the present invention provides a method for targeted degradation of IRAK4 protein kinase, comprising contacting the IRAK4 protein kinase with the compound of formula (A) according to the present invention as described above or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof in the presence of an E3 ubiquitin ligase. In some embodiments, the method is performed in vitro or ex vivo. In some other embodiments, the method is performed in vivo.

In some embodiments, the disease, disorder, or condition related to an IRAK4 protein kinase is selected from: autoimmune condition, inflammatory condition, cancer, graft rejection, thromboembolism, atherosclerosis, myocardial infarction, and metabolic syndrome.

In some embodiments, the inflammatory condition is selected from: osteoarthritis, gout, gouty arthritis, chronic obstructive pulmonary disease, periodic fever, atopic dermatitis, hidradenitis suppurativa, chronic nephritis, allergic eczema, lymphadenectasis, pyemia, irritable bowel syndrome (IBD), ulcerative colitis, asthma, and allergies, preferably osteoarthritis, chronic obstructive pulmonary disease, atopic dermatitis, hidradenitis suppurativa, and chronic nephritis.

In some embodiments, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis, preferably psoriasis.

In some embodiments, the graft rejection is selected from graft versus host disease and allogeneic graft rejection.

In some embodiments, the cancer is selected from: brain cancer, kidney cancer, liver cancer, stomach cancer, vaginal cancer, ovarian cancer, gastric tumor, breast cancer, bladder and colon cancer, prostate cancer, pancreatic cancer, lung cancer, cervical cancer, testicular cancer, skin cancer, bone cancer, thyroid cancer, sarcoma, glioblastoma, neuroblastoma, multiple myeloma, gastrointestinal cancer, neck and head tumors, adenoma, adenocarcinoma, keratoacanthoma, epidermoid carcinoma, large cell carcinoma, non-small cell lung cancer, Hodgkin and Non-Hodgkin lymphoma, breast cancer, follicular carcinoma, papillary carcinoma, seminoma, melanoma, acute myelogenous leukemia, chronic myelogenous leukemia, diffuse large B-cell lymphoma, activated B-cell-like diffuse large B-cell lymphoma, chronic lymphocytic leukemia, chronic lymphocytic lymphoma, primary effusion lymphoma, Burkitt's lymphoma/leukemia, acute lymphoblastic leukemia, B-cell prolymphocytic leukemia, lymphoplasmacytic lymphoma, Waldenstrom macroglobulinemia, splenic marginal zone lymphoma, intravascular large B-cell lymphoma, plasmacytoma, and multiple myeloma.

In the present invention, "pharmaceutically acceptable carrier" refers to a diluent, adjuvant, excipient or vehicle administered together with a therapeutic agent, which is suitable, to the extent of a reasonable medical judgment, for contacting the tissues of humans and/or other animals without excessive toxicity, irritation, allergic reactions, or other problems or complications commensurate with a reasonable benefit/risk ratio.

Unless otherwise stated, as used herein, the term "treatment" means reversing, alleviating, inhibiting the disorder or condition to which such term applies or the progression of one or more symptoms of such disorder or condition, or preventing such disorder or condition or one or more symptoms of such disorder or condition.

As used herein, "individuals" include human beings or non-human animals. Exemplary human individuals include human individuals suffering from a disease (for example the diseases described herein) (referred to as patients) or normal individuals. In the present invention, "non-human animals" include all vertebrates, for example non-mammals (e.g., birds, amphibians, reptiles) and mammals, for example non-human primates, domestic animals and/or domesticated animals (e.g., sheep, dogs, cats, cows, pigs, etc.).

In another embodiment, the pharmaceutical composition of the present invention may further comprise one or more additional therapeutic agents or prophylactic agents.

### Examples

The embodiments of the present invention will be described in detail below with reference to examples. However, those skilled in the art will understand that the following examples are only used to illustrate the present invention and should not be regarded as limiting the scope of the present invention. If a particular condition is not specified in the examples, it is carried out according to a conventional condition or the condition suggested by the manufacturer. The used reagents or instruments whose manufacturers are not specified are all commercially available conventional products.

NMR was determined using a Bruker Avance III 400 nuclear magnetic instrument, and the chemical shift (δ) is given in unit of 10⁻⁶ (ppm). The solvent was deuterated methanol (CD₃OD), deuterated chloroform (CDCl₃), or hexadeuterated dimethyl sulfoxide (DMSO-d6), etc., with tetramethylsilane (TMS) as the internal reference.

MS was determined using an Agilent (ESI) mass spectrometer (Agilent 1260, Agilent 6125B).

High performance liquid chromatography (HPLC) analytical conditions: Gilson highpressure liquid chromatograph (Gilson GX-281), C18 column (10 µM, 19 mm × 250 mm), ultraviolet detection wavelengths of 220 and 254 nm, the elution conditions being gradient elution of 5-95% acetonitrile (containing 0.05% v/v formic acid or ammonium bicarbonate) for 15 minutes.

Reversed-phase purification was performed using Biotage Isolera rapid purificatcion system.

Separation and purification by thin layer chromatography were performed on a thin layer chromatography silica gel plate (an aluminium plate from Meck (20 cm × 20 cm × 1 mm) or GF 254 from Yantai).

Biotage Initiator + (400 W, RT - 300°C) microwave reactor was used for a microwave reaction.

The reaction was routinely monitored by TLC or LCMS, common developer system including: dichloromethane/methanol, n-hexane/ethyl acetate, petroleum ether/ethyl acetate, and the volume ratio of the solvents was adjusted according to the different polarities of the compounds, or with additives such as triethylamine as needed.

The silica gel used in column chromatography is generally 100-200 mesh silica gel. Commonly used eluant system included: dichloromethane/methanol, petroleum ether/ethyl acetate, and the volume ratio of the solvents was adjusted according to the different polarities of the compounds, and a small amount of triethylamine was also added as needed.

The reagents and solvents and the like of the present invention were purchased from Aldrich Chemical Company, Energy, J&K Scientific, Shanghai Bide Pharmaceutical Technology Co., Ltd., PharmaBlock, and Shanghai Titan Technology Co., Ltd., among others.

### Synthesis examples

### Example 1

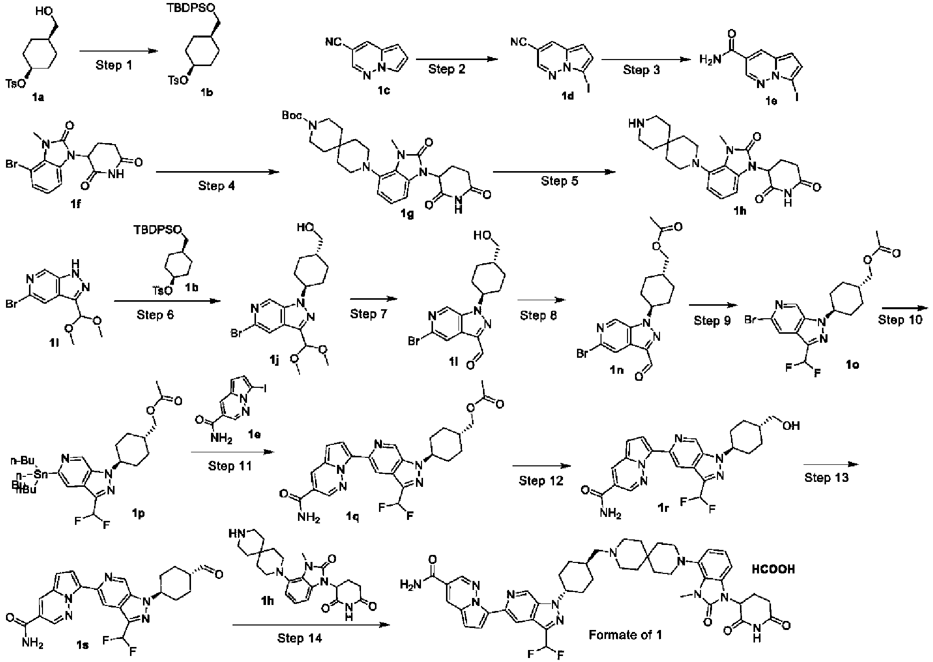

1) Step 1: Compound 1a (14.2 g, 49.9 mmol, prepared using the method disclosed for intermediate ARD in paragraph 001207 on page 586 of the specification of patent application "WO2020264499A1") was dissolved in dichloromethane (150 mL). Imidazole (6.80 g, 99.9 mmol), and tert-butyldiphenylsilyl chloride (16.5 g, 59.9 mmol) were added. After reacting under stirring at 20°C for 2 hours, water (300 mL) was added to the reaction mixture, followed by extraction with ethyl acetate (100 mL×3). The organic phases were combined, dried, filtered, and concentrated under reduced pressure. The crude product and petroleum ether (200 mL) were stirred, and filtered. The solid was collected and dried under vacuum to afford compound 1b. ¹H NMR (400 MHz, DMSO-*d₆*) δ 7.78 (d, *J* = 8.1 Hz, 2H), 7.61 - 7.58 (m, 4H),7.47 - 7.43 (m, 8H), 4.71 (s, 1H), 3.47 (d, *J* = 6.0 Hz, 2H), 2.42 (s, 3H), 1.75 - 1.65 (m, 2H), 1.60 - 1.44 (m, 5H), 1.32 - 1.20 (m, 2H), 1.00 (s, 9H).
2) Step 2: At room temperature, compound 1c (300 mg, 2.10 mmol, prepared using the method disclosed for intermediate pyrrolo[1,2-b]pyndazine-3- carbonitrile on page 75 of the specification of patent application "WO2015117563A1"), and *N*-iodosuccinimide (707 mg, 3.14 mmol) were added to acetonitrile (10 mL). The reaction solution was stirred at 60°C for 1 hour. The reaction mixture was cooled to room temperature. Saturated sodium sulfite solution (10 mL) was carefully added to the reaction solution. Then, the mixture was extracted with ethyl acetate (100 mL). The organic phases were washed with saturated saline (100 mL× 2), dried over anhydrous sodium sulfate, filtered and concentrated under reduced pressure. The residue was subjected to flash chromatographic column (petroleum ether/ethyl acetate=10/1) to afford compound 1d. ¹H NMR (400 MHz, CDCl₃): δ 8.27 (d, *J* = 2.1 Hz, 1H), 8.05 (d, *J* = 2.1 Hz, 1H), 7.24 (d, *J* = 4.7 Hz, 1H), 6.95 (d, *J* = 4.7 Hz, 1H).
3) Step 3: Compound 1d (1.50 g, 5.58 mmol) was dissolved in a mixed solution of ethanol (15 mL) and dimethyl sulfoxide (3 mL). At 0°C, hydrogen peroxide aqueous solution (35%, 3 mL) and sodium hydroxide aqueous solution (0.5 M, 13.4 mL, 6.69 mmol) were added, followed by reacting under stirring at 0°C for 30 minutes. A large amount of solid precipitated out, followed by filtering directly. The filter cake was washed twice with (PE/EA=1/1, 10 mL), and filtered. The filter cake was used directly as the raw material for the next step, without further purification, to afford compound 1e, MS m/z(ESI):287.9[M+1]⁺.
4) Step 4: At room temperature, compound 1f (200 mg, 0.590 mmol, prepared using the method disclosed for the product of step 3 on page 88 of the specification of patent application "WO2022012623A"), tert-butyl 3,9-diazaspiro[5.5]undecan-3-carboxylate (301 mg, 1.18 mmol, Bide), 2-dicyclohexylphosphino-2',6'-diisopropoxy-1,1'-biphenyl (55.0 mg, 0.120 mmol), and methanesulfonato (2-dicyclohexylphosphino-2',6'-diisopropoxy-1,1'-biphenyl) (2-amino-1,1'-biphenyl-2-yl) palladium(II) (25.0 mg, 30.0 µmol) were dissolved in toluene (3 mL). Under nitrogen protection, lithium bis(trimethylsilyl)amide (3 mL, 3.00 mmol, 1.0M tetrahydrofuran solution, Energy) was added dropwise. The reaction solution was stirred at 80°C for 2 hours. The reaction solution was cooled to room temperature. Water (20 mL) was added. The aqueous phase was extracted with dichloromethane/methanol (15/1, 15 mL×3). The combined organic phase was washed with saturated saline (15 mL), and dried over anhydrous sodium sulfate. After filtering off the drying agent, the filtrate was concentrated under reduced pressure. The residue was purified by silica gel column chromatography (dichloromethane/methanol=100/1 to 10/1) to afford compound 1g. MS m/z(ESI):510.4[M-1]⁻.
5) Step 5: At room temperature, 1g (120 mg, 0.210 mmol) was dissolved in dichloromethane (2 mL). Trifluoroacetic acid (2 mL) was added. The reaction solution was stirred at 25°C for 1 hour. The solvent was removed from the reaction solution under reduced pressure. Compound 1h was afforded without purification. MS m/z(ESI):412.6[M+1]⁺.
6) Step 6: Compound 1i (7.20 g, 26.5 mmol) and 1b (18.0 g, 34.4 mmol) were dissolved in N,N-dimethylformamide (150 mL). At 25°C, cesium carbonate (25.9 g, 79.4 mmol, Bide) was added. The reaction solution was stirred at 100°C for 12 hours. The reaction solution was cooled to room temperature, and filtered. The filtrate was concentrated under reduced pressure. The residue was diluted with dichloromethane (100 mL) and water (100 mL). The aqueous phase was extracted with dichloromethane (100 mL×2). The combined organic phase was washed with saturated saline (100 mL), and dried over anhydrous sodium sulfate. After filtering off the drying agent, the filtrate was concentrated under reduced pressure. The residue was purified by silica gel column chromatography (petroleum ether/ethyl acetate=100/1 to 2/5), to afford a crude product of compound 1j. MS m/z(ESI): 385.6[M+H]⁺.
7) Step 7: The crude product of compound 1j (11.5 g, 15.0 mmol) was dissolved in glacial acetic acid (100 mL) and water (20 mL). The reaction solution was stirred at 25°C for 12 hours. The reaction solution was concentrated under reduced pressure. The residue was diluted with dichloromethane(100 mL). Saturated sodium bicarbonate (80 mL) was slowly added dropwise. The aqueous phase was separated and then extracted with dichloromethane (100 mL×2). The combined organic phase was washed with saturated saline (100 mL), and dried over anhydrous sodium sulfate. After filtering off the drying agent, the filtrate was concentrated under reduced pressure and purified to afford a crude product of compound 1l. MS m/z(ESI): 339.9[M+H]⁺.
8) Step 8: The crude product of compound 11 (9.70 g, 14.3 mmol), 4-dimethylaminopyridine (180 mg, 1.43 mmol, Bide), and triethylamine (6.00 mL, 43.0 mmol, Bide) were dissolved in dichloromethane(150 mL). At 0°C, acetyl chloride (1.58 g, 20.1 mmol, Bide) was slowly added dropwise. The reaction solution was stirred at 0°C for 1 hour. The reaction solution was quenched with water (100 mL). The aqueous phase was extracted with dichloromethane (100 mL×3). The combined organic phase was washed with saturated saline (100 mL), and dried over anhydrous sodium sulfate. After filtering off the drying agent, the filtrate was concentrated under reduced pressure. The residue was purified by silica gel column chromatography (petroleum ether/tetrahydrofuran=100/1 to 10/1). The afforded compound was identified as compound 1n by nuclear magnetic two-dimensional spectrum. MS m/z(ESI):380.0[M+H]⁺. ¹H NMR (400 MHz, CDCl₃): δ 10.22 (s, 1H), 8.84 (s, 1H), 8.39 (s, 1H), 4.67 - 4.54 (m, 1H), 4.02 (d, *J =* 6.4 Hz, 2H), 2.27 - 2.14 (m, 4H), 2.12 - 2.06 (m, 5H), 1.92 - 1.83 (m, 1H), 1.41 - 1.32 (m, 2H).
9) Step 9: Compound 1n (2.40 g, 6.31 mmol) was dissolved in dichloromethane (40 mL). At 0°C, diethylaminosulfur trifluoride (5.80 mL, 44.2 mmol, Bide) was slowly added dropwise. The reaction solution was heated to 25°C, and stirred at this temperature for 4 hours. The reaction solution was slowly poured into cooled saturated sodium bicarbonate aqueous solution (100 mL). The aqueous phase was separated and extracted with dichloromethane (100 mL×2). The combined organic phase was washed with saturated saline (100 mL), and dried over anhydrous sodium sulfate. After filtering off the drying agent, the filtrate was concentrated under reduced pressure. The residue was purified by silica gel column chromatography (petroleum ether/tetrahydrofuran=100/1 to 10/1), to afford compound 1o. MS m/z(ESI): 403.8[M+H]⁺.
   ¹H NMR (400 MHz, CDCl₃): δ 8.81 (d, *J* = 1.0 Hz, 1H), 8.01 (s, 1H), 6.96 (t, *J* = 54.3 Hz, 1H), 4.56 - 4.46 (m, 1H), 4.00 (d, *J* = 6.4 Hz, 2H), 2.20 - 2.14 (m, 2H), 2.12 - 2.04 (m, 6H), 1.89 - 1.78 (m, 1H), 1.39 - 1.25 (m, 3H).
10) Step 10: Compound 1o (1.00 g, 2.49 mmol), hexa-n-butylditin (1.6 mL, 3.23 mmol, Bide), lithium chloride (0.530 g, 12.4 mmol, Bide), tris(dibezylideneacetone)dipalladium (230 mg, 0.250 mmol, Bide), and tricyclohexylphosphine (140 mg, 0.500 mmol, Bide) were dissolved in 1,4-dioxane (15 mL). The reaction solution was purged with nitrogen three times, and stirred at 100°C for 12 hours. The reaction solution was filtered. The filtrate was concentrated under reduced pressure. The residue was purified by silica gel column chromatography (petroleum ether/ethyl acetate=100/1 to 1/1) to afford compound 1p. MS m/z(ESI):614.3[M+H]⁺. ¹H NMR (400 MHz, CDCl₃): δ 9.22 (d, *J =* 1.3 Hz, 1H), 7.89 (s, 1H), 6.99 (t, *J =* 54.5 Hz, 1H), 4.58 - 4.47 (m, 1H), 4.00 (d, *J =* 6.4 Hz, 2H), 2.20 - 2.12 (m, 3H), 2.11 - 2.01 (m, 6H), 1.88 - 1.81 (m, 1H), 1.66 - 1.54 (m, 8H), 1.38 - 1.31 (m, 8H), 1.19 - 1.15 (m, 4H), 0.89 (t, *J =* 7.3 Hz, 9H).
11) Step 11: Compound 1p (660 mg, 1.08 mmol), compound 1e (371 mg, 1.29 mmol), cuprous iodide (41.1 mg, 0.220 mmol, Bide), and tetrakis(triphenylphosphine)palladium (125 mg, 0.110 mmol, Bide) were dissolved in dioxane (10 mL). The reaction solution was bubbled with nitrogen for 1 minute, and stirred at 100°C for 4 hours. The reaction solution was filtered. The filtrate was concentrated under reduced pressure. The residue was purified by silica gel column chromatography (dichloromethane/methanol=30/1 to 1/1), to afford compound 1q. MS m/z(ESI):483.2[M+H]⁺.
12) Step 12: Compound 1q (900 mg, 1.87 mmol) was dissolved in methanol (10 mL). At 25°C, potassium carbonate (773 mg, 5.60 mmol, Bide) was added. The reaction solution was stirred at 25°C for 3 hours. The reaction solution was concentrated under reduced pressure. The residue was diluted with dichloromethane (50 mL) and methanol (5 mL), followed by acidifying with hydrogen chloride in ethanol (4.0 M) to pH of about 6-7. The reaction solution was concentrated under reduced pressure. The residue was purified by silica gel column chromatography (dichloromethane/methanol=100/1 to 10/1), to afford compound 1r. MS m/z(ESI):441.3[M+H]⁺. ¹H NMR (400 MHz, DMSO-*d*₆): δ 9.48 (d, *J =* 1.2 Hz, 1H), 9.18 (s, 1H), 8.88 (d, *J* = 2.3 Hz, 1H), 8.64 (d, *J* = 2.3 Hz, 1H), 8.23 - 7.95 (m, 1H), 7.82 (d, *J=* 4.7 Hz, 1H), 7.63 - 7.33 (m, 2H), 7.04 (d, *J* = 4.7 Hz, 1H), 4.95 - 4.85 (m, 1H), 4.54 (t, *J =* 5.3 Hz, 1H), 3.17 (d, *J* = 5.3 Hz, 2H), 2.17 - 2.09 (m, 2H), 1.99 - 1.89 (m, 4H), 1.28 - 1.21 (m, 3H).
13) Step 13: Compound 1r (150 mg, 0.340 mmol) was dissolved in *N,N-*dimethylformamide (3 mL) and dichloromethane (3 mL). At 25°C, Dess-Martin Periodinane (289 mg, 0.680 mmol, Bide) was added. The reaction solution was stirred at 25°C for 3 hours. The reaction solution was concentrated under reduced pressure. The residue was purified by silica gel column chromatography (dichloromethane/methanol=100/1 to 15/1), to afford compound 1s. MS m/z(ESI):439.1[M+H]⁺.
14) Step 14: Compound 1h (134 mg, 0.330 mmol) was dissolved in *N,N-*dimethylformamide (2 mL). At 25°C, triethylamine (25.4 mg, 0.250 mmol, Bide) was added. After the reaction solution was stirred at 25°C for 5 minutes, acetic acid (15.1 mg, 0.250 mmol, Bide) and sodium triacetoxyborohydride (237 mg, 1.13 mmol, Bide) were added. The reaction solution was stirred for 5 minutes, and then compound 1s (110 mg, 0.250 mmol) was added. The reaction solution was stirred for 3 hours. One drop of water was added to the reaction solution to quench the reaction, followed by filtration to afford the residue. The residue was purified by high performance liquid chromatography (Waters-2545, chromatographic column:SharpSil-T C18,30×150 mm, 5µm; mobile phase: water (containing 10 mmol/L formic acid) and acetonitrile, gradient ratio: acetonitrile 38%-45%, flow rate:30 mL/min) to afford the formate of compound 1. MS m/z(ESI):832.6[M-H]⁻. ¹H NMR (400 MHz, DMSO-*d*₆): δ 11.09 (s, 1H), 9.48 (s, 1H), 9.18 (s, 1H), 8.88 (d, *J =* 1.9 Hz, 1H), 8.64 (d, *J* = 2.1 Hz, 1H), 8.16 - 8.05 (m, 1H), 7.82 (d, *J* = 4.7 Hz, 1H), 7.62 - 7.34 (m, 2H), 7.04 (d, *J* = 4.7 Hz, 1H), 6.99 - 6.94 (m, 2H), 6.89 - 6.84 (m, 1H), 5.36 - 5.31 (m, 1H), 4.94 - 4.84 (m, 1H), 3.63 (s, 3H), 2.88 (s, 6H), 2.70 - 2.61 (m, 3H), 2.43 - 2.36 (m, 4H), 2.25 - 2.19 (m, 2H), 2.14 - 2.08 (m, 2H), 2.02 - 1.94 (m, 5H), 1.71 - 1.65 (m, 3H), 1.54 - 1.40 (m, 4H), 1.25 - 1.20 (m, 2H).

### Example 2

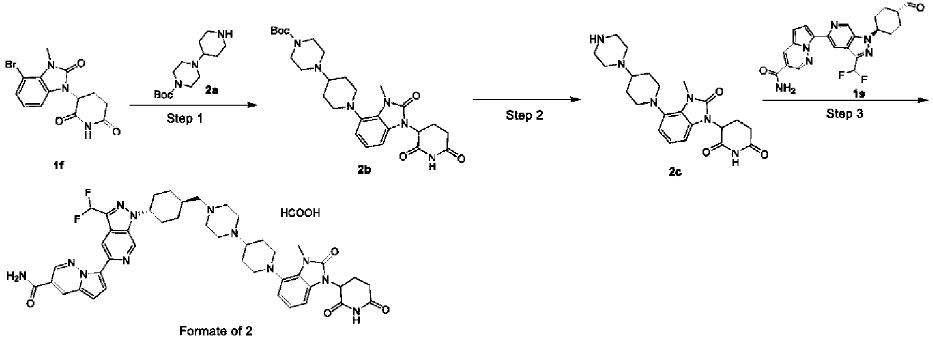

1) Step 1: Compound 2a (398 mg, 1.48 mmol, Bide) was dissolved in toluene (5 mL). Compound 1f (500 mg, 1.48 mmol, prepared using the method disclosed for the product of step 3 on page 88 of the specification of patent application "WO2022012623 A"), 2-dicyclohexylphosphino-2,6-diisopropoxy-1,1-biphenyl (138 mg, 0.300 mmol), and chloro(2-amino-[1,1-biphenyl]-2-yl)(dicyclohexyl(2,6-diisopropoxy-[1,1-biphenyl]-2-yl)phosphoryl)palladium (230 mg, 0.300 mmol) were added. Under nitrogen protection, lithium bis(trimethylsilyl)amide (7.40 mL, 7.40 mmol, 1.0M tetrahydrofuran solution) was added dropwise to the reaction solution. Under a nitrogen atmosphere, the reaction was carried out at 80°C for 12 hours. The reaction solution was concentrated under reduced pressure, and purified by silica gel column chromatography with an eluant system (petroleum ether/ethyl acetate=3/1 to 1/1) to afford compound 2b. MS m/z(ESI):527.3[M+1]⁺.
2) Step 2: Compound 2b (60.0 mg, 0.110 mmol) was dissolved in dichloromethane (2 mL), and trifluoroacetic acid (0.4 mL) was added. The reaction was carried out at room temperature for 1 hour. The reaction solution was concentrated under reduced pressure, to afford compound 2c. MS m/z(ESI):427.3[M+1]⁺.
3) Step 3: Compound 2c (24.3 mg, 0.057 mmol) was dissolved in a mixed solution of *N,* N-dimethylformamide (1 mL) and tetrahydrofuran (4 mL). Triethylamine (5.77 mg, 0.057 mmol) was added, followed by reacting under stirring at room temperature for 15 minutes. Then, 1s (25.0 mg, 0.06 mmol), acetic acid (6.85 mg, 0.11 mmol) were added, followed by reacting under stirring at room temperature for 30 minutes. Then, sodium triacetoxyborohydride (36.1 mg, 0.17 mmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate:30 mL/min), to afford the formate of compound 2. MS m/z(ESI):849.6[M+1]⁺. ¹H NMR (400 MHz, DMSO-*d*₆): δ 11.11 (s, 1H), 9.49 (d, *J* = 1.2 Hz, 1H), 9.19 (s, 1H), 8.89 (d, *J =* 2.3 Hz, 1H), 8.65 (d, *J =* 2.3 Hz, 1H), 8.17 (s, 1H), 7.83 (d, *J* = 4.7 Hz, 1H), 7.63 - 7.36 (m, 2H), 7.04 (d, *J* = 4.7 Hz, 1H), 6.99 - 6.94 (m, 1H), 6.91 - 6.85 (m, 2H), 5.39 - 5.31 (m, 1H), 4.96 - 4.86 (m, 1H), 3.63 (s, 3H), 3.17 - 3.12 (m, 2H), 2.97 - 2.83 (m, 2H), 2.72 - 2.60 (m, 6H), 2.45 - 2.39 (m, 2H), 2.36 - 2.29 (m, 2H), 2.20 - 2.16 (m, 2H), 2.14 - 2.09 (m, 2H), 2.03 - 1.86 (m, 8H), 1.76 (s, 1H), 1.70 - 1.58 (m, 3H), 1.25 - 1.15 (m, 2H).

### Example 3

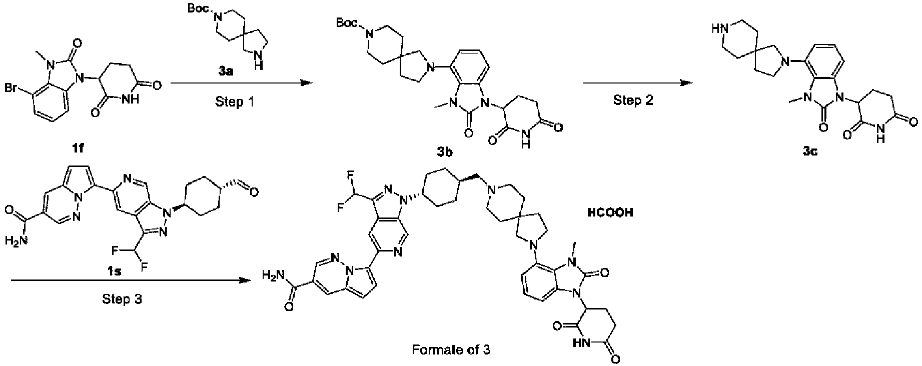

1) Step 1: Compound 1f (500 mg, 1.48 mmol, prepared using the method disclosed for the product of step 3 on page 88 of the specification of patent application "WO2022012623 A"), compound3a (533 mg, 2.22 mmol, Bide), methanesulfonato(2-dicyclohexylphosphino-2',6'-diisopropoxy-1,1'-biphenyl)(2-amino-1,1'-biphenyl-2-yl)palladium(II) (248 mg,0.300 mmol, Bide) and 2-dicyclohexylphosphino-2',6'-diisopropoxy-1,1'-biphenyl(138 mg,0.300 mmol, Bide) were dissolved in toluene(5 mL). Under nitrogen protection, lithium bis(trimethylsilyl)amide (7.40 mL, 7.40 mmol, 1.0M tetrahydrofuran solution, Energy) was added dropwise. The reaction solution was reacted at 80°C for 2 hours under a nitrogen atmosphere. The reaction solution was quenched with saturated ammonium chloride (20 mL). The aqueous phase was extracted with dichloromethane (20 mL×2). The organic phases were combined, and dried over anhydrous sodium sulfate. After filtering off the drying agent, the filtrate was concentrated under reduced pressure. The residue was purified by silica gel column chromatography (dichloromethane/methanol=100/1 to 30/1) to afford compound3b. MS m/z(ESI):498.4[M+1]⁺.
2) Step 2: Compound 3b (80.0 mg, 0.160 mmol) was dissolved in dichloromethane (2 mL), and trifluoroacetic acid (1 mL) was added. The resulting mixture was reacted under stirring at 25°C for 1 hour. The reaction solution was concentrated under reduced pressure to afford compound 3c.
   MS m/z(ESI):398.5[M+1]⁺
3) Step 3: Compound 3c (36.3 mg, 0.091 mmol) was dissolved in *N,N-*dimethylformamide (1 mL), and sodium triacetoxyborohydride (76.9 mg, 0.361 mmol, Bide) was added. The reaction solution was stirred at 25°C for 5 minutes, and then 1s (40.1 mg, 0.091 mmol) was added. The reaction solution was stirred at 25°C for another 30 minutes. The reaction solution was concentrated under reduced pressure to afford a residue. The residue was purified by high performance liquid chromatography (Waters-2545, chromatographic column:SharpSil-T C18,30×150mm, 5µm; mobile phase:water (containing 10 mmol/L formic acid) and acetonitrile, gradient ratio : acetonitrile 38%-45%, flow rate: 30 mL/min) to afford the formate of compound 3. MS m/z(ESI):820.5[M+1]⁺. ¹H NMR (400 MHz, DMSO-*d*₆): δ 11.09 (s, 1H), 9.49 (s, 1H), 9.19 (s, 1H), 8.89 (d, *J* = 2.2 Hz, 1H), 8.65 (d, *J* = 2.1 Hz, 1H), 8.36 (s, 1H), 8.11 (s, 1H), 7.83 (d, *J* = 4.7 Hz, 1H), 7.65 - 7.33 (m, 2H), 7.05 (d, *J* = 4.7 Hz, 1H), 6.99 - 6.94 (m, 2H), 6.89 - 6.83 (m, 1H), 5.38 - 5.30 (m, 1H), 4.97 - 4.86 (m, 1H), 3.61 (s, 3H), 3.11 - 3.04 (m, 2H), 2.90 (s, 3H), 2.75 - 2.58 (m, 3H), 2.43 - 2.36 (m, 3H), 2.35 - 2.32 (m, 1H), 2.20 - 2.15 (m, 2H), 2.15 - 2.08 (m, 2H), 2.05 - 1.95 (m, 5H), 1.78 - 1.74 (m, 2H), 1.70 - 1.60 (m, 4H), 1.27 - 1.13 (m, 2H).

### Example 4

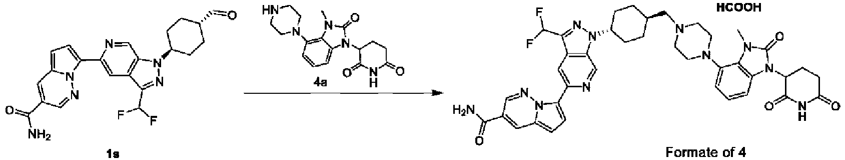

Compound 4a (28.2 mg, 0.091 mmol, prepared using the method disclosed for intermediate GS on page 550 of the specification of patent application "WO2022236058 A1") was dissolved in *N,N*-dimethylformamide (1 mL). Sodium triacetoxyborohydride (57.7 mg, 0.271 mmol, Bide) was added. The reaction solution was stirred at 25°C for 5 minutes, and then 1s (30.1 mg, 0.071 mmol) was added. The reaction solution was stirred at 25°C for another 30 minutes. The reaction solution was concentrated under reduced pressure to afford a residue. The residue was purified by high performance liquid chromatography (Waters-2545, chromatographic column:SharpSil-T C18, 30×150 mm, 5 µm; mobile phase:water (containing 10 mmol/L formic acid) and acetonitrile, gradient ratio : acetonitrile 38%-45%, flow rate: 30 mL/min)to afford the formate of compound 4. MS m/z(ESI):766.4[M+1]⁺. ¹H NMR (400 MHz, DMSO-*d*₆): δ 11.10 (s, 1H), 9.51 (d, *J =* 1.2 Hz, 1H), 9.20 (s, 1H), 8.90 (d, *J =* 2.3 Hz, 1H), 8.65 (d, *J* = 2.3 Hz, 1H), 8.45 (s, 1H), 8.11 (s, 1H), 7.83 (d, *J =* 4.7 Hz, 1H), 7.66 - 7.33 (m, 2H), 7.05 (d, *J =* 4.7 Hz, 1H), 7.02 - 6.94 (m, 2H), 6.92 - 6.87 (m, 1H), 5.48 - 5.26 (m, 1H), 5.01 - 4.85 (m, 1H), 3.65 (s, 3H), 3.06 - 2.86 (m, 8H), 2.75 - 2.68 (m, 2H), 2.30 - 2.26 (m, 2H), 2.21 - 2.10 (m, 3H), 2.07 - 1.97 (m, 5H), 1.79 - 1.69 (m, 1H), 1.32 - 1.19 (m, 2H).

### Example 5

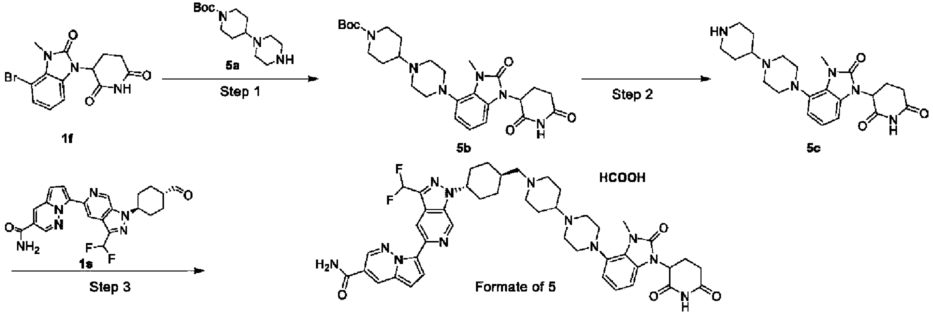

1) Step 1: Compound 1f (150 mg, 0.440 mmol, prepared using the method disclosed for the product of step 3 on page 88 of the specification of patent application "WO2022012623 A"), compound 5a (179 mg, 0.660 mmol, Bide), 2-dicyclohexylphosphino-2',6'-diisopropoxy-1,1'-biphenyl (62.0 mg, 0.130 mmol, Bide), and methanesulfonato(2-dicyclohexylphosphino-2',6'-diisopropoxy-1,1'-biphenyl)(2-amino-1,1'-biphenyl-2-yl)palladium(II) (111 mg, 0.130 mmol, adamas) were dissolved in toluene (5 mL). Under nitrogen protection, lithium bis(trimethylsilyl)amide (2.6 mL, 2.60 mmol, 1.0M tetrahydrofuran solution, Energy) was added dropwise. The reaction solution was reacted under stirring at 80°C for 2 hours under a nitrogen atmosphere. The mixture was poured into water (20 mL), and the aqueous phase was extracted with ethyl acetate (30 mLx3). The combined organic phase was washed with saturated saline (10 mL), and dried over anhydrous sodium sulfate. After filtering off the drying agent, the solvent was removed by concentration under reduced pressure. The residue was purified by silica gel chromatography (petroleum ether/ethyl acetate=10/1 to 0/1) to afford compound 5b. MS m/z(ESI): 527.3[M+1]⁺.
2) Step 2: Compound 5b (78.0 mg, 0.150 mmol) was dissolved in dichloromethane (5 mL). Trifluoroacetic acid (1 mL) was added, followed by stirring at 25°C for 2 hours. The mixture was concentrated to afford compound 5c. MS m/z(ESI): 427.3[M+1]⁺.
3) Step 3: Compound 5c (46.7 mg, 0.110 mmol) was dissolved in *N,N-*dimethylformamide (1 mL). At 25°C, triethylamine (9.23 mg, 91 µmol) was added. After the reaction solution was stirred at 25°C for 5 minutes, acetic acid (5.48 mg, 91 µmol) and sodium triacetoxyborohydride (115 mg, 0.540 mmol, Bide) were added. After the reaction solution was stirred for 5 minutes, compound 1s (40.0 mg, 91 µmol) was added. The reaction solution was stirred for 3 hours. One drop of water was added to the reaction solution to quench the reaction, followed by filtration to afford the residue. The residue was purified by high performance liquid chromatography (Waters-2545, chromatographic column: SharpSil-T C18,30×150 mm, 5 µm; mobile phase: water (containing 10mmol/L formic acid) and acetonitrile, gradient ratio: acetonitrile 38%-45%, flow rate: 30mL/min) to afford the formate of compound 5. MS m/z(ESI):425.2[M/2+H]⁺. ¹H NMR (400 MHz, DMSO-*d*₆): δ 11.08 (s, 1H), 9.49 (d, *J =* 1.1 Hz, 1H), 9.18 (s, 1H), 8.89 (d, *J* = 2.2 Hz, 1H), 8.64 (d, *J* = 2.3 Hz, 1H), 8.10 (s, 1H), 7.82 (d, *J* = 4.7 Hz, 1H), 7.65 - 7.30 (m, 2H), 7.04 (d, *J =* 4.7 Hz, 1H), 7.01 - 6.95 (m, 1H), 6.94 - 6.90 (m, 1H), 6.89 - 6.85 (m, 1H), 5.38 - 5.32 (m, 1H), 4.96 - 4.84 (m, 1H), 3.62 (s, 3H), 2.95 - 2.80 (m, 7H), 2.75 - 2.57 (m, 3H), 2.25 - 2.10 (m, 5H), 2.03 - 1.85 (m, 8H), 1.81 - 1.75 (m, 2H), 1.72 - 1.64 (m, 1H), 1.51 - 1.42 (m, 2H), 1.28 - 1.12 (m, 4H).

### Example 6

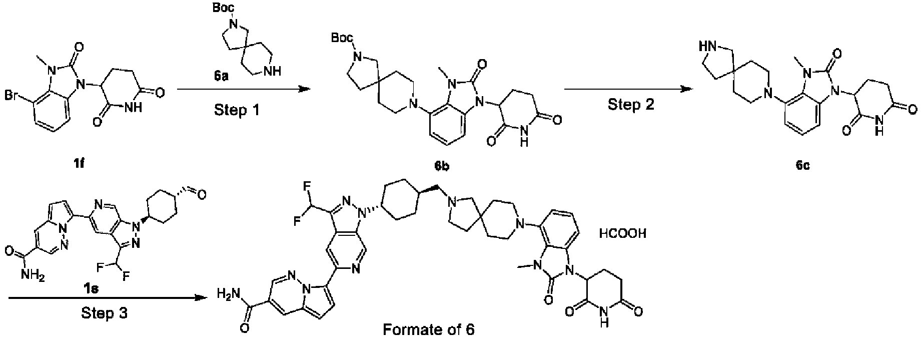

1) Step 1: Compound 1f (300 mg, 0.890 mmol, prepared using the method disclosed for the product of step 3 on page 88 of the specification of patent application "WO2022012623 A"), compound 6a (426 mg, 1.77 mmol, Bide), methanesulfonato(2-dicyclohexylphosphino-2',6'-diisopropoxy-1,1'-biphenyl)(2-amino-1,1'-biphenyl-2-yl)palladium(II) (149 mg, 0.180 mmol, adamas), and 2-dicyclohexylphosphino-2',6'-diisopropoxy-1,1'-biphenyl (83.0 mg, 0.180 mmol, Bide) were dissolved in toluene (3 mL). Under nitrogen protection, lithium bis(trimethylsilyl)amide (4.4 mL, 4.44 mmol, 1.0M tetrahydrofuran solution, Energy) was added dropwise. The reaction solution was reacted at 80°C for 2 hours. The reaction solution was quenched with saturated ammonium chloride aqueous solution (20 mL). The aqueous phase was extracted with dichloromethane (20 mL×2). The organic phases were combined, and dried over anhydrous sodium sulfate. After filtering off the drying agent, the filtrate was concentrated under reduced pressure. The residue was purified by silica gel column chromatography (dichloromethane/methanol=100/1 to 30/1) to afford compound 6b. MS m/z(ESI):498.3[M+1]⁺.
2) Step 2: Compound 6b (100 mg, 0.200 mmol) was dissolved in dichloromethane (2 mL), and trifluoroacetic acid (1 mL) was added. The reaction solution was stirred at 20°C for 1 hour. The reaction solution was concentrated under reduced pressure to afford crude compound 6c. MS m/z(ESI):398.2[M+1]⁺.
3) Step 3: At room temperature, compound 6c (55.0 mg, 0.14 mmol), and triethylamine (7.00 mg, 0.069 mmol) were added to N,N-diisopropylethylamine (3 mL), followed by stirring for 2 minutes. Acetic acid (8.00 mg, 0.13 mmol), sodium triacetoxyborohydride (43.0 mg, 0.20 mmol), and compound 1s (30.0 mg, 0.068 mmol) were added. The reaction solution was stirred at room temperature for 15 minutes. The reaction solution was added to water (10 mL), followed by extraction with ethyl acetate (3 mL×3). The organic phases were washed by adding saturated saline (10 mL), dried over anhydrous sodium sulfate, and filtered. The solvent was removed under reduced pressure. The residue was purified by high performance liquid chromatography (Gilson_306_1741, chromatographic column: Waters-SunFire-C18-10 µm-19×250 mm; mobile phase: water (containing 0.1% formic acid) and acetonitrile, gradient ratio: acetonitrile 10%-90%, flow rate: 25 mL/min), to afford the formate of compound 6. MS m/z(ESI):821.0[M+1]⁺. ¹H NMR (400 MHz, DMSO-*d₆*) δ 11.09 (s, 1H), 9.50 - 9.47 (m, 1H), 9.19 (s, 1H), 8.89 (d, *J* = 2.3 Hz, 1H), 8.65 (d, *J* = 2.3 Hz, 1H), 8.11 (s, 1H), 7.83 (d, *J* = 4.6 Hz, 1H), 7.67 - 7.30 (m, 2H), 7.05 (d, *J* = 4.7 Hz, 1H), 7.01 - 6.97 (m, 2H), 6.88 - 6.86 (m, 1H), 5.38 - 5.33 (m, 1H), 4.95 - 4.88 (m, 1H), 3.65 (s, 3H), 2.99 - 2.85 (m, 4H), 2.77 - 2.65 (m, 5H), 2.38 - 2.33 (m, 3H), 2.18 - 2.12 (m, 2H), 2.03 - 2.00 (m, 5H), 1.80 - 1.60 (m, 8H), 1.24 - 1.21 (m, 2H).

### Example 7

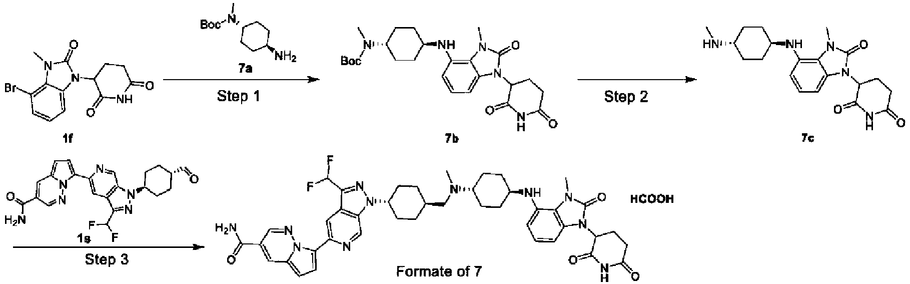

1) Step 1: Compound 1f (518 mg, 1.53 mmol, prepared using the method disclosed for the product of step 3 on page 88 of the specification of patent application "WO2022012623 A"), compound 7a (698 mg, 3.06 mmol, prepared using the method disclosed for intermediate 4 on page 858 of the specification of patent application "US 2019/0192668 A1"), 2-dicyclohexylphosphino-2',6'-diisopropoxy-1,1'-biphenyl (140 mg, 0.300 mmol, Bide), methanesulfonato(2-dicyclohexylphosphino-2',6'-diisopropoxy-1,1'-biphenyl)(2-amino-1,1'-biphenyl -2-yl)palladium(II) (74.2 mg, 90.0µmol, adamas) were dissolved in toluene (8 mL). Under nitrogen protection, lithium bis(trimethylsilyl)amide (8.9 mL, 8.87 mmol, 1M tetrahydrofuran solution, Energy) was added dropwise. The reaction solution was reacted at 80°C for 1 hour under a nitrogen atmosphere. Water (10 mL) was added to the reaction solution. The aqueous phase was extracted with dichloromethane (50 mL×3). The organic phases were combined, and dried over anhydrous sodium sulfate. After filtering off the drying agent, the filtrate was concentrated under reduced pressure. The residue was purified by silica gel column chromatography with an eluant system (dichloromethane/methanol=20/1) to afford compound 7b. MS m/z(ESI):486.4[M+1]⁺.
2) Step 2: Compound 7b (350 mg, 0.721 mmol) was dissolved in dichloromethane (6 mL). Trifluoroacetic acid (2 mL) was added. The reaction solution was reacted at 25°C for 2 hours. The reaction solution was concentrated under reduced pressure to afford compound 7c. MS m/z(ESI):386.7[M+1]⁺.
3) Step 3: Compound 7c (26.3 mg, 0.068 mmol) was dissolved in N,N-dimethylformamide (1 mL). Sodium triacetoxyborohydride (57.7 mg, 0.271 mmol, Bide) was added. The reaction solution was stirred at 25°C for 5 minutes, and then 1s (30.1 mg, 0.071 mmol) was added. The reaction solution was stirred at 25°C for another 30 minutes. The reaction solution was concentrated under reduced pressure to afford a residue. The residue was purified by high performance liquid chromatography (Waters-2545, chromatographic column: SharpSil-T C18,30×150 mm, 5 µm; mobile phase: water (containing 10 mmol/L formic acid) and acetonitrile, gradient ratio: acetonitrile 38%-45%, flow rate: 30 mL/min)to afford the formate of compound 7. MS m/z(ESI):808.5[M+1]⁺. ¹H NMR (400 MHz, DMSO-*d*₆): δ 11.07 (s, 1H), 9.49 (d, *J* = 1.2 Hz, **1H**), 9.19 (s, 1H), 8.89 (d, *J =* 2.2 Hz, 1H), 8.65 (d, *J =* 2.3 Hz, 1H), 8.33 (s, 1H), 8.11 (s, 1H), 7.83 (d, *J =* 4.6 Hz, 1H), 7.66 - 7.32 (m, 2H), 7.05 (d, *J =* 4.7 Hz, 1H), 6.91 - 6.83 (m, 1H), 6.57 - 6.47 (m, 2H), 5.34 - 5.25 (m, 1H), 4.97 - 4.85 (m, 1H), 4.60 - 4.53 (m, 1H), 3.62 (s, 3H), 2.99 - 2.81 (m, 2H), 2.70 - 2.61 (m, 2H), 2.36 - 2.25 (m, 3H), 2.24 (s, 3H), 2.15 - 2.05 (m, 4H), 2.03 - 1.94 (m, 5H), 1.81 - 1.74 (m, 2H), 1.64 - 1.53 (m, 1H), 1.44 - 1.15 (m, 6H).

### Example 8

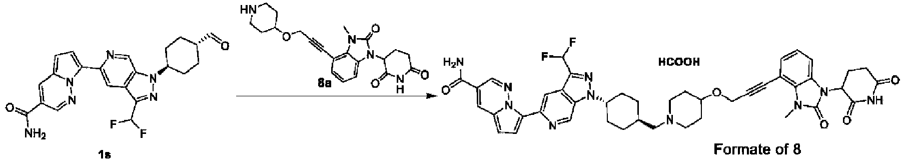

Compound 8a (36.2 mg, 0.091 mmol, prepared using the method disclosed for intermediate APT on page 91 of the specification of patent application "WO2021247899 A1") was dissolved in *N,N-*dimethylformamide (1 mL). Sodium triacetoxyborohydride (76.9 mg, 0.361 mmol, Bide) was added. The reaction solution was stirred at 25°C for 5 minutes, and then 1s (40.1 mg, 0.091 mmol) was added. The reaction solution was stirred at 25°C for another 30 minutes. The reaction solution was concentrated under reduced pressure to afford a residue. The residue was purified by high performance liquid chromatography (Waters-2545, chromatographic column: SharpSil-T C18,30×150 mm, 5 µm; mobile phase: water (containing 10 mmol/L formic acid) and acetonitrile, gradient ratio: acetonitrile 38%-45%, flow rate: 30 mL/min)to afford the formate of compound 8. MS m/z(ESI):819.5[M+1]⁺. ¹H NMR (400 MHz, DMSO-*d*₆): δ 11.13 (s, 1H), 9.49 (s, 1H), 9.19 (s, 1H), 8.89 (d, *J* = 2.*2* Hz, 1H), 8.65 (d, *J* = 2.*2* Hz, 1H), 8.41 (s, 1H), 8.11 (s, 1H), 7.83 (d, *J* = 4.7 Hz, 1H), 7.64 - 7.35 (m, 2H), 7.21 - 7.16 (m, 1H), 7.15 - 7.11 (m, 1H), 7.07 - 7.01 (m, 2H), 5.45 - 5.37 (m, 1H), 4.95 - 4.83 (m, 1H), 4.49 (s, 2H), 3.66 (s, 3H), 3.60 - 3.53 (m, 1H), 2.94 - 2.68 (m, 4H), 2.20 - 2.09 (m, 5H), 2.05 - 1.90 (m, 8H), 1.77 - 1.59 (m, 2H), 1.56 - 1.45 (m, 2H), 1.24 - 1.15 (m, 2H).

### Example 9

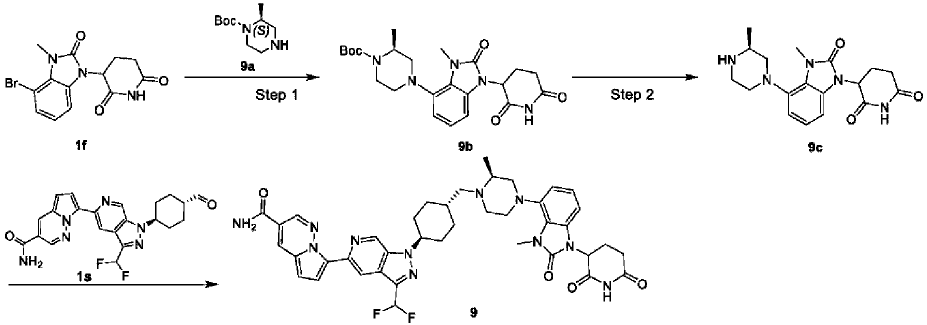

1) Step 1: Compound 1f (200 mg, 0.590 mmol, prepared using the method disclosed for the product of step 3 on page 88 of the specification of patent application "WO2022012623 A"), compound 9a (355 mg, 1.77 mmol, Bide), 2-dicyclohexylphosphino-2',6'-diisopropoxy-1,1'-biphenyl (55.0 mg, 0.120 mmol), and methanesulfonato(2-dicyclohexylphosphino-2',6'-diisopropoxy-1,1'-biphenyl)(2-amino-1,1'-biphenyl -2-yl)palladium(II) (74.0 mg, 90.0 µmol) were dissolved in toluene (3 mL). Under nitrogen protection, lithium bis(trimethylsilyl)amide (4 mL, 4.00 mmol, 1.0M tetrahydrofuran solution, Energy) was added dropwise. The reaction solution was stirred at 80°C for 2 hours under a nitrogen atmosphere. Water (10 mL) was added to the reaction solution. The aqueous phase was extracted with dichloromethane/methanol (15/1, 15 mL×3). The combined organic phase was washed with saturated saline (10 mL), dried over anhydrous sodium sulfate, filtered, and concentrated to afford the residue. The residue was purified by silica gel column chromatography (dichloromethane/methanol=100/1 to 10/1)to afford compound 9b. MS m/z(ESI):458.3[M+1]⁺.
2) Step 2: Compound 9b (110 mg, 0.240 mmol) was dissolved in dichloromethane (2 mL). Trifluoroacetic acid (2 mL) was added. The reaction solution was stirred at 25°C for 1 hour. The reaction solution was concentrated under reduced pressure to remove the solvent, affording compound 9c. MS m/z(ESI):358.4[M+1]⁺.
3) Step 3: Compound 9c (56.3 mg, 0.16 mmol) was dissolved in a mixed solution of *N,N-*dimethylformamide (1 mL) and tetrahydrofuran (4 mL). Triethylamine (15.9 mg, 0.16 mmol) was added, followed by reacting under stirring at room temperature for 15 minutes. Then, 1s (69.0 mg, 0.16 mmol), and acetic acid (18.9 mg, 0.31 mmol) were added, followed by reacting under stirring at room temperature for 30 minutes. Then, sodium triacetoxyborohydride (166 mg, 0.79 mmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min), to afford compound 9. MS m/z(ESI):780.7[M+1]⁺. ¹H NMR (400 MHz, DMSO-*d*₆): δ 11.09 (s, 1H), 9.49 (s, 1H), 9.19 (s, 1H), 8.89 (s, 1H), 8.65 (s, 1H), 8.11 (s, 1H), 7.87 - 7.77 (m, 1H), 7.61 - 7.33 (m, 2H), 7.07 - 6.86 (m, 4H), 5.42 - 5.29 (m, 1H), 5.00 - 4.87 (m, 1H), 3.65 (s, 3H), 3.11 - 2.81 (m, 5H), 2.72 - 2.58 (m, 3H), 2.32 - 2.05 (m, 5H), 2.03 - 1.91 (m, 4H), 1.75 - 1.65 (m, 1H), 1.27 - 1.19 (m, 5H), 1.07 (s, 2H).

### Example 10

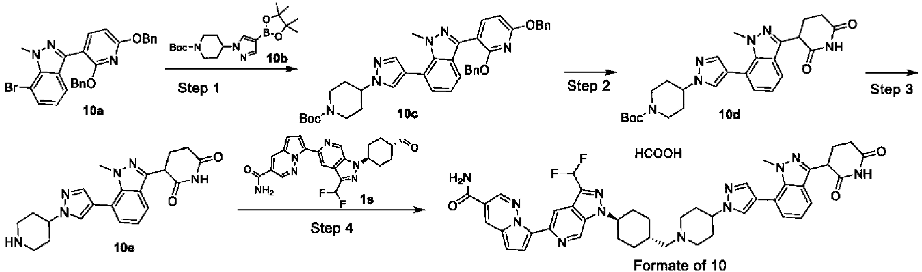

1) Step 1: [1,1'-bis(diphenylphosphino)ferrocene]dichloropalladium dichloromethane complex (88.0 mg, 120 µmol) was added to a solution containing compound 10a (600 mg, 1.20 mmol, prepared using the method disclosed for the product of step 00146 on page 48 of the specification of patent application "WO2021262812A1"), compound 10b (905.0 mg, 2.40 mmol, Bide), potassium carbonate (500 mg, 3.60 mmol), dioxane (10 mL), and water (2 mL). The reaction solution was stirred at 110°C for 12 hours under a nitrogen atmosphere. Water was added to the reaction solution, followed by extraction with ethyl acetate (50 mL×3), washing with saturated saline (20 mL×1), drying over anhydrous sodium sulfate, and concentrating under reduced pressure. The residue was purified by column chromatography (0-30% petroleum ether/ethyl acetate) to afford compound 10c. MS m/z(ESI):671.0 [M+1]⁺. ¹H NMR (400 MHz, DMSO-*d₆*) δ 8.11 (s, 1H), 7.87 (d, *J* = 8.1 Hz, 1H), 7.69 (s, 1H), 7.61 (d, *J* = 8.1 Hz, 1H), 7.51 - 7.46 (m, 2H), 7.44 - 7.27 (m, 8H), 7.20 - 7.15 (m, 1H), 7.07 - 6.99 (m, 1H), 6.61 (d, *J* = 8.1 Hz, 1H), 5.45 (d, *J* = 2.7 Hz, 4H), 4.48 - 4.38 (m, 1H), 4.13 - 3.98 (m, 2H), 3.78 (s, 3H), 3.07 - 2.84 (m, 2H), 2.13 - 2.02 (m, 2H), 1.93 - 1.79 (m, 2H), 1.43 (s, 9H).
2) Step 2: At room temperature, palladium on carbon (30 mg, 10%) was added to a solution containing compound 10c (300.0 mg, 450 µmol), ethyl acetate (3 mL), and ethanol (3 mL). After purging with hydrogen three times, the reaction was carried out for 12 hours under a condition of 25°C. The resulting mixture was filtered. The filtrate was concentrated under reduced pressure, to afford compound 10d. The product was used directly in the next step without purification. MS m/z(ESI):493.4 [M+1]⁺.
3) Step 3: Trifluoroacetic acid (1 mL) was added to a solution containing compound 10d (140.0 mg, 280 µmol) and dichloromethane (2 mL). After reacting under stirring at 25°C for 1 hour, the reaction solution was concentrated under reduced pressure to afford crude compound 10e. The product was used directly in the next step without purification. MS m/z(ESI):393.6 [M+1]⁺.
4) Step 4: Triethylamine (0.30 mL, 2.26 mmol) was added to a solution containing compound 10e (80.0 mg, 200 µmol), tetrahydrofuran (1 mL), and N,N-dimethylformamide (2 mL), followed by stirring for 30 minutes. Then, compound 1s (108.0 mg, 240 µmol) and acetic acid (0.5 mL) were added to the above solution, followed by stirring for 30 minutes. Finally, sodium triacetoxyborohydride (129.0 mg, 610 µmol) was added to the above reaction solution. The resulting mixture was also reacted under stirring for 2 hours under a condition of 25°C. The reaction solution was concentrated under reduced pressure and purified by high performance liquid chromatography (A: 0.1% FA/H₂O B:ACN, chromatographic column: Waters-SunFire-C18-10 µm-19×250 mm; mobile phase: water (containing 0.1% formic acid) and acetonitrile, gradient ratio: acetonitrile 32%-48%, flow rate: 25 mL/min) to afford the formate of compound 10. MS m/z(ESI):815.6 [M+1]⁺;¹H NMR (400 MHz, DMSO-*d₆*) δ 10.91 (s, 1H), 9.50 (d, *J* = 1.2 Hz, 1H), 9.19 (s, 1H), 8.89 (d, *J* = 2.3 Hz, 1H), 8.65 (d, *J* = 2.3 Hz, 1H), 8.10 (s, 2H), 7.83 (d, *J* = 4.7 Hz, 1H), 7.71 - 7.66 (m, 2H), 7.63 - 7.34 (m, 2H), 7.23 - 7.19 (m, 1H), 7.16 - 7.10 (m, 1H), 7.05 (d, *J =* 4.7 Hz, 1H), 4.97 - 4.87 (m, 1H), 4.44 - 4.37 (m, 1H), 4.28 - 4.20 (m, 1H), 3.73 (s, 3H), 3.01 (d, *J =* 10.5 Hz, 2H), 2.74 - 2.60 (m, 2H), 2.43 - 2.33 (m, 1H), 2.27 - 2.22 (m, 2H), 2.15 - 2.05 (m, 8H), 2.03 - 1.96 (m, 5H), 1.83 - 1.66 (m, 1H), 1.29 - 1.20 (m, 2H).

### Example 11

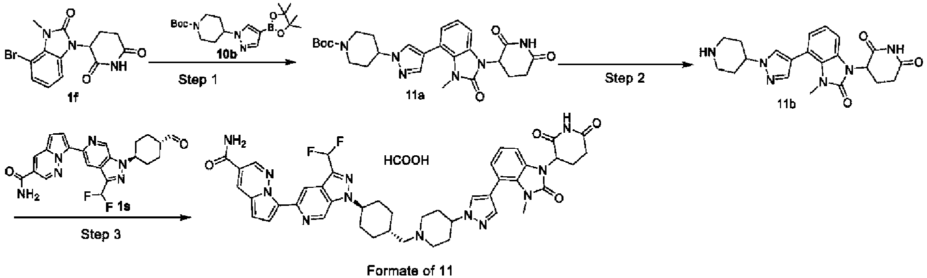

1) Step 1: Compound 1f (400 mg, 1.18 mmol, prepared using the method disclosed for the product of step 3 on page 88 of the specification of patent application "WO2022012623 A") was dissolved in a mixed solution of dioxane (10 mL) and water (1 mL). 10b (580 mg, 1.54mmol, Bide), potassium phosphate (753 mg, 3.55 mmol), and methanesulfonato(2-dicyclohexylphosphino-2',4',6'-tri-isopropyl-1,1'-biphenyl)(2'-amino-1,1'-biphenyl-2-yl)palladium(II) (150 mg, 0.18 mmol) were added. After reacting under stirring at 60°C for 3 hours, the reaction solution was poured into water (20 mL), followed by extraction with ethyl acetate (20 mL×3). The organic phases were combined. The organic phases were dried over anhydrous sodium sulfate. After filtering off the drying agent, the filtrate was concentrated under reduced pressure. The residue was purified by silica gel column chromatography with an eluant system (dichloromethane/methanol=100/1 to 20/1) to afford compound 11a. MS m/z(ESI):453.3[M-55]⁺.
2) Step 2: Compound 11a (100 mg, 0.20 mmol) was dissolved in dichloromethane (4 mL). Hydrogen chloride/dioxane (4M, 4 mL) was added. After reacting under stirring at room temperature for 1 hour, the reaction solution was directly concentrated under reduced pressure, to afford compound 11b. MS m/z(ESI):409.7[M+1]⁺.
3) Step 3: Compound 11b (55.9 mg, 0.14 mmol) was dissolved in a mixed solution of *N,N-*dimethylformamide (1 mL) and tetrahydrofuran (4 mL). Triethylamine (13.9 mg, 0.14 mmol) was added, followed by reacting under stirring at room temperature for 15 minutes. Then, 1s (60.0 mg, 0.14 mmol), and acetic acid (16.4 mg, 0.27 mmol) were added, followed by reacting under stirring at room temperature for 30 minutes. Then, sodium triacetoxyborohydride (144 mg, 0.68 mmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mM formic acid) and acetonitrile, gradient ratio: acetonitrile 38%-45%, flow rate: 30 mL/min), to afford the formate of compound 11. MS m/z(ESI):416.2[M/2+1]⁺. ¹H NMR (400 MHz, DMSO-*d*₆): δ 11.11 (s, 1H), 9.52 - 9.47 (m, 1H), 9.19 (s, 1H), 8.89 (d, *J* = 2.3 Hz, 1H), 8.64 (d, *J =* 2.3 Hz, 1H), 8.10 (s, 1H), 8.03 (m, 1H), 7.82 (d, *J =* 4.7 Hz, 1H), 7.62 - 7.35 (m, 3H), 7.15 - 7.08 (m, 1H), 7.07 - 7.01 (m, 2H), 6.92 - 6.88 (m, 1H), 5.44 - 5.39 (m, 1H), 4.96 - 4.86 (m, 1H), 4.25 - 4.16 (m, 1H), 3.06 (s, 3H), 3.02 - 2.97 (m, 2H), 2.94 - 2.87 (m, 1H), 2.79 - 2.70 (m, 1H), 2.67 - 2.60 (m, 1H), 2.27 - 2.20 (m, 2H), 2.14 - 1.96 (m, 13H), 1.76 - 1.67 (m, 1H), 1.28 - 1.16 (m, 2H).

### Example 12

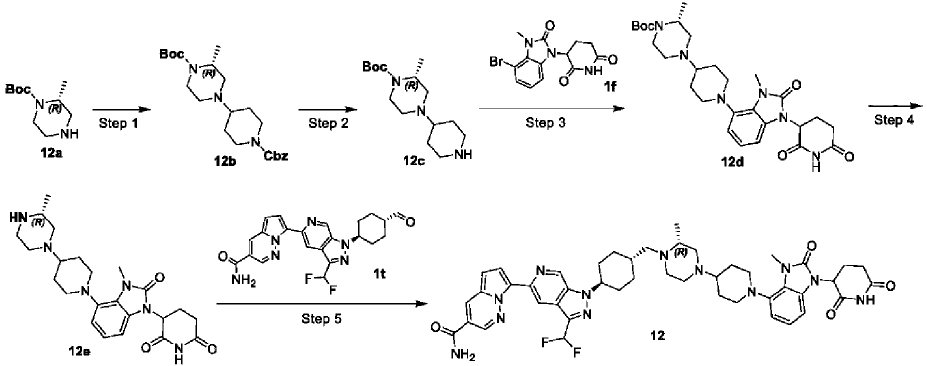

1) Step 1: At room temperature, compound 1-benzyloxycarbonyl-4-piperidone (5.00 g, 21.4 mmol), acetic acid (4.90 mL, 85.7 mmol) were added to a solution of compound 12a (5.15 g, 25.7 mmol) in methanol(50 mL), followed by stirring at room temperature for 1 hour. Sodium cyanoborohydride (6.73 g, 107 mmol) was added. After stirring at room temperature for 18 hours, water (1 mL) was added to quench the reaction. The reaction solution was evaporated to dryness under reduced pressure. The residue was purified by column chromatography (dichloromethane/methanol=10/1) to afford compound 12b. MS m/z(ESI):418.7[M+1]⁺.
2) Step 2: At room temperature, wet palladium on carbon (50.0 mg, 10%) was added to a solution of compound 12b (2.00 g, 4.79 mmol) in methanol (30 mL). The system was purged with hydrogen 3 times, followed by reacting under stirring under a condition of hydrogen for 18 hours. The reaction solution was evaporated to dryness under reduced pressure. The residue was used directly in the next reaction without further purification, to afford compound 12c. MS m/z(ESI):284.3 [M+1]⁺.
3) Step 3: At room temperature, compound 1f (600 mg, 1.78 mmol), 2-dicyclohexylphosphino-2',6'-diisopropoxy-1,1'-biphenyl (82.8 mg, 0.18 mmol), methanesulfonato(2-dicyclohexylphosphino-2',6'-diisopropoxy-1,1'-biphenyl)(2-amino-1,1'-biphenyl-2-yl)palladium(II) (148 mg, 0.18 mmol), lithium bis(trimethylsilyl)amide (8.87 mL, 8.87 mmol, 1M tetrahydrofuran solution ) were added to a solution of compound 12c (754 mg, 2.66 mmol) in toluene (10 mL). The system was purged with nitrogen 3 times, followed by reacting under stirring at 80°C for 2 hours. The reaction solution was poured into saturated ammonium chloride solution (20 mL), followed by extraction with dichloromethane (20 mL×3). The organic phases were combined, dried over anhydrous sodium sulfate, filtered, and evaporated to dryness under reduced pressure. The residue was purified by column chromatography (dichloromethane/methanol=20/1) to afford compound 12d. MS m/z(ESI):541.5[M+1]⁺.
4) Step 4: Trifluoroacetic acid (1 mL) was added to a solution containing compound 12d (100.0 mg, 185 µmol) and dichloromethane (2 mL). The resulting mixture was reacted under stirring at 25°C for 1 hour. The reaction solution was concentrated under reduced pressure to afford compound 12e. The product was used directly in the next step without purification.
5) Step 5: Compound 12e (42.2 mg, 0.120 mmol) was dissolved in *N,N-*dimethylformamide (1 mL). Triethylamine (9.69 mg, 0.100 mmol) was added. The reaction solution was stirred at 25°C for 5 minutes. Acetic acid (5.66 mg, 0.100 mmol) and sodium triacetoxyborohydride (101 mg, 0.480 mmol) were added. After stirring for 5 minutes, compound 1t (35.0 mg, 80.0 µmol) was added finally. The reaction solution was stirred for 1 hour. Two drops of water were added to the reaction solution to quench the reaction, followed by filtration to afford the residue. The residue was purified by high performance liquid chromatography (Waters-2545, chromatographic column: SharpSil-T C18,30×150 mm, 5 µm; mobile phase: water (containing 10 mmol/L formic acid) and acetonitrile, gradient ratio: acetonitrile 38%-45%, flow rate: 30 mL/min) to afford compound 12. MS m/z(ESI):863.8[M+1]⁺. ¹H NMR (400 MHz, DMSO-*d*₆): δ 11.08 (s, 1H), 9.48 (s, 1H), 9.18 (s, 1H), 8.89 (d, *J =* 2.3 Hz, 1H), 8.64 (d, *J* = 2.2 Hz, 1H), 8.10 (s, 1H), 7.82 (d, *J =* 4.6 Hz, 1H), 7.70 - 7.29 (m, 2H), 7.04 (d, *J* = 4.7 Hz, 1H), 6.99 - 6.93 (m, 1H), 6.92 - 6.84 (m, 2H), 5.37 - 5.32 (m, 1H), 4.98 - 4.82 (m, 1H), 3.62 (s, 3H), 3.16 - 3.11 (m, 2H), 2.91 - 2.81 (m, 2H), 2.77 - 2.59 (m, 6H), 2.35 - 2.26 (m, 3H), 2.17 - 2.08 (m, 4H), 2.05 - 1.96 (m, 5H), 1.91 - 1.84 (m, 3H), 1.69 - 1.55 (m, 3H), 1.25 - 1.21 (m, 3H), 1.01 (d, *J* = 6.0 Hz, 3H).

### Example 13

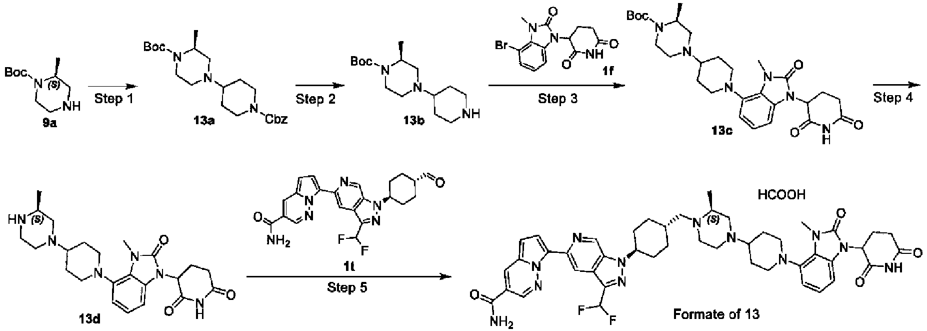

1) Step 1: Compound 9a (4.50 g, 22.5 mmol) and 1-benzyloxycarbonyl-4-piperidone (5.00 g, 21.4 mmol) were dissolved in methanol (80 mL). Acetic acid (2 mL) was added to the reaction solution. After the reaction solution was stirred at 25°C for 1 hour, sodium cyanoborohydride (3.00 g, 62.8 mmol) was added. The reaction solution was stirred for another 2 hours. The reaction solution was poured into water (100 mL), followed by extraction three times with ethyl acetate (80 mL). The organic phases were washed with saturated saline (100 mL), dried over anhydrous sodium sulfate, and filtered. The filtrate was concentrated under reduced pressure. The residue was purified by silica gel column chromatography (dichloromethane/methanol=20/1 to 15/1) to afford compound 13a. MS m/z(ESI):418.7[M+H]⁺.
2) Step 2: Compound 13a (2.80 g, 2.71 mmol) was dissolved in methanol (30 mL). Wet palladium on carbon (200 mg, 10%) was added to the reaction solution. The reaction solution was stirred at 25°C for 18 hours under a hydrogen (1 atm) atmosphere. The reaction solution was filtered. The filtrate was concentrated under reduced pressure to afford compound 13b, which was used directly in the next reaction, without purification. MS m/z(ESI):284.2[M+H]⁺.
3) Step 3: Compound 1f (450 mg, 1.33 mmol), 13b (565.7 mg, 2.01 mmol), 2-dicyclohexylphosphino-2',6'-diisopropoxy-1,1'-biphenyl (124 mg, 0.27 mmol), methanesulfonato(2-dicyclohexylphosphino-2',6'-diisopropoxy-1,1'-biphenyl)(2-amino-1,1'-biphenyl-2-yl)palladium(II) (223 mg, 0.27 mmol) were dissolved in toluene (8mL). Lithium bis(trimethylsilyl)amide (6.65 mL, 6.65 mmol, 1M tetrahydrofuran solution) was added, followed by reacting at 80°C for 1 hour under a nitrogen atmosphere. Water (10 mL) was added to the reaction solution, followed by extraction with dichloromethane (50 mL×3). The organic phases were combined. The organic phases were dried over anhydrous sodium sulfate, and filtered. The filtrate was concentrated under reduced pressure. The residue was purified by silica gel column chromatography with an eluant system (dichloromethane/methanol=20/1) to afford compound 13c. MS m/z(ESI):541.6[M+1]⁺.
4) Step 4: Compound 13c (90 mg, 0.171 mmol) was dissolved in dioxane (0.5 mL). Hydrogen chloride/dioxane (2 mL, 4M) was added. The reaction was carried out at room temperature for 2 hours. The reaction solution was concentrated under reduced pressure, to afford compound 13d. MS m/z(ESI):441.4[M+1]⁺;
5) Step 5: Compound 13d (50.2 mg, 0.111 mmol) was dissolved in *N,N-*dimethylformamide (1 mL). Sodium triacetoxyborohydride (72.5 mg, 0.341 mmol) was added. The reaction solution was stirred at 25°C for 5 minutes, and then 1t (50 mg, 0.111 mmol) was added. The reaction solution was stirred at 25°C for another 30 minutes. The reaction solution was concentrated under reduced pressure. The residue was purified by high performance liquid chromatography (Waters-2545, chromatographic column: SharpSil-T C18,30×150 mm, 5 µm; mobile phase: water (containing 10 mmol/L formic acid) and acetonitrile, gradient ratio: acetonitrile 38%-45%, flow rate: 30 mL/min) to afford the formate of compound 13. MS m/z(ESI):863.8[M+1]⁺. ¹H NMR (400 MHz, DMSO-*d*₆): δ 11.09 (s, 1H), 9.49 (s, 1H), 9.19 (s, 1H), 8.89 (d, *J =* 2.2 Hz, 1H), 8.65 (d, *J* = 2.3 Hz, 1H), 8.11 (s, 1H), 7.83 (d, *J =* 4.7 Hz, 1H), 7.65 - 7.33 (m, 2H), 7.05 (d, *J =* 4.7 Hz, 1H), 6.97 (d, *J =* 7.8 Hz, 1H), 6.93 - 6.84 (m, 2H), 5.41 - 5.32 (m, 1H), 4.98 - 4.86 (m, 1H), 3.63 (s, 3H), 3.18 - 3.12 (m, 2H), 2.94 - 2.84 (m, 2H), 2.81 - 2.55 (m, 7H), 2.39 - 2.23 (m, 4H), 2.19 - 2.08 (m, 4H), 2.05 - 1.96 (m, 4H), 1.95 - 1.83 (m, 4H), 1.66 - 1.58 (m, 2H), 1.32 - 1.22 (m, 1H), 1.19 - 1.07 (m, 1H), 1.01 (d, *J* = 6.1 Hz, 3H).

### Example 14

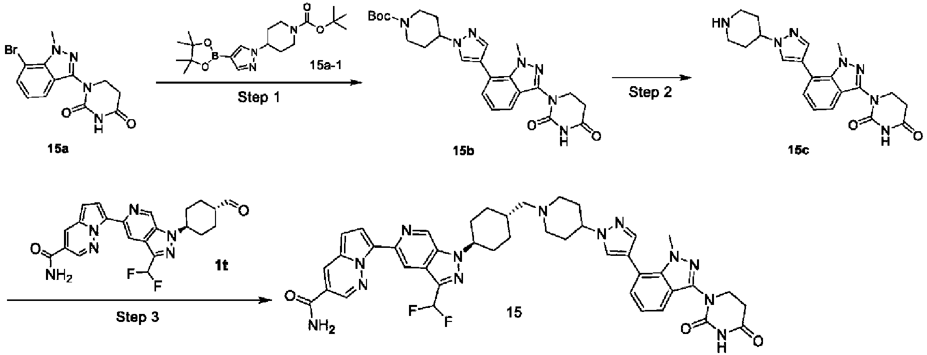

1) Step 1: Compound 15a (100 mg, 309 µmol, prepared using the method disclosed for product B25 of step 5 on page 209 of the specification of patent application "WO2023083194 A1") was dissolved in a mixed solution of dioxane (4 mL) and water (0.5 mL). 15a-1 (234 mg, 618 µmol), potassium carbonate (128 mg, 927 µmol) and 1,1-bis(diphenylphosphino)ferrocenedichloropalladium (II) (22.6 mg, 30.9 µmol) were added, followed by purging with nitrogen three times. After reacting under stirring at 100°C for 2 hours, the reaction solution was poured into water (20 mL), followed by extraction with ethyl acetate (20 mL×3). The organic phases were combined. The organic phases were dried over anhydrous sodium sulfate. After filtering off the drying agent, the filtrate was concentrated under reduced pressure. The residue was purified by silica gel column chromatography with an eluant system (dichloromethane/methanol=100/1 to 20/1) to afford compound 15b. MS m/z(ESI):516.3[M+23]⁺.
2) Step 2: Compound 15b (100 mg, 203 µmol) was dissolved in dichloromethane (4 mL). Hydrogen chloride/dioxane (4 mL, 4 M) was added. After reacting under stirring at room temperature for 1 hour, the reaction solution was directly concentrated under reduced pressure, to afford compound 15c, which was used directly as the raw material for the next step, without purification. MS m/z(ESI):394.6[M+1]⁺.
3) Step 3: Compound 15c (44.9 mg, 114 µmol) was dissolved in a mixed solution of *N,N-*dimethylformamide (1 mL) and tetrahydrofuran (4 mL). Triethylamine (11.5 mg, 114 µmol) was added, followed by reacting under stirring at room temperature for 15 minutes. Then, 1t (50.0 mg, 114 µmol), acetic acid (13.7 mg, 228 µmol) were added, followed by reacting under stirring at room temperature for 30 minutes. Then, sodium triacetoxyborohydride (120 mg, 570 µmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min), to afford compound 15. MS m/z(ESI):816.6[M+1]⁺. ¹H NMR (400 MHz, DMSO-*d*₆): δ 10.57 (s, 1H), 9.50 - 9.48 (m, 1H), 9.19 (s, 1H), 8.89 (d, *J* = 2.3 Hz, 1H), 8.64 (d, *J* = 2.3 Hz, 1H), 8.16 - 8.03 (m, 2H), 7.89 - 7.78 (m, 1H), 7.67 (s, 1H), 7.63 - 7.34 (m, 3H), 7.28 - 7.20 (m, 1H), 7.18 - 7.10 (m, 1H), 7.07 - 7.01 (m, 1H), 5.05 - 4.81 (m, 1H), 4.33 - 4.17 (m, 1H), 3.90 (t, *J =* 6.7 Hz, 2H), 3.72 (s, 3H), 3.12 - 2.93 (m, 2H), 2.78 (t, *J =* 6.7 Hz, 2H), 2.29 - 2.21 (m, 2H), 2.16 - 1.93 (m, 12H), 1.79 - 1.66 (m, 1H), 1.38 - 1.08 (m, 2H).

### Example 15

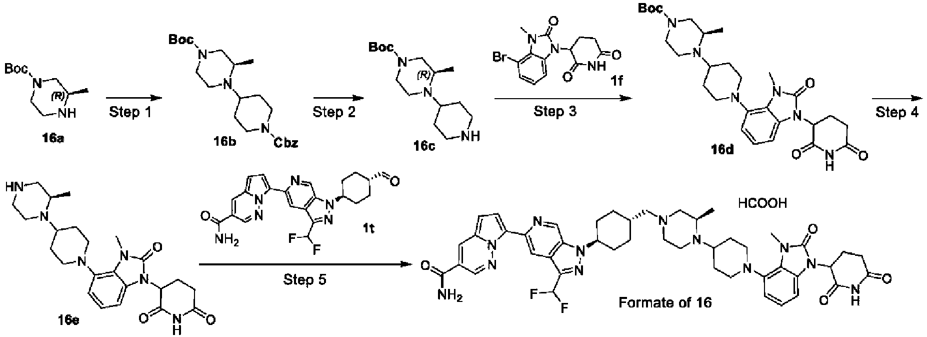

1) Step 1: Compound 1-Cbz-4-piperidone (5.00 g, 21.4 mmol), compound 16a (5.15 g, 25.7 mmol), and anhydrous methanol (100 mL), acetic acid (1.21 mL, 21.4 mmol) were added to a single-necked flask, followed by stirring until dissolution. Sodium cyanoborohydride (2.69 g, 42.8 mmol) was added. The reaction solution was stirred at 45°C for18 hours. Sodium hydroxide aqueous solution (1M) was added dropwise to the reaction solution to adjust the pH to about 7, followed by extraction with ethyl acetate (200 mL×3). The organic phases were combined, washed with saturated saline (200 mL), dried over anhydrous sodium sulfate, and filtered. The filtrate was evaporated to dryness under reduced pressure. The residue was purified by reversed-phase (acetonitrile/ formic acid aqueous solution (0.1%) =0-100%) to afford compound 16b. MS m/z(ESI):418.4 [M+1]⁺.
2) Step 2: Compound 16b (1.50 g, 3.59 mmol), and anhydrous methanol (30 mL) were added to a single-necked flask, followed by stirring until dissolution. Wet palladium on carbon (150 mg, 10%) was added. The reaction system was purged with hydrogen three times, followed by stirring at room temperature for 18 hours under a hydrogen atmosphere(15 psi). The reaction solution was filtered through celite. The filter cake was washed with methanol (10 mL×3). The filtrates were combined, and evaporated to dryness under reduced pressure, to afford compound 16c. MS m/z(ESI):284.2 [M+1]⁺.
3) Step 3: Compound 16c (1.02 g, 3.59 mmol), compound 1f (950 mg, 2.81 mmol, prepared using the method disclosed for the product of step 3 on page 88 of the specification of patent application "WO2022012623 A"), 2-dicyclohexylphosphino-2',6'-diisopropoxy-1,1'-biphenyl (0.26 g, 0.56 mmol), methanesulfonato(2-dicyclohexylphosphino-2',6'-diisopropoxy-1,1'-biphenyl)(2-amino-1,1'-biphenyl-2-yl)palladium(II) (0.47 g, 0.56 mmol) and anhydrous toluene (20 mL) were added to a three-necked flask. The reaction system was purged with nitrogen three times. Lithium bis(trimethylsilyl)amide (14.1mL, 14.1mmol, 1M tetrahydrofuran solution) was added. The reaction solution was reacted at 80°C for 2 hours. After the reaction was completed, the reaction solution was poured into water (100 mL), followed by extraction three times with ethyl acetate (100 mL). The organic phases were combined, washed with saturated saline (100 mL), dried over anhydrous sodium sulfate, filtered, and evaporated to dryness under reduced pressure. The residue was separated by silica gel column chromatography (dichloromethane/methanol=100/1 to 20/1), to afford compound 16d. MS m/z(ESI):541.4 [M+1]⁺.
4) Step 4: Trifluoroacetic acid (1 mL) was added to a solution containing compound 16d (100.0 mg, 185 µmol) and dichloromethane (2 mL), followed by reacting under stirring at 25°C for 1 hour. The reaction solution was concentrated under reduced pressure to afford compound 16e. The product was used directly in the next step without purification. MS m/z(ESI):441.3 [M+1]⁺.
5) Step 5: Triethylamine (0.20 mL, 1.44 mmol) was added to a solution containing compound 16e (50.0 mg, 110 µmol), tetrahydrofuran (2 mL), and N,N-dimethylformamide (1 mL), followed by stirring for 30 minutes. Then, compound 1t (60.0 mg, 137 µmol) and acetic acid (0.5 mL) were added to the above solution, followed by stirring for 30 minutes. Finally, sodium triacetoxyborohydride (73.0 mg, 340 µmol) was added to the above reaction solution. Then, the resulting mixture was stirred for 2 hours under a condition of 25°C. The reaction solution was concentrated under reduced pressure. The residue was purified by high performance liquid chromatography (A: 0.1% FA/H₂O B:ACN, chromatographic column: Waters-SunFire-C18-10 µm-19×250 mm; mobile phase: water (containing 0.1% formic acid) and acetonitrile, gradient ratio: acetonitrile 32%-48%, flow rate: 25mL/min)to afford the formate of compound 16. MS m/z(ESI):863.8 [M+1]⁺. ¹H NMR (400 MHz, DMSO-*d₆*) δ 11.09 (s, 1H), 9.50 (d, *J* = 1.3 Hz, 1H), 9.19 (d, *J* = 1.2 Hz, 1H), 8.89 (d, *J* = 2.3 Hz, 1H), 8.65 (d, *J* = 2.3 Hz, 1H), 7.85 - 7.81 (m, 1H), 7.64 - 7.33 (m, 2H), 7.05 (d, *J =* 4.7 Hz, 1H), 7.01 - 6.85 (m, 3H), 5.38 - 5.33 (m, 1H), 4.96 - 4.86 (m, 1H), 3.64 (s, 3H), 3.19 - 3.11 (m, 2H), 2.81 - 2.60 (m, 8H), 2.17 - 2.11 (m, 4H), 2.02 - 1.97 (m, 4H), 1.89 - 1.73 (m, 3H), 1.73 - 1.55 (m, 3H), 1.51 - 1.39 (m, 1H), 1.27 - 1.19 (m, 6H), 1.10 - 1.01 (m, 3H), 0.92 - 0.81 (m, 1H).

### Example 16

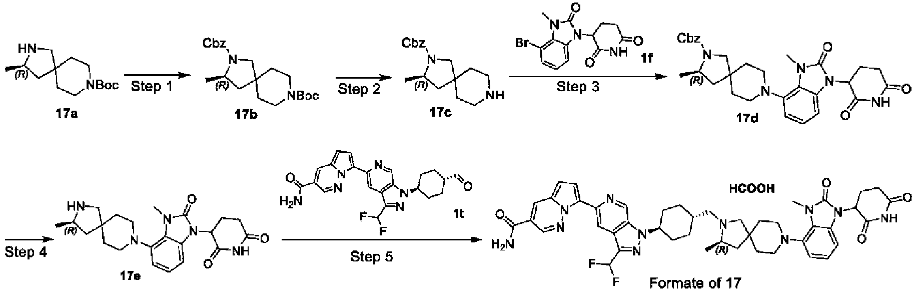

1) Step 1: Compound 17a (2.27 g, 8.92 mmol, using the synthetic method for compound R-3 on page 225 of the specification of patent application "WO2021055756 A1"), anhydrous acetonitrile (20 mL), and water (20 mL) were added to a single-necked flask, followed by stirring until dissolution. Sodium carbonate (2.84 g, 26.8 mmol), and benzyl chloroformate (2.54 mL, 17.9 mmol) were added. The reaction solution was stirred at room temperature for 18 hours. Then, the reaction solution was extracted with ethyl acetate (30 mL×3). The organic phases were combined, washed with saturated saline (30 mL), dried over anhydrous sodium sulfate, and filtered. The filtrate was evaporated to dryness under reduced pressure. The residue was separated by reversed-phase column (acetonitrile/0.1% formic acid aqueous solution = 0-100%), to afford compound 17b. MS m/z(ESI):333.2[M-55]⁺.
2) Step 2: Compound 17b (2.05 g, 5.28 mmol) was dissolved in hydrogen chloride/dioxane solution (4.0 M, 20 mL), followed by stirring at 25°C for 1 hour. The reaction mixture was concentrated under reduced pressure, to afford compound 17c. MS m/z(ESI):289.6[M+H]⁺.
3) Step 3: Compound 1f (1.20 g, 3.55 mmol), compound 17c (1.54 g, 5.32 mmol), 2-dicyclohexylphosphino-2',6'-diisopropoxy-1,1'-biphenyl (170 mg, 0.350 mmol, Bide), and methanesulfonato(2-dicyclohexylphosphino-2',6'-diisopropoxy-1,1'-biphenyl)(2-amino-1,1'-biphenyl-2-yl)palladium(II) (300 mg, 0.350 mmol, Bide) were dissolved in toluene (20 mL). The reaction solution was purged with nitrogen three times at 25°C. Under a nitrogen atmosphere, lithium bis(trimethylsilyl)amide (17.7 mL, 17.7 mmol, 1.0 M tetrahydrofuran solution, Bide) was added. The reaction solution was heated to 80°C, and reacted under stirring for 2 hours. The reaction solution was poured into cooled saturated ammonium chloride aqueous solution (50 mL), followed by diluting with dichloromethane (100 mL). The aqueous phase was separated and extracted with dichloromethane (50 mL×3). The combined organic phase was washed with saturated saline (50 mL), and dried over anhydrous sodium sulfate. After filtering off the drying agent, the filtrate was concentrated under reduced pressure. The residue was purified by silica gel column chromatography (dichloromethane/methanol=100/1 to 50/1), to afford compound 17d. MS m/z(ESI):546.4[M+H]⁺. ¹H NMR (400 MHz, DMSO-*d₆):* δ 11.10 - 11.07 (m, 1H), 7.43 - 7.23 (m, 4H), 7.20 - 6.84 (m, 4H), 5.38 - 5.31 (m, 1H), 5.09 - 5.02 (m, 1H), 3.86 (s, 1H), 3.62 (s, 2H), 3.32 (s, 4H), 3.20 - 2.80 (m, 4H), 2.74 - 2.58 (m, 3H), 2.49 - 2.31 (m, 2H), 2.06 - 1.89 (m, 2H), 1.68 - 1.42 (m, 3H), 1.24 (s, 3H).
4) Step 4: Compound 17d (860 mg, 1.58 mmol) and wet palladium on carbon (168 mg, 10%) were mixed with methanol (15 mL). The mixture was purged with hydrogen three times at 25°C, followed by stirring for 3 hours under a hydrogen atmosphere. The reaction solution was filtered through celite. The filtrate was concentrated under reduced pressure. The residue was purified by silica gel column chromatography (dichloromethane/methanolic ammonia solution (7.0 M)=100/1 to 10/1), to afford compound 17e. MS m/z(ESI):412.3[M+H]⁺. ¹H NMR (400 MHz, CDCl₃) δ 7.02 - 6.96 (m, 1H), 6.93 - 6.88 (m, 1H), 6.56 (d, *J* = 7.2 Hz, 1H), 5.23 - 5.02 (m, 1H), 3.77 (s, 3H), 3.30 - 3.23 (m, 1H), 3.05 (s, 2H), 2.87 - 2.72 (m, 4H), 2.47 - 2.34 (m, 4H), 2.24 - 2.19 (m, 1H), 1.90 - 1.73 (m, 3H), 1.71 - 1.55 (m, 2H), 1.21 (s, 3H).
5) Step 5: Compound 17e (70.1 mg, 0.171 mmol) was dissolved in *N,N-*dimethylformamide (2 mL). Sodium triacetoxyborohydride (180.1 mg, 0.851 mmol, Bide) was added. The reaction solution was stirred at 25°C for 5 minutes, and then 1t (74.5 mg, 0.171 mmol) was added. The reaction solution was stirred at 25°C for another 30 minutes. The reaction solution was concentrated under reduced pressure. The residue was purified by high performance liquid chromatography (Waters-2545, chromatographic column: SharpSil-T C18, 30×150 mm, 5 µm; mobile phase: water (containing 10 mmol/L formic acid) and acetonitrile, gradient ratio: acetonitrile 38%-45%, flow rate: 30 mL/min) to afford a formate of compound 17. MS m/z(ESI):832.8[M-1]⁻;¹H NMR (400 MHz, DMSO-*d*₆): δ 11.09 (s, 1H), 9.50 (s, 1H), 9.20 (s, 1H), 8.89 (d, *J* = 2.*2* Hz, 1H), 8.65 (d, *J* = 2.*2* Hz, 1H), 8.15 (s, 1H), 8.12 (s, 1H), 7.83 (d, *J* = 4.7 Hz, 1H), 7.66 - 7.32 (m, 2H), 7.07 - 6.85 (m, 4H), 5.41 - 5.30 (m, 1H), 4.99 - 4.86 (m, 1H), 3.65 (s, 3H), 3.08 - 2.96 (m, 2H), 2.95 - 2.76 (m, 3H), 2.75 - 2.58 (m, 4H), 2.27 - 2.06 (m, 5H), 2.06 - 1.82 (m, 6H), 1.81 - 1.66 (m, 4H), 1.61 - 1.27 (m, 3H), 1.25 - 1.05 (m, 4H).

### Example 17

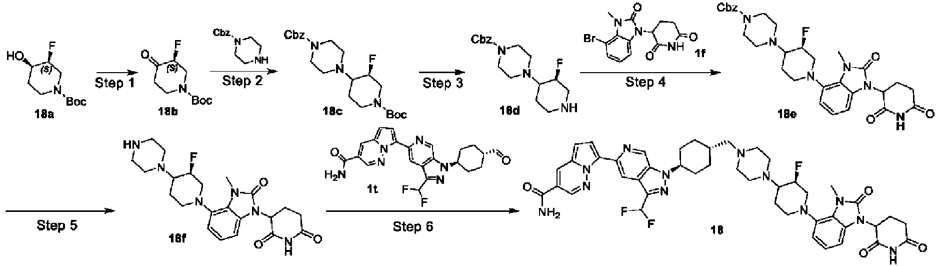

1) Step 1: Compound 18a (3.00 g, 13.7 mmol, Energy) was dissolved in dichloromethane (35 mL), followed by cooling to 0°C. Dess-Martin Periodinane (8.70 g, 20.5 mmol) was added, followed by reacting under stirring at room temperature for 16 hours. Then, the reaction system was poured into sodium bicarbonate aqueous solution (100 mL), followed by extraction with dichloromethane (50 mL×3). The organic phases were combined, dried over anhydrous sodium sulfate, and filtered. The filtrate was concentrated under reduced pressure. The residue was purified by silica gel column chromatography (petroleum ether/ethyl acetate=100/1 to 4/1), to afford compound 18b. ¹H NMR (400 MHz, DMSO-*d*₆): δ 5.18 - 5.02 (m, 1H), 4.36 - 4.25 (m, 1H), 4.06 - 3.98 (m, 1H), 3.30 - 3.18 (m, 2H), 2.60 - 2.54 (m, 1H), 2.40 - 2.35 (m, 3H), 1.45 (s, 9H).
2) Step 2: Compound 18b (2.00 g, 9.21 mmol) was dissolved in 1,2-dichloroethane (40 mL). Benzyl-1-piperazinecarbonate (4.06 g, 18.4 mmol) and acetic acid (1.11 g, 18.4 mmol) were added, followed by reacting under stirring at room temperature for 1 hour. Sodium triacetoxyborohydride (3.90 g, 18.4 mmol) was added, followed by reacting under stirring at room temperature for 16 hours. The reaction system was poured into sodium bicarbonate aqueous solution (100 mL), followed by extraction with dichloromethane (50 mL×3). The organic phases were combined, dried over anhydrous sodium sulfate, and filtered. The filtrate was concentrated under reduced pressure. The residue was purified by silica gel column chromatography with an eluant system (petroleum ether/ethyl acetate=100/1 to 1/1)to afford compound 18c. MS m/z(ESI):422.7[M+1]⁺.
3) Step 3: Compound 18c (1.60 g, 3.80 mmol) was dissolved in dichloromethane (10 mL). Hydrogen chloride/dioxane (4 M, 10 mL) was added. After reacting under stirring at room temperature for 1 hour, the reaction solution was directly concentrated under reduced pressure, to afford compound 18d, followed by using directly in the next reaction, without purification. MS m/z(ESI):322.6[M+1]⁺.
4) Step 4: Compound 1f (300 mg, 887 µmol), 18d (570 mg, 1.77 mmol), 2-dicyclohexylphosphino-2',6'-diisopropoxy-1,1'-biphenyl (41.4 mg, 88.7 µmol), and methanesulfonato(2-dicyclohexylphosphino-2',6'-diisopropoxy-1,1'-biphenyl)(2-amino-1,1'-biphenyl-2-yl)palladium(II) (74.2 mg, 88.7 µmol) were dissolved in toluene (15 mL). Under nitrogen protection, lithium bis(trimethylsilyl)amide (4.44 mL, 4.44 mmol, 1.0M tetrahydrofuran solution, Energy) was added dropwise. The reaction solution was stirred at 80°C for 2 hours. The reaction solution was cooled to room temperature. Water (40 mL) was added. The aqueous phase was extracted with ethyl acetate (30 mL×3). The combined organic phase was washed with saline (15 mL), dried over anhydrous sodium sulfate, and filtered. The filtrate was concentrated under reduced pressure. The residue was purified by silica gel column chromatography (dichloromethane/methanol=100/1 to 20/1), to afford compound 18e. MS m/z(ESI):579.4[M+1]⁺.
5) Step 5: Compound 18e (100 mg, 173 µmol) was dissolved in methanol (10 mL). Palladium on carbon (10%, 100 mg) was added, followed by purging with hydrogen three times, and stirring for 18 hours under a hydrogen (1 atm) atmosphere. The reaction system was filtered. The organic phases were concentrated. The residue was used directly as the raw material for the next step, without purification, to afford compound 18f. MS m/z(ESI):445.5[M+1]⁺.
6) Step 6: Compound 18f (30.4 mg, 68.4 µmol) was dissolved in a mixed solution of *N,N-*dimethylformamide (1 mL) and tetrahydrofuran (4 mL). 1t (30.0 mg, 68.4 µmol), and acetic acid (8.22 mg, 137 µmol) were added, followed by reacting under stirring at room temperature for 30 minutes. Then, sodium triacetoxyborohydride (72.2 mg, 342 µmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to afford compound 18. MS m/z(ESI):867.8[M+1]⁺. ¹H NMR (400 MHz, DMSO-*d*₆): δ 11.09 (s, 1H), 9.54 - 9.45 (m, 1H), 9.28 - 9.15 (m, 1H), 8.89 (d, *J* = 2.3 Hz, 1H), 8.64 (d, *J* = 2.3 Hz, 1H), 8.10 (s, 1H), 7.89 - 7.78 (m, 1H), 7.67 - 7.34 (m, 2H), 7.09 - 7.02 (m, 1H), 7.02 - 6.88 (m, 3H), 5.42 - 5.29 (m, 1H), 5.16 - 4.99 (m, 1H), 4.96 - 4.84 (m, 1H), 3.65 (s, 3H), 3.21 - 2.78 (m, 5H), 2.75 - 2.58 (m, 5H), 2.48 - 2.31 (m, 4H), 2.28 - 1.92 (m, 11H), 1.82 - 1.62 (m, 2H), 1.27 - 1.11 (m, 3H).

### Example 18

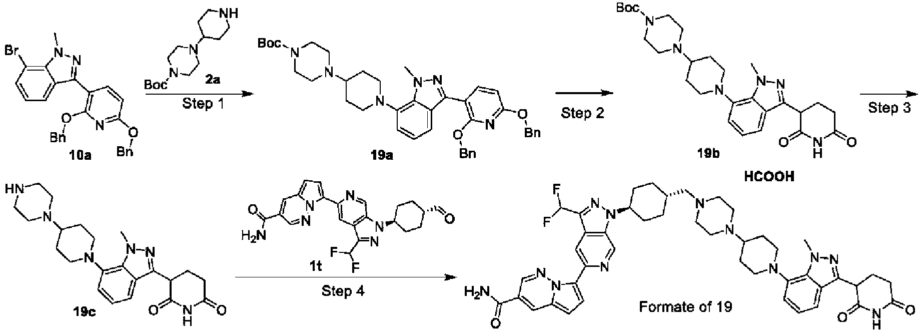

1) Step 1: At room temperature, compound 10a (800 mg, 1.60 mmol), toluene (5 mL) were added to a single-necked flask under stirring. Compound 2a (862 mg, 3.20 mmol), tris(dibenzylideneacetone)dipalladium (147 mg, 0.16 mmol), sodium tert-butoxide (460.0 mg, 4.80 mmol), and 1,1'-binaphthyl-2,2'-bis(diphenylphosphine) (100.0 mg, 0.16 mmol) were added. The reaction solution was stirred at 80°C for 6 hours under a nitrogen atmosphere. Water (10 mL) was added to the reaction solution, followed by extraction with ethyl acetate (20 mL×3). The organic phases were combined, washed with saturated saline (20 mL), dried over anhydrous sodium sulfate, and filtered. The filtrate was concentrated under reduced pressure. The residue was purified by column chromatography (0-5% dichloromethane/methanol) to afford compound 19a. MS m/z(ESI):690.0[M+1]⁺.
2) Step 2: At room temperature, palladium on carbon 10% (20 mg) was added to a solution containing compound 19a (120.0 mg, 170 µmol), ethyl acetate (2 mL), and ethanol (2 mL). After purging with hydrogen three times, the reaction was carried out for 16 hours under a condition of 25°C. After filtration, the filtrate was concentrated under reduced pressure. The residue was purified by silica gel column chromatography (dichloromethane/methanol=100/1 to 30/1) to afford compound 19b.
3) Step 3: Trifluoroacetic acid (1 mL) was added to a solution containing compound 19b (80.0 mg, 160 µmol) and dichloromethane (2 mL). The resulting mixture was reacted under stirring at 25°C for 1 hour. The reaction solution was concentrated under reduced pressure to afford crude compound 19c. MS m/z(ESI):411.7[M+1]⁺.
4) Step 4: Triethylamine (0.30 mL, 2.15 mmol) was added to a solution containing compound 19c (50.0 mg, 120 µmol), tetrahydrofuran (2 mL), and *N,N*-dimethylformamide (1 mL), followed by stirring for 30 minutes. Then, compound 1t (81.0 mg, 180 µmol) and acetic acid (0.5 mL) were added to the above solution, followed by stirring for 30 minutes. Finally, sodium triacetoxyborohydride (77.0 mg, 360 µmol) was added, Then, the resulting mixture was stirred for 2 hours under a condition of 25°C. The reaction solution was concentrated under reduced pressure and purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30mL/min) to afford the formate of compound 19. MS m/z(ESI):833.7[M+1]⁺. ¹H NMR (400 MHz, DMSO-*d₆*) δ 10.89 (s, 1H), 9.50 (d, *J* = 1.3 Hz, 1H), 9.19 (d, *J* = 1.3 Hz, 1H), 8.89 (d, *J* = 2.3 Hz, 1H), 8.65 (d, *J* = 2.3 Hz, 1H), 8.35 (s, 1H), 7.83 (d, *J* = 4.7 Hz, 1H), 7.65 - 7.34 (m, 3H), 7.05 - 7.01 (m, 3H), 4.95 - 4.89 (m, 1H), 4.37 - 4.31 (m, 1H), 4.25 (s, 3H), 3.29 - 3.26 (m, 1H), 2.73 - 2.55 (m, 8H), 2.44 - 2.30 (m, 6H), 2.25 - 2.10 (m, 6H), 2.08 - 1.95 (m, 6H), 1.75 - 1.62 (m, 3H), 1.51 - 1.39 (m, 1H), 0.93 - 0.82 (m, 1H).

### Example 19

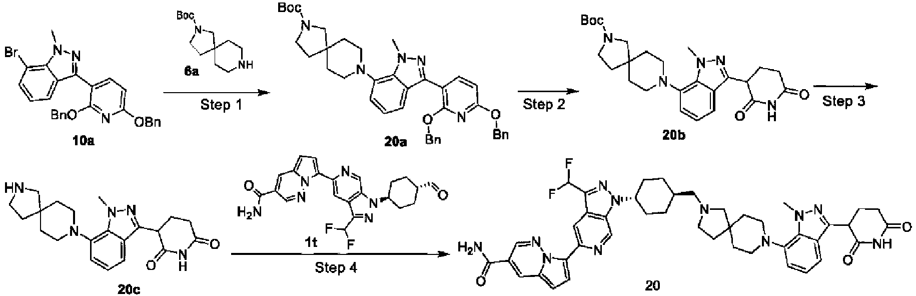

1) Step 1: Tris(dibenzylideneacetone)dipalladium (64 mg, 0.070 mmol) was added to a mixture containing compound 10a (350 mg, 0.70 mmol), compound 6a (252 mg, 1.05 mmol), 1,1'-binaphthyl-2,2'-bis(diphenylphosphine) (44 mg, 0.07 mmol), sodium tert-butoxide (134 mg, 1.40 mmol), and toluene (20 mL), followed by purging with nitrogen 3 times, and then stirring at 80°C for 18 hours under a condition of nitrogen protection. Water (20 mL) was added to the reaction mixture, followed by extraction with ethyl acetate (30 mL×3). The organic phases were combined, dried, filtered, and concentrated under reduced pressure. The residue was purified by silica gel column chromatography (petroleum ether/ethyl acetate=100/1 to 10/1) to afford compound 20a. MS m/z(ESI):660.6[M+1]+. ¹H NMR (400 MHz, DMSO-*d₆*) δ 7.85 (d, *J* = 8.0 Hz, 1H), 7.51 - 7.45 (m, 2H), 7.43 - 7.23 (m, 10H), 6.96 - 6.90 (m, 1H), 6.59 (d, *J* = 8.0 Hz, 1H), 5.51 - 5.35 (m, 4H), 4.33 (s, 3H), 3.26 (s, 2H), 3.22 - 3.04 (m, 3H), 2.89 - 2.66 (m, 2H), 2.58 - 2.52 (m, 1H), 1.91 - 1.59 (m, 6H), 1.42 (s, 9H).
2) Step 2: Under nitrogen protection, at room temperature, palladium on carbon (10 mg, 10%) was added to a solution containing compound 20a (90 mg, 0.14 mmol), ethanol (2 mL), and ethyl acetate (2 mL), followed by purging with hydrogen three times, and then stirring at 25°C for 18 hours under a condition of hydrogen (15 psi). The reaction solution was filtered. The filter cake was washed twice with ethyl acetate (10 mL). The filtrate was concentrated under reduced pressure to afford compound 20b. MS m/z(ESI):482.4[M+1]⁺.
3) Step 3: Compound 20b (65 mg, 0.13 mmol) was mixed with hydrogen chloride/dioxane (3 mL, 4 M), followed by reacting under stirring at 25°C for 2 hours. The reaction solution was concentrated under reduced pressure, to afford compound 20c. MS m/z(ESI):382.6[M+1]⁺;
4) Step 4: Compound 20c (39 mg, 0.10 mmol) was dissolved in *N,N-*dimethylformamide (3 mL). Triethylamine (10.4 mg, 0.103 mmol) was added, followed by reacting under stirring at room temperature for 5 minutes. Then, acetic acid (9.2 mg, 0.15 mmol) and sodium triacetoxyborohydride (56 mg, 0.26 mmol) were added, followed by reacting under stirring at room temperature for 10 minutes. Finally, compound 1t (45 mg, 0.103 mmol) was added, followed by reacting under stirring at room temperature for 15 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min), to afford compound 20. MS m/z(ESI):804.7[M+1]⁺. ¹H NMR (400 MHz, DMSO-*d*₆): δ 10.89 (s, 1H), 9.50 (s, 1H), 9.19 (s, 1H), 8.93 - 8.87 (m, 1H), 8.68 - 8.62 (m, 1H), 8.11 (s, 1H), 7.86 - 7.81 (m, 1H), 7.**65** - 7.33 (m, 3H), 7.08 - 6.98 (m, 3H), 4.99 - 4.87 (m, 1H), 4.36 - 4.31 (m, 1H), 4.26 (s, 3H), 3.15 - 3.08 (m, 2H), 2.84 - 2.71 (m, 2H), 2.70 - 2.56 (m, 5H), 2.43 - 2.27 (m, 4H), 2.20 - 2.09 (m, 3H), 2.06 - 1.95 (m, 4H), 1.84 - 1.62 (m, 7H), 1.29 - 1.16 (m, 2H).

### Example 20

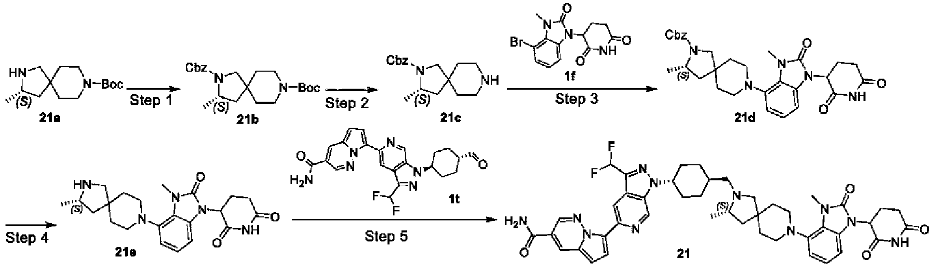

1) Step 1: Compound 21a (3.30 g, 13.0 mmol, prepared using the method disclosed for the product R-3 of step on page 225 of the specification of "WO2021055756A1") was dissolved in a mixed solution of water (15 mL) and acetonitrile (15 mL). Sodium carbonate (4.13 g, 38.9 mmol) was added, followed by cooling to 0°C. Benzyl chloroformate (3.69 mL, 26.0 mmol) was added dropwise. After reacting under stirring at room temperature for 5 hours, the reaction solution was poured into water (50 mL). The aqueous phase was extracted with ethyl acetate (30 mL×3). The combined organic phase was washed with saline (50 mL), dried over anhydrous sodium sulfate, and filtered. The filtrate was concentrated under reduced pressure. The residue was purified by thin layer chromatography silica gel plate (dichloromethane/methanol=100/1 to 20/1) to afford compound 21b.
2) Step 2: Compound 21b (1.10 g, 2.83 mmol) was dissolved in dichloromethane (5 mL). Hydrogen chloride/dioxane (5 mL, 4 M) was added. After reacting under stirring at room temperature for 1 hour, the reaction solution was directly concentrated under reduced pressure to afford compound 21c, which was used directly in the next step, without purification. MS m/z(ESI):289.4[M+1]⁺.
3) Step 3: Compound 1f (300 mg, 887 µmol), 21c (512 mg, 1.77 mmol), 2-dicyclohexylphosphino-2',6'-diisopropoxy-1,1'-biphenyl (41.4 mg, 88.7 µmol), and methanesulfonato(2-dicyclohexylphosphino-2',6'-diisopropoxy-1,1'-biphenyl)(2-amino-1,1'-biphenyl-2-yl)palladium(II) (74.2 mg, 88.7 µmol) were dissolved in toluene (15 mL). Under nitrogen protection, lithium bis(trimethylsilyl)amide (4.44 mL, 4.44 mmol, 1.0 M tetrahydrofuran solution, Energy) was added dropwise. The reaction solution was stirred at 80°C for 2 hours. The reaction solution was cooled to room temperature. Water (40 mL) was added, followed by extraction with ethyl acetate (30 mL×3). The combined organic phase was washed with saturated saline(15 mL), dried over anhydrous sodium sulfate, and filtered. The filtrate was concentrated under reduced pressure. The residue was purified by thin layer chromatography silica gel plate (dichloromethane/methanol=20/1) to afford compound 21d. MS m/z(ESI):546.4[M+1]⁺.
4) Step 4: Compound 21d (60 mg, 110 µmol) was dissolved in methanol (5 mL). Palladium on carbon (10%, 60 mg) was added, followed by purging with hydrogen three times, and stirring for 3 hours under a hydrogen (1 atm) atmosphere. The reaction system was filtered. The organic phases were concentrated. The residue was used directly as the raw material for the next step, without purification, to afford compound 21e. MS m/z(ESI):412.4[M+1]⁺.
5) Step 5: Compound 21e (28.2 mg, 68.4 µmol) was dissolved in a mixed solution of *N,N-*dimethylformamide (1 mL) and tetrahydrofuran (4 mL). Compound 1t (30.0 mg, 68.4 µmol), and acetic acid (8.22 mg, 137 µmol) were added, followed by reacting under stirring at room temperature for 30 minutes. Then, sodium triacetoxyborohydride (72.3 mg, 342 µmol) was added, followed by reacting under stirring at room temperature for 30 minutes. The reaction system was directly concentrated. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min), to afford compound 21. MS m/z(ESI):833.7[M+1]⁺. ¹H NMR (400 MHz, DMSO-*d*₆): δ 11.08 (s, 1H), 9.55 - 9.46 (m, 1H), 9.21 - 9.15 (m, 1H), 8.89 (d, *J* = 2.3 Hz, 1H), 8.64 (d, *J =* 2.3 Hz, 1H), 8.10 (s, 1H), 7.87 - 7.79 (m, 1H), 7.61 - 7.35 (m, 2H), 7.06 - 7.02 (m, 1H), 7.00 - 6.85 (m, 2H), 5.46 - 5.27 (m, 1H), 4.97 - 4.82 (m, 1H), 3.69 - 3.61 (m, 3H), 3.45 (s, 1H), 3.07 - 2.82 (m, 4H), 2.78 - 2.57 (m, 3H), 2.48 - 2.26 (m, 2H), 2.23 - 1.58 (m, 17H), 1.35 - 1.22 (m, 2H), 1.12 - 0.99 (m, 3H).

### Example 21

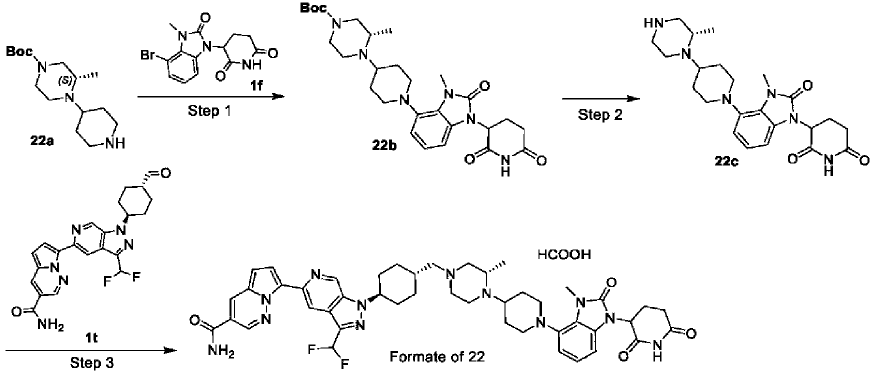

1) Step 1: Compound 22a (440 mg, 1.55 mmol, prepared using the method disclosed for the product of Preparation 23 on page 45 of the specification of patent application "WO2013163262A1"), compound 1f (350 mg, 1.04 mmol, prepared using the method disclosed for the product of step 3 on page 88 of the specification of patent application "WO2022012623 A"), 2-dicyclohexylphosphino-2',6'-diisopropoxy-1,1'-biphenyl (100 mg, 0.214 mmol), methanesulfonato(2-dicyclohexylphosphino-2',6'-diisopropoxy-1,1'-biphenyl)(2-amino-1,1'-biphenyl-2-yl)palladium(II) (175 mg, 0.209 mmol), and toluene (10 mL) were added to a three-necked flask. The reaction system was purged with nitrogen three times. Lithium bis(trimethylsilyl)amide (5.20 mL, 5.20 mmol, 1M tetrahydrofuran solution) was added. The reaction solution was reacted at 80°C for 2 hours. Then, the reaction solution was poured into water (30 mL), followed by extraction with ethyl acetate (30 mL×3). The organic phases were combined, washed with saline (50 mL), dried over anhydrous sodium sulfate, and filtered. The filtrate was concentrated under reduced pressure. The residue was separated by silica gel column chromatography (dichloromethane/methanol=20/1 to 10/1), to afford compound 22b. MS m/z(ESI):541.6 [M+1]⁺.
2) Step 2: Compound 22b (250 mg, 0.462 mmol) was dissolved in dichloromethane (3 mL), and trifluoroacetic acid (1 mL) was added. After reacting under stirring at 25°C for 1 hour, the reaction solution was concentrated under reduced pressure to afford compound 22c. The product was used directly in the next step without purification. MS m/z(ESI):441.5 [M+1]⁺.
3) Step 3: Compound 22c (150 mg, 0.340 mmol) and compound 1t (150 mg, 0.342 mmol) were dissolved in *N,N*-dimethylformamide (5 mL). Acetic acid (0.5 mL) and sodium triacetoxyborohydride (300 mg, 1.42 mmol) were added sequentially. The reaction solution was stirred at 25°C for 2 hours. The reaction solution was poured into water (30 mL), followed by extraction with ethyl acetate (30 mL×3). The combined organic phase was washed with saturated saline (50 mL), dried over anhydrous sodium sulfate, and filtered. The filtrate was concentrated under reduced pressure. The residue was purified by high performance liquid chromatography (A: 0.1% FA/H₂O B:ACN, chromatographic column: Waters-SunFire-C18-10 µm-19×250 mm; mobile phase: water (containing 0.1% formic acid) and acetonitrile, gradient ratio: acetonitrile 32%-48%, flow rate: 25 mL/min) to afford the formate of compound 22. MS m/z(ESI):863.8 [M+1]⁺. ¹H NMR (400 MHz, DMSO-*d₆*) δ 11.09 (s, 1H), 9.49 (s, 1H), 9.19 (s, 1H), 8.89 (d, *J* = 2.2 Hz, 1H), 8.65 (d, *J =* 2.2 Hz, 1H), 8.11 (s, 1H), 7.83 (d, *J* = 4.6 Hz, 1H), 7.62 - 7.37 (m, 2H), 7.05 (d, *J* = 4.7 Hz, 1H), 7.00 - 6.94 (m, 1H), 6.93 - 6.89 (m, 1H), 6.89 - 6.85 (m, 1H), 5.40 - 5.31 (m, 1H), 4.96 - 4.86 (m, 1H), 3.64 (s, 3H), 3.18 - 3.12 (m, 2H), 2.97 - 2.75 (m, 5H), 2.74 - 2.52 (m, 6H), 2.23 - 2.08 (m, 5H), 2.05 - 1.94 (m, 6H), 1.87 - 1.59 (m, 5H), 1.24 - 1.15 (m, 2H), 1.06 (d, *J=* 6.1 Hz, 3H).

### Biological test:

### Experimental Example 1: detection of proteolysis by Hibit fluorescence

Using the HiBiT tag technology, the sequence containing the HiBiT tag (Seq. ID. No. 1, 5'-GTGAGCGGCTGGCGGCTGTTCAAGAAGATTAGC-3', Beijing Tsingke Biotech Co., Ltd.) was ligated to the IRAK4 plasmid (Seq. ID. No. 2, 5'-EcoRIGCCACCATGAACAAACCCATAACACCATCAACATATGTGCGCTGCCTCAATG TTGGACTAATTAGGAAGCTGTCAGATTTTATTGATCCTCAAGAAGGATGGAAGAAG TTAGCTGTAGCTATTAAAAAACCATCTGGTGATGATAGATACAATCAGTTTCACATA AGGAGATTTGAAGCATTACTTCAAACTGGAAAAAGTCCCACTTCTGAATTACTGTT TGACTGGGGCACCACAAATTGCACAGTTGGTGATCTTGTGGATCTTTTGATCCAAA ATGAATTTTTTGCTCCTGCGAGTCTTTTGCTCCCAGATGCTGTTCCCAAAACTGCTA ATACACTACCTTCTAAAGAAGCTATAACAGTTCAGCAAAAACAGATGCCTTTCTGT GACAAAGACAGGACATTGATGACACCTGTGCAGAATCTTGAACAAAGCTATATGC CACCTGACTCCTCAAGTCCAGAAAATAAAAGTTTAGAAGTTAGTGATACACGTTTT CACAGTTTTTCATTTTATGAATTGAAGAATGTCACAAATAACTTTGATGAACGACCC ATTTCTGTTGGTGGTAATAAAATGGGAGAGGGAGGATTTGGAGTTGTATATAAAGG CTACGTAAATAACACAACTGTGGCAGTGAAGAAGCTTGCAGCAATGGTTGACATTA CTACTGAAGAACTGAAACAGCAGTTTGATCAAGAAATAAAAGTAATGGCAAAGTG TCAACATGAAAACTTAGTAGAACTACTTGGTTTCTCAAGTGATGGAGATGACCTCT GCTTAGTATATGTTTACATGCCTAATGGTTCATTGCTAGACAGACTCTCTTGCTTGGA TGGTACTCCACCACTTTCTTGGCACATGAGATGCAAGATTGCTCAGGGTGCAGCTA ATGGCATCAATTTTCTACATGAAAATCATCATATTCATAGAGATATTAAAAGTGCAA ATATCTTACTGGATGAAGCTTTTACTGCTAAAATATCTGACTTTGGCCTTGCACGGG CTTCTGAGAAGTTTGCCCAGACAGTCATGACTAGCAGAATTGTGGGAACAACAGC TTATATGGCACCAGAAGCTTTGCGTGGAGAAATAACACCCAAATCTGATATTTACA GCTTTGGTGTGGTTTTACTAGAAATAATAACTGGACTTCCAGCTGTGGATGAACAC CGTGAACCTCAGTTATTGCTAGATATTAAAGAAGAAATTGAAGATGAAGAAAAGAC AATTGAAGATTATATTGATAAAAAGATGAATGATGCTGATTCCACTTCAGTTGAAGC TATGTACTCTGTTGCTAGTCAATGTCTGCATGAAAAGAAAAATAAGAGACCAGACA TTAAGAAGGTTCAACAGCTGCTGCAAGAGATGACAGCTTCTGTGAGCGGCTGGCG GCTGTTCAAGAAGATTAGCTAA NOT1-3', Beijing Tsingke Biotech Co., Ltd.). The plasmid was transfected into HEK293T cells (ATCC), and the HiBiT fluorescence intensity was measured to characterize the compound-induced IRAK4 degradation. 300,000 HEK293T cells in 1 mL of DMEM medium (Gbico, 11965-092) were seeded into a 6-well plate (Corning 3516) , followed by being placed overnight in a constant incubator at 37°C, 5% CO2. 3 µL X-tremeGENE^{™} 9 DNA Transfection Reagent (Roche #06365787001), and 1 µg IRAK4 plasmid were added to 200 µL Opti-MEM (Gibico 31985062), followed by gentle mixing. The 6-well plate that had been incubated overnight was taken out and allowed to stand at room temperature for 15 minutes, followed by dropwise addition to the 6-well plate. After transient transfection for 6 hours, cells were digested with trypsin (Gibco, 25200-056) for one minute, followed by centrifugation at 1000 rpm for 3 minutes. 5000 HEK293T cells in 40 µL DMEM medium were seeded into a 384-well plate (Corning 3765), followed by being placed overnight in a constant incubator at 37°C, 5% CO2. Then, 40 nL of the compound was added to the 384-well plate using an Echo 655 Liquid Handler, with a highest final concentration of 10 µM, and a 1:3 serial dilution to give a total of 10 concentrations, followed by incubation at 37°C, 5% CO2 for 24 hours. The 384-well plate was removed and equilibrated to room temperature. 40 µL Nano-Glo^{®} HiBiT Lytic Detection System (Promega N3030) was added to each well. After incubation at room temperature for 15 minutes, the fluorescence values were recorded using EnVision Xcite Multilabel Reader (PerkinElmer 2105-0020). Data were fitted and analyzed using Prism 9.3 software with non-linear regression (non-liner inhibitor(Bell-shaped)) and a 4 parameter, with parameters as follows:
Span1=Plateau1=Dip
Span2=Plateau2-Dip $Section 1 = Span 1 / \left(1+{10}^{∧}\left(\left(LogEC50_1-X\right)*nH1\right)\right)$ $Section 2 = Span 2 / \left(1+{10}^{∧}\left(\left(X-LogEC50_2\right)*nH2\right)\right)$

The equation is as follows: Y=Dip+Section1+Section2.

Some Hibit test results are shown in Table 2.

**Table 2**

| Example | DC₅₀ (nM) | Dₘₐₓ | Example | DC₅₀ (nM) | Dₘₐₓ |
|---|---|---|---|---|---|
| 1 | A | A | 5 | A | A |
| 2 | A | A | 6 | A | A |
| 3 | A | B | 7 | A | C |
| 4 | C | D | 8 | A | B |

| | | | | | |
|---|---|---|---|---|---|
| Note: Hibit DC₅₀≤0.5 nM is A, 0.5nM < DC₅₀≤1 nM is B, 1nM < DC₅₀≤5 nM is C, 5nM < DC₅₀ is D; Hibit Dₘₐₓ≥80% is A, 70%≤Dₘₐₓ < 80% is B, 60%≤Dₘₐₓ < 70% is C, Dₘₐₓ < 60% is D. | | | | | |

### Experimental Example 2: detection of proteolysis by WB

The degradation level of IRAK4 in THP-1 cells was detected by Western Blot technique. 1,000,000 THP-1 cells were seeded in a 12-well plate (Corning 3513) with 1 mL per well. A stock solution of the compound in 1000× gradient dilution was prepared, with a maximum concentration of 300 µM, and serially diluted with DMSO in 1:3, for a total of 9 doses. The compound in 1000× gradient dilution was added to the 12-well plate with 1 µL per well. After incubation under a condition of 37°C, 5% CO₂ for 24 hours, the cells were collected into a 2 mL centrifuge tube, and centrifuged at 5,000 rpm for 5 minutes. The supernatant was discarded. 100 µL RIPA lysis solution (Beyotime, P0013C) was added. After mixing well, the cells were lysed on ice for 10 min, and centrifuged at 14,000 rpm at 4°C for 10 min. The supernatant was collected. Protein content was determined using BCA (Solarbio, PC0020-10*50T). 75 µL of the supernatant was taken, and 25 µL NuPAGE LDS Sample Buffer (4X) (Thermo B0007) (with 5% β-mercaptoethanol added before use) was added to each tube to prepare samples, followed by heating in a metal bath at 100°C for 5 min. 20 µL was loaded into a 4-12% Bis-Tris gel (Invitrogen, WG1402BOX) to prepare 1x NuPAGE^{™} MOPS buffer (Invitrogen, NP0001). The electrophoresis apparatus was set to a constant voltage of 120V for 1.5 hours. 1x NuPAGE^{™} MOPS SDS electrophoresis buffer (20X) (Invitrogen, NP0001) was prepared. The electrophoresis apparatus was set to a constant current of 300 mA for 100 minutes. The protein was transferred to a PVDF membrane (Millipore, IPVH00010). The PVDF membrane was blocked with a blocking solution (Licor, 927-60001) at room temperature for 1 h, IRAK4 antibody (Abcam ab32511) was diluted 1:1,000, GAPDH (Abcam ab8245) was diluted 1:3,000, and then the antibody was incubated overnight at 4°C. The next day, the membrane was washed three times with 1× TBST (Elabscience E-BC-R335), for 5 min each time. Secondary antibodies IR Dye 800 CW Goat anti-rabbit (Licor #926-32211) and IRDye^{®} 680RD Goat anti-Mouse (Licor #926-68070) were diluted 1:10,000, followed by incubation in the dark at room temperature for 1 h. The membrane was washed three times with TBST, for 5 min each time. Using Odyssey imaging system Li-cor Odyssey Clx, signals were detected and data were analyzed. Data were analyzed using GraphPad (Prism 9.3) software with non-linear regression (non-liner inhibitor(Bell-shaped)) and a 4 parameter, with parameters as follows:
Span 1 =Plateau1-Dip
Span2=Plateau2-Dip $Section 1 = Span 1 / \left(1+{10}^{∧}\left(\left(LogEC50_1-X\right)*nH1\right)\right)$ $Section 2 = Span 2 / \left(1+{10}^{∧}\left(\left(X-LogEC50_2\right)*nH2\right)\right)$

The equation is as follows: Y=Dip+Section1+Section2.

Some of the activity results from WB test are shown in Table 3.

**Table 3**

| Example | DC₅₀ (nM) | Dₘₐₓ | Example | DC₅₀ (nM) | Dₘₐₓ |
|---|---|---|---|---|---|
| 1 | A | A | 13 | C | A |
| 2 | A | A | 16 | C | A |
| 5 | A | B | 17 | A | A |
| 6 | A | A | 18 | A | A |
| 9 | C | | 19 | B | C |
| 10 | A | A | 20 | B | A |
| 11 | C | C | 22 | C | C |
| 12 | C | B | | | |

| | | | | | |
|---|---|---|---|---|---|
| Note: WB DC₅₀≤10 nM is A, 10 nM<DC₅₀≤30 nM is B, 30 nM<DC₅₀ is C; WB Dₘₐₓ≥70% is A, 60%≤Dₘₐₓ<70% is B, Dₘₐₓ<60% is C. | | | | | |

The results of this experimental example show that the compounds of the present application exhibit excellent proteolysis-targeting effect in THP-1 cells, in the experiment for IRAK4 degradation detected by Western Blot technique.

The results show that the compounds of the present invention have good IRAK4 proteolysis ability and possess efficacy as a proteolysis targeting chimera for IRAK4 kinase.

In addition to those described herein, various modifications of the present invention will be apparent to those skilled in the art based on the foregoing description. Such modifications are also intended to fall within the scope of the appended claims. All references cited in the present application (including all patents, patent applications, journal articles, books, and any other publications) are incorporated herein by reference in their entirety.

## Claims

1. A compound of formula (A): 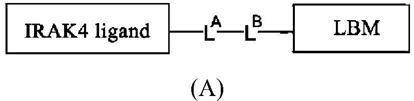
or a stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof,
wherein:
L^{A} is selected from a bond and straight or branched chain C₁₋₄ alkylene, the C₁₋₄ alkylene is optionally substituted by one or more substituents independently selected from C₁₋₄ alkyl, C₁₋₄ haloalkyl, halogen, oxo (=O), OH, CN, NH₂, -NH(C₁₋₄ alkyl), and -N(C₁₋₄ alkyl)₂;
L^{B} is selected from groups (1)-(21):
(1) -CyL1-,
(2) -CyL1-La-,
(3) -CyL1-Lb-,
(4) -CyL1-La-CyL2-La-,
(5) -CyL1-NR^{L1}-,
(6) -CyL1-C(O)-,
(7) -CyL1-C(O)-NR^{L1}-,
(8) -CyL1-NR^{L1}-C(O)-,
(9) -CyL1-CyL2-,
(10) -NR^{L1}-CyL1-La-,
(11) -NR^{L1}-CyL1-Lb-,
(12) -NR^{L1}-CyL3-NR^{L2}-,
(13) -NR^{L1}-CyL3-La-NR^{L2}-,
(14) -NR^{L1}-CyL1-C(O)-,
(15) -NR^{L1}-La-CyL1-La-,
(16) -La-CyL1-,
(17) -O-La-,
(18) -S-La-,
(19) -NR^{L1}-La-,
(20) -CyL1-La-CyL2-,
and
(21) -CyL1-Lc-CyL4-,
wherein:
In the groups (1)-(21), the bond extending from the leftmost end of each group is attached to L^{A} and the bond extending from the rightmost end is attached to the LBM moiety, or the bond extending from the leftmost end of each group is attached to the LBM moiety and the bond extending from the rightmost end is attached to L^{A},
CyL1 and CyL2 at each occurrence are each independently selected from C₃₋₁₂ cycloalkylene or 3-12-membered heterocycloalkylene, wherein the heterocycloalkylene preferably has 1, 2, or more nitrogen heteroatoms and 0, 1, or 2 heteroatoms selected from O and S,
CyL3 at each occurrence is independently selected from C₃₋₁₂ cycloalkylene,
CyL4 at each occurrence is independently selected from 5-12-membered heteroarylene,
La at each occurrence is independently selected from C₁₋₄ hydrocarbylene,
Lb at each occurrence is independently selected from straight chain C₂₋₄ hydrocarbylene, wherein 1 or 2, but not all, of the CH₂ groups in the straight chain C₂₋₄ hydrocarbylene are replaced by 1 or 2 groups selected from O, S, NR^{L1}, and C(O),
Lc at each occurrence is independently selected from a bond or C₁₋₄ hydrocarbylene,
each of CyL1, CyL2, CyL3, CyL4, La, Lb, and Lc is optionally substituted by one or more groups independently selected from the following: C₁₋₄ alkyl, C₁₋₄ haloalkyl, halogen, OH, CN, NH₂, -NH(C₁₋₄ alkyl), and -N(C₁₋₄ alkyl)₂, preferably methyl, ethyl, F, Cl, Br, OH, CN, and NH₂, more preferably methyl, F, Cl, and OH; and
R^{L1} and R^{L2} at each occurrence are each independently selected from H and C₁₋₄ alkyl;
the 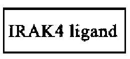 moiety has the structure of formula (1): 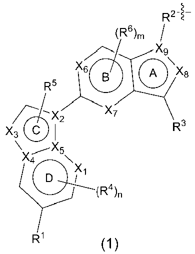
wherein: X₁, X₂, X₃, X₄, X₅, X₆, X₇, X₈, and X₉ can each independently be C, CR, or N, with the proviso that the valencies of all atoms are satisfied, and the X₄ and X₅ are not simultaneously N;
R, R¹, R⁴, and R⁵ are each independently selected from: hydrogen, deuterium, R⁷, halogen, CN, NO₂, C₁₋₆ alkyl, -OR^{1f}, -SR^{1f}, -NR^{1d}R^{1e}, -S(O)₂R^{1f}, -S(O)R^{1f}, -S(O)₂-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), -CF₃, -CCl(R^{1f})₂, -CCl₂(R^{1f}),-CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)R^{1f}, -C(O)OR^{1f},-C(O)NR^{1d}R^{1e}, -C(O)NR^{1f}-OR^{1f}, -OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, and -NR^{1f}-S(O)₂R^{1f},
p is 1, 2, or 3;
n is 0, 1, or 2, wherein when n is 1 or 2, R⁴ is attached to an available ring member of ring D; and
R⁵ is attached to an available ring member of ring C;
R² is selected from: saturated or partially unsaturated C₃₋₇ cyclohydrocarbylene; 3-7-membered saturated or partially unsaturated heterocyclylene having 1-3 heteroatoms independently selected from nitrogen, oxygen, and sulfur; C₆₋₁₀ arylene; and 5-10-membered heteroarylene having 1-4 heteroatoms independently selected from nitrogen, oxygen, and sulfur, wherein the cyclohydrocarbylene, heterocyclylene, arylene, and heteroarylene are optionally substituted by 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and-C(O)NR^{2d}R^{2e}, and in the case where two substituents are attached to the same ring carbon atom of the cyclohydrocarbylene or heterocyclylene, the two substituents optionally together with the ring carbon atoms that they are attached to, form saturated or partially unsaturated optionally substituted C₃₋₇ cyclohydrocarbyl, or 3-7-membered saturated or partially unsaturated optionally substituted heterocyclyl having 1-3 heteroatoms independently selected from nitrogen, oxygen, and sulfur;
R³ and R⁶ are each independently selected from: H, R⁸, halogen, CN, NO₂, C₁₋₆ alkyl,-OR^{3f}, -SR^{3f}, -NR^{3d}R^{3e}, -S(O)₂R^{3f}, -S(O)R^{3f}, -S(O)₂-NR^{3d}R^{3e}, -S(O)-NR^{3d}R^{3e}, -P(O)(OR^{3f})₂,-P(O)(NR^{3d}R^{3e})₂, -CF(R^{3f})₂, -CF₂(R^{3f}), -CF₃, -CCl(R^{3f})₂, -CCl₂(R^{3f}), -CCl₃, -(CR^{3a}R^{3b})_{q}-OR^{3f},-(CR^{3a}R^{3b})_{q}-C(O)OR^{3f}, -(CR^{3a}R^{3b})_{q}-NR^{3d}R^{3e}, -C(O)R^{3f}, -C(O)OR^{3f}, and -C(O)NR^{3d}R^{3e},
q is 1, 2, or 3; and
m is 0, 1, 2, or 3, wherein when m is 1, 2, or 3, R⁶ is attached to an available ring member of ring B;
R^{1a}, R^{1b}, R^{3a}, and R^{3b} are each independently selected from hydrogen, deuterium, halogen, CN, OH, SH, NO₂, C₁₋₆ alkyl, C₁₋₆ haloalkyl, C₁₋₆ alkoxy, C₁₋₆ alkylthio, and -NR^{7d}R^{7e}; or R^{1a} and R^{1b}, or R^{3a} and R^{3b}, together with the carbon atoms that they are commonly attached to, form R⁹;
R^{1d}, R^{1e}, R^{2d}, R^{2e}, R^{3d}, R^{3e}, R^{7d}, and R^{7e} are each independently selected from hydrogen, deuterium, R¹¹, C₁₋₆ alkyl, and C₁₋₆ haloalkyl; or, R^{1d} and R^{1e}, or R^{2d} and R^{2e}, or R^{3d} and R^{3e}, or R^{7d} and R^{7e}, together with the nitrogen atoms that they are commonly attached to, form R¹⁰;
R^{1f}, R^{2f}, and R^{3f} are independently selected from hydrogen, deuterium, C₁₋₆ alkyl, C₁₋₆ haloalkyl, and R¹²;
R⁷, R⁸, R⁹, R¹¹, and R¹² are each independently selected from: saturated or partially unsaturated C₃₋₇ cyclohydrocarbyl; 3-12-membered saturated or partially unsaturated heterocyclyl having 1-3 heteroatoms independently selected from nitrogen, oxygen, and sulfur; C₆₋₁₀ aryl; and 5-10-membered heteroaryl having 1-4 heteroatoms independently selected from nitrogen, oxygen, and sulfur, wherein the cyclohydrocarbyl, heterocyclyl, aryl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
R¹⁰ is selected from: 3-7-membered saturated or partially unsaturated heterocyclyl having one nitrogen heteroatom and optionally 1-2 additional heteroatoms independently selected from nitrogen, oxygen, and sulfur; and 5-10-membered heteroaryl having one nitrogen heteroatom and optionally 1-3 additional heteroatoms independently selected from nitrogen, oxygen, and sulfur, wherein the heterocyclyl and heteroaryl are optionally substituted by one or more substituents independently selected from halogen, CN, OH, SH, NO₂, oxo (=O), C₁₋₆ alkyl, C₁₋₆ haloalkyl, C₁₋₆ alkoxy, C₁₋₆ alkylthio, and -NR^{10d}R^{10e}; and
R^{8d}, R^{8e}, R^{10d}, and R^{10e} are each independently selected from hydrogen, deuterium, C₁₋₆ alkyl, and C₁₋₆ haloalkyl; and
the 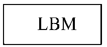 moiety is a ligase binding moiety.

2. The compound according to claim 1, wherein L^{A} is selected from a bond, C₁₋₂ alkylene, the C₁₋₂ alkylene is optionally substituted by one or more substituents independently selected from C₁₋₂ alkyl, C₁₋₂ haloalkyl, halogen, oxo (=O), OH, CN, NH₂, -NH(C₁₋₂ alkyl), and -N(C₁₋₂ alkyl)₂; preferably, L^{A} is selected from a bond, C₁₋₂ alkylene, the C₁₋₂ alkylene is optionally substituted by one or more substituents independently selected from C₁₋₂ alkyl, halogen, oxo (=O); further preferably, L^{A} is selected from a bond, -CH₂-, -CH₂-CH₂-, -CH(CH₃)-, and-C(=O)-; further preferably, L^{A} is a bond, -CH₂-, or -CH₂-CH₂-, more preferably -CH₂-.

3. The compound according to any one of claim 1 or 2, wherein the 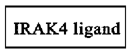 moiety has the structure of formula (1), wherein:
R¹ and R⁴ are not simultaneously hydrogen or deuterium;
and/or
R^{1a}, R^{1b}, R^{3a}, and R^{3b} are each independently selected from hydrogen, halogen, CN, OH, SH, NO₂, C₁₋₄ alkyl, C₁₋₄ haloalkyl, C₁₋₄ alkoxy, C₁₋₄ alkylthio, and -NR^{7d}R^{7e}; or R^{1a} and R^{1b}, or R^{3a} and R^{3b}, together with the carbon atoms that they are commonly attached to, form R⁹;
and/or
R^{1d}, R^{1e}, R^{2d}, R^{2e}, R^{3d}, R^{3e}, R^{7d}, and R^{7e} are each independently selected from hydrogen, R¹¹, C₁₋₄ alkyl, and C₁₋₄ haloalkyl; or, R^{1d} and R^{1e}, or R^{2d} and R^{2e}, or R^{3d} and R^{3e}, or R^{7d} and R^{7e}, together with the nitrogen atoms that they are commonly attached to, form R¹⁰;
and/or
R^{1f}, R^{2f}, and R^{3f} are independently selected from hydrogen, C₁₋₄ alkyl, C₁₋₄ haloalkyl, and R¹²;
and/or
R⁷, R⁸, R⁹, R¹¹, and R¹² are each independently selected from: saturated or partially unsaturated C₃₋₆ cyclohydrocarbyl; 3-10-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cyclohydrocarbyl, heterocyclyl, aryl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
and/or
R¹⁰ is selected from: 3-6-membered saturated or partially unsaturated heterocyclyl having one nitrogen heteroatom and optionally 1-2 additional heteroatoms independently selected from nitrogen, oxygen, and sulfur; and 5-6-membered heteroaryl having one nitrogen heteroatom and optionally 1-2 additional heteroatoms independently selected from nitrogen, oxygen, and sulfur, wherein the heterocyclyl and heteroaryl are optionally substituted by one or more substituents independently selected from halogen, CN, OH, SH, NO₂, oxo (=O) , C₁₋₆ alkyl, C₁₋₆ haloalkyl, C₁₋₆ alkoxy, C₁₋₆ alkylthio, and -NR^{10d}R^{10e};
and/or
R^{8d}, R^{8e}, R^{10d}, and R^{10e} are each independently selected from hydrogen, C₁₋₆ alkyl, and C₁₋₆ haloalkyl.

4. The compound according to any one of claims 1 to 3, wherein the 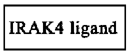 moiety has the structure of formula (1), wherein:
R¹ is not hydrogen; or
R¹ is selected from: R⁷, halogen, CN, NO₂, C₁₋₄ alkyl, -OR^{1f}, -SR^{1f}, -NR^{1d}R^{1e}, -S(O)₂R^{1f},-S(O)R^{1f}, -S(O)₂-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), -CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)R^{1f}, -C(O)OR^{1f}, -C(O)NR^{1d}R^{1e}, -C(O)NR^{1f}-OR^{1f}; -OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, or -NR^{1f}-S(O)₂R^{1f}; or
R¹ is selected from: R⁷, halogen, CN, NO₂, C₁₋₄ alkyl, -OR^{1f}, -SR^{1f}, -NR^{1d}R^{1e}, -CF(R^{1f})₂,-CF₂(R^{1f}), -CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f},-(CR^{1a}R^{1b})ₚ-NR^{1d}R^{1e}, -C(O)OR^{1f}, or -C(O)NR^{1d}R^{1e}; or
R¹ is selected from: R⁷, halogen, CN, -OR^{1f}, -NR^{1d}R^{1e}, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e}.

5. The compound according to any one of claims 1 to 4, wherein the 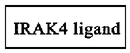 moiety has the structure of formula (1), wherein:
R⁷ is selected from: C₃₋₆ cycloalkyl; 4-6-membered saturated or partially unsaturated heterocyclyl having 1-3 heteroatoms independently selected from nitrogen, oxygen, and sulfur; phenyl; and 5-6-membered heteroaryl having 1-3 heteroatoms independently selected from nitrogen, oxygen, and sulfur, wherein the cycloalkyl, heterocyclyl, phenyl, and heteroaryl are optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}; or
R⁷ is selected from: C₃₋₆ cycloalkyl; 4-6-membered heterocycloalkyl having 1-3 heteroatoms independently selected from nitrogen, oxygen, and sulfur; phenyl; and 5-6-membered heteroaryl having 1-3 heteroatoms independently selected from nitrogen, oxygen, and sulfur, wherein the cycloalkyl, heterocycloalkyl, phenyl, and heteroaryl are optionally substituted by one or more substituents independently selected from halogen, CN, OH, NO₂, C₁₋₄ alkyl, C₁₋₄ haloalkyl, C₁₋₄ alkoxy, and -NR^{8d}R^{8e};
wherein: preferably, R^{8d} and R^{8e} are each independently selected from hydrogen and C₁₋₆ alkyl; or, preferably, R^{8d} and R^{8e} are each independently selected from hydrogen and C₁₋₄ alkyl;
or
R⁷ is selected from: 4-6-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
R⁷ is selected from: azetidinyl, pyrrolidinyl, and piperidinyl.

6. The compound according to any one of claims 1 to 5, wherein the 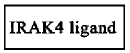 moiety has the structure of formula (1), wherein:
p is 1;
and/or
R^{1a} and R^{1b} are each independently selected from hydrogen and C₁₋₆ alkyl; or
R^{1a} and R^{1b} are each independently selected from hydrogen and C₁₋₄ alkyl; or
R^{1a} and R^{1b} are each independently hydrogen; or
R^{1a} and R^{1b} together with the carbon atoms that they are commonly attached to, form R⁹;
wherein:
R⁹ is selected from: C₃₋₆ cycloalkyl; 3-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e};
preferably, R^{8d} and R^{8e} are each independently selected from hydrogen and C₁₋₆ alkyl; or preferably, R^{8d} and R^{8e} are each independently selected from hydrogen and C₁₋₄ alkyl;
or
R⁹ is selected from: C₃₋₆ cycloalkyl, wherein the cycloalkyl is optionally substituted by one or more substituents independently selected from F, Cl, Br, CN, OH, NO₂, C₁₋₄ alkyl, C₁₋₄ haloalkyl, C₁₋₄ alkoxy, NH₂, -NH(C₁₋₄ alkyl), and N(C₁₋₄ alkyl)₂; or
preferably, R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R⁹ is cyclopropyl.

7. The compound according to any one of claims 1 to 6, wherein the 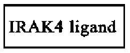 moiety has the structure of formula (1), wherein:
R^{1d} and R^{1e} are each independently selected from hydrogen, R¹¹, and C₁₋₆ alkyl; or
R^{1d} and R^{1e} are each independently selected from hydrogen, R¹¹, and C₁₋₄ alkyl; or
R^{1d} and R^{1e} are each independently selected from hydrogen, R¹¹, methyl, ethyl, propyl, isopropyl, n-butyl, isobutyl, sec-butyl, and tert-butyl; or
R^{1d} and R^{1e} together with the nitrogen atoms that they are commonly attached to, form R¹⁰;
wherein:
preferably, R¹¹ is selected from: C₃₋₆ cycloalkyl; 3-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e};
more preferably, R^{8d} and R^{8e} are each independently selected from hydrogen and C₁₋₆ alkyl; or preferably, R^{8d} and R^{8e} are each independently selected from hydrogen and C₁₋₄ alkyl;
or
preferably, R¹¹ is selected from: C₃₋₆ cycloalkyl; and 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R¹¹ is selected from: cyclopropyl, cyclobutyl, cyclopentyl, cyclohexyl, azetidinyl, pyrrolidinyl, and piperidinyl, each of which is optionally substituted by one or more substituents independently selected from F and Cl; or
preferably, R¹¹ is selected from cyclopropyl and 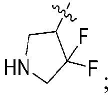 and/or
preferably, R¹⁰ is selected from: 3-6-membered heterocycloalkyl having one nitrogen heteroatom and optionally 1-2 additional heteroatoms independently selected from nitrogen, oxygen, and sulfur, and 5-6-membered heteroaryl having one nitrogen heteroatom and optionally 1-2 additional heteroatoms independently selected from nitrogen, oxygen, and sulfur, wherein the heterocycloalkyl and heteroaryl are optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: 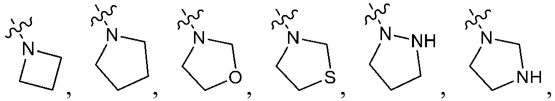 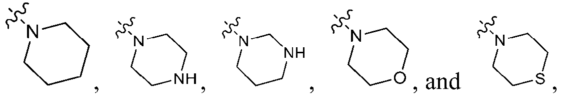 each of which is optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R¹⁰ is selected from: 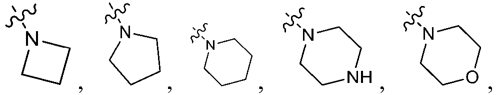 and 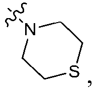 each of which is optionally substituted by one or more substituents independently selected from F, Cl, Br, CN, OH, NO₂, oxo, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

8. The compound according to any one of claims 1 to 7, wherein 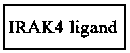 the moiety has the structure of formula (1), wherein: R^{1f} is selected from hydrogen and C₁₋₆ alkyl; or R^{1f} is selected from hydrogen and C₁₋₄ alkyl; or R^{1f} is hydrogen.

9. The compound according to any one of claims 1 to 8, wherein the 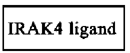 moiety has the structure of formula (1), wherein R¹ is selected from: F, Cl, Br, CN, OH, NH₂, -NHCH₃, -C(O)OH, -C(O)NH₂, -CH₂NH₂, -CH₂OH, 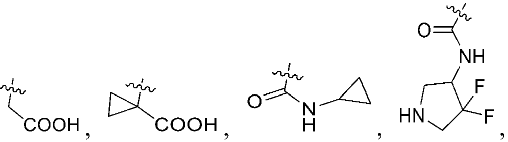 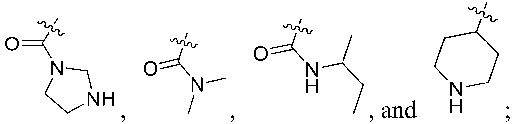 or , R¹ is selected from -CN, -C(O)NH₂, F, -NHCH₃, -C(O)OH,

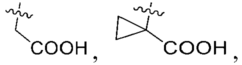

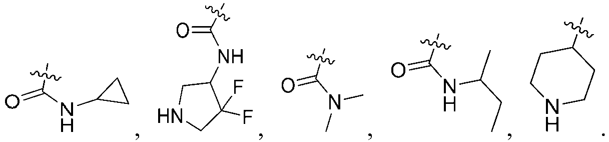

10. The compound according to any one of claims 1 to 9, wherein the 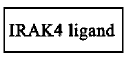 moiety has the structure of formula (I-1): 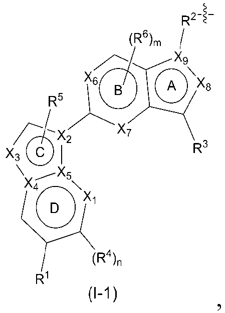 wherein, n is 0 or 1.

11. The compound according to any one of claims 1 to 9, wherein:
R¹ is selected from hydrogen and deuterium, and n is 1 or 2; or
R¹ is hydrogen, and n is 1; or
the 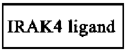 moiety has the structure of formula (I-2): 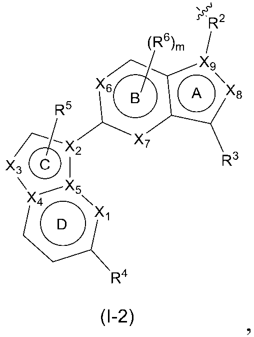 with the proviso that: R⁴ is not hydrogen or deuterium.

12. The compound according to any one of claims 1 to 11, wherein:
R⁴ is selected from: hydrogen, R⁷, halogen, CN, NO₂, -OR^{1f}, -SR^{1f}, and -NR^{1d}R^{1e};
preferably, R^{1d} and R^{1e} are each independently selected from hydrogen and C₁₋₆ alkyl; or
preferably, R^{1d} and R^{1e} are each independently selected from hydrogen and C₁₋₄ alkyl;
and/or
preferably, R^{1f} is selected from hydrogen and C₁₋₆ alkyl; or preferably, R^{1f} is selected from hydrogen and C₁₋₄ alkyl;
or
R⁴ is selected from: hydrogen, R⁷, F, Cl, Br, CN, NO₂, OH, and NH₂; or
R⁴ is selected from: hydrogen, R⁷, and CN;
wherein:
preferably, R⁷ is selected from: 4-6-membered saturated monocyclic heterocyclyl, 8-10-membered saturated fused bicyclic heterocyclyl, 6-11-membered saturated spiro heterocyclyl, and 7-10-membered saturated bridged heterocyclyl, each of which has 1-3 heteroatoms independently selected from nitrogen, oxygen, and sulfur, and is optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}; or
preferably, R⁷ is selected from: 4-6-membered saturated monocyclic heterocyclyl and 7-10-membered saturated bridged heterocyclyl, the monocyclic heterocyclyl and bridged heterocyclyl have one nitrogen heteroatom and optionally 1-2 additional heteroatoms independently selected from nitrogen, oxygen, and sulfur, and are optionally substituted by one or more substituents independently selected from halogen, CN, OH, NO₂, C₁₋₄ alkyl, C₁₋₄ haloalkyl, C₁₋₄ alkoxy, and -NR^{8d}R^{8e};
wherein: preferably, R^{8d} and R^{8e} are each independently selected from hydrogen and C₁₋₆ alkyl; or preferably, R^{8d} and R^{8e} are each independently selected from hydrogen and C₁₋₄ alkyl;
or
preferably, R⁷ is selected from: 4-6-membered saturated monocyclic heterocyclyl (for example azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl) and 7-10-membered saturated bridged heterocyclyl, the monocyclic heterocyclyl and bridged heterocyclyl have one nitrogen heteroatom and optionally 1-2 additional heteroatoms independently selected from nitrogen, oxygen, and sulfur, and are optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R⁷ is selected from: 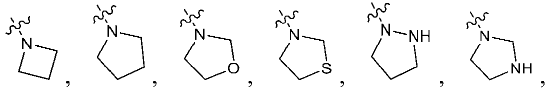 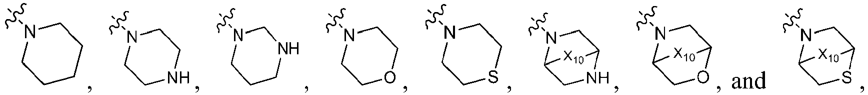 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂, and wherein X₁₀ is CH₂, (CH₂)₂, or (CH₂)₃; or
preferably, R⁷ is selected from: 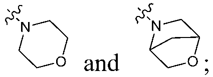
preferably, R⁴ is selected from: hydrogen, CN, 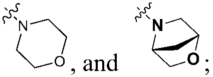
with the proviso that: when R¹ is hydrogen or deuterium, R⁴ is not hydrogen.

13. The compound according to any one of claims 1 to 12, wherein:
R and R⁵ are each independently selected from hydrogen;
and/or
R² is selected from: C₃₋₆ cycloalkylene; 4-6-membered saturated or partially unsaturated heterocyclylene having 1-3 heteroatoms independently selected from nitrogen, oxygen, and sulfur; C₆₋₁₀ arylene; and 5-6-membered heteroarylene having 1-3 heteroatoms independently selected from nitrogen, oxygen, and sulfur, wherein the cycloalkylene, heterocyclylene, arylene, and heteroarylene are optionally substituted by 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cycloalkylene or heterocyclylene, the two substituents optionally together with the ring carbon atoms that they are attached to, form optionally substituted C₃₋₆ cycloalkyl, or optionally substituted 4-6-membered heterocycloalkyl having 1-3 heteroatoms independently selected from nitrogen, oxygen, and sulfur; wherein preferably, R^{2d} and R^{2e} are each independently selected from hydrogen, C₁₋₆ alkyl, and C₁₋₆ haloalkyl, and/or R^{2f} is selected from hydrogen and C₁₋₆ alkyl; or
R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₄ alkyl, -C(O)OH, -C(O)O-C₁₋₄ alkyl, -C(O)NH₂,-C(O)NH(C₁₋₄ alkyl), and -C(O)NH(C₁₋₄ alkyl)₂, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl; or
R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylidene, piperazinylene, hexahydropyrimidinylidene, triazinylene, morpholinylene, thiomorpholinylidene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1, 2, or more substituents independently selected from F, Cl, Br, CN, OH, NH₂, NO₂, methyl, ethyl, isopropyl, tert-butyl, C(O)OH, -C(O)OCH₃, -C(O)OCH₂CH₃,-C(O)NH₂, -C(O)NHCH₃, and -C(O)N(CH₃)₂, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylidene, piperazinylene, hexahydropyrimidinylidene, triazinylene, morpholinylene, or thiomorpholinylidene, the two substituents optionally together with the ring carbon atoms that they are attached to, form optionally substituted C₃₋₆ cycloalkyl; or
R² is selected from: cyclohexylidene, pyrrolidinylene, piperidinylene, phenylene, and pyridylene, each of which is optionally substituted by 1 or 2 substituents independently selected from F, Cl, Br, OH, NH₂, and methyl, and in the case where two substituents are attached to the same ring carbon atom of the cyclohexylidene, pyrrolidinylene, or piperidinylene, the two substituents optionally together with the ring carbon atoms that they are attached to, form optionally substituted C₃₋₆ cycloalkyl; or
R² is selected from: 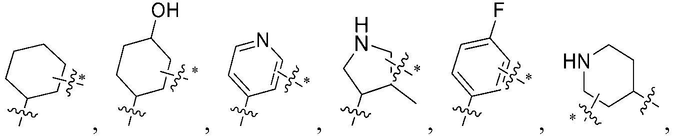 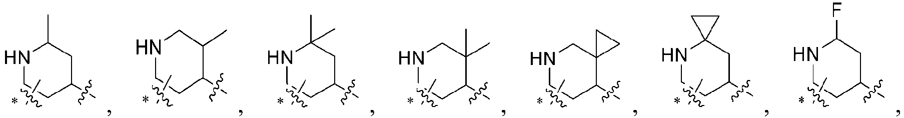 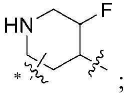 or
R² is selected from: 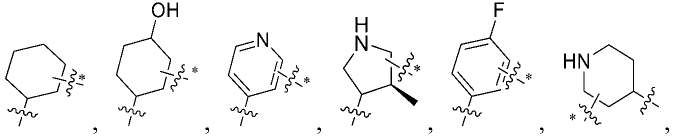 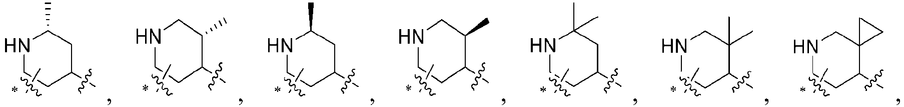 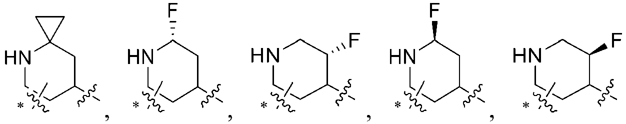 ; wherein, the wavy line " 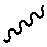 " represents the site of attachment, the bond indicated by * is attached to L^{A};
and/or
R³ is selected from: halogen, CN, NO₂, C₁₋₆ alkyl, -OR^{3f}, -SR^{3f}, -NR^{3d}R^{3e}, -S(O)₂-NR^{3d}R^{3e}, -S(O)-NR^{3d}R^{3e}, -CF(R^{3f})₂, -CF₂(R^{3f}), -CF₃, -CCl(R^{3f})₂, -CCl₂(R^{3f}), -CCl₃, -C(O)OR^{3f}, and-C(O)NR^{3d}R^{3e}; or
R³ is selected from: halogen, CN, C₁₋₄ alkyl, -OR^{3f}, -NR^{3d}R^{3e}, -CF(R^{3f})₂, -CF₂(R^{3f}), -CF₃, -CCl(R^{3f})₂, -CCl₂(R^{3f}), -CCl₃, -C(O)OR^{3f}, and -C(O)NR^{3d}R^{3e}, or
R³ is selected from: halogen, CN, C₁₋₄ alkyl, -OR^{3f}, -NR^{3d}R^{3e}, -CF(R^{3f})₂, -CF₂(R^{3f}), -CF₃, -CCl(R^{3f})₂, -CCl₂(R^{3f}), -CCl₃, and -C(O)NR^{3d}R^{3e};
wherein: preferably, R^{3d} and R^{3e} are each independently selected from hydrogen, deuterium, R¹¹, C₁₋₄ alkyl, and C₁₋₄ haloalkyl, wherein R¹¹ is preferably selected from: C₃₋₆ cycloalkyl; 4-6-membered saturated or partially unsaturated heterocyclyl having 1-3 heteroatoms independently selected from nitrogen, oxygen, and sulfur; C₆₋₁₀ aryl; and 5-6-membered heteroaryl having 1-3 heteroatoms independently selected from nitrogen, oxygen, and sulfur, wherein the cycloalkyl, heterocyclyl, aryl, and heteroaryl are optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}, wherein preferably, R^{8d} and R^{8e} are each independently selected from hydrogen, C₁₋₄ alkyl, and C₁₋₄ haloalkyl; or
preferably, R^{3d} and R^{3e} are each independently selected from hydrogen, methyl, and ethyl;
and/or
wherein:
preferably, R^{3f} is selected from hydrogen and C₁₋₆ alkyl; or preferably, R^{3f} is selected from hydrogen and C₁₋₄ alkyl;
or
wherein:
preferably, R^{3d} and R^{3e} together with the nitrogen atoms that they are commonly attached to, form R¹⁰, wherein R¹⁰ is preferably selected from: 3-6-membered heterocycloalkyl having one nitrogen heteroatom and optionally 1-2 additional heteroatoms independently selected from nitrogen, oxygen, and sulfur, and 5-6-membered heteroaryl having one nitrogen heteroatomand optionally 1-2 additional heteroatoms independently selected from nitrogen, oxygen, and sulfur, wherein the heterocycloalkyl and heteroaryl are optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂;
or
R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, -C(O)NH₂, -C(O)NH(C₁₋₄ alkyl), and -C(O)N(C₁₋₄ alkyl)₂;
or
R³ is selected from: F, Cl, Br, OH, NH₂, CN, methyl, ethyl, propyl, isopropyl, n-butyl, isobutyl, sec-butyl, tert-butyl, methoxy, ethoxy, -N(CH₃)₂, -CHF₂, -CHCl₂, and -C(O)NH₂;
and/or
R⁶ is selected from: halogen, CN, NO₂, C₁₋₄ alkyl, -OR^{3f}, -SR^{3f}, -NR^{3d}R^{3e}, -CF(R^{3f})₂,-CF₂(R^{3f}), -CF₃, -CCl(R^{3f})₂, -CCl₂(R^{3f}), and -CCl₃, wherein R^{3f} is preferably selected from hydrogen and C₁₋₆ alkyl, or R^{3f} is preferably selected from hydrogen and C₁₋₄ alkyl; or
R⁶ is selected from: F, Cl, Br, OH, NH₂, CN, NO₂, methyl, ethyl, propyl, isopropyl, n-butyl, isobutyl, sec-butyl, tert-butyl, methoxy, ethoxy, -CH₂F, -CHF₂, -CF₃, -CH₂Cl, -CHCl₂, and-CCl₃;
and/or
m is 0.

14. The compound according to any one of claims 1 to 13, wherein:
at least two of X₁, X₂, X₃, X₄, and X₅ are N, with the proviso that X₄ and X₅ are not simultaneously N; or
three of X₁, X₂, X₃, X₄, and X₅ are N, with the proviso that X₄ and X₅ are not simultaneously N; or
four of X₁, X₂, X₃, X₄, and X₅ are N, with the proviso that X₄ and X₅ are not simultaneously N;
wherein preferably, X₁ is N;
and/or
at least two of X₆, X₇, X₈, and X₉ are N; or
at least three of X₆, X₇, X₈, and X₉ are N;
at least four of X₆, X₇, X₈, and X₉ are N;
wherein preferably, X₈ and X₉ are each N;
preferably, two or three of X₁, X₂, X₃, X₄, and X₅ are N, and X₁ is N, with the proviso that X₄ and X₅ are not simultaneously N; and
three or four of X₆, X₇, X₈, and X₉ are N, and X₈ and X₉ are each N.

15. The compound according to any one of claims 1 to 14, wherein:
X₁ and X₂ are each N; X₃ is CH; X₄ and X₅ are each C; X₆, X₈, and X₉ are each N; and X₇ is CH;
and/or
the IRAK4 ligand moiety has the structure of formula (I-i) or formula (I-ii): 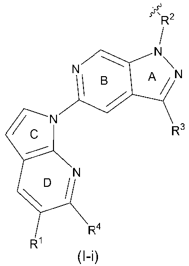
wherein:
R¹, R², R³, and R⁴ are each as defined in any one of claims 1 to 14, with the proviso that: R¹ is not hydrogen or deuterium; 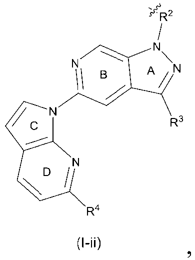
wherein:
R², R³, and R⁴ are each as defined in any one of claims 1 to 13, with the proviso that: R⁴ is not hydrogen or deuterium;
preferably, R¹ is selected from: halogen, CN, NO₂, -OR^{1f}, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)OR^{1f,} or -C(O)NR^{1d}R^{1e}; or
R¹ is selected from: halogen, CN, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e};
more preferably, p is 1;
and/or
preferably, R^{1a} and R^{1b} are each independently selected from hydrogen and C₁₋₄ alkyl, preferably each independently are hydrogen; or R^{1a} and R^{1b} together with the carbon atoms that they are commonly attached to, form R⁹;
wherein:
preferably, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R⁹ is cyclopropyl;
and/or
preferably, R^{1d} and R^{1e} are each independently selected from hydrogen and R¹¹; or R^{1d} and R^{1e} together with the nitrogen atoms that they are commonly attached to, form R¹⁰;
wherein:
preferably, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂; or
R¹¹ is selected from: cyclopropyl, cyclobutyl, cyclopentyl, cyclohexyl, azetidinyl, pyrrolidinyl, piperidinyl, piperazinyl, and morpholinyl, each of which is optionally substituted by one or more substituents independently selected from F, Cl, Br, CN, OH, NO₂, and NH₂; or
R¹¹ is selected from cyclopropyl and 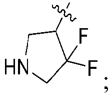 and/or
preferably, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R¹⁰ is selected from: 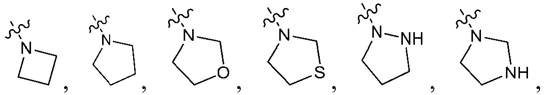 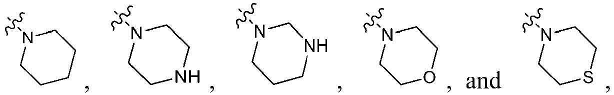 preferably 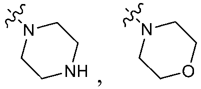 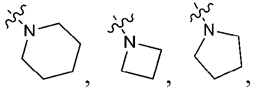 each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
R^{1f} is selected from hydrogen and C₁₋₄ alkyl, preferably hydrogen;
or
R¹ is selected from: F, Cl, Br, CN, OH, NH₂, C(O)OH, -C(O)NH₂, 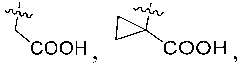 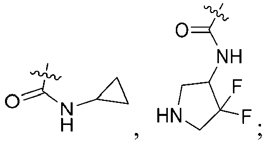
preferably, R¹ is selected from CN and -C(O)NH₂;
and/or
preferably, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl; or
R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl; or
R² is selected from cyclopentylidene, cyclohexylidene, pyrrolidinylene, piperidinylene, phenylene, and pyridylene, each of which is optionally substituted by 1 or 2 substituents independently selected from F, Cl, Br, CN, OH, NH₂, NO₂, methyl, ethyl, and isopropyl, and in the case where two substituents are attached to the same ring carbon atom of the cyclopentylidene, cyclohexylidene, pyrrolidinylene, or piperidinylene, the two substituents optionally together with the ring carbon atoms that they are attached to, form optionally substituted C₃₋₆ cycloalkyl; or
R² is selected from: 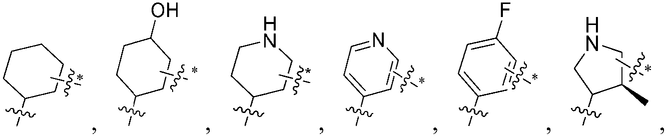 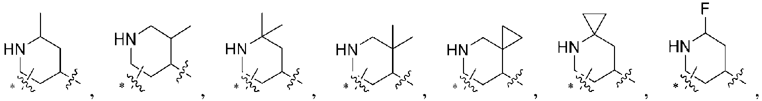 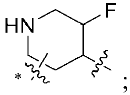 or
R² is selected from 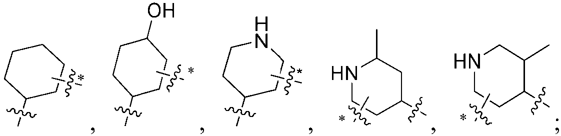 or
R² is 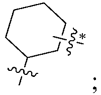 wherein, the wavy line " 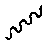 " represents the site of attachment, the bond indicated by * is attached to L^{A};
and/or
preferably, R³ is selected from: halogen, CN, NO₂, C₁₋₆ alkyl, OH, NH₂, -CH₂F, -CHF₂, - CF₃, -CH₂Cl, -CHCl₂, and -CCl₃; or
R³ is -CHF₂;
and/or
preferably, R⁴ is selected from: hydrogen, R⁷, halogen, CN, NO₂, OH, and NH₂; or
R⁴ is selected from: hydrogen, R⁷, F, Cl, Br, CN, NO₂, OH, and NH₂;
wherein: preferably, R⁷ is selected from: 4-6-membered saturated monocyclic heterocyclyl, the heterocyclyl has one nitrogen heteroatom and optionally 1-2 additional heteroatoms independently selected from nitrogen, oxygen, and sulfur, and is optionally substituted by one or more substituents independently selected from halogen, CN, OH, SH, NO₂, C₁₋₆ alkyl, C₁₋₆ haloalkyl, C₁₋₆ alkoxy, C₁₋₆ alkylthio, NH₂, NH(C₁₋₄ alkyl), and N(C₁₋₄ alkyl)₂; or
R⁷ is selected from: 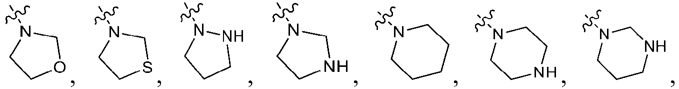 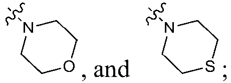
preferably, R⁴ is selected from: hydrogen, CN, and 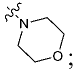
with the proviso that: when R¹ is hydrogen, R⁴ is not hydrogen.

16. The compound according to any one of claims 1 to 14, wherein:
X₁ and X₅ are each N; X₂ and X₄ are C; X₃ is CH; X₆, X₈, and X₉ are each N; and X₇ is CH;
and/or
the 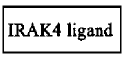 moiety has the structure of formula (I-iii): 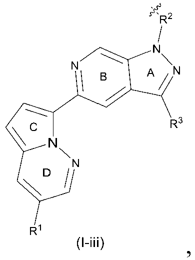
wherein:
R¹, R², and R³ are each as defined in any one of claims 1 to 13, with the proviso that: R¹ is not hydrogen or deuterium;
preferably, R¹ is selected from: R⁷, halogen, NO₂, -OR^{1f}, -NR^{1d}R^{1e}, -(CR^{1a}R^{1b} )ₚ-OR^{1f}, - (CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}; or
R¹ is selected from: R⁷, halogen, -NR^{1d}R^{1e}, -(CR^{1a}R^{1b})ₚ-OR^{1f}, -(CR^{1a}R^{1b})ₚ-C(O)OR^{1f}, - C(O)OR^{1f}, or -C(O)NR^{1d}R^{1e};
wherein:
preferably, p is 1;
and/or
wherein:
preferably, R⁷ is selected from: 4-6-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
R⁷ is piperidinyl;
and/or
wherein:
preferably, R^{1a} and R^{1b} are each independently selected from hydrogen and C₁₋₄ alkyl, preferably each independently are hydrogen; or R^{1a} and R^{1b} together with the carbon atoms that they are commonly attached to, form R⁹;
wherein:
preferably, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
preferably, R^{1d} and R^{1e} are each independently selected from hydrogen, C₁₋₄ alkyl, and R¹¹; or R^{1d} and R^{1e} together with the nitrogen atoms that they are commonly attached to, form R¹⁰;
wherein:
preferably, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂;
and/or
preferably, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
R^{1f} is selected from hydrogen and C₁₋₄ alkyl, preferably hydrogen;
preferably, R¹ is selected from: F, Cl, Br, OH, NH₂, -NHCH₃, C(O)OH, -C(O)NH₂, - CH₂OH, 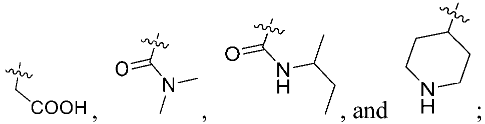
more preferably, R¹ is selected from -CN and -C(O)NH₂;
and/or
wherein:
preferably, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl; or
R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl; or
R² is selected from: cyclopentylidene, cyclohexylidene, pyrrolidinylidene, piperidinylidene, phenylene, and pyridylene, each of which is optionally substituted by 1 or 2 substituents independently selected from F, Cl, Br, CN, OH, NH₂, NO₂, methyl, ethyl, and isopropyl, and in the case where two substituents are attached to the same ring carbon atom of the cyclopentylidene, cyclohexylidene, pyrrolidinylidene, or piperidinylidene, the two substituents optionally together with the ring carbon atoms that they are attached to, form optionally substituted C₃₋₆ cycloalkyl; or
R² is selected from: 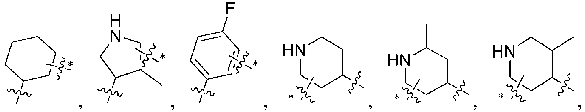 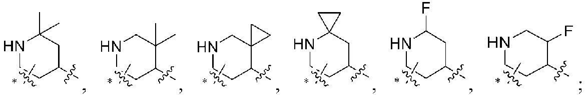 or
R² is selected from: 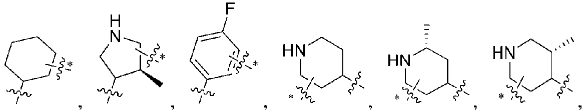 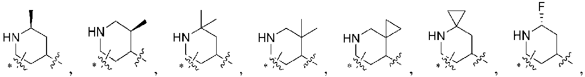 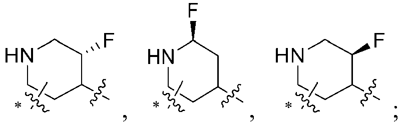
wherein, the wavy line " 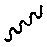 " represents the site of attachment, the bond indicated by * is attached to L^{A};
and/or
wherein:
preferably, R³ is selected from: halogen, CN, C₁₋₄ alkyl, -OR^{3f}, -SR^{3f}, -NR^{3d}R^{3e}, -CF(R^{3f})₂, -CF₂(R^{3f}), -CF₃, -CCl(R^{3f})2, -CCl₂(R^{3f}), -CCl₃, and -C(O)NR^{3d}R^{3e},
wherein: preferably, R^{3d} and R^{3e} are each independently selected from hydrogen and C₁₋₄ alkyl, preferably hydrogen and methyl;
and/or
preferably, R^{3f} is selected from hydrogen and C₁₋₄ alkyl, preferably hydrogen and methyl;
preferably, R³ is selected from: halogen, OH, SH, NH₂, CN, C₁₋₄ alkyl, -OC₁₋₄ alkyl, - NH(C₁₋₄ alkyl), -N(C₁₋₄ alkyl)₂, -CHF₂, -CH₂F, -CF₃, -CH₂Cl, -CHCl₂, -CCl₃, -C(O)NH₂, - C(O)NH(C₁₋₄ alkyl), and -C(O)N(C₁₋₄ alkyl)₂; or
R³ is selected from: F, OH, NH₂, CN, methyl, ethyl, isopropyl, tert-butyl, methoxy, - N(CH₃)₂, -CHF₂, and -C(O)NH₂; or
R³ is selected from: F, CN, methyl, isopropyl, methoxy, -N(CH₃)₂, -CHF₂, and -C(O)NH₂.

17. The compound according to any one of claims 1 to 14, wherein:
X₁, X₃, and X₄ are each N; X₂ and X₅ are C; X₆, X₈, and X₉ are each N; and X₇ is CH;
and/or
the 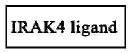 moiety has the structure of formula (I-iv): 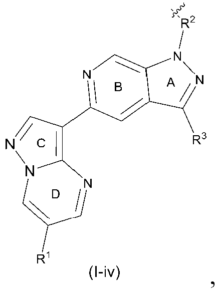
wherein:
R¹, R², and R³ are each as defined in any one of claims 1 to 13, with the proviso that: R¹ is not hydrogen or deuterium;
preferably, R¹ is selected from: halogen, CN, NO₂, -OR^{1f}, -(CR^{1a}R^{1b})ₚ-OR^{1f} -(CR^{1a}R^{1b})ₚ-C(O)OR^{1f}; -(CR^{1a}R^{1b})ₚ-NR^{1d}R^{1e}, or -C(O)OR^{1f}; or
R¹ is selected from: halogen, CN, -(CR^{1a}R^{1b})ₚ-OR^{1f}, -(CR^{1a}R^{1b})ₚ-C(O)OR^{1f}, or -C(O)OR^{1f};
wherein:
preferably, p is 1;
and/or
preferably, R^{1a} and R^{1b} are each independently selected from hydrogen and C₁₋₄ alkyl, preferably each independently are hydrogen; or R^{1a} and R^{1b} together with the carbon atoms that they are commonly attached to, form R⁹;
wherein:
preferably, R⁹ is selected from: C₃₋₆ cycloalkyl, wherein the cycloalkyl is optionally substituted by one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
preferably, R^{1d} and R^{1e} are each independently selected from hydrogen and R¹¹; or R^{1d} and R^{1e} together with the nitrogen atoms that they are commonly attached to, form R¹⁰;
wherein:
preferably, R¹¹ is selected from: C₃₋₆ cycloalkyl, and 3-6-membered heterocycloalkyl having 1-3 heteroatoms independently selected from nitrogen, oxygen, and sulfur; wherein the cycloalkyl and heterocycloalkyl are optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more substituents independently selected from F, Cl, Br, CN, OH, NO₂, and NH₂;
and/or
preferably, R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted by one or more independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
R^{1f} is selected from hydrogen and C₁₋₄ alkyl, preferably hydrogen;
or
R¹ is selected from: F, Cl, Br, CN, OH, NH₂, C(O)OH, 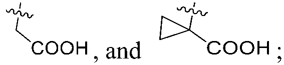 or
R¹ is CN;
and/or
wherein:
preferably, R² is selected from: C₃₋₆ cycloalkylene; 4-6-membered heterocycloalkylene having one nitrogen heteroatom and optionally 1-2 additional heteroatoms independently selected from nitrogen, oxygen, and sulfur; phenylene; and 5-6-membered heteroarylene having one nitrogen heteroatom and optionally 1-2 additional heteroatoms independently selected from nitrogen, oxygen, and sulfur, wherein the cycloalkylene, heterocycloalkylene, phenylene, and heteroarylene are optionally substituted by 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkylene or heterocycloalkylene, the two substituents optionally together with the ring carbon atoms that they are attached to, form optionally substituted C₃₋₆ cycloalkyl; or
R² is selected from: cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, thiomorpholinylene, phenylene, pyrazolylene, oxazolylene, isoxazolylene, thiazolylene, isothiazolylene, oxadiazolylene, thiadiazolylene, pyridylene, pyrazinylene, pyridazinylene, and pyrimidinylene, each of which is optionally substituted by 1 or 2 substituents independently selected from F, Cl, Br, CN, OH, NH₂, NO₂, and C₁-C₄ alkyl, and in the case where two substituents are attached to the same ring carbon atom of the cyclopropylidene, cyclobutylidene, cyclopentylidene, cyclohexylidene, azetidinylene, pyrrolidinylene, oxazolidinylene, thiazolidinylene, pyrazolidinylene, imidazolidinylene, piperidinylene, piperazinylene, hexahydropyrimidinylene, triazinylene, morpholinylene, or thiomorpholinylene, the two substituents optionally together with the ring carbon atoms that they are attached to, form optionally substituted C₃₋₆ cycloalkyl; or
R² is selected from: 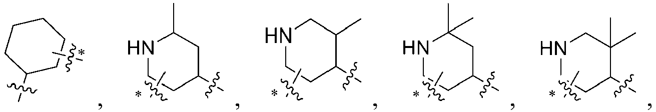 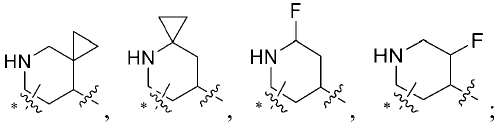 or
R² is selected from: 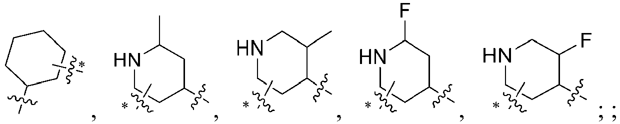 or
R² is 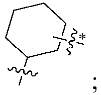 wherein, the wavy line " 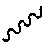 " represents the site of attachment, the bond indicated by * is attached to L^{A};
and/or
wherein:
preferably, R³ is selected from: halogen, CN, NO₂, C₁₋₆ alkyl, OH, NH₂, -CH₂F, -CHF₂, - CF₃, -CH₂Cl, -CHCl₂, and -CCl₃; or
R³ is -CHF₂.

18. The compound according to any one of claims 1 to 14, wherein:
X₁ and X₅ are each N; X₂ and X₄ are C; X₃ is CH; X₆ is CH; and X₇, X₈, and X₉ are each N;
and/or
the 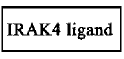 moiety has the structure of formula (I-v): 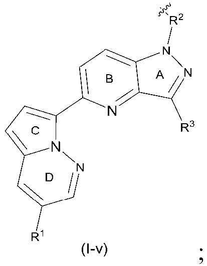
wherein:
R¹, R², and R³ are each as defined in any one of claims 1 to 17, with the proviso that: R¹ is not hydrogen or deuterium;
preferably, R¹, R², and R³ are each as defined in claim 15, 16, or 17; or
R¹ is -C(O)NH₂; and/or R² is 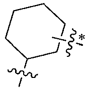 ; wherein, the wavy line " 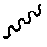 " represents the site of attachment, the bond indicated by * is attached to L^{A}; and/or R³ is -CHF₂;
or
wherein:
X₁ and X₅ are each N; X₂ and X₄ are C; X₃ is CH; and X₆, X₇, X₈, and X₉ are each N;
and/or
the 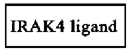 moiety has the structure of formula (I-vi): 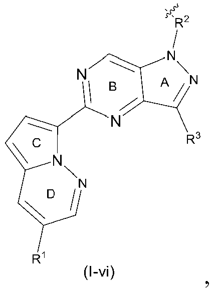
R¹, R², and R³ are each as defined in any one of claims 1 to 17, with the proviso that: R¹ is not hydrogen or deuterium;
wherein:
preferably, R¹, R², and R³ are each as defined in claim 15, 16, or 17; or
R¹ is -C(O)NH₂; and/or R² is 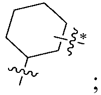 wherein, the wavy line " 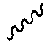 " represents the site of attachment, the bond indicated by * is attached to L^{A}; and/or R³ is -CHF₂;
or
wherein:
X₁ and X₂ are each N; X₃ is CH; X₄ and X₅ are each C; and X₆, X₇, X₈, and X₉ are each N;
and/or
the 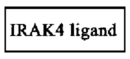 moiety has the structure of formula (I-vii): 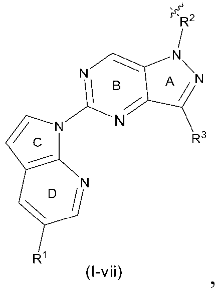
wherein:
R¹, R², and R³ are each as defined in any one of claims 1 to 17, with the proviso that: R¹ is not hydrogen or deuterium;
preferably, R¹, R², and R³ are each as defined in claim 15, 16, or 17; or
R¹ is -CN; and/or R² is 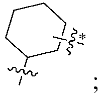 wherein, the wavy line " 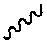 " represents the site of attachment, the bond indicated by * is attached to L^{A}; and/or R³ is -CHF₂.

19. The compound according to any one of claims 1 to 14, wherein:
X₁, X₃, and X₄ are each N; X₂ and X₅ are C; and X₆, X₇, X₈, and X₉ are each N;
and/or
the 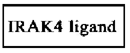 moiety has the structure of formula (I-viii): 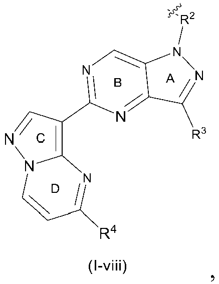
wherein:
R², R³, and R⁴ are each as defined in any one of claims 1 to 17, with the proviso that: R⁴ is not hydrogen or deuterium;
preferably, R² and R³ are each as defined in claim 15, 16, or 17; or
R² is 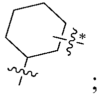 wherein, the wavy line " 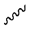 " represents the site of attachment, the bond indicated by * is attached to L^{A}; and/or R³ is -CHF₂;
and/or
preferably, R⁴ is selected from: R⁷, halogen, CN, NO₂, OH, and NH₂;
or
R⁴ is selected from: R⁷, F, Cl, Br, CN, NO₂, OH, and NH₂; wherein:
wherein:
preferably, R⁷ is selected from: 7-10-membered saturated bridged heterocyclyl, the bridged heterocyclyl has one nitrogen heteroatom and optionally 1-2 additional heteroatoms independently selected from nitrogen, oxygen, and sulfur, and is optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R⁷ is selected from: 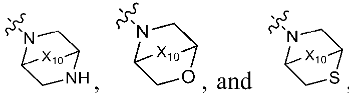 , wherein X₁₀ is CH₂, (CH₂)₂, or (CH₂)₃;
preferably, R⁴ is selected from: CN and 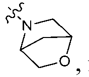 more preferably

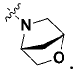

20. The compound according to claim 1, wherein the 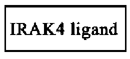 moiety is selected from: 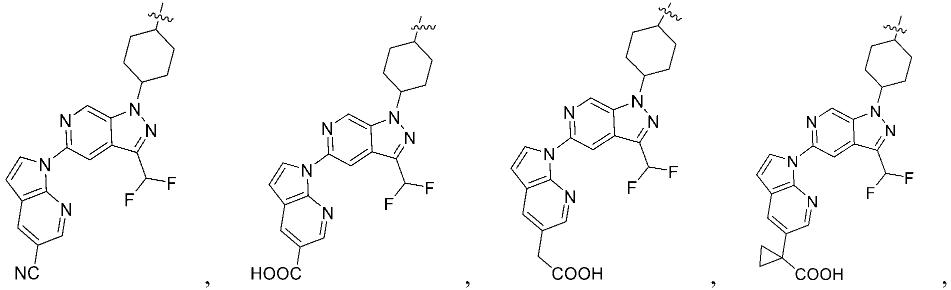 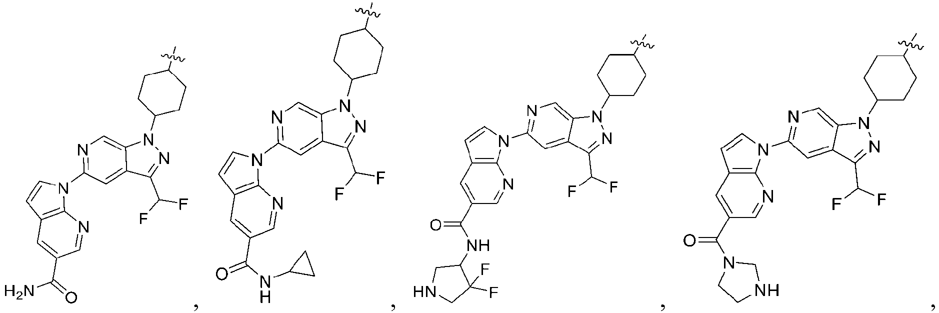 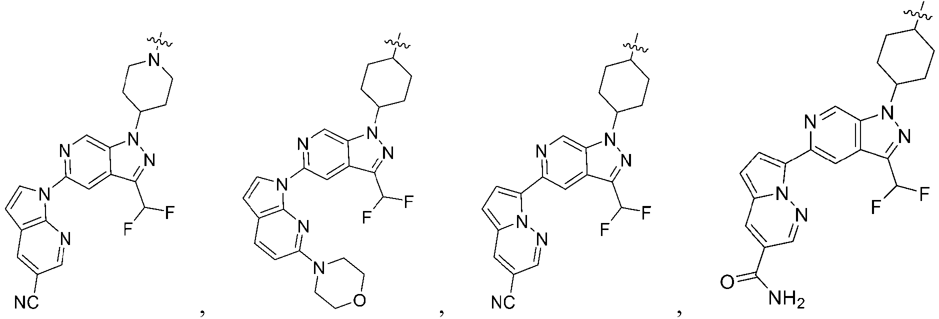 (preferably 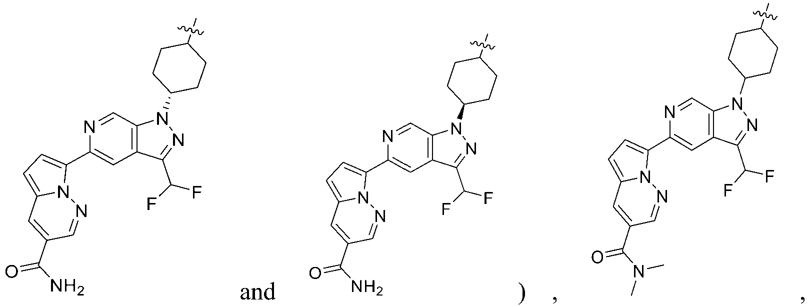 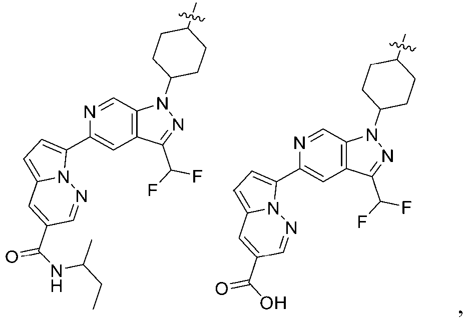 (preferably 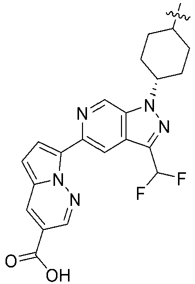 and 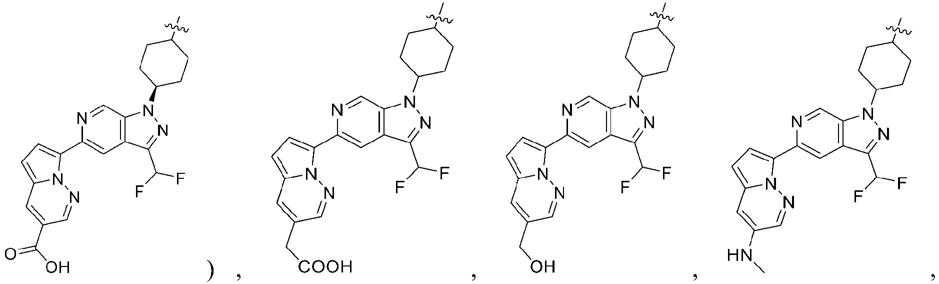 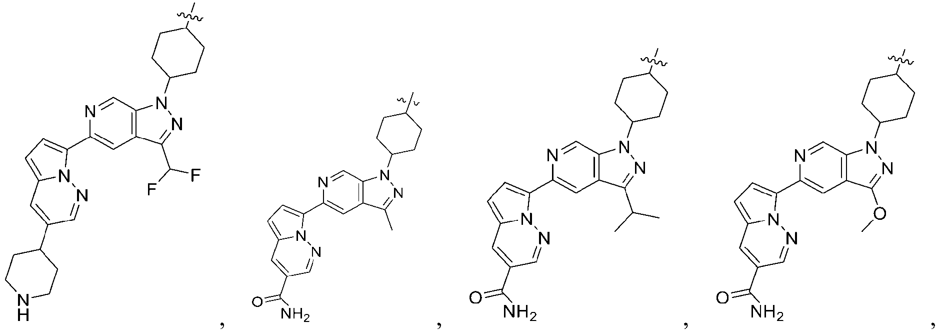 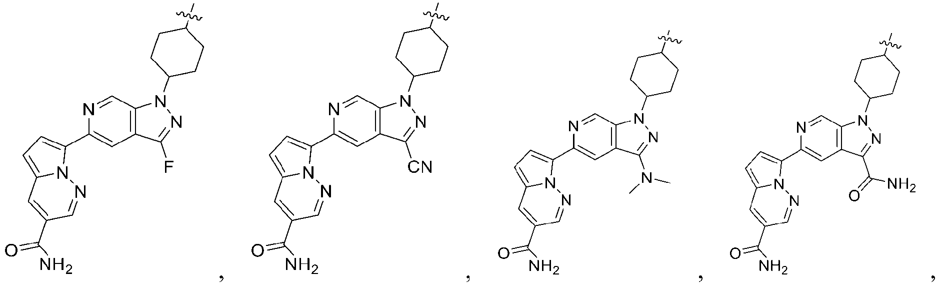 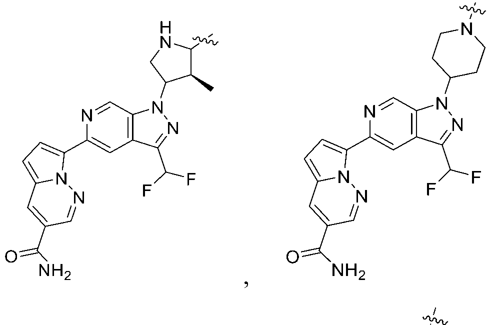 (preferably 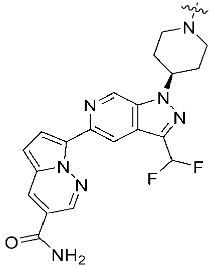 and 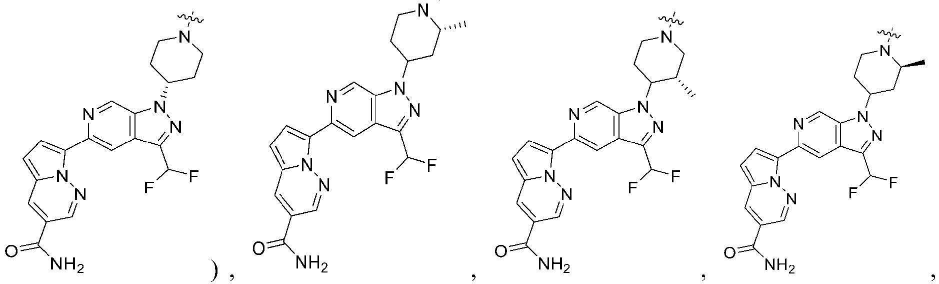 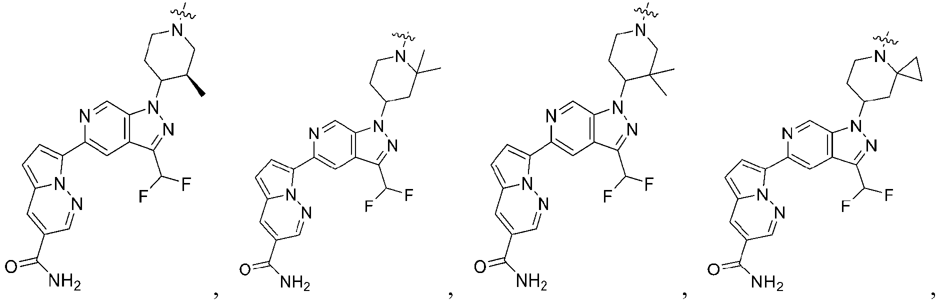 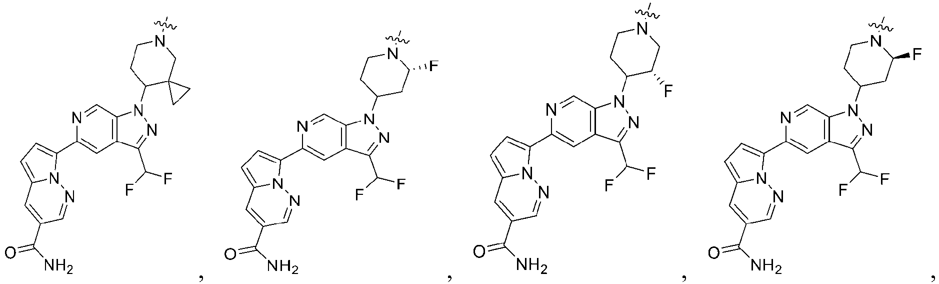 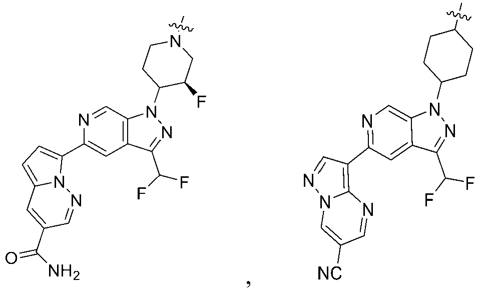 (preferably 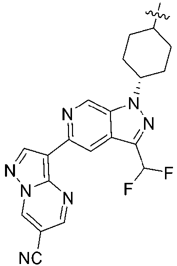 and 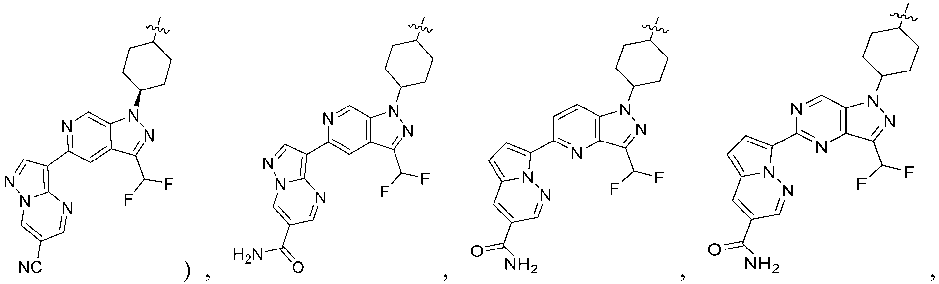 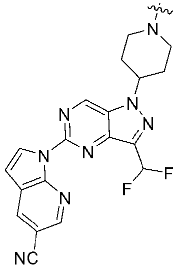 (preferably 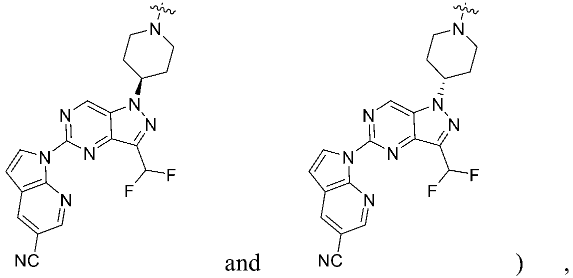 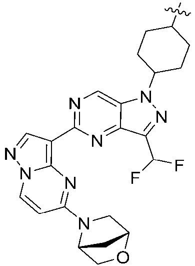 (preferably 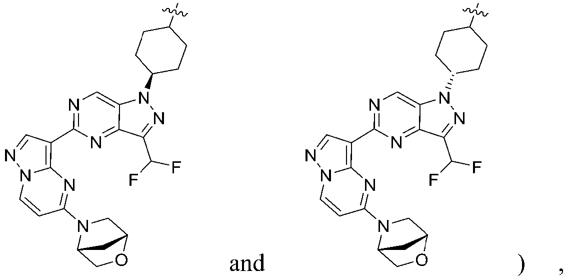 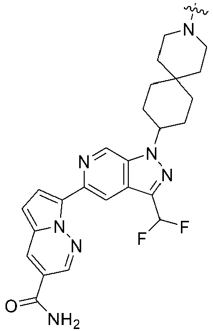 (preferably 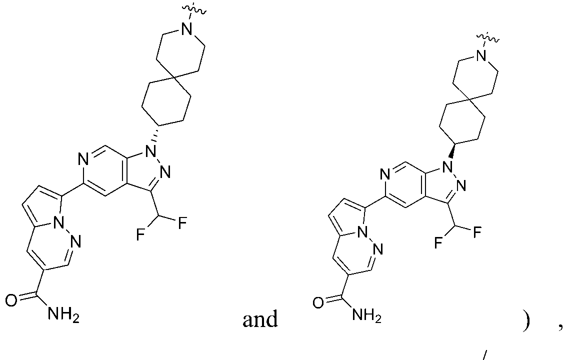 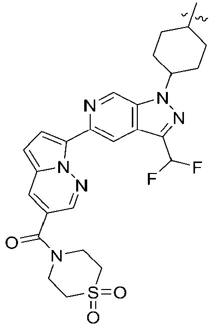 (preferably 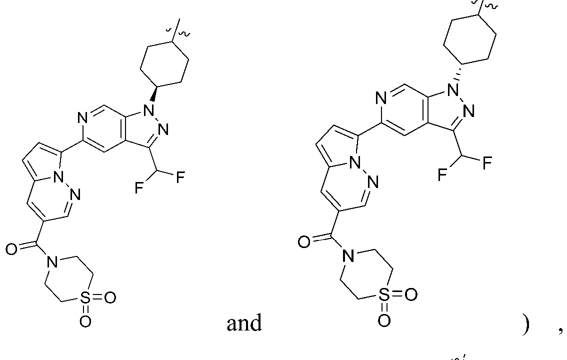 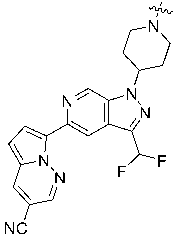 (preferably 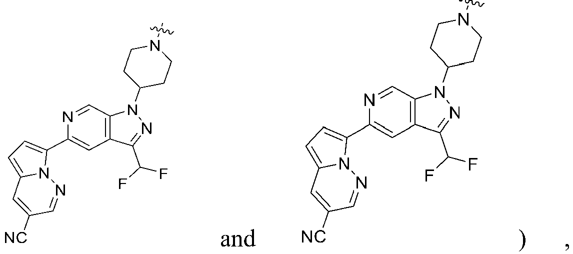 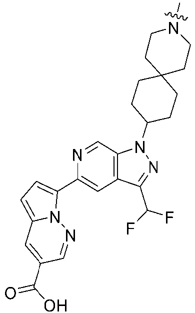 (preferably 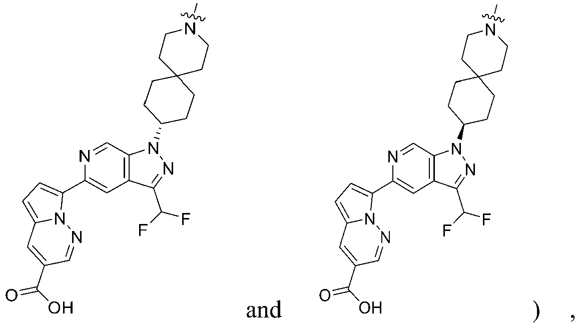 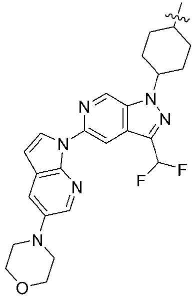 (preferably 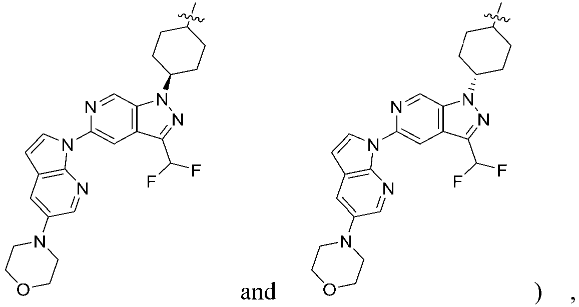 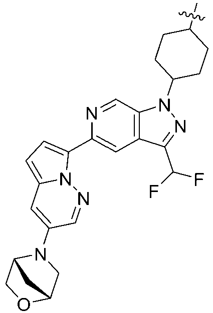 (preferably 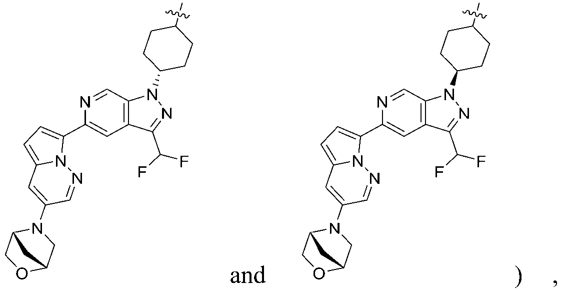 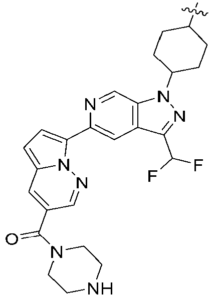 (preferably 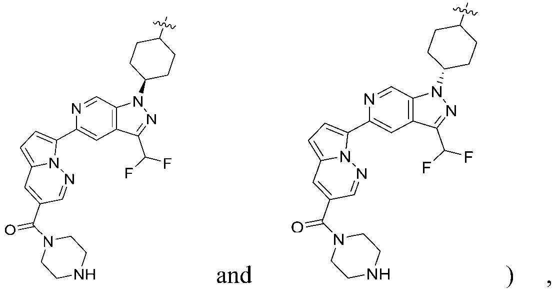 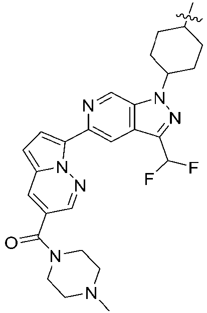 (preferably 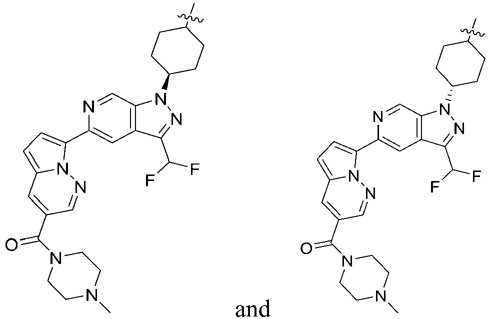 ), and 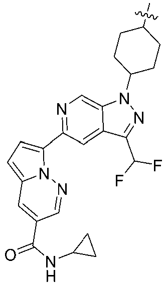 (preferably

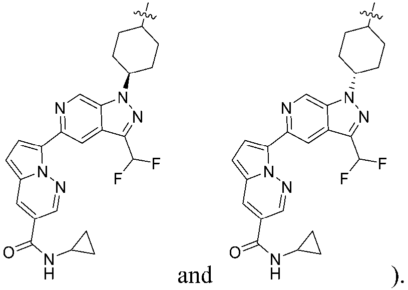

21. The compound according to any one of claims 1-20, wherein:
the CyL1 and CyL2 groups at each occurrence are each independently selected from C₄₋₁₁ cycloalkylene, 4-11-membered heterocycloalkylene, preferably C₄₋₇ monocyclic cycloalkylene, 4-7-membered monocyclic heterocycloalkylene, 6-10-membered fused bicyclic heterocycloalkylene, 6-9-membered bridged heterocycloalkylene, and 5-12-membered spiro heterocycloalkylene, more preferably C₄₋₆ monocyclic cycloalkylene, 4-6-membered monocyclic heterocycloalkylene, 8-10-membered fused bicyclic heterocycloalkylene, 6-8-membered bridged heterocycloalkylene, and 7-11-membered spiro heterocycloalkylene, further preferably, CyL1 at each occurrence is independently selected from C₅₋₆ monocyclic cycloalkylene, 4-6-membered monocyclic heterocycloalkylene, 8-10-membered fused bicyclic heterocycloalkylene, 7-8-membered bridged bicyclic heterocycloalkylene, 7-11-membered spiro bicyclic heterocycloalkylene, CyL2 at each occurrence is independently selected from 4-6-membered monocyclic heterocycloalkylene, wherein any of the above heterocycloalkylene preferably has 1, 2, or more nitrogen heteroatoms and 0, 1, or 2 heteroatoms selected from O and S;
and/or
the CyL3 group at each occurrence is independently selected from C₄₋₁₁ cycloalkylene, preferably C₄₋₆ monocyclic cycloalkylene, C₆₋₁₀ fused bicyclic cycloalkylene, C₆₋₉ bridged cycloalkylene, and C₅₋₁₂ spiro cycloalkylene, more preferably C₅₋₆ monocyclic cycloalkylene, C₈₋₁₀ fused bicyclic cycloalkylene, C₆₋₈ bridged cycloalkylene, and C₇₋₁₁ spiro cycloalkylene, further preferably C₅₋₆ monocyclic cycloalkylene, C₇₋₁₁ spiro bicyclic cycloalkylene;
and/or
the CyL4 group at each occurrence is independently selected from 5-10-membered heteroarylene, preferably 5-6-membered heteroarylene, more preferably 5-6-membered nitrogen-containing heteroarylene;
and/or
La at each occurrence is independently selected from C₁₋₄ alkylene, C₂₋₄alkenylene, and C₂₋₄ alkynylene, preferably -CH₂-, -(CH₂)₂-, -(CH₂)₃-, -(CH₂)₄-, -CH=CH-, -CH₂-CH=CH-, - CH=CH-CH₂-, -C≡C-, -CH₂-C≡C-, -C≡C-CH₂-, -C≡C-CH₂CH₂-, -CH₂CH₂-C≡C-, and -CH₂-C≡C-CH₂-, more preferably -CH₂-, -(CH₂)₂-, -(CH₂)₃-, -C≡C-, -CH₂-C≡C-, -C≡C-CH₂-, -C≡C-CH₂CH₂-, and -CH₂CH₂-C≡C-;
and/or
Lb at each occurrence is independently selected from -O-straight chain C₁₋₃ alkylene-, - straight chain C₁₋₃ alkylene-O-, -O-C₂₋₃ alkenylene-, -C₂₋₃ alkenylene-O-, -O-C₂₋₃ alkynylene-, -C₂₋₃ alkynylene-O-, -NR^{8'}-straight chain C₁₋₃ alkylene-, -straight chain C₁₋₃ alkylene-NR^{8'}-, - straight chain C₁₋₂ alkylene-NR^{8'}-straight chain C₁₋₂ alkylene-, -straight chain C₁₋₂ alkylene-C(O)-NR^{8'}-, -NR^{8'}-C(O)-straight chain C₁₋₂ alkylene-, -straight chain C₁₋₂ alkylene-NR^{8'}-C(O)-, -C(O)-straight chain C₁₋₃ alkylene-, and -straight chain C₁₋₃ alkylene-C(O)-, preferably -O-C₂₋₃ alkynylene-, -NR^{8'}-straight chain C₁₋₃ alkylene-, -straight chain C₁₋₃ alkylene-NR^{8'}-, -straight chain C₁₋₂ alkylene-NR^{8'}-straight chain C₁₋₂ alkylene-, -straight chain C₁₋₂ alkylene-C(O)-NR^{8'}-, -straight chain C₁₋₂ alkylene-NR^{8'}-C(O)-, and -C(O)-straight chain C₁₋₃ alkylene-, wherein R^{8'} at each occurrence is independently selected from H and C₁₋₄ alkyl;
and/or
Lc at each occurrence is independently selected from a bond or straight chain C₁₋₃ alkylene, preferably a bond, methylene, or ethylene, more preferably a bond or methylene;
and/or
R^{L1}, R^{L2}, and R^{8'} at each occurrence are each independently selected from H, methyl, and ethyl, more preferably H and methyl;
and/or L^{B} is selected from groups (1)-(21):
(1) 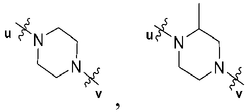 (preferably 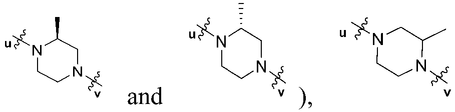 (preferably 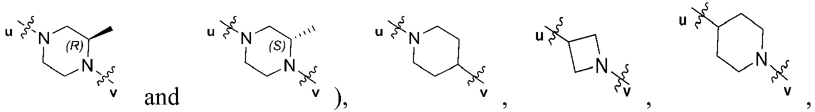 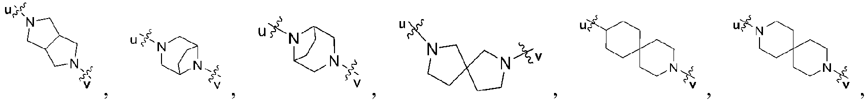 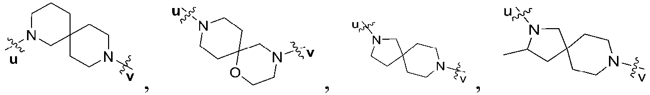 (preferably 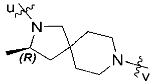 and 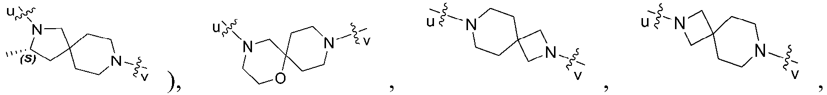 and 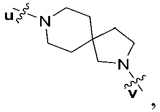
(2) 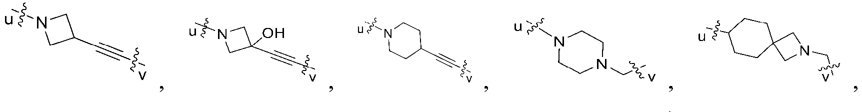 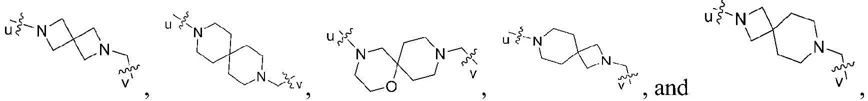
(3) 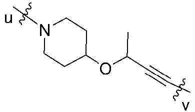 (preferably 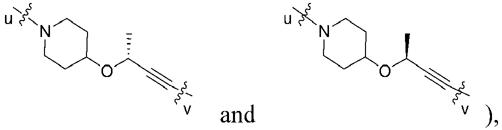 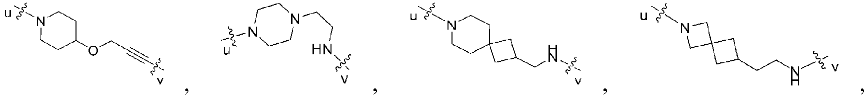 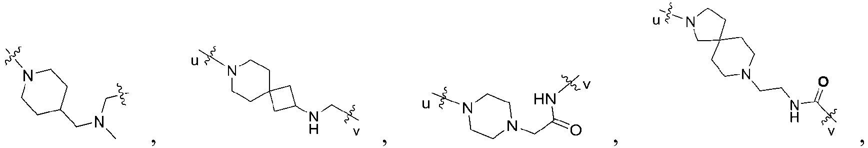 and 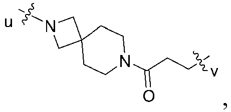
(4) 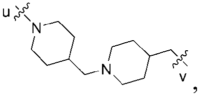
(5) 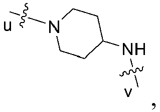
(6) 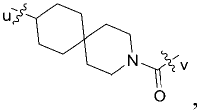
(7) 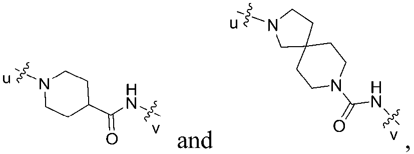
(8) 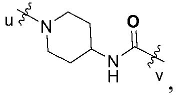
(9) 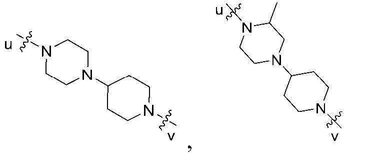 (preferably 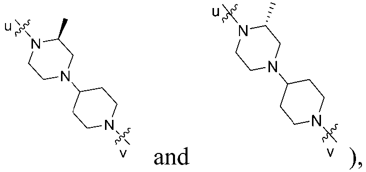 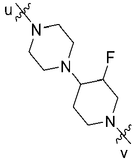 (preferably 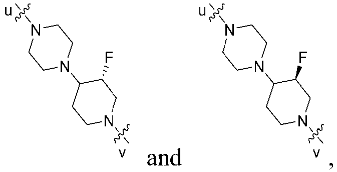 further preferably 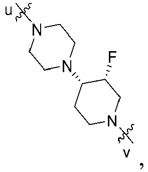 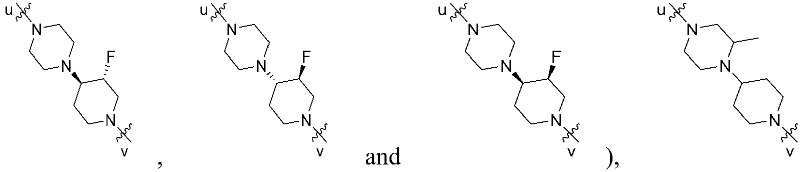 (preferably 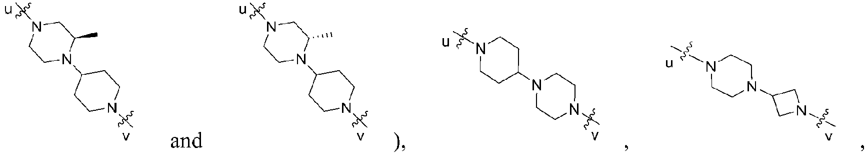 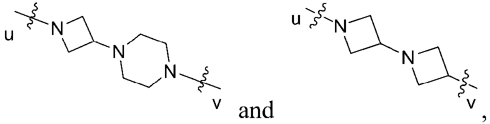
(10) 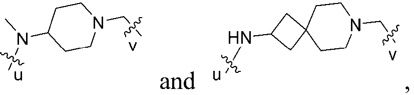
(11) 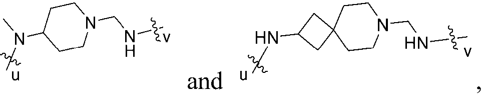
(12) 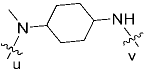 (preferably 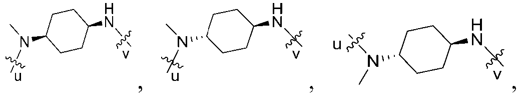 and 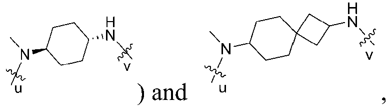
(13) 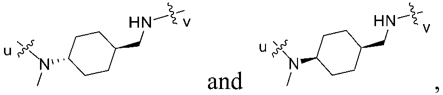
(14) 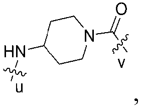
(15) 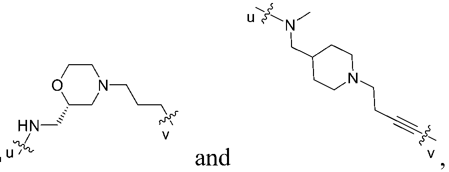
(16) 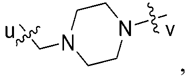
(17) 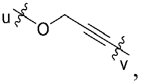
(18) 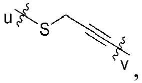
(19) 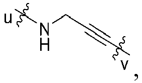
(20) 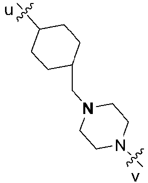 (preferably 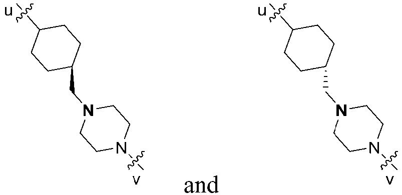 ) and 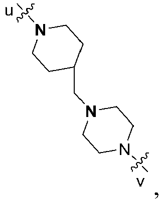 and
(21) 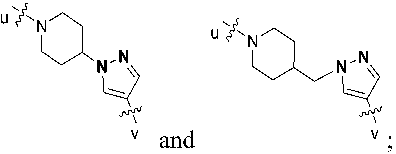 wherein preferably, in any group of the above groups (1)-(21), the bond marked with "u" is attached to the L^{A}, and the bond marked with "v" is attached to the 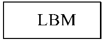 moiety.

22. The compound according to any one of claims 1-21, wherein:
the 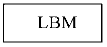 moiety is an E3 ubiquitin ligase ligand,
preferably, the 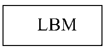 moiety is selected from: 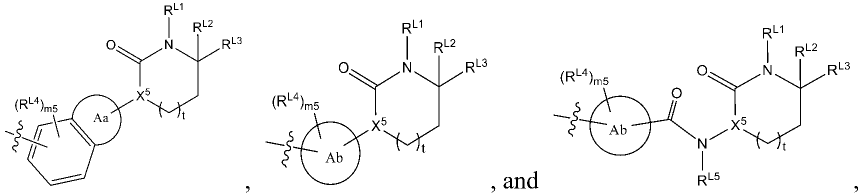
wherein:
ring Aa 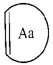 is 5-membered heterocyclyl or 5-membered heteroaryl, preferably 5-membered heterocyclyl or 5-membered heteroaryl having 1, 2, or more N heteroatoms, wherein the 5-membered heterocyclyl and 5-membered heteroaryl are optionally substituted by one or more substituents independently selected from H, halogen, OH, NH₂, CN, oxos and C₁₋₄ alkyl,
preferably, 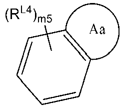 moiety is selected from 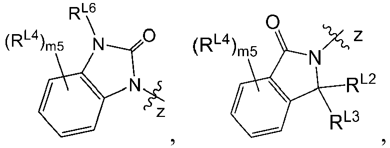 and 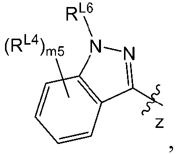 wherein the bond marked with "z" is attached to X⁵;
each ring 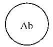 is independently phenyl or 5-6-membered heteroaryl, preferably phenyl;
X₅ is CR^{L7} or N;
t is 0 or 1, preferably 1;
R^{L1}, R^{L5}, and R^{L6} at each occurrence are each independently selected from H and C₁₋₄ alkyl, preferably H and methyl;
R^{L2} and R^{L3} at each occurrence are each independently selected from H and C₁₋₄ alkyl, preferably H and methyl; or R^{L2} and R^{L3} together form oxo;
R^{L4} and R^{L7} at each occurrence are each independently selected from H, halogen, OH, NH₂, CN, and C₁₋₄ alkyl, preferably H, F, Cl, Br, and C₁₋₂ alkyl, more preferably H, F, Cl, and methyl;
m5 is 0, 1, 2, 3, or 4, preferably 0 or 1;
preferably, the 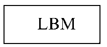 moiety is selected from: 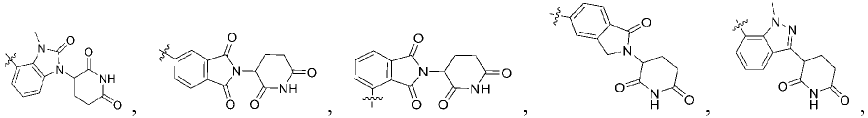 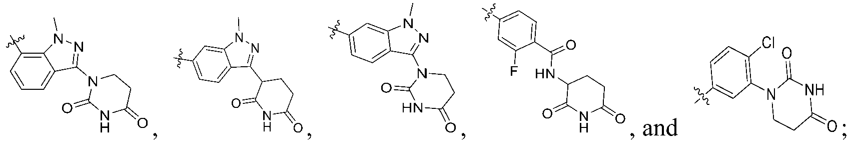
more preferably, the 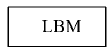 moiety is selected from 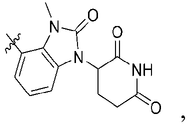 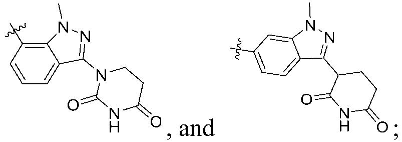
further preferably, the 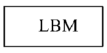 moiety is selected from

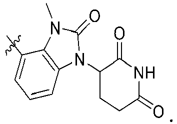

23. The compound according to any one of claims 1-22, wherein moiety is selected from:

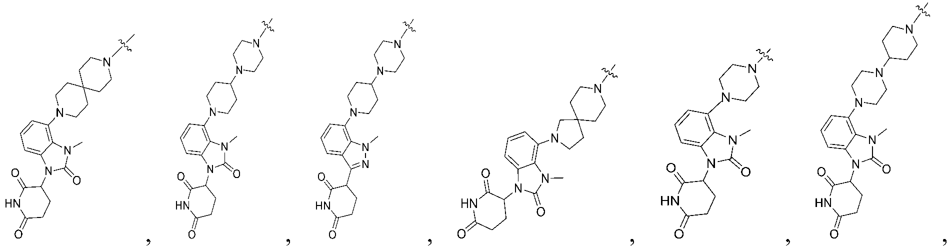

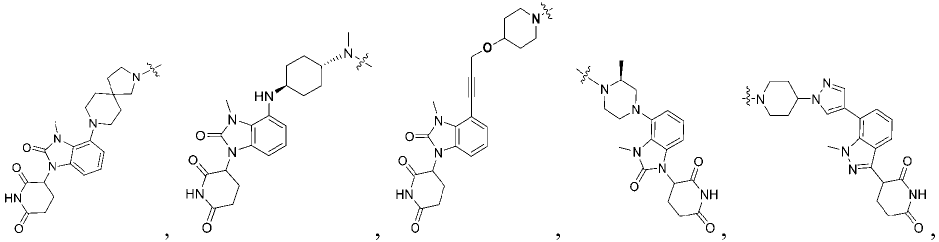

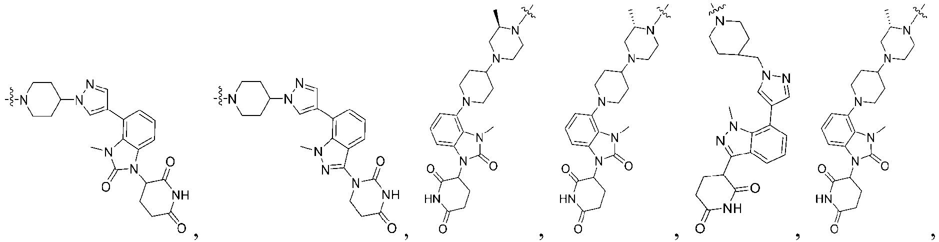

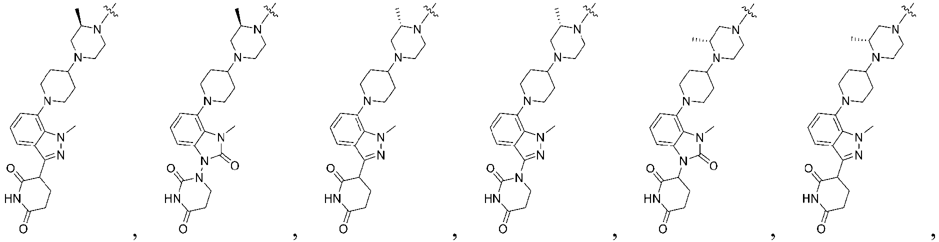

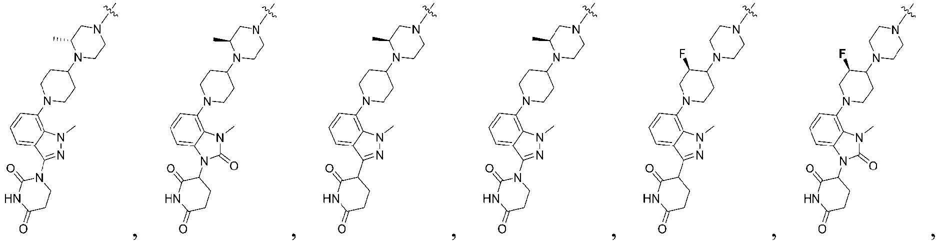

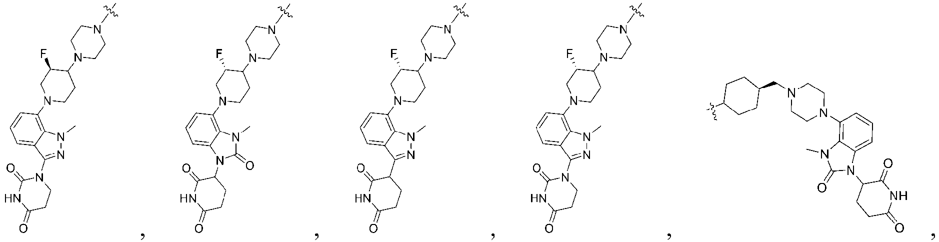

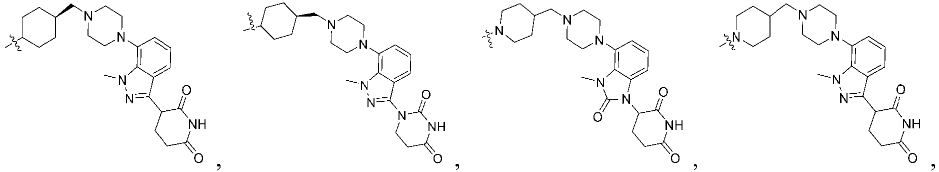

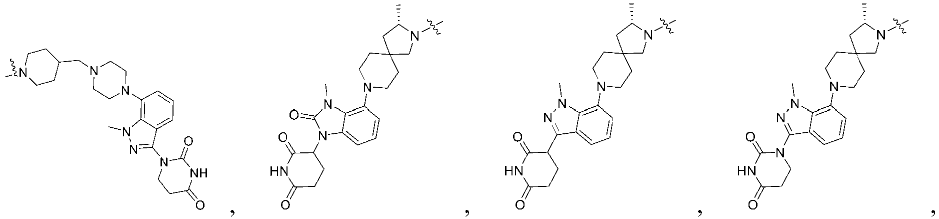

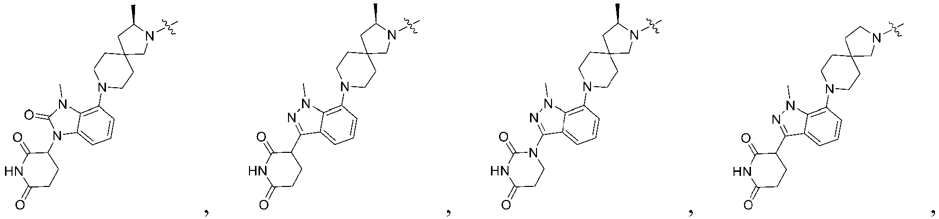

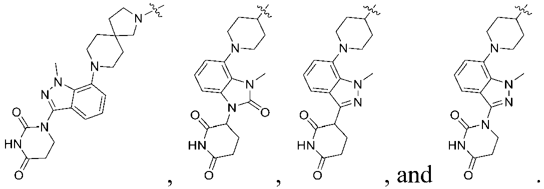

24. The compound according to claim 1, wherein the compound is selected from the compounds listed in table 1 of the specification.

25. The compound according to any one of claims 1-23, wherein the compound has the structure represented by formula (B0): 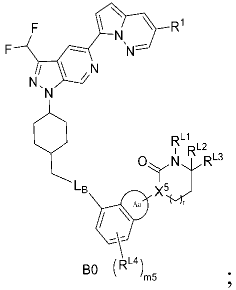
wherein, R¹, L_{B}, Aa, R^{L1}, R^{L2} R^{L3}, R^{L4}, X⁵, t, m5 are as defined in any one of claims 1-23;
preferably, R¹ is selected from CN, R⁷, -C(O)OR^{1f} or -C(O)NR^{1d}R^{1e}; more preferably, R¹ is selected from -C(O)NR^{1d}R^{1e};
wherein:
preferably, R⁷ is selected from: 4-7-membered heterocycloalkyl having one N atom and optionally 1-2 additional heteroatoms independently selected from nitrogen, oxygen, and sulfur, wherein the heterocycloalkyl is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, morpholinyl, thiomorpholinyl, and 2-oxa-5-azabicyclo[2.2.1]heptane, each of which is optionally substituted by one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
R⁷ is selected from piperidinyl, morpholinyl, and 2-oxa-5-azabicyclo[2.2.1]heptane; and/or
preferably, R^{1f} is selected from hydrogen and C₁₋₄ alkyl, preferably hydrogen; and/or
preferably, R^{1d} and R^{1e} are each independently selected from hydrogen, C₁₋₆ alkyl, and R¹¹; or R^{1d} and R^{1e} together with the nitrogen atoms that they are commonly attached to, form R¹⁰;
wherein:
preferably, R¹¹ is selected from: C₃₋₆ cycloalkyl; wherein the cycloalkyl is optionally substituted by one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, each of which is optionally substituted by one or more substituents independently selected from F, Cl, Br, CN, OH, NO₂, and NH₂; and/or
R¹⁰ is selected from: 3-6-membered heterocycloalkyl having one nitrogen heteroatom and optionally 1-2 additional heteroatoms independently selected from nitrogen, oxygen, and sulfur, wherein the heterocycloalkyl is optionally substituted by one or more substituents independently selected from F, Cl, Br, CN, OH, oxo, C₁₋₄ alkyl, C₁₋₄ haloalkyl, C₁₋₄ alkoxy, - NH₂, -NH(C₁₋₄ alkyl), and -N(C₁₋₄ alkyl)₂; or
R¹⁰ is selected from: piperidinyl, piperazinyl, morpholinyl, and thiomorpholinyl, each of which is optionally substituted by one or more substituents independently selected from F, Cl, Br, CN, OH, oxo, C₁₋₄ alkyl, C₁₋₄ haloalkyl, C₁₋₄ alkoxy, -NH₂, -NH(C₁₋₄ alkyl), and -N(C₁₋₄ alkyl)₂;
preferably, R¹ is selected from CN, 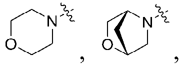 -C(O)OH, -C(O)NH₂, 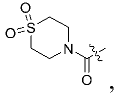 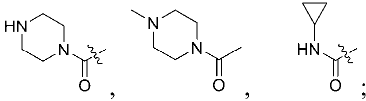 more preferably, R¹ is selected from -C(O)NH₂; and/or
L_{B} is selected from:
(1) -CyL1-, wherein the CyL1 here is selected from 4-7-membered monocyclic heterocycloalkylene, 7-11-membered spiro bicyclic heterocycloalkylene; preferably 5-6-membered monocyclic heterocycloalkylene, 9-11-membered spiro bicyclic heterocycloalkylene; wherein any of the heterocycloalkylenes each has 1 or 2, preferably 2 nitrogen heteroatoms, and any of the heterocycloalkylenes is optionally substituted by 1 or more groups independently selected from the following: C₁₋₄ alkyl and halogen, preferably methyl, F, and Cl, more preferably methyl and F;
(3) -CyL1-Lb-, wherein the CyL1 here is selected from 4-7-membered monocyclic heterocycloalkylene; preferably 5-6-membered monocyclic heterocycloalkylene; Lb group is selected from -O-C₂₋₃ alkynylene;
(9) -CyL1-CyL2-, wherein the CyL1 and CyL2 groups here are each independently selected from 4-7-membered monocyclic heterocycloalkylene, more preferably 5-6-membered monocyclic heterocycloalkylene, wherein any of the heterocycloalkylenes each has 1 or 2 nitrogen heteroatoms, and is optionally substituted by one or more groups independently selected from the following: C₁₋₄ alkyl and halogen, preferably methyl, F, and Cl, more preferably methyl and F;
(12) -NR^{L1}-CyL3-NR^{L2}-, wherein the CyL3 group here is selected from C₅₋₆ monocyclic cycloalkylene, R^{L1}, R^{L2} groups are each independently selected from H and C₁₋₄ alkyl, preferably H and methyl;
(20) -CyL1-La-CyL2-, wherein the CyL1 and CyL2 groups here are each independently selected from 4-7-membered monocyclic heterocycloalkylene or C₄₋₇ monocyclic cycloalkylene; preferably 5-6-membered monocyclic heterocycloalkylene or C₅₋₆ monocyclic cycloalkylene; La is independently selected from C₁₋₄ alkylene, preferably methylene and ethylene;
(21) -CyL1-Lc-CyL4-, wherein the CyL1 group here is selected from 4-7-membered heterocycloalkylene, preferably 5-6-membered heterocycloalkylene; the CyL4 here is selected from 5-6-membered monocyclic heteroarylene, preferably 5-membered monocyclic nitrogen-containing heteroarylene, the Lc group here is selected from a bond or C₁₋₄ alkylene, preferably a bond, methylene, or ethylene; or
L_{B} is selected from: 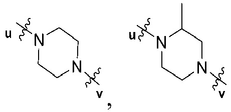 (preferably 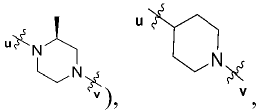 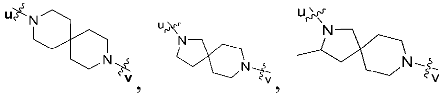 (preferably 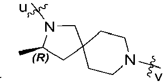 and 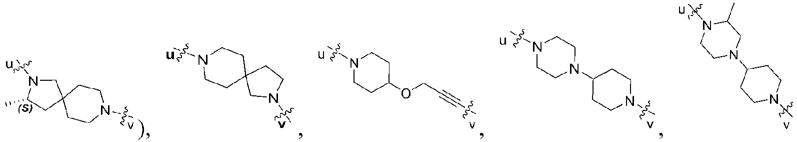 (preferably 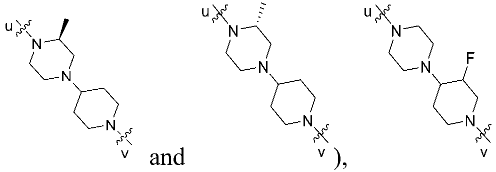 (preferably 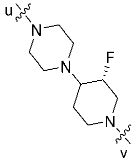 and 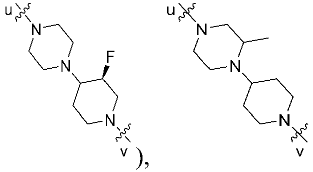 (preferably 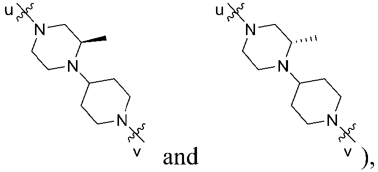 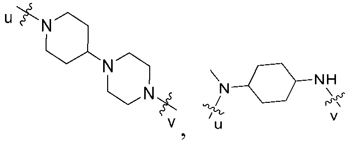 (preferably 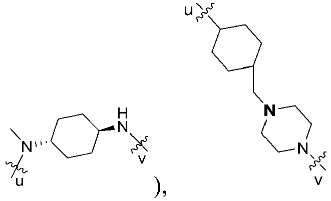 (preferably 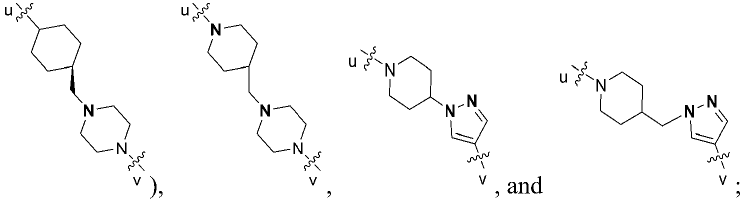 wherein in any of the above groups, the bond marked with "u" is attached to CH₂ moiety, and the bond marked with "v" is attached to the moiety containing phenyl; and/or
preferably, 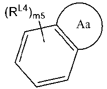 moiety is selected from 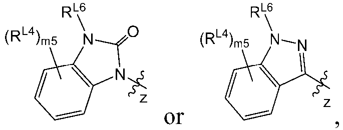 wherein the bond marked with "z" is attached to X⁵;
wherein:
preferably, R^{L4} is selected from H, halogen, and C₁₋₄ alkyl, more preferably H, F, Cl and methyl; and/or
preferably, R^{L6} is selected from H and C₁₋₄ alkyl, more preferably H and methyl; and/or
preferably, m5 is selected from 0 or 1, more preferably 0; and/or
X⁵ is CR^{L7} or N;
wherein: R^{L7} is selected from H, halogen, and C₁₋₄ alkyl, more preferably H; and/or
t is preferably 1; and/or
preferably, R^{L1} is selected from H and C₁₋₄ alkyl, more preferably H and methyl; and/or
preferably, R^{L2} and R^{L3} at each occurrence are each independently selected from H and C₁₋₄ alkyl, preferably H and methyl; or R^{L2} and R^{L3} together form oxo; more preferably R^{L2} and R^{L3} together form oxo;
preferably, the formula (B0) is selected from the structure represented by formula (B0-1), (B0-2), or (B0-3):

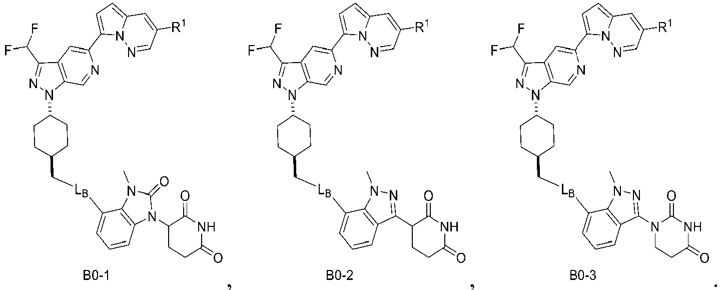

26. The compound according to claim 1, wherein the compound has the structure represented by formula (B): 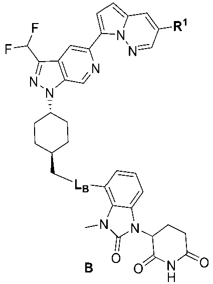 wherein:
L^{B} is selected from:
(1) -CyL1-, wherein the CyL1 group here is selected from 5-9-membered monocyclic heterocycloalkylene, 7-11-membered spiro heterocycloalkylene; preferably 5-6-membered monocyclic heterocycloalkylene, 9-11-membered spiro heterocycloalkylene; more preferably 9-11-membered spiro heterocycloalkylene; wherein the 7-11-membered spiro heterocycloalkylene and 9-11-membered spiro heterocycloalkylene each have 1 or 2, preferably 2 nitrogen heteroatoms, and the 7-11-membered spiro heterocycloalkylene and 9-11-membered spiro heterocycloalkylene are optionally substituted by one or more groups independently selected from the following: C₁₋₄ alkyl and halogen, preferably methyl, F, and Cl, more preferably methyl and F; wherein the 5-9-membered monocyclic heterocycloalkylene and 5-6-membered monocyclic heterocycloalkylene each have 1 or 2, preferably 2 nitrogen heteroatoms, and the 5-9-membered monocyclic heterocycloalkylene and 5-6-membered monocyclic heterocycloalkylene are optionally substituted by 1 or more groups independently selected from the following: C₁₋₄ alkyl and halogen, preferably methyl, F, and Cl, more preferably methyl and F;
(2) -CyL1-Lb-, wherein the CyL1 group here is selected from 5-9-membered monocyclic heterocycloalkylene, more preferably 5-6-membered monocyclic heterocycloalkylene; Lb group is selected from -O-C₂₋₃ alkynylene;
(3) -CyL1-CyL2-, wherein the CyL1 and CyL2 groups here are each independently selected from 4-7-membered monocyclic heterocycloalkylene, more preferably 4-6-membered monocyclic heterocycloalkylene, wherein the 4-7-membered monocyclic heterocycloalkylene and 4-6-membered monocyclic heterocycloalkylene each have 1 or 2 nitrogen heteroatoms, and are optionally substituted by one or more groups independently selected from the following: C₁₋₄ alkyl and halogen, preferably methyl, F, and Cl, more preferably methyl and F;
(4) -NR^{L1}-CyL3-NR^{L2}-, wherein the CyL3 group here is selected from C₄₋₆ monocyclic cycloalkylene, R^{L1}, R^{L2} groups are each independently selected from H, methyl, and ethyl, preferably H and methyl;
(5) -CyL1-La-CyL2-, wherein the CyL1 and CyL2 groups here are each independently selected from 4-7-membered monocyclic heterocycloalkylene or C₄₋₇ monocyclic cycloalkylene; La is independently selected from C₁₋₄ alkylene, preferably methylene and ethylene;
(6) -CyL1-Lc-CyL4-, wherein the CyL1 group here is selected from 4-6-membered monocyclic heterocycloalkylene, preferably CyL1 group is selected from 5-6-membered monocyclic heterocycloalkylene; the CyL4 group here is selected from 5-6-membered monocyclic heteroarylene, preferably 5-membered monocyclic nitrogen-containing heteroarylene, the Lc group here is selected from a bond or C₁₋₄ hydrocarbylene, preferably a bond, methylene, or ethylene;
and, R¹ is selected from 3-10-membered saturated or partially unsaturated heterocyclyl having 1-3 heteroatoms independently selected from nitrogen, oxygen, and sulfur, CN, - C(O)R^{1f}, -C(O)OR^{1f}, -C(O)NR^{1d}R^{1e};
R^{1d}, R^{1e} are each independently selected from hydrogen, deuterium, R¹¹, C₁₋₆ alkyl, and C₁₋₆ haloalkyl; preferably hydrogen, R¹¹, C₁₋₃ alkyl, and C₁₋₃ haloalkyl;
R^{1f} is independently selected from hydrogen, deuterium, C₁₋₆ alkyl, C₁₋₆ haloalkyl, and R¹²; preferably hydrogen, C₁₋₃ alkyl, C₁₋₃ haloalkyl, and R¹²;
R¹¹, R¹² are independently selected from: saturated or partially unsaturated C₃₋₇ cyclohydrocarbyl; 3-10-membered saturated or partially unsaturated heterocyclyl having 1-3 heteroatoms independently selected from nitrogen, oxygen, and sulfur; wherein the cyclohydrocarbyl and heterocyclyl are optionally substituted by one or more substituents independently selected from C₁₋₆ alkyl, C₁₋₆ haloalkyl; preferably saturated C₃₋₇ cyclohydrocarbyl; 3-10-membered saturated heterocyclyl having 1-3 heteroatoms independently selected from nitrogen, oxygen, and sulfur; preferably the cyclohydrocarbyl and heterocyclyl are optionally substituted by one or more substituents independently selected from C₁₋₃ alkyl, C₁₋₃ haloalkyl;
R¹ is preferably selected from -CONH₂, -CN, -COOH, 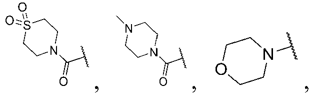 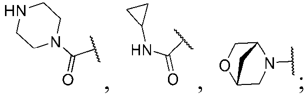 more preferably R¹ is preferably selected from -CONH₂.

27. The compound according to claim 25 or 26, wherein
L^{B} is selected from: 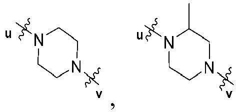 (preferably 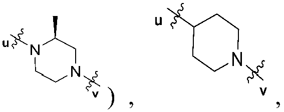 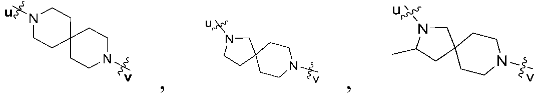 (preferably 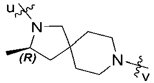 and 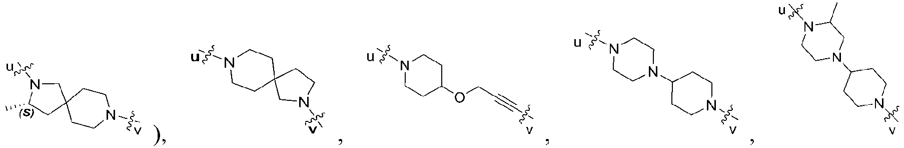 (preferably 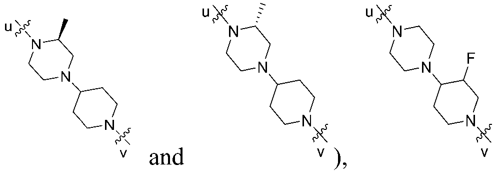 (preferably 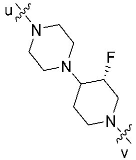 and 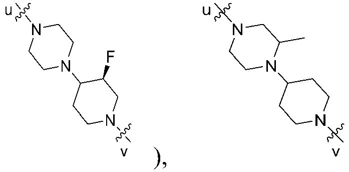 (preferably 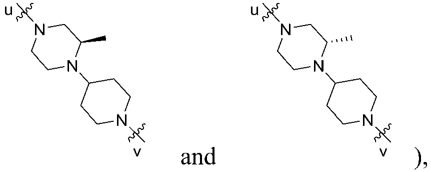 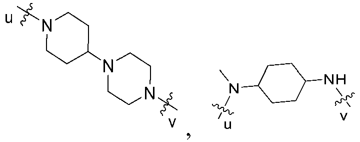 (preferably 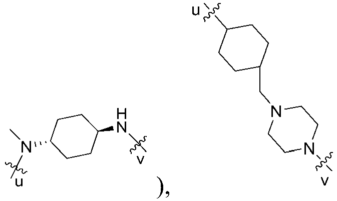 preferably 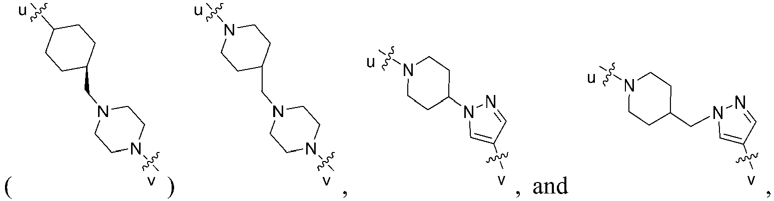 more preferably 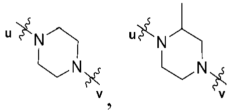 (preferably 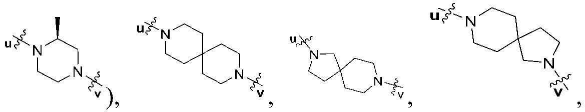 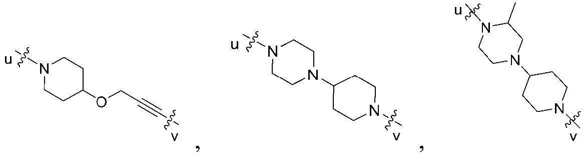 (preferably 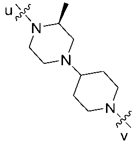 and 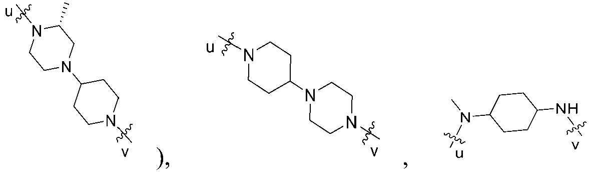 (preferably 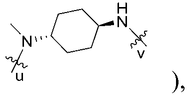 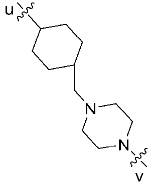 preferably 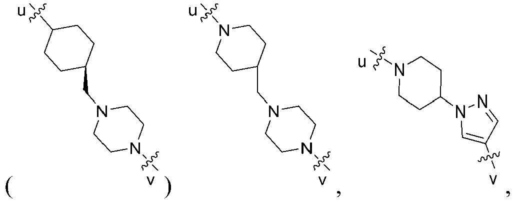 and , wherein in any of the above groups, the bond marked with "u" is attached to CH₂ moiety, and the bond marked with "v" is attached to the moiety containing phenyl;
and/or
R¹ is selected from -CONR_{Na}R_{Nb}, wherein R_{Na}, R_{Nb} are each independently H or C₁₋₄ alkyl, preferably -CONH₂.

28. The compound according to any one of claims 25 to 27, wherein the compound is selected from:

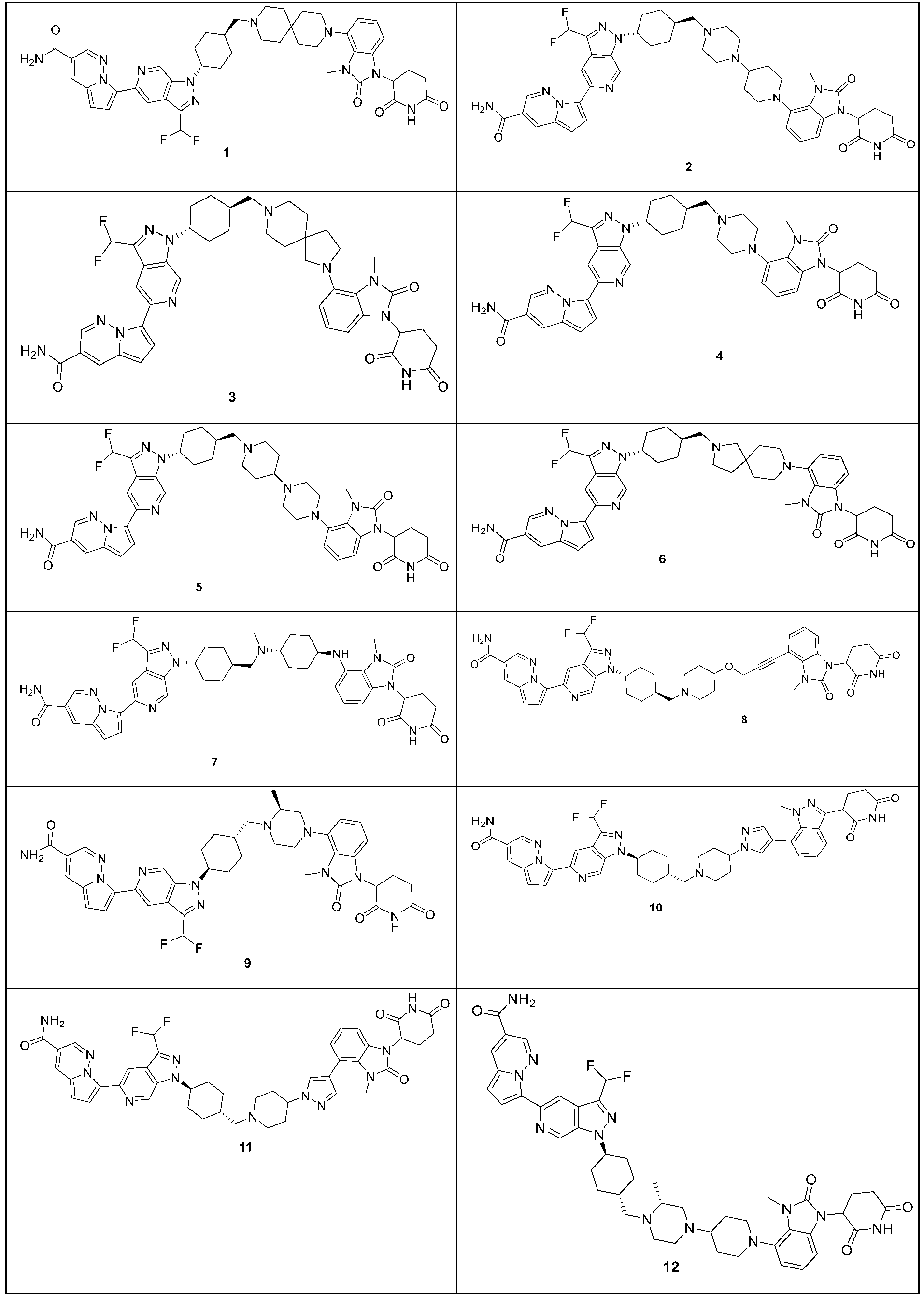

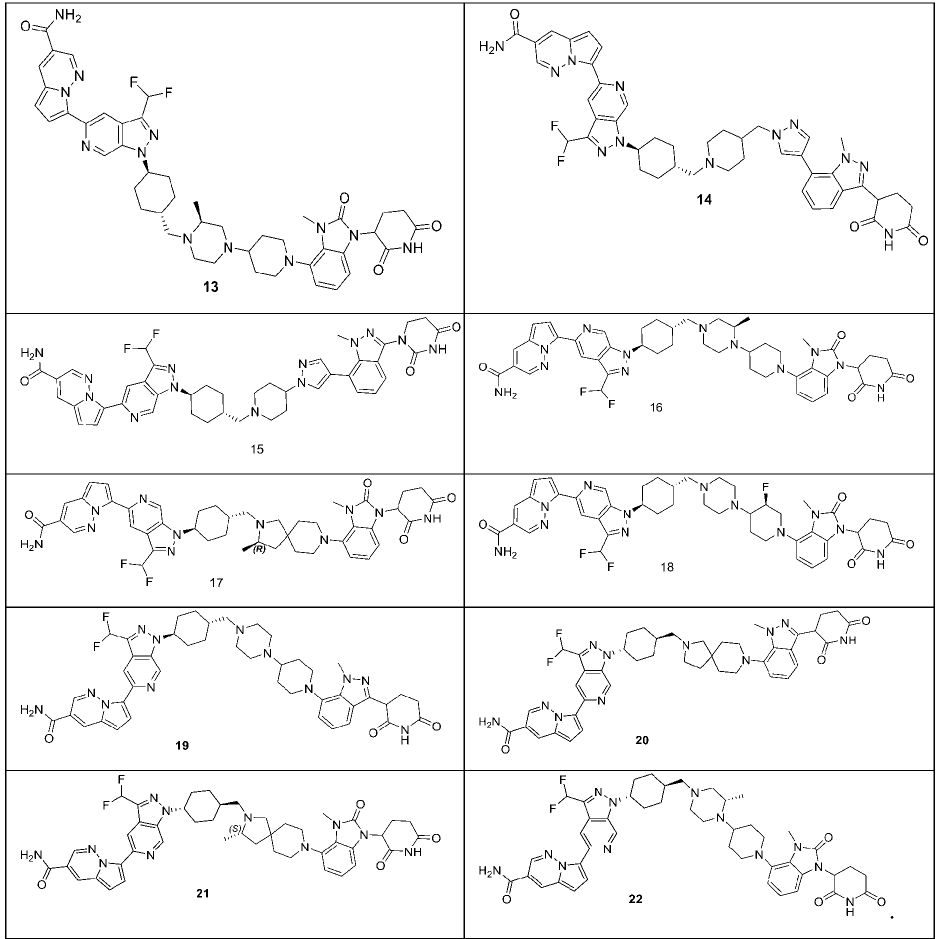

29. A pharmaceutical composition, comprising the compound or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof according to any one of claims 1 to 28, and a pharmaceutically acceptable excipient, carrier, or diluent.

30. Use of the compound or stereoisomer, tautomer, diastereomer, racemate, cis/trans isomer, isotope-labeled compound (preferably deuterated compound), N-oxide, metabolite, ester, prodrug, crystal form, hydrate, solvate, or pharmaceutically acceptable salt thereof according to any one of claims 1 to 28, or the pharmaceutical composition according to claim 29 in the preparation of a medicament for treating a disease, disorder, or condition related to an IRAK4 protein kinase.

31. A method for treating a disease, disorder, or condition related to an IRAK4 protein kinase, comprising administering to an individual in need thereof a therapeutically effective amount of the compound or stereoisomer, tautomer, diastereomer, racemate, cis/trans isomer, isotope-labeled compound (preferably deuterated compound) , N-oxide, metabolite, ester, prodrug, crystal form, hydrate, solvate or pharmaceutically acceptable salt thereof according to any one of claims 1 to 28, or the pharmaceutical composition according to claim 29.

32. The use according to claim 30, or the method according to claim 31, wherein the disease, disorder, or condition related to an IRAK4 protein kinase is selected from: autoimmune condition, inflammatory condition, cancer, graft rejection, thromboembolism, atherosclerosis, myocardial infarction, and metabolic syndrome;
wherein:
preferably, the inflammatory condition is selected from: osteoarthritis, gout, gouty arthritis, chronic obstructive pulmonary disease, periodic fever, atopic dermatitis, hidradenitis suppurativa, chronic nephritis, allergic eczema, lymphadenectasis, pyemia, irritable bowel syndrome (IBD), ulcerative colitis, asthma, and allergies, preferably osteoarthritis, chronic obstructive pulmonary disease, atopic dermatitis, hidradenitis suppurativa, and chronic nephritis; and/or
preferably, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis, preferably psoriasis; and/or
preferably, the graft rejection is selected from graft versus host disease and allogeneic graft rejection; and/or
preferably, the cancer is selected from: brain cancer, kidney cancer, liver cancer, stomach cancer, vaginal cancer, ovarian cancer, gastric tumor, breast cancer, bladder and colon cancer, prostate cancer, pancreatic cancer, lung cancer, cervical cancer, testicular cancer, skin cancer, bone cancer, thyroid cancer, sarcoma, glioblastoma, neuroblastoma, multiple myeloma, gastrointestinal cancer, neck and head tumors, adenoma, adenocarcinoma, keratoacanthoma, epidermoid carcinoma, large cell carcinoma, non-small cell lung cancer, Hodgkin and Non-Hodgkin lymphoma, breast cancer, follicular carcinoma, papillary carcinoma, seminoma, melanoma, acute myelogenous leukemia, chronic myelogenous leukemia, diffuse large B-cell lymphoma, activated B-cell-like diffuse large B-cell lymphoma, chronic lymphocytic leukemia, chronic lymphocytic lymphoma, primary effusion lymphoma, Burkitt's lymphoma/leukemia, acute lymphoblastic leukemia, B-cell prolymphocytic leukemia, lymphoplasmacytic lymphoma, Waldenstrom macroglobulinemia, splenic marginal zone lymphoma, intravascular large B-cell lymphoma, plasmacytoma, and multiple myeloma.
